# EUROPEAN PATENT APPLICATION

(11) **EP 1 557 425 A2**
(43) Date of publication of application: **27.07.2005**
(21) Application number: 04007560.8
(22) Date of filing: 07.07.1998
(51) Int. Cl.: C07K 14/47, C12N 15/12, C12N 15/63, A61K 38/00

(54) **123 human secreted proteins**

(30) Priority: 08.07.1997 US 51926 P; 08.07.1997 US 51929 P; 08.07.1997 US 52793 P; 08.07.1997 US 51925 P; 08.07.1997 US 51931 P; 08.07.1997 US 51932 P; 08.07.1997 US 52803 P; 08.07.1997 US 52732 P; 08.07.1997 US 51916 P; 08.07.1997 US 51930 P; 08.07.1997 US 51918 P; 08.07.1997 US 51920 P; 08.07.1997 US 51919 P; 08.07.1997 US 51928 P; 08.07.1997 US 52795 P; 08.07.1997 US 52733 P; 18.08.1997 US 55948 P; 18.08.1997 US 55722 P; 18.08.1997 US 55723 P; 18.08.1997 US 55949 P; 18.08.1997 US 55964 P; 18.08.1997 US 55947 P; 18.08.1997 US 55950 P; 18.08.1997 US 55953 P; 18.08.1997 US 56360 P; 18.08.1997 US 55684 P; 18.07.1997 US 55984 P; 18.08.1997 US 55954 P; 12.09.1997 US 58785 P; 12.09.1997 US 58664 P; 12.09.1997 US 58660 P; 12.09.1997 US 58661 P
(62) Divisional of application: 98935509.4
(71) Applicant: Human Genome Sciences, Inc., Rockville, MD 20850 (US)
(72) Inventor: Lafleur, David, Washington, D.C. 20015 (US); Ruben, Steven M., Brookeville, MD 20833 (US); Rosen, Craig A., Laytonsville, MD 20882 (US)
(74) Representative: VOSSIUS & PARTNER

(57) **Abstract**

The present invention relates to novel human secreted proteins and isolated nucleic acids containing the coding regions of the genes encoding such proteins. Also provided are vectors, host cells, antibodies, and recombinant methods for producing human secreted proteins. The invention further relates to diagnostic and therapeutic methods useful for diagnosing and treating disorders related to these novel human secreted proteins.

<110> Human Genome Sciences, Inc.

<120> 123 Human Secreted Proteins

<130> PZ010PCT

<140> Unassigned
<141> 98-07-07

<150> 60/051,926
<151> 97-07-08

<150> 60/051,929
<151> 97-07-08

<150> 60/052,793
<151> 97-07-08

<150> 60/051,925
<151> 97-07-08

<150> 60/051,931
<151> 97-07-08

<150> 60/051,932
<151> 97-07-08

<150> 60/052,803
<151> 97-07-08

<150> 60/052,732
<151> 97-07-08

<150> 60/051,916
<151> 97-07-08

<150> 60/051,930
<151> 97-07-08

<150> 60/051,918
<151> 97-07-08

<150> 60/051,920
<151> 97-07-08

<150> 60/051,919
<151> 97-07-08

<150> 60/051,928
<151> 97-07-08

<150> 60/052,795
<151> 97-07-08

<150> 60/052,733

<151> 97-07-08

<150> 60/055,948
<151> 97-08-18

<150> 60/055,722
<151> 97-08-18

<150> 60/055,723
<151> 97-08-18

<150> 60/055,949
<151> 97-08-18

<150> 60/055,964
<151> 97-08-18

<150> 60/055,947
<151> 97-08-18

<150> 60/055,950
<151> 97-08-18

<150> 60/055,953
<151> 97-08-18

<150> 60/056,360
<151> 97-08-18

<150> 60/055,684
<151> 97-08-18

<150> 60/055,984
<151> 97-08-18

<150> 60/055,954
<151> 97-08-18

<150> 60/058,785
<151> 97-09-12

<150> 60/058,664
<151> 97-09-12

<150> 60/058,660
<151> 97-09-12

<150> 60/058,661
<151> 97-09-12

<160> 516

<170> PatentIn Ver. 2.0

<210> 1
<211> 733
<212> DNA
<213> Homo sapiens

<400> 1
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180
tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat 733

<210> 2
<211> 5
<212> PRT
<213> Homo sapiens

<220>
<221> misc feature
<222> (3)
<223> Xaa equals any one of the twenty naturally ocurring L-amino acids

<400> 2
Trp Ser Xaa Trp Ser
1               5

<210> 3
<211> 86
<212> DNA
<213> Homo sapiens

<400> 3
gcgcctcgag atttccccga aatctagatt tccccgaaat gatttccccg aaatgatttc 60
cccgaaatat ctgccatctc aattag 86

<210> 4
<211> 27
<212> DNA
<213> Homo sapiens

<400> 4
   gcggcaagct ttttgcaaag cctaggc 27

<210> 5
<211> 271
<212> DNA
<213> Homo sapiens

<400> 5
ctcgagattt ccccgaaatc tagatttccc cgaaatgatt tccccgaaat gatttccccg 60
aaatatctgc catctcaatt agtcagcaac catagtcccg cccctaactc cgcccatccc 120

gcccctaact ccgcccagtt ccgcccattc tccgccccat ggctgactaa ttttttttat 180
ttatgcagag gccgaggccg cctcggcctc tgagctattc cagaagtagt gaggaggctt 240
ttttggaggc ctaggctttt gcaaaaagct t 271

<210> 6
<211> 32
<212> DNA
<213> Homo sapiens

<400> 6
gcgctcgagg gatgacagcg atagaacccc gg 32

<210> 7
<211> 31
<212> DNA
<213> Homo sapiens

<400> 7
gcgaagcttc gcgactcccc ggatccgcct c 31

<210> 8
<211> 12
<212> DNA
<213> Homo sapiens

<400> 8
ggggactttc cc 12

<210> 9
<211> 73
<212> DNA
<213> Homo sapiens

<400> 9
gcggcctcga ggggactttc ccggggactt tccggggact ttccgggact ttccatcctg 60
ccatctcaat tag 73

<210> 10
<211> 256
<212> DNA
<213> Homo sapiens

<400> 10
ctcgagggga ctttcccggg gactttccgg ggactttccg ggactttcca tctgccatct 60
caattagtca gcaaccatag tcccgcccct aactccgccc atcccgcccc taactccgcc 120
cagttccgcc cattctccgc cccatggctg actaattttt tttatttatg cagaggccga 180
ggccgcctcg gcctctgagc tattccagaa gtagtgagga ggcttttttg gaggcctagg 240
cttttgcaaa aagctt 256

<210> 11
<211> 1882
<212> DNA
<213> Homo sapiens

<220>
<221> misc feature
<222> (12)
<223> n equals a,t,g, or c

<220>
<221> misc feature
<222> (565)
<223> n equals a,t,g, or c

<400> 11
ttcggcacgg anactggaag gaaagaaaga aaggtcagct ttggcccaga tgtggttacc 60
ccttggtctc ctgtctttat gtctttctcc tcttcctatt ctgtcatctc cctcacttaa 120
gtctcaggcc tgtcagcagc tcctgtggac attgccatcc cctctggtag ccttcagagc 180
aaacaggaca acctatgtta tggatgtttc caccaaccag ggtagtggca tggagcaccg 240
taaccatctg tgcttctgtg atctctatga cagagccact tctccacctc tgaaatgttc 300
cctgctctga aatctggcat gagatggcac aggtgaccac gcagaagcca ccagaatytt 360
gcctgcccta ttcctcctcc caagtctgtt ctcttattgt caacctcagc acaacaggct 420
ggcgccaatg gcattacaga gaaagcaatc tgtgtggcta gtgggcagat taccatgcaa 480
gccccaggag aaatggagga gctttgtagc cacctccctg tcasccagta ttaacatgtc 540
cccttccccc tgccccgccg taganttcag gacattcgcc cctgtgtgcc accaaaccag 600
ggactttccc cttsssttgg gttggcatcc ctgggctctc tcctggtacc cagcaagacg 660
tctgttccag ggcagggcac gagctttcaa gctccgttac tatggcgatg gccatgatgt 720
tacaatccca cttgcctgaa taatcaagtg ggaasgggaa gcasagggaa atggggccat 780
gtgaatgcag ctgctctgtt ctccctaccc tgaggaaaaa ccaaagggaa gcaacaggaa 840
cttctgcaac tggtttttat cggaaagatc atcctgcctg cagatgctgt tgaaggggca 900
caagaaattg gagctggaga agattgatga aagtgcaggt gtgtaaggaa atagaacagt 960
ctgctgggag tcagacctgg aattctgatt ccaaactctt tattactttg ggaagtcact 1020
cagcctcccc gtagccatct ccagggtgac ggaacccagt gtattacctg ctggaaccaa 1080
ggaaactaac aatgtaggtt actagtgaat accccaatgg tttctccaat tatgcccatg 1140
ccaccaaaac aataaaacaa aattctctaa cactgcaaag agtgagccat gcctgttaac 1200
actgtaaaga atgtaacatg tgggggacac acaggggcag atgggatggt ttagtttagg 1260
attttattag tgcatgccct accctctggg cgaacgtccc ctctgaggtt ttcttctcgg 1320
tggggggatt taacttctgt cctagggaaa acagtgtctg atgaggagtg tttccaacac 1380
aggctacatg aattccccta taccagtgcg aaagcagcca ggagtccccg ttggaaaaga 1440
acaatgccac tctcttttat gtatcttggt tctgcaactc atttgttgta agtagggtta 1500
atcgagtatc aggttcacag tatcctgccc ttattatttt atgattcact gactcaagtt 1560
ccacgaagtc cttagaaatg gacctcttca tgtaaaatat cttgagaata ataaatgtga 1620
gggaataaga aaggcaagct ttggacacag atatgatagg tgcatcagct tcggaagaga 1680
agaatgatgt gcagagtgtt aggaagacat ccgggctgct gagactcggg attagaagaa 1740
agagaggtaa ataaagtggg tcctggaatc ttttaggact tctgctgtag gacaaacagc 1800
tgcctttggt gttttaatgt ctcccaaagt acccttcagc caataaatac catctgttgg 1860
tgcaaaaaaa aaaaaaaaaa aa 1882

<210> 12
<211> 1590
<212> DNA
<213> Homo sapiens

<220>
<221> misc feature
<222> (1374)
<223> n equals a,t,g, or c

<220>
<221> misc feature
<222> (1397)
<223> n equals a,t,g, or c

<220>
<221> misc feature
<222> (1516)
<223> n equals a,t,g, or c

<400> 12
gggtcgaccc acgcgtccgg agattctggg aggatcccga gtacccccca gttgcagtca 60
tgtcaagact gatgctcagg aggatcccaa ctgtcatgag caacacccat cgaacacagc 120
catccacctg ggaacagatc aagaagctgt cacagatggt gggagaaaac ctgaggaaag 180
cgggacaacc agtcacaatg agtaatttaa tggtagctat gatagcagtg atcaccattg 240
ccgtgagtat tccttcaaca agggctgaca cagagatcag ttatacttat tgggcatatt 300
tgtcaatttt ggctggcaat aatgcctgga tataatcact ttatgacaca gttacacatg 360
ctttctggtc tcaatattta ccataataag tctgctccta taattgaggc ataccaccct 420
caaaaatcta tttgtaaaca aaattgaacc tggccagaaa aaatgaatgt actttttag 480
gaaggttgca ttgcagaaca ggcagaggtg ctgcacaacg attcctatgg aatcattatt 540
gattggtccc ctaaggggat gtttagcttg aattgcacct cttagtctgc atgtcacagc 600
cacactgtgt tcaactggtc tgaacagaat ggtcagatgg tacaaatggt aagacgtatg 660
gcaagagttc ctattatctg gaaccatggc agtatagggg cacctcaacc tcaaatgata 720
tggcccattg taggagctaa acataaggat ttgtggcaac tgttaatagc tcttaataag 780
atcaaaattt gggaaagaat aaaaaagcat ctagaaggac actctgcaaa cttgtctttg 840
gatattgcaa aatatatata tatatttaaa gcatcccagg cacacctgac cttaatgcca 900
gaactggagt gctcgaagga gctgcagaca gattagcagc tagtaaccca ttaaaatgga 960
taaaaacact tagaagctct gtgatttcaa tgatgattgt gcttttaatc tgtgttgttt 1020
gtctttatat agtctgcaga tgctgatctt gactcctgtg agaagtagct caccgtgaca 1080
aagctgcctt ggctttttat cgctttgcaa aacaagagaa tggggacaag ttgggaacag 1140
gccccaaaat ctggccataa actggcccct aaactggtca taaacaaaat ctctgcagca 1200
ctgtcacatg cttgtgatag cctgacgccc acgttggaag gctgtcggtt taccggaatg 1260
agggcaagga acaactggcc cactcagggc ggataaccac ttaaggcatt cttaaaccac 1320
aaacaatagc atgagctatc tgtgccttaa ggacatgttc atgctgcaga taantagcca 1380
gagcccatcc ctttacntcg gcccatccct ttatttccca taaggaatac ttatagttaa 1440
tctatagaaa caatgcttat cactggcttg ctgtcaataa atatgtgggt aaatctctgt 1500
tcaaggctct cagctntgaa ggctgtgaga cccctgattt cccactccac aatctaaaaa 1560
aaaaataaaa acaaaaaaaa aaaaaaaaaa 1590

<210> 13
<211> 1373
<212> DNA
<213> Homo sapiens

<400> 13
tcgacccacg cgtccgttca gaaaaaggat ttgacaaaat tcagtgccca ttcatggtta 60
aaaaaaaaaa aaactttcag aaaaatgata atggaggaga tctttctcaa cttgataaag 120
aacatctaca aaagccccta cagccaatgt aacacataat agtaaaagac taattgcttt 180
tctccaatat cagggatatt agggacagag atgtctgtcc tcaccactct tattcaacat 240
attgctggaa gttctgtcta gtgcagtgag gaaagaaaag gaattaaaaa gcatgcagac 300
aaaaagaagg aaacaaaact gtctctattt gcaaatgaca tgattctcta aataaaaaat 360
cccaaggaat ctacaaaaaa aactagagct aggtggggtg tggtggctca tgcctgtaat 420
cccagcactt tgggaggctg aattaagagg attacctaaa ccaagaagtt caagaccagc 480
ctgcgcaaca tagtaagacc cccatctcta caaaaaattg aaaaattagc tggatgtatt 540
agctactcag ggagctgagc tgggagggat tgtttgagcc agagaggtca gggctctggt 600
gatccatgat cacatcacca tactccagcc tgggcaaccg agtgagacct gtccttaaaa 660
aacaaacaaa aacaaactag atctagtgag agttcagcaa gccctcaagc tacaagacct 720
atataccaaa aatcaacttg catttctata tactattaat gaacatatgg gaaacctaaa 780
tttaaaagat agtaccactt aacaattgtt tcacaaaaat gaattacctg ggcataaatt 840
aaataaacat atacaggatc tgtatgctaa aaattgcaaa atactgataa aagaaatcaa 900
agcaaaccca aagaagtgga gacacatacc gtgttcatgt actggaaggc tcagcagaga 960
cgtgggttcc ctccagactg atgtacaggt ttgatgtact tgctagcaaa aatcccagca 1020
aggtattttt ttgtagatgc gcaagattat tctaaaattt gtatggaagg gcagtgaaac 1080
taaaagtcac gaaaataatc ttgaaaaaga aaaagaaaat gggcagaatc actgtatttg 1140

ataacatacc ttgttatata actgcagtaa tcaagacagt atagtgttgg tgaagggaca 1200
gacacaaggt caatgaaaca gaatagagaa cccagacata gacccacaca agtaccacca 1260
gtggatttgg acaaggtgca aaagcaactc attggaggaa ggcagcctat ttagccaatg 1320
tgactggagc actggatacc cataagccaa aaaaagaaaa aaaaaaaaaa agg 1373

<210> 14
<211> 1142
<212> DNA
<213> Homo sapiens

<220>
<221> misc feature
<222> (341)
<223> n equals a,t,g, or c

<220>
<221> misc feature
<222> (369)
<223> n equals a,t,g, or c

<220>
<221> misc feature
<222> (386)
<223> n equals a,t,g, or c

<220>
<221> misc feature
<222> (408)
<223> n equals a,t,g, or c

<220>
<221> misc feature
<222> (412)
<223> n equals a,t,g, or c

<220>
<221> misc feature
<222> (526)
<223> n equals a,t,g, or c

<220>
<221> misc feature
<222> (598)
<223> n equals a,t,g, or c

<220>
<221> misc feature
<222> (676)
<223> n equals a,t,g, or c

<220>
<221> misc feature
<222> (739)
<223> n equals a,t,g, or c

<400> 14
tcgacccacg cgtccgtctt cctcctgcgt cctcccccgc tgcctccgct gctcccgacg 60
cggagcccgg agcccgcgcc gagcccctgg cctcgcggtg ccatgctgcc ccggcggcgg 120
cgctgaagga tggcgacgcc gctgcctccg ccctccccgc ggcacctgcg gctgctgcgg 180

ctgctgctct ccggcctcgt cctcggcgcc gccctgcgtg gagccgccgc cggccacccg 240
gaatgttgcc gcctgtcccg ggagcctgga ctgtgccctg aagaagcggg caagtgtcct 300
cctggtgcac atgcctgtgg gcctgccttc agcccttcca naaggaacag caaaggcttg 360
ttttgccang atgcgccggg cttcangcgg gggccgggcc caacccanac tngaaatgag 420
attgattcct ggcccaaggg agcttgcccg gaaaggaatt tggacatcaa ttccgcccta 480
acccaaggac ggacagcggt tcccggagct tgccaccttg ggattntcgg cacgggggca 540
ggggctggag ctgggcttcc cttccactcc aggaaccccc acgcccacgc cccacacnta 600
ccatgggtta cccctgtgtc atccgacccg gtgcacatgt cgcccctgga gccccgggga 660
gggcaaggcg acggcntcgc ccttgtgctg atcctggcgt tctgtgtggc cggtgcagcc 720
gccctctccg tagcctccnt ctgctggtgc aggctgcagc gtgagatccg cctgactcag 780
aaggccgagt acgccactgc gaaggccctg gctacacctg cagctacccc ggatctcgct 840
tggggaccag cgcctggcac agagcgcgga gatgtaccac taccagcacc aacggcaaca 900
gatgttgtcc ctggagcggc ataaagagcc acccaaggag ctggacacgg ctcttcggat 960
gaggagaatg aggacggaga cttcacggtg tacgagtgcc cgggcatggc cccgaccggg 1020
gaaatggagg tgcgcaacca tctgttcgac cacgccgcac tgtccgcgcc cctgccggcc 1080
cccagctcac cgcttgcact gccatgacct ggaggcagac agacgcccac ttgctccccg 1140
ac 1142

<210> 15
<211> 1034
<212> DNA
<213> Homo sapiens

<400> 15
gaattcggca cgaggaacca ccttctgtag gacagtcacc aggccagatc cagaaggctt 60
gaggccctgt ggtccccatc cttgggagaa gtcagctcca gcaccatgaa gggcatcctc 120
gttgctggta tcactgcagt gcttgttgca gctgtagaat ctctgagctg cgtgcagtgt 180
aattcatggg aaaaatcctg tgtcaacagc attgcctctg aatgtccctc acatgccaac 240
accagctgta tcagctcctc agccagctcc tctctagaga caccagtcag attataccag 300
aatatgttct gctcagcgga gaactgcagt gaggagacac acattacagc cttcactgtc 360
cacgtgtctg ctgaagaaca ctttcatttt gtaagccagt gctgccaagg aaaggaatgc 420
agcaacacca gcgatgccct ggaccctccc ctgaagaacg tgtccagcaa cgcagagtgc 480
cctgcttgtt atgaatctaa tggaacttcc tgtcrtggga agccctggaa atgctatgaa 540
gaagaacagt gtgtcyttct agttgcagaa cttaagaatg acattgagtc taagagtctc 600
gtgctgaaag gctgttccaa cgtcagtaac gccacctgtc agttcctgtc tggtgaaaac 660
aagactcttg gaggagtcat ctttcgaaag tttgagtgtg caaatgtaaa cagcttaacc 720
cccacgtctg caccaaccac ttcccacaac gtgggctcca aagcttccct ctacctcttg 780
gcccttgcca gcctccttct tcggggactg ctgccctgag gtcctggggc tgcactttgc 840
ccagcacccc atttctgctt ctctgaggtc cagagcatcc cctgcggtgc tgacaccctc 900
tttccctgct ctgccccgtt taactgccca gtaagtggga gtcacaggtc tccaggcaat 960
gccgacagct gccttgttct tcattattaa agcactggtt cattcactga aaaaaaaaaa 1020
aaaaaaaaac tcga 1034

<210> 16
<211> 1198
<212> DNA
<213> Homo sapiens

<400> 16
   cccacgcgtc cgggagaaag ctgcactctg ttgagctcca gggcgcagtg gagggaggga 60
   gtgaaggagc tctctgtacc caaggaaagt gcagctgaga ctcagacaag attacaatga 120
   accaactcag cttcctgctg tttctcatag cgaccaccag aggatggagt acagatgagg 180
   ctaatactta cttcaaggaa tggacctgtt cttcgtctcc atctctgccc agaagctgca 240
   aggaaatcaa agacgaatgt cytagtgcat ttgatggcct gtattttctc cgcactgaga 300
   atggtgttat ctaccagacc ttctgtgaca tgacctctgg gggtggcggc tggaccctgg 360
   tggccagcgt gcatgagaat gacatgcgtg ggaagtgcac ggtgggcgat cgctggtcca 420
   gtcagcaggg cagcaaagca gactacccag agggggacgg caactgggcc aactacaaca 480
   cctttggatc tgcagaggcg gccacgagcg atgactacaa gaaccctggc tactacgaca 540
   tccaggccaa ggacctgggc atctggcacg tgcccaataa gtcccccatg cagcactgga 600
   gaaacagctc cctgmtgagg taccgcacgg acactggctt cctccagaca ctgggacata 660
   atctgtttgg catctaccag aaatatccag tgaaatatgg agaaggraag tgttggactg 720
   acaacggccc ggtgatccct gtggtctatg attttggcga cgcccagaaa acagcatctt 780
   attactcacc ctatggccag cgggaattca ctgcgggatt tgttcagttc agggtattta 840
   ataacgagag agcagccaac gccttgtgtg ctggaatgag ggtcaccgga tgtaacactg 900
   agcaccactg cattggtgga ggaggatact ttccagaggc cagtccccag cagtgtggag 960
   atttttctgg ttttgattgg agtggatatg gaactcatgt tggttacagc agcagccgtg 1020
   agataactga ggcagctgtg cttctattct atcgttgaga gttttgtggg agggaaccca 1080
   gacctctcct cccaaccatg agatcccaag gatggagaac aacttaccca gtagctagaa 1140
   tgttaatggc agaagagaaa acaataaatc atattgactc aaaaaaaaaa aaaaaaaa 1198

<210> 17
<211> 1447
<212> DNA
<213> Homo sapiens

<220>
<221> misc feature
<222> (1420)
<223> n equals a,t,g, or c

<220>
<221> misc feature
<222> (1432)
<223> n equals a,t,g, or c

<220>
<221> misc feature
<222> (1436)
<223> n equals a,t,g, or c

<400> 17
caagcgcgca agggcgcggg cgagcaggcc tgtgaattcg caggatcatt tcagacccgc 60
acttcggcag ccaactcgaa agcaggcggt tgtgtgcggc agcagttggc gtttgctttg 120
cacttcggaa cctgttgcgt tttgacccac ggaggtggag gagtaacttt ttgacatgtt 180
ggcctttcca gttttgttgg aagtttcatg gtcggttttg tttygtttct cattcttctc 240
tccksgcccc tcagcccccc aacccccaac cccctcccgg tccgtgttgc atgcacgctg 300
ttcaaatgtg aggtctgaaa tggctggcac acgggaaaag ctgcttgtgt cattcgtttc 360
tgggagtggg atggctctga gcagcctcgc ctccctgttt gtactatttg aactttgcag 420
atctctgttc tctcaagcag aactcccaac cagatccatt cttgaccagt gaccggctcg 480
gaatctggcc ttttgtgtga gatgatcacg gtttcttttg tttatcacgc catttgcaaa 540
tcagagcaag agctctttct caagggcaag aaacgcaaac aagaaatatt tgtgagatga 600
aagttgtcaa ttggattttc ttcctaaaca aacaacaaca acaaactact agaagtctcc 660
ctgagtccac tcgcttggat ttctgacaca gtttacaaaa aaggaaaaag gcactgctcc 720
tattttccct tatggctgag ttcaccttaa gattgtaaat gtgtatatgt cagtgaaaac 780
attgaggctt ggaaaatgtg ttattttcgt tgccctaagt ttgagtcgac tttagactca 840
aaaacatttt gagcgaatat caaagttaac ttttaaaaat tgcgaaacta tttcagaatc 900
gcaattttat cgaagattaa atcagacttt tttgtctggt aattatatat ttattattta 960
gcaaaactga agaaaaaaag cacagaattg tttcaacaga tgtctctcat tttcagctag 1020
catttctctc ccaagttgag ctggtttaat gtgttttgga tttccctcct caattggctt 1080
attttttaga tcacctgcaa ttcatttgca aattgcaata aaacacattt tagaaaaaag 1140
gaaccttcaa ttattagctt tgtttctttt taaatgtata taaaaagact aatgtttgtg 1200
aatgaagttg gctaacatgt atttagtttc attttggctt tatgtaatat aaagttttta 1260
aaattttaaa tatggtttta acctttatgt gtaaatgatt ttctagtgtg accttctaat 1320
ttaatattag acgtctaagg tatatctgta aattagaatc cgactatcac tctgttcatt 1380
ttttttgaac aaagagttta aataaagcct gaaccagggn acagataaag anaatnaaaa 1440
aaaaaaa 1447

<210> 18
<211> 1422
<212> DNA
<213> Homo sapiens

<220>
<221> misc feature
<222> (1397)
<223> n equals a,t,g, or c

<400> 18
gtttctctta ggttttgaag gtataagtgt aaagtgaagc atctctcgat gattctttcc 60
aagataggtt taaaaactat gaatccattt tcagtattty cttctctctg tttgaaacag 120
tttgaggatg tgtytctttt tcttggcttg atgtttggta sgtccttgaa tgggcaagag 180
ggcacatgaa gtacggcgtc ctccacattc acggcctcta cacgggaccc ctgcggggtg 240
ggtgctggac ccatcggggt ataaagacgt cactcaagac gcagaagtca tggaagtcct 300
ccagaactta taccgcacca agtcctttct gtttgtgggc tgtggggaga cccttcgtga 360
tcagatattc caggccctct ttctttactc cgtgccgaat aaggtggatt tggagcacta 420
catgcttgtg ctgaaggaga atgaagacca tttctttaag catcaggcag atatgcttct 480
gcacggaatc aaagttgtat cctacgggga ctgttttgac cactttccag gatatgtgca 540
agaccttgcc actcagatct gcaaacagca aagcccagga catttgtact cgaattcatg 600
gagtgccact cctgatggga gaggaggccc atgacagtga cagtcatgct agtgatcgcg 660
gacaccacac catgctgcct ttgccagctg gctccttcag cgagtcctcg caccaagcct 720
gggaggtaga gatgctgatc gcgtggacag caccacatta ttgggtaatg catgccagga 780
ctgtgcaaag aggaagttag aagagaatgg aattgaagtt tcaaaaaaac gcacacaatc 840
agatactggt gtctgtgcca tcctcatgct cgcgggagtt ttggcatggg attctccgtt 900
gtgattcccc cggactccac tgtctgaaga ccaggtttcc tatgaagagg gtctgatggg 960
aacctgttcc cagtgatttg aagatgatgc tggagggtct tgaaatcttt acagtaaaac 1020
ctgcaacttg aaaactagcc tttctgtaac cacagtgccc aaacgaagag gaatgtatgg 1080
agaactccac gtggatctct gattgggaaa ccgtcacata caccaagaga gccacatggg 1140
catgtggccc tcaaggctgg gtgagagggc tcccctgtgt gttgaactat gcaggagggt 1200
gacgcggaca catttcaggt ggactttgca aggactgatg gatagctacc tcagggacca 1260
gaatccgtgg gaagggatgg acctggtgtt cccgttccca tctgacaggc tctcttttgt 1320
ccaaggtggt atttttcgta ataaaagggg aagagtaaar amwrwmmaar maamagtagc 1380
tgccaaagag aaaatangaa atagacactt tttttttttg gg 1422

<210> 19
<211> 1107
<212> DNA
<213> Homo sapiens

<400> 19
ctaaactatt tagttcaaaa gtaacccaac taattaaagt gaaaaaaaat tgttgaatca 60
caatgaacaa acataaaaca atacttaaat gagaattctg tgtctttttt ggttttatct 120
gtgatttatt ttgtccagta ttaaggaatg gttatcttta tcattcttct aacatgtttt 180
ggtttctcta atggttcatt ttcctttagc ttgtgaaaat tagggcagtt tgtccagagc 240
cttactcgca ggagacacca gacccaaccc atgcttagat ttctgttaat aaaagggaga 300
agggtatttg aataggtagt aaaggcaggt acaagtttaa gggagcaggg ctatcatatg 360
tactaggtga gattactata aatgtctgaa aagttacatg catagtcatt ggctcaggta 420
atttctctga atttgaactt atttgattta tttaaccaag ttattataat atgcagttct 480
ctttaatcaa tcttctatta ttcaatcatc tatccattta ttaattcaac aaatatttat 540
taaagtgcct accatgatta tgtgctgtag aaaagacaag gacatttact aggggggatt 600
gtgggcccaa tcggcatcat aagcatgttc tgaaagccaa agacaataat cacatccaac 660
ggcaccagtt cagctcaact ttagaattca gcagtaacag tacagatggc ctaaagtaca 720
tctgtgtgta tctgtacgtg tgcacacacc catgtatata tatttatcta tctgcacaca 780
cactacatat gtatacacac tatctatgta aaatataata tatgtataat gcatataaat 840
tctaacaagt gtatttgtgt tatctttaaa atagaacaat tgtatcttga agtggtaaat 900
gcagagaatt ggttttattg ttgatctgtg gatttaatga tttctaggtg aaaaggacgt 960

ttaagtgtac aatttctttt cttaatttaa tatatttatg taaatgcatg cctgaaattt 1020
ggttagattg gctgtgtttt gtgtctttta acatgatcaa atgattaaac tttatgctta 1080
tgacttgaaa aaaaaaaaaa aaaaaaa 1107

<210> 20
<211> 1183
<212> DNA
<213> Homo sapiens

<220>
<221> misc feature
<222> (266)
<223> n equals a,t,g, or c

<220>
<221> misc feature
<222> (426)
<223> n equals a,t,g, or c

<220>
<221> misc feature
<222> (1170)
<223> n equals a,t,g, or c

<220>
<221> misc feature
<222> (1178)
<223> n equals a,t,g, or c

<400> 20
gtgattcaaa gccatcacaa aacactataa gactgaccaa aatttagata acctttgaac 60
cacgattttt ttccacatct gtytgtgaga cacagcgcaa tgctactgcc cttccagaaa 120
ctgtgctaaa aagagaaagt ccaaaagact ctaaacaaaa acctcgacgc cgttgaggat 180
gtgtttcatt ctggtggtct gttttgcaag cttgataaca gaatgtccgt gccattgtaa 240
atgttgtaga gatgtgggcc gtggcncaac cgtcctatat gwgtgtagca tggtacagaa 300
caaactgctt acacaggtct cactagttag aaacctgtgg gccatggagg tcagacatcc 360
atcttgtmcm tctataggca agaagtgttt ccagatcctt tggaaaggtg ggcatggggc 420
aggtsnttgg agagtggcgt ttgagcagag cgaccccatt tccgtgtgaa ccataggcac 480
aacccaggaa gtttccccac ttgtaggagt gtgggtattc cagagcaaga ctgtggccac 540
catcttcccc tcttggtgtt ttccgaaagt gacagtgttg gtcatcccat gaccactgaa 600
gcttagtaac cagcgccaaa aagtagattc atcaaactag agaccccagc tccccttctc 660
gccatcttct ttctcaagtt gaccgtggtg ctgtttctgg aaggcatctg caactccaag 720
tccatgcaga actctggaag gccaagttca tcgcagcatg ttcaccatat cccagcctcc 780
aaatctatcc tcctaccttc caacgcatga cctgttgggg agcagagact taacccccaa 840
ctcagaggaa cccttcctcc agcgtctttg gcatggtttc tagggtgaga gttcccaatt 900
tggatagaac ggccaccata ttggttactg aatctctctc ccttgttttt attacgtttc 960
ctttttcaaa ctgtccatgg gaaggctgaa ttgagtgact ccccagaatg aagatgagaa 1020
ggtgaatata atcaatgcca atgtaatgcc agcgggtgar gatggccgat ggraggtttt 1080
caaagatgta gctagcattt tggaaaccat atgggcaaaa cccgggcaac cagagggggg 1140
aacaggttaa gggaccgttt cccaggaaan tccccaantt ttt 1183

<210> 21
<211> 1420
<212> DNA
<213> Homo sapiens

<220>
<221> misc feature

<222> (524)
<223> n equals a,t,g, or c

<220>
<221> misc feature
<222> (585)
<223> n equals a,t,g, or c

<220>
<221> misc feature
<222> (596)
<223> n equals a,t,g, or c

<220>
<221> misc feature
<222> (1042)
<223> n equals a,t,g, or c

<220>
<221> misc feature
<222> (1062)
<223> n equals a,t,g, or c

<220>
<221> misc feature
<222> (1144)
<223> n equals a,t,g, or c

<220>
<221> misc feature
<222> (1171)
<223> n equals a,t,g, or c

<220>
<221> misc feature
<222> (1286)
<223> n equals a,t,g, or c

<220>
<221> misc feature
<222> (1350)
<223> n equals a,t,g, or c

<400> 21
gtctgcatcc cgggcgcggc tgggttgagt gttctcttag gaatggtgga gaactgggtc 60
cttgaggagt caccggggag actgctcgca ctgtttgtgg tgcgacgggc actggcccag 120
ggacagaggg aagagaaggg ccagccagcg gcagtggagt cggcaggctg gctgcccact 180
cgctttctct cctcacaaga ctcgcttccc ctgtcttcga ggatctcgaa cggactatag 240
tctggactcg ctgggctgga ggaaacttgg ccgctggcca cccggaggag actgaaaatc 300
ctttggtcaa cagggcgcct ttccttgaac caaaacaaaa ctttccgaag ccggaaagga 360
aacgcccagt gtcgcctgag agccctggag ctgcgcgaga cccaggcact gagtgcggcc 420
tcggcctctg acctctaaca cgccgggaac aaaccatctg gggcggcccg caggcctgcg 480
ggagcggaat gtgacccgaa accgaccgac ttcctgaccc atantccata gttctcttca 540
gcaacttgaa cattttggaa aaagaaacaa tcttaacatg ccacnaccta atgganaaac 600
taaatcccct tcctacacct tgctttccaa aagttaaaaa aaaatagtta aacgctatta 660
gaggtctcaa gttcactgtc accagatcag ctaggtccag aatcttcagt tcttgaagcc 720
aagccctaca aatagattta ttgtagcata tcacacctct tcaggtgact taaaacaatg 780
agaattcatg agaaattatc ttcatcctca agtaaaaatc atgaggtgcc tttcacatgg 840
atgaaattgt aagtgcttgt tgaacaagga ataattggat aatggtattg tggtcatact 900
ttttaagaat atctgttaga aagatatagg atgcagaaca tctaggattt gctgaaagtc 960

atttattatg gatagggggt atgagtaagt tcatagatga aaagggatga aacaagattg 1020
gccatagttg ctctattttt gngtatcttg tttctttatt tngtttcttt aaaaagtcct 1080
catatcactg acatttacac ttagttttag ggaaagtcaa atttagaaat aagctacagc 1140
tctntaagct atcggtctaa ctggattttt ntcgatgctg aagaactttt taaaaaattc 1200
agccatttag gtcacacagc aaatacattt ggcattaaat tcctagtatc actaaagtac 1260
tccctcccac cgccgcgccc cccccnttcc ccccgcaccc ttagacctgg gcaagagaga 1320
cttctatcct ggactccatg ctttaaaggn acttacatat cacacacaca cattaattta 1380
aaaaggaaaa aaaaaaaaaa aaaaaaaaaa aaaaaaaaaa 1420

<210> 22
<211> 1575
<212> DNA
<213> Homo sapiens

<400> 22
gtccattctt ccggtggaga tggctgcggc cgtggcgggg atgctgcgag gggtctcct 60
gccccaggcg ggccggctgc ctaccctcca gactgtccgc tatggctcca aggctgttac 120
ccgccaccgt cgtgtgatgc actttcagcg gcagaagctg atggctgtga ctgaatatat 180
ccccccgaaa ccagccatcc acccatcatg cctgccatct cctcccagcc ccccacagga 240
ggagataggc ctcatcaggc ttctccgccg ggagatagca gcagttttcc aggacaaccg 300
aatgatagcc gtctgccaga atgtggctct gagtgcagag gacaagcttc ttatgcgaca 360
ccagctgcgg aaacacaaga tcctgatgaa grtcttcccc aaccaggtcc tgaagccctt 420
cctggaggat tccaagtacc aaaatctgct gccccttttt gtggggcaca acatgctgct 480
ggtcagtgaa gagcccaagg tcaagg[?]gat ggtacggatc ttaaggactg tgccattcct 540
gccgctgcta ggtggctgca ttgatgacac catcctcagc aggcagggct ttatcaacta 600
ctccaagctc cccagcctgc ccctggtgca gggggagctt gtaggaggcc tcacctgcct 660
cacagcccag acccactccc tgctccagca ccagcccctc cagctgacca ccctgttgga 720
ccagtacatc agagagcaac gcgagaagga ttctgtcatg tcggccaatg ggaagccaga 780
tcctgacact gttccggact cgtagccagc ctgtttagcc agccctgcgc ataaatacac 840
tctgcgttat tggctgtgct ctcctcaatg ggacatgtgg aagaacttgg ggtcggggag 900
tgtgtttgtc acttggtttt cactagtaat gatattgtca ggtatagggc cacttggaga 960
tgcagaggat tccatttcag atgtcagtca ccggcttcgt ccttagtttt cccaacttgg 1020
gacgtgatag gagcaaagtc tctccattct ccaggtccaa ggcagagatc ctgaaaagat 1080
agggctattg tcccctgcct ccttggtcac tgcctcttgc tgcacgggct cctgagccca 1140
ccccttgggg gcacaacctg ccactgccac agtagctcaa ccaagcagtt gtgctgagaa 1200
tggcacctgg tgagagcctg ctgtgtgcca ggctttgtgc tgagtgctgt acatgtatta 1260
gttcctttac tgctgaccac attgtaccca tttcacagag aaggagcaga gaaattaagt 1320
ggcttgctca aggtcatgca gttagtaagt ggcagaacag ggacttgaac caagccctct 1380
gctctgaaga ccgcgtcctg aatttcttca ctagagcttc ctcatcaggt tacccagaag 1440
tgggtcccat ccaccatcca ggtgtgcttg gatgttagtt ctccaccctc gaggtgtacg 1500
ctgtgaaaag tttgggagca ctgctttata ataaaatgaa atatattcta maaaaaaaaa 1560
aaaaaaaaaa ykcga 1575

<210> 23
<211> 541
<212> DNA
<213> Homo sapiens

<400> 23
caggagcaag gctttgtgct atatctacat aatcttagac cctgttcctt ccaattccag 60
ggatatgctc ttaaccactg cagtataagc ctccccgcya cactctgagt ggagcagagg 120
aaggtgtttt tgtctttgag aaaggcaagg atgaagggca agatttgagc catggtggta 180
gatcagaaag aagatctgat aacaggctta gggatcaaaa tggtaaggaa atggcttcag 240
gggagtcagg cctggcccct ggagagggag gagagggaag ggctaggctc tttatgtaca 300
tgctgtccat ggggcctggt aagattcmtg gaatcactaa cccatttcac aggtgaggca 360
attgagcctc tcagagctga agtaactgac ccaaagcatc cgtgctcttg tgtggcagag 420
ccagaagtca aatccaggtc tctgtgamct caaggggcac caaartgagt atcaaaaagg 480
cagaaaggga cttatccctt cactcactca gcaaaagcat agtaagcagg tggcgtgcct 540

t 541

<210> 24
<211> 833
<212> DNA
<213> Homo sapiens

<400> 24
gatcttgtcc aagcagtcgg ggctacttcc aagaatgtca gctcctgtta gcaaccagtg 60
gagtctggcc ttgggctcta agttgacctc tctatagctc caaatcctac caatctcaga 120
aaactgtaag aggcacagat gactccacca gctgcagagt gactctgaag agagtcttca 180
cttactgcac aggcaaagaa aggcacagga atatttccta cctctccctc ctgtgagtcc 240
cacctccccc cacccccatc tccaggaggc aggtagagca gttctraccg agaggataga 300
ctgctgttgc tgtctttccc cagctctgaa ctagttttaa ggtagcttag gatgaaaaat 360
ggagaatgat tgggggttcc aaaccacttt yttctccctt ggcttatatc tcttcaccat 420
ttggtggtca actgtgggsc taccctggac ctcatctact cagcgagaat tggacatgaa 480
gctagaggca gctgccttgg aagggaagtm aggctcactt ggacagccca ggccatggca 540
ggaagaatcc cttcctcttg gggtccttga tgggcatgtg tgatggggaa ggagcagtct 600
cccagccctg ggtctgctcc ccacatctct cctaattcca cttcaccttt tgccaccccc 660
tccccaccag aggcctagcc cttttgtcac cgaaggcccc cagagtgttt ctgtgtgaaa 720
ccctctcatt tacactgtgg catcaaaatc cacaaaagat ggattaattg cactctggtt 780
aatagcagca gcacaatgat taaaatctat attcctaaaa aaaaaaaaaa aaa 833

<210> 25
<211> 1555
<212> DNA
<213> Homo sapiens

<220>
<221> misc feature
<222> (1248)
<223> n equals a,t,g, or c

<220>
<221> misc feature
<222> (1389)
<223> n equals a,t,g, or c

<220>
<221> misc feature
<222> (1391)
<223> n equals a,t,g, or c

<220>
<221> misc feature
<222> (1393)
<223> n equals a,t,g, or c

<220>
<221> misc feature
<222> (1396)
<223> n equals a,t,g, or c

<220>
<221> misc feature
<222> (1551)
<223> n equals a,t,g, or c

<400> 25
ggcacgagct gggcagatgc aaaatctgga gagcgcgagg gccgggcggt cagtcagcac 60
ccagactggc agcatgaccg gtcagatacc aaggctttct aaagtcaacc ttttcactct 120
gctcagcctc tggatggagc tctttccagc agaagcccag cggcaaaaat ctcagaaaaa 180
tgaagaggga aagcatggac ccttaggaga taatgaagag aggaccagag tatctactga 240
caaaagacag gattactggg agcagctaag atgcctarat gaaaggttta ccatcactgc 300
tggttaggaa atggattatg agaactcgaa cagagggaag gtgaaatgca accggaggaa 360
acactctgat atgaggtttg aggccttcaa aattgctttg cagcataagc cacagtgagt 420
caggagtacc agggagtgga tagaatgttt atttgtttaa ctgagacttt ttagttcatc 480
aattattttg aagggtagaa cactctgtgg gctctctttc tatttccttc tgggtacaat 540
cacaaaaaaa aaatctctcc tagctgaaat tacatgcagt actagcaaag ggtctctttg 600
ttataaactg ttcattaatt gacgaacatt tgtgtactta actatgtata aggcatctca 660
tcgttcaatt tcaaatacaa attaaaatat tttttcacat ttgttatcct gttatgtttt 720
ctcttttaca aattgtctgt tcgtatcttt ttgtctctct ttaggcctta ttcttgtcaa 780
ttcatatgtg ctctaatgaa ttgaaatatt ttctgtatat taaacattac taacctttcc 840
tctgtcacac tgattgaaaa atgatctatt tagtttgttg ttttgtcttt aattttgtaa 900
gctttaaaaa gttaatattg cccttcagac accatcccaa catcacataa gaattttttc 960
atgttataaa ttctttgtgg acatatttga taactgtttt attatgagga ggaccataat 1020
taattcaacc attcccctat tttggtcatt taggtttttg ggtttgggtt ttttgtttgt 1080
ttaacgtctt tgcttgctat ttttaaagaat gctgcactaa atgtgaatgc ttgagatttc 1140
ttctctgtat ttagaatatt ttcctagaat ggattctcag aagaattctc agtctgtgga 1200
gaggaacatt tttaatgcat ggaagagctg gagtgaaccg aatttcanac tgccctgctg 1260
atccagaaat aagtttgctt acggaggctt ctagttctga agatgcaaag ttagatgcca 1320
aagcagtgga aagattgaag tcaaacagtc gggcccatgt gtgtgtctta cttcaacctt 1380
tggtgtgtna nanggngcag tttgtagagg agacctctta caaatgtgac tttattcaaa 1440
aaattacaaa aacattgccg gatgctaaca ctgactttta ttatgaatgt aaacaagaaa 1500
gaataaaaga atatgaaatg ttaaaaaaaa aaaaaaaaaa aaaaaaaaaa naaaa 1555

<210> 26
<211> 1543
<212> DNA
<213> Homo sapiens

<220>
<221> misc feature
<222> (69)
<223> n equals a,t,g, or c

<220>
<221> misc feature
<222> (717)
<223> n equals a,t,g, or c

<220>
<221> misc feature
<222> (899)
<223> n equals a,t,g, or c

<400> 26
   gagcgggata acaatttcac acagggaaca agctatgacc atggattacg ccaagcttgg 60
   aattaaccnt cactaaaggg aacaaaagct gggagctccc accgcggtgg cggccgttct 120
   agaactagtg gatcccccgg gctgcaggaa ttcggcacga gccgaacagt aggacatgtc 180
   atggcatttt tgctcaccct tgttccactc ctccccagcc gttgtcttgg tttggaggag 240
   atggcagttc ctaattccac ctgtattagt ccattctcat gctgctatgg ataaatatct 300
   aagactgggt aatttataaa ggaaagaggt ttagttgact cacagttctg catagctgag 360
   gagacctcag gaaccttata atcatggcaa aaggcaaagg agaagcagac aggacagagt 420
   gaatgccagc aggagaaatg ccagacgctt ataaaaccat caaatcttgt gagaactctt 480
   cactatcata agaacggcat ggggaaaact gcccccatga ttcagttacc tccacctggt 540
   cccacccttg acatgtggga attattacaa ttcaaggtga gatttgggtg gggacacaca 600
   gccaaatcat atcaccatcc cttgaaccaa aacgaacaag gctgacctta tttgcaacat 660
   tctaacttgt ctaaaggctg cctgaagaat tgatccctga ttcacctaac tcagatntct 720
   gctaggagac aagcatggcc ttaatctcag atgaggagaa gcagtagtca tggctcagaa 780
   agctgcagag agaccctaca gattcctggg gcaaaagatt ataggtggag acatatgaca 840
   gaccatcaag accccacaaa gatctcttgg gaaatttaag acaattaaaa gcagccatnt 900
   atacagagat tcaaaaaacc acaaacaggt ccaggcaagg atgcatgctc agtaaagacc 960
   tgagaagacc tttagctttc tctttgatgt gatctcaaaa ttcagaagca aggccaagat 1020
   aattaggaaa ggacttccat ggcaaagagc cagtctacag agaatgggag aagtagctgt 1080
   ttttcttttt gttttcaaat ccccaacata actattgtga atttaaaatc ccaaaatcac 1140
   aatgcataaa aagaaacgga aacatggacc attcaataat aataataaat tggcagaaac 1200
   tatccctgaa aaaatacagg tatcagactt actagaaaaa gattttcaaa caatgtttta 1260
   aatatgttca aacagtaaag aacaacatgg acagagacct aaaggaaatc aggtaaatta 1320
   tatgaacaaa ggggaatttc aacagagata gacattatta aaagaaccaa ccagaaattc 1380
   tggaaatgaa aaatataatg ggcacactgg ggatagtcaa acttaaaaat tcactagagg 1440
   gattcaatag cagacttgga cagaagaaag aataaacaag cttaaagata acttatttga 1500
   aattatctag tatgaggtac aaaaaaaaaa aaaaaaaaaa aaa 1543

<210> 27
<211> 1262
<212> DNA
<213> Homo sapiens

<220>
<221> misc feature
<222> (621)
<223> n equals a,t,g, or c

<220>
<221> misc feature
<222> (641)
<223> n equals a,t,g, or c

<220>
<221> misc feature
<222> (722)
<223> n equals a,t,g, or c

<220>
<221> misc feature
<222> (723)
<223> n equals a,t,g, or c

<220>
<221> misc feature
<222> (726)
<223> n equals a,t,g, or c

<220>
<221> misc feature
<222> (730)
<223> n equals a,t,g, or c

<220>
<221> misc feature
<222> (1259)
<223> n equals a,t,g, or c

<220>
<221> misc feature

<222> (1261)
<223> n equals a,t,g, or c

<400> 27
ggcaccagaa aaaaaaaaga taatccaaag aatttaaatt gtaatcatgt ttcatgtatt 60
tgttttatta cttactttta tagcacttag tcccagtggt attagactgc tatttggttt 120
catacaaaaa ggattaaatt taaattcatt catgtttaga cttgagttat tacattttta 180
aaactatcat cttgccttta atgtttgtgg tcctacacaa actattagta catttcagta 240
tcctcttacc cctttgtttt taagtttttg attgctaaag caagactttt ttcttctaga 300
atttaagtca accaagtgtt atctatgttg taaaaatgga taatagtaga ttttaggtga 360
taaaacaact tgttagtaag acatttccta gcttaaaaaa aaaaatcaaa aattccatga 420
tagaaatgca gacctgtgag ggaaactcct gaaaagcata agaagcatcc cagagagcca 480
tgggttttct agaccagaga atttagaggg agattgtgga actgaggctt aggtggtcag 540
atcgtttccc ttatcactgt aatattttct gggggaaaaa tgctttctga gttgtttaaa 600
caagcatcct tacatttttt ntttaattaa aacagcctgt ntagggcttg ggattcccta 660
atactacagt agcagtatat gaatatgatt ttgtgattgt gtttttaaaa agataagtaa 720
tnngangaan tgttcttttg cagtcagaaa acactcacaa aaagacaaaa aaagttccac 780
agtattatat ttcatgtcag ttcaggccta aaatcctttg caaataagat gtttataggc 840
tggtcacaat taacaatgtt attattggca gcacttcttg gatggatacc ttttgggacc 900
tttcattaga aagagggaaa gaatggggtg gttttgtatg ggctcctgtt tggggtaaaa 960
atagcagagt cagttgctga ggaccaatga cctttcctta taaacattta gtttcatacc 1020
catattaggt cttgtcttga ggacccttta tatgtgcttg tttactagtg gccttccagc 1080
catagcattc ttaccttttt ttcctattct aagaattaaa aaaaaaaatt atagagccag 1140
caagggagga ggcaggaaac agaaatcgaa tttcatcatt ccagtatagt tgtccctttt 1200
tttgtatttc tgacttggtt ttataattat atttacttac taaaaaaaaa aaaaagggna 1260
na 1262

<210> 28
<211> 753
<212> DNA
<213> Homo sapiens

<400> 28
ggcacgaggt gatgacttca ctcccaattc tggcatttgg ggctgtctat tggccagacc 60
ttgcttcaca tagtttctca ccctcaagga gtctagccca gactccccat atgtcagtct 120
cagggtagca tttcaagagg gataaggtag acgtttctgg cctgttgtgg tgtaggctgt 180
gaattaccat aacatcactt ctttgagatt ttcttggtca aggcaaatca catgacaagg 240
actcaagagg gtagagaaat aggttctact atttagtgga aaggacagca aagtgacatc 300
acaaagagga atgcatatag agatgggggg aatatgtgac caactttagt aatcactgta 360
attctgaatt gactcacaaa cactatcaag acggatcatt gtcataccct agttcaaaaa 420
gcagtccttg cagcaataca gaacagatag aagtgaagag aatgtgattt tgctaaaaat 480
gacatattta catgaccagt gatgggtgag acctatgaaa aatccccaga gattctcaag 540
aactcataaa gtgcatttcc atatttatgt agaatatcaa tctcctgctg tctttgactt 600
cacctagtat attcctaggt atgtgtatct aagcccaagt tggtctcacg tttttgccta 660
cttccgagtc aatatgtgac atgccatccc acctttttgt gttaccacat tattataaca 720
taaggggtgg ttatgtttcc tggatatctt gag 753

<210> 29
<211> 1621
<212> DNA
<213> Homo sapiens

<220>
<221> misc feature
<222> (527)
<223> n equals a,t,g, or c

<220>

<221> misc feature
<222> (542)
<223> n equals a,t,g, or c

<220>
<221> misc feature
<222> (553)
<223> n equals a,t,g, or c

<220>
<221> misc feature
<222> (701)
<223> n equals a,t,g, or c

<220>
<221> misc feature
<222> (731)
<223> n equals a,t,g, or c

<220>
<221> misc feature
<222> (906)
<223> n equals a,t,g, or c

<400> 29
ggcacaggcg cggcctgcgg cttcttggga actctagggc cgcggccggg cctggctctg 60
ccggcggcct gttgggagct ggatcggagc ggtttggaac gacaagcccg acaaagagac 120
ttttaaaaaa ccatggcaga tgtggaccca gatacattgc tggaatggct acagatggga 180
casggragat saaaaggaca tgcaactaat accccttgaa cagctatgca tgctgctttt 240
gatgtctgac aacgtggatc gttgttttga aacatgtcct cctcgcactt tcttaccagc 300
cctttgcaaa atttttcttg atgaaagtgc tccagacaat gtattagagg tgacagcccg 360
tgccataaca tactacctgg atgtatctgc ggaatgtacc cgaaggattg ttggggtaga 420
tggagctata aaagcacttt gtaatcsttt ggttgtagtt gaacttaaca acaggactag 480
cagagactta gctgaacagt gtgtaaaggt attagaactg atatgtnctc ctgagtccgg 540
ancagtcttt gangctggtg gtttgaatcg tgttgcttac cttccaagcg tgaacagtgg 600
acatctagtt cataaagaca ccttgcactc tgctatggct gtggtatcaa gactctgtgg 660
caaaatggag cctcaagatt cttctttaga aatttgtgta naatctctgt ctagtttatg 720
aaagcatgaa natcatcagg tttcagatgg agctctgcga tgctttgcat cactggctga 780
ccgatttacc cgtcgtggtg ttgacccagc tccattagcc aagcatggat taactgagga 840
gctgttatct cgaatggctg ctgctggtgg tactgtttca ggaccatcat cagcatgcaa 900
accagntcgc agcaccacag gagctccatc caccactgca gattccaaat tgagtaatca 960
ggtgtcaaca attgtaagtc tgctctcaac actttgcaga ggctctccgg tagtaacaca 1020
tgatcttctg aggtcggagc ttccagattc aattgaaagt gcattgcagg gtgatgaaag 1080
atgtgtgctt gatactatgc gtttggttga ctttctcttg gtgctattat ttgaaggacg 1140
aaaagctttg ccaaagtcta gtgctggatc tacaggcaga atcccaggac tccggagatt 1200
agatagttct ggggagcgct cacatcggca gcttatagat tgtattcgaa gtaaagatac 1260
cgatgcactt atagatgcaa ttgacacagg agcctttgaa gtaaatttta tggatgatgt 1320
aggtcagact ctattaaact gggcctctgc ttttggaact caggaaatgg tagaatttct 1380
ttgtgagaga ggtgcagatg ttaatagagg tcaaaggtca tcatcattac attatgctgc 1440
atgttttgga agacctcaag tagcaaagac tctgttacgg catggtgcaa atccagatct 1500
gagagatgaa gatgggaaaa ctccattaga taaagctcga gaaaggggcc atagtgaagt 1560
ggtagctatt cttcagtctc caggtgattg gatgtgtcca gttaataaag gagatgataa 1620
g 1621

<210> 30
<211> 921
<212> DNA
<213> Homo sapiens

<400> 30
ccacgcgtcc gaccatgcca aatttcttgt ggttccctaa atgcgccatg tttgaagata 60
ctctgaggac attgtatata cttttgttct acctgagata catttgctta ctttctccac 120
atattgccct catgacactt atccttattg atggatttct tcaatgctac tattgtgcct 180
tacatgtgcc ttgtattata gcatttttat agcatttctc acccaattgt ggctatttgt 240
ttacatgtct gtctccttgg tggaactgtg aactctgtca taacagatgc cattttatgt 300
cagttagact tctttggttg ccagtaagag aagctgactc taatctaaac caaaaggaat 360
tcattggacg gatgtgggtt ggctcacaaa atcaaaggga caactgcgga ccgatcttgg 420
aatgatgctc tgacaccaga acagctctgt gaattcagat aggggtagtg aattgaccat 480
ttcatcaaat gctgcagcaa gctaggtggt ttccccaaag gaaattgagg agtgttacaa 540
gaagaccatt aggggaacgg ttatctggtg gctgataata acaaatttcc atggcagtct 600
ctttgctctc tgttggaaga ggtactccac catgggcctt gagcatctct acacatcctt 660
gctaagcgtg tcaaatttca agtcctaact gtcctctgtc tctggaggag gagacaggtt 720
tggttactgt ttgttgtaaa aattactgag cccttcacca tgggtgcctc agctgtatgc 780
aaagcccctt gtattgctgg gggacagagc aactggtact gccatgctgg tgctctggct 840
gtttgctgtt ggcaataaac tattctgttt tggttcaaaa aaaaaaaaaa aaaaaaaaaa 900
aaaaaaaaaa aaaaaaaaaa a 921

<210> 31
<211> 2095
<212> DNA
<213> Homo sapiens

<220>
<221> misc feature
<222> (14)
<223> n equals a,t,g, or c

<400> 31
cccacgcggt ccgnatcgtc cttccctcac ttcagagggt ggccagagct gaatacccag 60
agagggacaa gtaagggtcc agttccaaaa catcatgagg atgtatcatc ccacgtgtct 120
cacctgacag ttacagagga aacccgcacc cagaatgcac gtgctgtctt atgggaacac 180
tcagcgcaga gtgctcaggt ccggccacac tcgggctgtg cttggtcgtg ccatggaatt 240
cctcaggact ttctcagcct ccctaatggc agaagcccct ttacagcaag acatttaccg 300
tttgtctgaa aatagccgaa ctgagccttt tcttcaggct atatgagaag tctctagaca 360
gtgggcaccg tcagaaagcc cagagccttg tgatagctcc caccctgcct ggctcagatc 420
ttcccatttt tttcctctgg cactaacctc accttttgtt tttttgtgtt tgtgtttgtt 480
tttgtttttg cagagttgga ttacagaaac tcctatgaaa ttgaatatat ggagaaaatt 540
ggctcctcct tacctcagga cgacgatgcc ccgaagaagc aggccttgta ccttatgttt 600
gacacttctc aggagagccc tgtcaagtca tctcccgtcc gcatgtcaga gtccccgacg 660
ccgtgttcag ggtcaagttt tgaagagact gaagcccttg tgaacactgc tgcgaaaaac 720
cagcatcctg tcccacgagg actggcccct aaccaagagt cacacttgca ggtgccagag 780
aaatcctccc agaaggagct ggaggccatg ggcttgggca ccccttcaga agcgattgaa 840
attagagagg ctgctcaccc aacagacgtc tccatctcca aaacagcctt gtactcccgc 900
atcrggaccr ctgaggtgga gaaacctgca ggccttctgt tccagcagcc cgacctggac 960
tctgccctcc agatcgccag agcagagatc ataaccaagg agagagaggt ctcagaatgg 1020
aaagataaat atgaagaaag caggcgggaa gtgatggaaa tgaggaaaat agtggccgag 1080
tatgagaaga ccatcgctca gatgatagag gacgaacaga gagagaagtc agtctcccac 1140
cagacggtgc agcagctggt tctggagaag gagcaagccc tggccgacct gaactccgtg 1200
gagaagtctc tggccgacct cttcagaaga tatgagaaga tgaaggaggt cctagaaggc 1260
ttccgcaaga atgaagaggt gttgaagaga tgtgcgcagg agtacctgtc ccgggtgaag 1320
aaggaggagc agaggtacca ggccctgaag gtgcacgcgg aggagaaact ggacagggcc 1380
aatgctgaga ttgctcaggt tcgaggcaag gcccagcagg agcaagccgc ccaccaggcc 1440
agcctgcgga aggagcagct gcgagtggac gccctggaaa ggacgctgga gcagaagaat 1500
aaagaaatag aagaactcac caagatttgt gacgaactga ttgccaaaat ggggaaaagc 1560
taactctgaa ccgaatgttt tggacttaac tgttgcgtgc aatatgaccg tcggcacact 1620
gctgttcctc cagttccatg gacaggttct gttttcactt tttygtatgc actactgtat 1680
ttcctttcta aataaaattg atttgattgt atgcagtact aaggagacta tcagaatttc 1740
ttgctattgg tttgcatttt cctagtataa ttcatagcaa gttgacctca gagttcctgt 1800

atcagggaga ttgtctgatt ctctaataaa agacacattg ctgaccttgg ccttgccctt 1860
tgtacacaag ttcccagggt gagcagcttt tggatttaat atgaacatgt acagcgtgca 1920
tagggactct tgccttaagg agtgtaaact tgatctgcat ttgctgattt gtttttaaaa 1980
aaacaagaaa tgcatgtttc aaataaaatt ctctattgta aataaaattt tttctttgga 2040
tcttggcaaw aaaaaaaaaa aaaaaaaaaa aaaaaaaaaa aaaaaaaaaa aattc 2095

<210> 32
<211> 1838
<212> DNA
<213> Homo sapiens

<220>
<221> misc feature
<222> (1076)
<223> n equals a,t,g, or c

<400> 32
tgttcaccag tagctgggat tacaggcatg tatcactatg cctggctaat tttgtatttt 60
ttagtagaaa tggggttttg ccatgttggc caggctggtc tcaaacttct gacctcaagt 120
gatccacctg cctcggcctc ccaaagtgct gggattacag gtgtgagcca ccatgcctgg 180
ggcaaaagat attttcaaaa cattgtmaat aacttctccc ccaaacccag acagggtctc 240
attctgttgc ccaggctgga gtggcagggg caccatcgta gctcactgca gccttgaaca 300
ccggggctca agcaatcctc ccgcctcagc ctgccaaagt gctgggatta cacacgtaag 360
ccagtgcact cagtcctaag taacttttta aataccaaag gtagaaaagg aagaagaggg 420
aaaaaaaaaa taagcccata tatggaaaag gaaaagacag cagataaata taggcaaata 480
gaggtggaaa atataatcac gtagaattta gtatagtaaa ggattatctc tgaaaaacaa 540
aaacagaaaa ctatcagagc caaataaaga aaaatggaaa tgactgggga aaaccactca 600
ctaatgagtt gaatgttcaa gagaaactga gaaagagtac tgcttatata aaaattatgt 660
gaaattaaac aaaaatgtag tttagtaatg aatggtgttt aagcacttat ggaatataaa 720
attatcacct gttaaataag aatgcatagt aaatggaatg gacaaagaat atgagtgaca 780
gataaaatca gtttttaaaa aattttatta aagttgatta agcctattag tgaaagaaag 840
caggccaggc acaatggctt gctcctgtaa tgccaatact ctgggaggtc aaggcaggaa 900
gatcacctga gcccaggagt ttgagataag cctgggtaac acagtgagac tccatctcta 960
aaaaaattaa aaagtaaaaa aaaattagct ggtcatggtg acacacacct gtsgkccyas 1020
skmctwkgga ggctgaggca agaggattac ataagcccag gaagatgaag ctgcantgac 1080
ccatgattgt gccactgcac tccggcttgg gtaacaaagt gagatcctat tttccatccc 1140
caaccagtcc ccccagaaaa ggccaggtgt ggtagctcat gcctgtaatc ccagcacttt 1200
gggaggccga ggtgggagga ttgcttgagc ccaggrgcyy ysagtascag tttaggcaac 1260
aaagtgaaac cctgtcttta caaaaggcaa tacagtgaaa ccttgtcttt acaaaaagtg 1320
caaaaataag ctgggcatgt gtgccacaac acctgtaatt gcagctactc aggaggcaga 1380
gacaggagga ttgcttgagc ccagaggtca agactgtaat gaaccatgat tgtgccattg 1440
cactccagtt taactgacag agtgagactc tgtcttaaaa aaaaaattat tttgatatta 1500
agtgataagt ggctatttgc ctagtagctt cctaaaataa actagcataa aatgaaactt 1560
attttccaac ctatccctaa gcccttggaa tttcagttct aataactaga atagttacat 1620
aaaaccagta aaaagttgtt taataagaat gtacacattt cccctactaa aatttattgc 1680
ttgtagtttc aaaataaaat cataaagtta tctcaaagcc aagcaaaaaa attatttggt 1740
acaaagtagc aaactcgctg cattagaaga aaaggccatt tcttcacata tttgaataca 1800
ggcaccaaca catagttcca catgaaatta tatttcgg 1838

<210> 33
<211> 782
<212> DNA
<213> Homo sapiens

<400> 33
tacgagtttt tttttttttt ttttgagaag gagtctcggg ctctgtcacc caggctggag 60
tgcagtggcg agactccgtt tcaaaacaaa aacaaagcat caattcctga tcatgaccca 120
ctgtaacttc aagcaagcta caagaatcta tactagggtt cagacctttg aggctgacag 180

cgagctttga gtttgatgac agtacctaaa atatattaag tgtactcagg aactggccaa 240
gcatggggtg gggcttgtca ggaaactggt atttctttct tctatttgta gtgaataaga 300
tgctcaatag acgactttta ctcctcgtca atggtcgcat aactgtctct ttttagacac 360
ttatgaaatt gtctgaactt cctcctctac ttctccaact cccagaagag tgaaggtaac 420
aaatgttatg tccaaaccac ggtttgttcc cagaccctgg tttccaatgc ccacctcttt 480
tccaagaagt ccaaagagac gcccctcatc gcaaaggaag tgctaccgtg ctgcctcgat 540
gtcccccttg ggtgccatcc ctgaaacatc gaacctccca tacctcttct ccagccgtcc 600
ccctcatcct cgttccccgc ctaccctctc ttcaacttca ttcattcatc caacattcgc 660
tgggggattt ctacattgac acgccccgga cagaagcctg gggtaaagat gatcaggaac 720
acgttccctc ccgctaagcg gcttggcaga gtaagaggca tcccaaaact cgtgccgaat 780
tc 782

<210> 34
<211> 1560
<212> DNA
<213> Homo sapiens

<220>
<221> misc feature
<222> (461)
<223> n equals a,t,g, or c

<220>
<221> misc feature
<222> (497)
<223> n equals a,t,g, or c

<220>
<221> misc feature
<222> (499)
<223> n equals a,t,g, or c

<220>
<221> misc feature
<222> (595)
<223> n equals a,t,g, or c

<220>
<221> misc feature
<222> (621)
<223> n equals a,t,g, or c

<220>
<221> misc feature
<222> (622)
<223> n equals a,t,g, or c

<400> 34
   ggctttttct gacattggtg aaccccctag actacaatta atccctttgc tacagacacc 60
   tgtagccctt gctgcctcct ttttgttaag aggtcttata attttatgtt tgatgtccat 120
   cttccccact tgattgaaat gcactattta tggaggagct atgtctgtat tgtttgttgc 180
   tgtatcccta ttgtctagca tagtgcctga catacagtac aggctcaaga catatttgca 240
   cattgattta tggaaaactg atactcaggt tctgaagaat aaataaatgc acctgaactg 300
   tcaaagtgct aaaagcaggc aatccagaaa tgtctggagg taggaatcac agctgcaaga 360
   ggcacttcct ggttaacctc gccctccgac ctctagttgg agccacccct ttggatccta 420
   cttcagcctt tctggagtca gtggctcaca ggtttcctga nacaaagaga agaggcttga 480
   gactattatt acatatnant cttctttaga agcaaagttg gttcgtggat tgaattttca 540
   accttacagt accaattata aatcctgagg cattctatca gttaagacaa cttanaatat 600
   ttgatcccat tcagaacttt nncatttgtt ttaaagcagg aaaagtaaar gmagtcaatg 660
   twmtaacyct tcttctttaa aatgtggatc atagtcctct tggggatgtt tgttcattta 720
   atattaacat tttttaagct tgscatgtwt cgtgggtgta tctgtttggt ttcctttggt 780
   aactgcattt tgccatgacc cttgatacca gctctactgc tacagcccta ggctaggcca 840
   ccgtcatctg tggcctggac cctttcagtc ctaactggtt gccgtgtctc ctttcttagg 900
   ccccccaaca gttcatcttc catatccaca cacagtagcc cttaatgatg tttttaaagg 960
   aatgagctat attaggatga tttctttgcc caaaaactcc ttcaatggtt ttccacttac 1020
   tccagagacc caaaaatcta aggcattttc cctatgggcc ctggatggcc ccacattccc 1080
   cctgaccccc gtctccagtg ctgtcccctc ctgcttgctg tgcttccagc ccacactggc 1140
   ttccttcctt accctcaggg ttccaccaat ctggatcttg tctcataaac tttgttcctc 1200
   tgacttcttc tttttgaatg ttcttttccc agaccttcac atggctcttt gctctcccct 1260
   tctgagtctg aacacaaagg tcactgactt aaagaggctt tttcccacca tccagttgaa 1320
   atcagcaccc tctctgtaac tgtgtaccac attgtcttat tctttctcat aggtctgaaa 1380
   ttgtcgtatt catttttaat gtattttttg cctttttgtc cctgctaaca tataagcttt 1440
   ttgaggtcag agactttctt ttcactgtag tattcccagt tcctaaaaca gggccctaca 1500
   catattggat gtttaataaa catttattga ctaatacaaa tgaaaaaaaa aaaaaaaaaa 1560

<210> 35
<211> 1092
<212> DNA
<213> Homo sapiens

<400> 35
ggcacgtgtt gtggagtctc ctaagtgctt gctggacaca atttcttgtc tatttttgct 60
gccttatgat tctccaaagg acatttcccc cacgggctct gaggacatct ccgtggcttt 120
ccaaccccat gggggttaaa gggaaaaaaa aaaaaggaac gtttatggaa atgatgctag 180
ggttgttctc tcctctttgc cttgtcactg gaattgctga aggcagggct gaagatgctt 240
ctctacatga catctgcacc acccaacaca cacttacctt cacaccttca taccctgttg 300
gagggtcctg atgactacag ggcagtaaat tcagccccac aggagggcca cagcagcccc 360
cagcctctag cctcctaccc tccttcttag gcaaccttga caggaaattt tccctctgcc 420
ttctccttga tcccaacggt agctgcataa tagctgagct cacataatcc ctgtcgccag 480
tgctagagtg cccttagatg gaggtagccc aggtttgact tcctgaatcc ccagcagcag 540
gccttttctt tctagagctc tttgcaggaa gagaaagctt tggaccagct catgctgggt 600
gtaatccttt gtggaagcct ccctgtttcc cttctctgat ctgccccgga gattcctgtg 660
tgtcccagtc tctagggagg gaggcttagc tggagaggtt caagggcagg agaaagcagg 720
agaatgcaga ggccgcgggg agaggacaga aagtatatca tttataacta acctttagcc 780
tttagccact caaaaatatt tcctaatagc ctaagggttc ttggcaggtc tttccccaca 840
tcagcaagaa atcttgggag ttgggaagag tcagaccttg ttccctgaac aagctttctg 900
ctttggccaa gagttgttag gagattaatg cctgtccctg aaaggcacag gttggagtgt 960
ttacttcttc ctctcctttc ctctctcccc ccttagagat cgtgaccctt cctgcttgcc 1020
tccctggtgg gctctttcag gctggacaca gggtttaaaa aaaaaaaaaa aaaaaaaaaa 1080
aaaaaaactc ga 1092

<210> 36
<211> 1153
<212> DNA
<213> Homo sapiens

<220>
<221> misc feature
<222> (409)
<223> n equals a,t,g, or c

<220>
<221> misc feature
<222> (511)
<223> n equals a,t,g, or c

<220>

<221> misc feature
<222> (1001)
<223> n equals a,t,g, or c

<220>
<221> misc feature
<222> (1089)
<223> n equals a,t,g, or c

<220>
<221> misc feature
<222> (1113)
<223> n equals a,t,g, or c

<400> 36
agactatcct caaggagctt acatatcagt aaataaatta ttaaaggtgg aaaatgtggt 60
aaaagagaca taatgtctcg gagagagaac aaatttctgc tttaggagtg ttcttagtta 120
aggtaacatt agcttctata atacgcacac tcccaaatct cagtatttca acatgagttt 180
ctctcttgct catgtaaaga ctggtcaggg acccaggttg acagaggctc ttcagtacat 240
agcttccaag attgctgtgg gtgtgacatc cagccagaaa tctggtgaag agagagcaat 300
grttacacag gaacttttaa tggaccaggc ctgggacagc gtatgtcact tccaccaaca 360
tcccactcac cagaatttsg tcacagggcc atagctatct gcagagaang ctgggaaatg 420
gaacttagct atgtgctcaa gaggaaaagt aaaacagtta ttgaataatt agtaataatt 480
agcaagtaac tacctagggg tcacagagga nctctcaggt agaatttaga cttaaagatg 540
atgggggagt gtgtggaaga gtggtgcaga atagggaaag gggggattga aggaagaaca 600
agctctagct tcacctgcat gggtagagcc cacagtgttg gtagggacat gttagctttc 660
aacatcagct tcttaacagt attattcttt catcggagga aattagtcta tttctgagga 720
aaaaaaaatc tgcaatacgt agcaatttac ttacttggat attgaatgtt aaagcagaga 780
gagactttgt cctcaaaacc ctcccatttc agaagtgagg agcctgggga ggtcatgctc 840
tctggatgtc acacagtgag tcactgtcaa agccagaata gaacccagac ctctcagttt 900
cccatwccag tgctctttct atgaggaaag tataagtttg agcattttta aaccttaatt 960
atgtagaaat aaccatgata ttttatcgta aattatttca ntcatctcat tttaaatttt 1020
actccaaact aaaggaaaac ggtactgatt taaaacatct atcataattc aatatagccc 1080
atatttctnc tttaggaaaa attttttttt gtnttttatc ctgaagaccc gtgccctctt 1140
cctgtgtctc atg 1153

<210> 37
<211> 985
<212> DNA
<213> Homo sapiens

<220>
<221> misc feature
<222> (633)
<223> n equals a,t,g, or c

<220>
<221> misc feature
<222> (642)
<223> n equals a,t,g, or c

<400> 37
ggcacgagca cacccccctg agaccaggga gcatttattc aaggaaacac ttgtctttag 60
aggatgttga cgatgcccca aacttactgt agctgtcagg aaaattaggt gagctattta 120
gtatcattga gcttcatttt acagaaccag catgttgtcc ttagacttcc ctctgatcct 180
tttaggtctc aacttacata ttgccctctt gagccttcta gttcccagac tgagttagga 240
accccaaccc atgctggact cagttagtcc tttccacatt gtgctgtaat tggctatacc 300
ccatctgtcc ttcctgccag actaggagtc tcctgcgggc cctaacgttc ccaatttccg 360
gtgtttggac tggtgctctg tagatgttta gggaatgaaa gggtaatgaa taaattaatg 420

aaacaaataa gaatcatata gtattagcag cactagataa aaggtgtaaa atcttaagtg 480
atccaccatc ttttaaataa ttcattcaaa cgatatttca aatgcatatc acctccaaga 540
aatcgtttct gcatttcrrs tgasttctac gatgccwwrt gaatgarraa rsrrgracak 600
ggyrtggttc tggggggctg tgagagtaac ggngcaatcc tngtcattgt cgtagttatc 660
tggccatcca gggcttctca ggttgccaaa tgccttgtga tagtctctgt tgcaatctta 720
gaggaaaaat aggcataatt aatgtacgca ttccaatatt tagtgctctt tcaacttaca 780
caggaatcat tcaaaaagat cattgcattt gataaacttt agaaaaaagt aatccagctt 840
cttcgtttac ctttgagata attgagaccc tgagcagtga agtgaattgc tcaagcagca 900
cacacaggtg caacgcaaca gctcgttcac acaaacacgc ctacaggaag catgacacag 960
gaggcttctc ctttaaagac gaata 985

<210> 38
<211> 1122
<212> DNA
<213> Homo sapiens

<220>
<221> misc feature
<222> (380)
<223> n equals a,t,g, or c

<220>
<221> misc feature
<222> (381)
<223> n equals a,t,g, or c

<220>
<221> misc feature
<222> (402)
<223> n equals a,t,g, or c

<220>
<221> misc feature
<222> (499)
<223> n equals a,t,g, or c

<220>
<221> misc feature
<222> (505)
<223> n equals a,t,g, or c

<400> 38
agtgaaaagc agatgttaag tggcatatgt gtcttcagtc acctctgtgt gggttgttct 60
gtagtataga gggtgttcta aaaatgatct ttaggaatgg agtgaggctt gtttttgttt 120
ttgttttgtt ttacacttcc acacaatccc ttttcaattc cttgcaaact gctgagtatg 180
tactattttg ccagcaaagg ctgagcctgt atgaacccag ccatgtgctt tgtctgtgca 240
tgtccccaca caggaagcac accagagaaa gcgatacttc agggtagatt gatttcatta 300
ggaacttcat tatcaccagc ctcaaatggt tctggccagc agtctttttc tatctgtatg 360
attaaccctt ctctgccgcn nagcacctcc tcccaccacc tnttctcagt gttaacaggt 420
gatctagact cctactctca gagaaaattg aagccaacaa gtagaaagtc ttttttgcta 480
ccaaagacac aaacctatnt tgttntgcat ccatcctcac ccccgctggt gcttgttcaa 540
cacaggagtc ctctctccac ctacccaaag cctgtcccct cctgctgtgc cctggatctt 600
atctctgtca ttgccttaga aacctttctt gtatatattc atctttttcc ttcaatagat 660
ctttcttatt ggattttaag catgttgcag cctcttctgt taataaaaca acaatcaaca 720
aaaacactct cccttaactg catgctttat tccagctact accttatatc attcctttcc 780
ttcaaggcca aagtcctcag aagaggtggc aatatcctcc atcatttctt cacttcatac 840
tcattcttca acacatacta atctagtctc ttaccccata attcattaaa acacttattc 900
ttgggtcatg ggtgacttct gtatagctaa atccagtgga tatttttcag gcctcctctt 960
ccttacattt tagtatttca ccctattggc cattcttttc ttcttgaaat actctctcct 1020

ttagctttta tgacactgta ctcctggttt ttctcccatt tcttgtctgc tcctgcttag 1080
ttccctctgt aaacttggcc tctttcacaa ggccagtaaa ca 1122

<210> 39
<211> 598
<212> DNA
<213> Homo sapiens

<400> 39
gaattcggca cgagctggct gcaaggtctg ttgggggagg gtcctcactt gacccttact 60
ggggtcagtg tgggtcaagg gttaagtgtc accctcggcc cttgggagcc tcattgctga 120
gggtctcagc gcttaccact ggtcctggcg tcacggactg tggagctggg ggcagcccgt 180
ggtgggtttt atagcaagtg gtgagatgtg ggcgctgtgc tccaaaccag accccgttaa 240
gtgccacatg gtcaacagtt tagtgtgcag aaatgaattt ccttctctta atttttcctt 300
atttttccag cctgttgggg gaggtggagg tggtgaaatg ttagcagtga ccagttcatc 360
ctgatctgct tgggaccttc cagttttagc actgaaagcc ccacagccca agaatccctt 420
ggatatcaac cacggttcct ccttccagaa tgtcccaaga gccttagggc ctggagacac 480
acaggtgggg gcctgagccc ctgtccccct cctccagatg gagcaggcag ggccccaggg 540
ccccagggct cacggtgttc tggggtccac agtgtgctgt gcggccaggc tggtcttc 598

<210> 40
<211> 1129
<212> DNA
<213> Homo sapiens

<220>
<221> misc feature
<222> (1053)
<223> n equals a,t,g, or c

<400> 40
ggcacgagct tatttatttc tgctgtcctt tttcttatta ttctgcctat attatcctga 60
aaatatttag tcagttctgt tttgcccaca attagcatgg ctaggtcatt gatttcagca 120
ctcaggtcag gtatgtcccc aggaagggtc tcagtggttt ctttgcaggg atcacagcta 180
tgtcttttgg tatctattgc aatcatgggt ttgcttctat tttgaatttg tctgtcttat 240
ctcttggaca tcaaaagtgc ccttcagggt aggcatgcta cttgttttat atctgccacc 300
caatttcaac tgtaaaatcc taatcacaag tggcaactag ataggttaaa atgatttctg 360
gaactttcct tctggacatg taagatccta aaatcttacg agaatttcag tgagttgatt 420
ttgtctttaa tatttttttct taggaaaaag aagacccatt ttgaatctgt tcaactgaaa 480
acctcaagat ccccaaatat atgaagagac agtgctgtag cccttgagac taatgaacaa 540
agaaacctgc tctagtttta caggaccata ttttagggtc tgtcctcata cctgtcacat 600
tggtgatctc acagaggagg gccatgccgc tgaaaaggga aggagattga aacatttgat 660
tgccttatca catggtcaag taccttgcca aataaaggaa agcaaatgat ttgggtctca 720
actgaagatg aagctcaact caggaagaga tttatctgta tatacacata actgaaaacc 780
aagtttaagc ccaccaatgc actgctgatg catgccatat aattaatggg taactttgat 840
tctttatgac gtctacataa caagtgtgat ttggaaggca catgtgagca tatgcattat 900
gatccaattt atgtttttttc tttgtttata ttttggggaa aattaaaatt tttttaaggt 960
atatttttcc cattatttat tttcctgacc ttaaaacagc ttttctacta aaaaatggtg 1020
agcaatgaag acaataaatt tttcattttt ccnaaaaaaa aaaaaaaaaa aaaaaaaaaa 1080
aaaaaaaaaa aaaaaaaaaa aaaaaaaaaa aaaaaaaaaa aaaaaaccc 1129

<210> 41
<211> 1158
<212> DNA
<213> Homo sapiens

<400> 41
   ttaacccaaa tgggttggga tggcacgagg ggaaatggga ggggaagaga acagctgaca 60
   tcttgaggaa agctttgggg tagtggagag gtaagggggt catggtcagt ctgaactcaa 120
   caatagggct gaatgaattt accaaaggaa gctgccttat attatatgcc aggctgctgg 180
   ggaaagcctc aggtcctggc cagcccctgt tctcacaaga acatgcaggt taccacataa 240
   ataatggcat atgccttcca taggacgtca acctgactta aatctaccta taccctactc 300
   tctattcttt ggtttttggt tctcatccct gtggaaggaa atgggcctct tctggcatct 360
   catgctactc tgtgcttttc cttgggctcc aaattctagc tcataaagat gcaagttttg 420
   caatttccta taaatggtta agaaaagagc aagctgtcca gagagtgaga agtttgaaaa 480
   gagaggtgca taagagagaa atgatgtcca tttgagcccc accacggagg ttatgtggtc 540
   ccaaaaggaa tgatggccaa gcaattaatt tttcctccta gttcttagct tgcttctgca 600
   ttgattggct ttacacaact ggcatttagt ctgcattaca caaatagaca ctaatttatt 660
   tggaacaagc agcaaaatga gaactttatt tggtgcagtc agggctccat ttagttccct 720
   cactctgctt ctaatcaccc cttctcccag ccctcttcta tttgatagag gtctgtccct 780
   cagatcagca atgtcttagc ccctctcctc tcttccattc cttcctgttg gtactcattt 840
   cttctaactt ttaataaaca tttaggtata atacattaca gtaagtgcta tttagataca 900
   aacttaaaac atactatata ttttaaggat ctaagaatcc tttagagaag gcacatgact 960
   gaagtacctc agctgcgcag cctgtagcca gtttttttaa tgtaaaagta agaatgccag 1020
   ccttaaccta gccctgcaga taaaagctaa cttttattaa taccagccct gaataatggc 1080
   actaatccac actcttcctt agagtgatgc tggaaaaata aaatcagggg cttcaggatt 1140
   aaaaaaaaaa aaaaaaaa 1158

<210> 42
<211> 1767
<212> DNA
<213> Homo sapiens

<220>
<221> misc feature
<222> (765)
<223> n equals a,t,g, or c

<220>
<221> misc feature
<222> (1130)
<223> n equals a,t,g, or c

<220>
<221> misc feature
<222> (1545)
<223> n equals a,t,g, or c

<220>
<221> misc feature
<222> (1658)
<223> n equals a,t,g, or c

<220>
<221> misc feature
<222> (1744)
<223> n equals a,t,g, or c

<220>
<221> misc feature
<222> (1748)
<223> n equals a,t,g, or c

<400> 42
ccgggtcgac ccacgcgtcc gattgaacgg tttggggcat ccttcctagg aaaagaatgt 60
cagttaaggt ggggtctctt ctggttttgg tgtattttac cctgggccca gttgttgcag 120

aactggaggt gaccctgcct tctcattcct aacatttttc tctactacca cccggatttt 180
gaggcagacc cccaactcgt catggctcca gctgaagttt gaaatataac gtcccggact 240
tctagcctgt aggagctgca gatgtagtgg ggcagacatg gggagggtca gtggtgagcc 300
tatagaaaca tctctttccg caggaaaaga taaaggatgt gatgtgtgta gctcacctcc 360
aggctgaaat gcagactttc ctcatcttcc acagtaagca ggattccctt ctgataacct 420
tgtcagaaat gttgtttttc aaagggcatg tatggtatct gtcactttca gtgatgattg 480
tgtcgtcagt tgatgtctct tgacctgaac tgagtatgcc tgtggaaggt cctcttagcc 540
ccctcacaga aataggaggg ggtgtcctcg ggctgtagct gtgcttcctc tgaaggtcac 600
tggggaaaag ggataccaag ggccgttgtc cagcttatta tcccagctgc tgcacaaagt 660
gtccaggaac tggtccttag agcttttgag ttttatcaga tcagtttgtt ccttgggttg 720
gccatcaaga tgggtctcaa tataaatgaa ggaatctgaa tagantccag ttttatgtgt 780
ttctagagaa aatgctcaag tgttcttatg caagtcatgt tagatttata tgatgtgtga 840
aatctgctta caaggaaatt ttcatgattt gtgttagatt agcatttaat tgtctgcttt 900
aacagatact taatttattt caaaaataag gaaaaataga ggaatcggtg tgaatgtttt 960
aagactgaga gatgatgatc ctttactttt cctgtaaaga agataatttt taaatctttc 1020
atatcctgta gagaaaacca acttttcctc tgtgatatag tacattatgt ttgcactact 1080
ataatgtcaa gactgaaagt ataaaaaatg tacatataag attaattttn atatcttttt 1140
ttttaaaggg gtttgaggtg cctgcctggc tcattcagta aaacatacaa ctctcgatct 1200
tgggatcatg agttcaagcc ccacgttagg ggtagagttt actttaaaaa taaataaaag 1260
gggttgagtc tattgcacta agctctacat gactaattta aagtggagag atgttgtgct 1320
agatttaaaa aaaataacta gttttcttaa tgtgtctttg tatgatcaac agcatgccat 1380
aagcaataca aaacaccaag ccttatactt acaagaaaaa aggttaacat actggtaaag 1440
ttctaaacat atcaaatgta cataagtgac aaaggtagga ttttaaggaa atgtcagtat 1500
atagagaagc tcagtactgc attaaggaac ttcttcagaa ctagngaagt attcctgtgt 1560
ttgaggagaa aacttagggg tttgagaagt tatatttttc tatttaaaag ggttaaatta 1620
ttgcataatt tggaaaaggt tgctttgaat gtaggacnaa actgtttcaa agatttttgt 1680
ttgaaaagtt tatgtatttt tgtgccttaa tatttgttct gacttttaat aaaatgcttt 1740
ctgnaaanaa aaaaaaaaaa aaaaaaa 1767

<210> 43
<211> 917
<212> DNA
<213> Homo sapiens

<400> 43
   ccacgcgtcc gggctcaccc caggccgaga ccaggtggtc cgaccccatc gctcttcacc 60
   aagggaagtc gccagcctcc atcgacacgt ggccagggcg acgcagtggt ggtatgatcg 120
   tcatcacctc tatcctctcc tccctggcca gcctcctgct cctggccttc ctggcagcgt 180
   ccaccgcacg cttgagccct cagtcacttc cagagacctg ataccggggt tagtcagggc 240
   aaccacctgg aggaagtggg ccaggagctg cttctagaag gaaggaaagg gagagactgc 300
   aggaggaccg gggacccagt gctgcctcct ctccccatcc agctccagcc tgtggtggcc 360
   ggaggaggcc ccggagcagc tgagaattgg ctccttcatg gggaagcgct acatgaccca 420
   ccacatccca cccagcgaag ccgccaccct gcccgtgggc tgtgagcctg gcctggaccc 480
   cctccccagc ctcagcccct agcctggccc ttgtggctgg ggcgtgtgtg gctgtggcca 540
   gtgtgggggc aaggacgtgg tagttattcc cagcccctgc accctcctcc tcacccctgc 600
   caaagtccca ctgatgtagg acagatgtca gggttctaga cgtctttggt gcaaaaaggg 660
   ggttttattc aagcacaggg acaggaccca tgggcaggga gagcggcacc ggggtggtga 720
   ggagtggccc gttatatata ctttcgagtt gggagggctt agagagagcg taagtctcta 780
   aggaatttgg gaagcaaggt ctccagggtc ctgagggggc tagctgttgt taggaaaagg 840
   tcatttatta ctgtttagta aaaactttca ccagaaaaaa aaaaaaaaaa aaaaaaaaaa 900
   aaaaaaaaaa aaaaaaa 917

<210> 44
<211> 1987
<212> DNA
<213> Homo sapiens

<220>

<221> misc feature
<222> (1554)
<223> n equals a,t,g, or c

<400> 44
ggcacagttt ccagggaaag aagggcgggg atgtcagggc tggagagtgc ccgtgtcctt 60
ctgtgtgcat tgggctcctt cctccttaat tctctgcttt ccacttttag gctgaactcc 120
agtgcaccca gttagacttg gagcggaaac tcaagttgaa tgaaaatgcc atctccaggc 180
tccaggctaa ccaaaagtct gttctggtgt cggtgtcaga ggtcaaagca gtggctgaaa 240
tgcagtttgg ggaactcctt gctgctgtga ggaaggccca ggccaatgtg atgctcttct 300
takakgagaa ggagcaagct gcgctgagcc aggccaacgg tatcaaggcc cacctggagt 360
acaagagtgc cgagatggag aagagtaagc aggagctgga gacgatggcg gccatcagca 420
acactgtcca gttcttggag gagtactgca agtttaagaa cactgaagac atcaccttcc 480
ctagtgttta catagggctg aaggataaac tctcgggcat ccgcaaagtt atcacggaat 540
ccactgtaca cttaatccak ttkytggaga actataagaa aaagctccag gagttttcca 600
aggaagagga gtatgacatc agaactcaag tgtctgccrt tgttcagcgc aaatattgga 660
cttccaaacc tgagcccagc accagggaac agttcctcca atatgtgyat gacatcacgt 720
tcgacccgga cacagcacac aagtatctcc ggctgcagga ggagaaccgc aaggtcacca 780
acaccacgcc ctgggagcat ccctacccgg acctccccag caggttcctg cactggcggc 840
aggtgctgtc ccagcagagt ctgtacctgc acaggtacta ttttgaggtg gagatcttcg 900
gggcaggcac ctatgttggc ctgacctgca aaggcatcga ccrgaaaggg gaggagcgca 960
rcagttgcat ttccggaaac aacttctcct ggagcctcca atggaacggg aaggagttca 1020
cggcctggta cagtgacatg gagaccccac tcaaagctgg ccctttctgg agctcggggt 1080
ctatattgac ttcccaggag ggatcctttc cttctatggc gtagagtatg attccatgac 1140
tctggttcac aagtttgcct gcaagttttc agaaccagtc tatgctgcct tctggctttc 1200
caagaaggaa aacgccatcc ggattgtaga tctgggagag gaacccgaga agccagcacc 1260
gtccttggtg gggactgctc cctagactcc aggagccata tcccagacct ttgccagcta 1320
cagtgatggg atttgcattt tagggtgatt tgggggcaaa aataactgct gatggtagct 1380
ggcttttgaa atcctatggg gtctctgaat gaaaacattc tccagctgct ctcttttgct 1440
ccatatggtg ctgttctcta tgtgtttggc agtaattctt tttttttttt tttttttgag 1500
acggagtctc gcactgttgc ccaggctgga gtgcagtggc gcgaatcttg gctncactgc 1560
caagtccgcc tcccgagttc caagccaatt ctcctgcctc agcctcccga gtagctggga 1620
ttacaggtgc ctgccaccac acccagctaa cgttttgtat ttttagtaga gatggggttt 1680
caccatgttg gccaggcaga tctcaaactt ctgacctcgt gatgcactca cctcggcctc 1740
ccaaagtgct gggattacag gcgtgagcca ctgcgccctg cctgtttgta gtaattttta 1800
ggcaccaaat ctccctcatc ttctagtgcc attctcctct ctgttcaggt aaatgtcaca 1860
ctgtgcccag aatggatgac caggaacctt caagagtggc tgaaaagatt gcagagttat 1920
cataataaat tgctaacttg cgtatwaaaa aaaaaaaaaa aaaaaaaaaa aaaaaaaaaa 1980
aaaaaaa 1987

<210> 45
<211> 2053
<212> DNA
<213> Homo sapiens

<400> 45
acgctgggac ttgggcggtg gtggaggtgg taaccgtgat agtagcagct ccggcgrcag 60
caacagcgac tacgagggat ggcggcggct gcagcaggaa ctgmarcatc ccagaggttt 120
ttccagagct tctcggatgc cctaatcgac gaggaccccc aggcggcgtt agargagctg 180
actaaggctt tggaacagaa accagatgat gcacagtatt attgtcaaag agcttattgt 240
cacattcttc ttgggaatta ctgtgttgct gttgctgatg caaagaagtc tctagaactc 300
aatccaaata attccactgc tatgctgaga aaaggaatat gtgaatacca tgaaaaaaac 360
tatgctgctg ccctagaaac ttttacagaa ggacaaaaat tagatagtgc agatgctaat 420
ttcagtgtct ggattaaaag gtgtcaagaa gctcagaatg gctcagaatc tgaggtggta 480
agtccaaagt tttcattctt catgttttta ttattttaaa tttcagctac caaatatatt 540
tgagacaaga ctcaggatga gctgtctgat atttaaatat taagcaattc catttaagtg 600
ctggttcctc taggcactga aataaaatca ttttttgata aatatagaag tttccagtca 660
tgaaaattat tggcctattt taatgaattt agtgtgtggt taaagttgat ttcgtgtgtt 720
ttaatatggt catgatgatc atttatcttt tccgttacta aaaccttatt gcatttattt 780

aggttcaaca gtttgaatca cttgtagggc tttttatgat aggctaagac aaaagttaaa 840
gaaaattgga aattgacagg gtcttgctct gtcatgcagg ctggagtgca gtggtgccat 900
catagtgcac ttgagcttca aactcctggg ctcaagcaat cttcccacct cagccttcca 960
agtagctggg actacaggtg tacaccacca agcctggcta attactctgt ttctttaaaa 1020
cgatttttaa aacaatgtta ttttagttta ggaagttgct gaatcttaga actggccatt 1080
ttatataagc aaccttttct aatcatgcct ttagaagttt tctgttattt aaagttctgt 1140
tattttagag caaaaatctt ttatgaaatt caatctaaga ttttttaaat gctgagcatt 1200
ctaatttttt tccgaaaact agtggtattt aacaattaca gttactatgt ctttggaagg 1260
aaaatttca tgtagttatt ttatatcaaa ataactgcag tgttgggtaa attaataata 1320
catgcatttt aataatacag ttgctaaact gacttgtaaa aatctttctc tttcaactta 1380
ccaaaatcaa tctgcatccc agtggactca tcagtcaaaa atcaagtatg actggtatca 1440
aacagaatct caagtagtca ttacacttat gatcaagaat gttcagaaga atgatgtaaa 1500
tgtggaattt tcagaaaaag agttgtctgc tttggttaaa cttccttctg gagaggatta 1560
caatttgaaa ctggaacttc ttcatcctat aataccagaa cagagcacgt ttaaagtact 1620
ttcaacaaag attgaaatta aactgaaaaa gccagaggct gtgagatggg aaaagctaga 1680
ggggcaagga gatgtgccta cgccaaaaca attcgtagca gatgtaaaga acctatatcc 1740
atcatcatct ccttatacaa gaaattggga taaattggtt ggtgagatca aagaagaaga 1800
aaagaatgaa aagttggagg gagatgcagc tttaaacaga ttatttcagc agatctattc 1860
agatggttct gatgaagtga aacgtgccat gaacaaatcc tttatggagt cgggtggtac 1920
agttttgagt accaactggt ctgatgtagg taaaaggaaa gttgaaatca atcctcctga 1980
tgatatggaa tggaaaaagt actaaataaa ttaatttgct ctcaaaaaaa aaaaaaaaaa 2040
aaaaaaaact cga 2053

<210> 46
<211> 1272
<212> DNA
<213> Homo sapiens

<220>
<221> misc feature
<222> (1264)
<223> n equals a,t,g, or c

<400> 46
aggctgctac actaatagga tgtagcaaag ggcggaggag gagatccaga agcaaacaca 60
agcaatgcaa gaactccaca gagtggagct ggagagagag aaagcgcgga taagagagga 120
gtatgaagag aaaatcagaa agctggaaga taaagtggag caggaaaaga gaaagaagca 180
aatggagaaa gaaactagca gaacaggagg ctcactatgc tgtaaggcag caaagggcaa 240
gaacggaagt ggagagtaag gatgggatac ttgaattaat catgacagcg ttacagattg 300
cttcctttat tttgttacgt ctgttcgcgg aagattaaac ttaatgaaaa tctgtttgta 360
ttttctgcat attctctggc aaccttgccc catacttact tatttagcat agtcgagtgc 420
tctagtttct gtctctcagg cactcgtaac taaggaccac cattggccat tggtagatgt 480
ttgattgact taacaagaga gggacaaatt ttcaatttgt gaaactccaa agcagaaagt 540
attggtgctt gctaccttgt gaattcttcc ttagacatgc agagaaaatg tatgcaagag 600
accaaaaaga tggctccaag ctatgtcatg ttacctgtaa taaaatcttt tcttctagat 660
tctttctatg ttggcagata atctcccctt gtagcttcca ctcacttatt cttgcattca 720
gagtcacaat gatcatctta cccatgtggt ttttgagaaa gaaagatcaa ttctttgttt 780
gcagtaggta atcttagaga tggagatgat tgtagaatta ttcctagatg agtgtcaatt 840
tatttaattc cattgtcata taaggagtca aattgtttct tatcatttgt tcattgaaga 900
acagagacct gtctggaaaa tcgatctcta caaattcaat taaataatga tccccaaatg 960
sykmaaaagt gaaatacagc aattcaacag ataatagagc aatgtttagt atattcagct 1020
gtatctgtag aaactctttg acgaacctca atttaaccaa tttgatgaat acccagttct 1080
cttcttttct agagaaagat agttgcaacc tcacctccct cactcaacac tttgaatact 1140
tattgtttgg caggtcatcc acacacttct gcccccactg cattgaattt tttgcttatg 1200
ttgtttataa taaaactttt caattatctc ataaaaaaaa aaaaaaaaaa agggggggcc 1260
cggnacccaa tt 1272

<210> 47

<211> 773
<212> DNA
<213> Homo sapiens

<220>
<221> misc feature
<222> (459)
<223> n equals a,t,g, or c

<220>
<221> misc feature
<222> (503)
<223> n equals a,t,g, or c

<400> 47
cggcacgagc ttttgcccat aggataagta caaactagat ctggttactg cctgccccac 60
cagcctcagt atctctcaca actaggacta actttttctt ctgacaacta taaaatattt 120
cccttgcctt ctcaagtttg ctcaaggtca agttatgcct tttgcctgga atgacttgac 180
ttctcttttg ttttacttag ctggctgctt ttcatcttgt aggttaggtc aagggactcc 240
aggaagtctt ccctggacaa gtaatgaaga gggcataatc caagggccaa ctcccatgtt 300
ttggaacctg actccatttt caggcacgta atattgtcaa attcctttta aaagcacctg 360
tctgtctgtt aacgttggtg cagatactgc tattcccctc ctccatacca ttgctgatgg 420
ttactgaggg tatgggaagg gccgactagt ccagctgtnc acaaacagcc cttaatgtca 480
aactgaatac tgccaacgta gtnccagttt ctgtatctaa agactcagct tggagtcact 540
tgtctggact aaaaaagtac ccctccttgt ctggtttgtg actttctgta ctctgatgcc 600
cccagctttc tgccttctag aaatttgtca gaatttccaa aattcttggg ccttccttct 660
tgctctatat atggttttgg attcattcct tttaaaaaat atttactgtc atttcagtag 720
aattttgaca caataaatat aagcacatca aaaaaaaaaa aaaaaaactc gga 773

<210> 48
<211> 2119
<212> DNA
<213> Homo sapiens

<220>
<221> misc feature
<222> (1424)
<223> n equals a,t,g, or c

<220>
<221> misc feature
<222> (1438)
<223> n equals a,t,g, or c

<400> 48
wgcaagcttt gggagttgtt cgctgtccct gccctgctct gctagggaga gaacgccaga 60
gggaggcggc tggcccggcg gcaggctctc agaaccgcta ccggcgatgc tactgctgtg 120
ggtgtcggtg gtcgcagcct tggcgctggc ggtactggcc cccggagcag gggagcagag 180
gcggagagca gccaaagcgc ccaatgtggt gctggtcgtg agcgactcct acgatggaag 240
gttaacattt catccaggaa gtcaggtagt gaaacttcct tttatcaact ttatgaagac 300
acgtgggact tcctttctga atgcctacac aaactctcca atttgttgcc catcacgcgc 360
agcaatgtgg agtggcctct tcactcactt aacagaatct tggaataatt ttaagggtct 420
agatccaaat tatacaacat ggatggatgt catggagagg catggctacc gaacacaaaa 480
atttgggaaa ctggactata cttcaggaca tcactccatt agtaatcgtg tggaagcgtg 540
gacaagagat gttgctttct tactcagaca agaaggcagg cccatggtta atcttatccg 600
taacaggact aaagtcagag tgatggaaag ggattggcag aatacagaca aagcagtaaa 660
ctggttaaga aaggaagcaa ttaattacac tgaaccattt gttatttact tgggattaaa 720
tttaccacac ccttaccctt caccatcttc tggagaaaat tttggatctt caacatttca 780
cacatctctt tattggcttg aaaaagtgtc tcatgatgcc atcaaaatcc caaagtggtc 840

acctttgtca gaaatgcacc ctgtagatta ttactcttct tatacaaaaa actgcactgg 900
aagatttacw aaaaaagaaa ttaakaatat tagagcattt tattatgcta tgtgtgctga 960
gacagatgcc atgcttggtg aaattatttt ggcccttcat caattagatc ttcttcagaa 1020
aactattgtc atatactcct cagaccatgg agagctggcc atggaacatc gacagtttta 1080
taaaatgagc atgtacgagg ctagtgcaca tgttccgctt ttgatgatgg gaccaggaat 1140
taaagccggc ctacaagtat caaatgtggt ttctcttgtg gatatttacc ctaccatgct 1200
tgatattgct ggaattcctc tgcctcagaa cctgagtgga tactcttcgt tgccgttatc 1260
atcagaaaca tttaagaatg aacataaagt caaaaacctg catccaccct ggattactga 1320
gtgaattacc atggatgtaa tgtgaatgcc tccacctaca tgcttcgaac taaccacttg 1380
gaaatatata gcctattcgg atgttgcatc aatgttgcct caantctttg atctttcntc 1440
ggatccagat gaattaacaa atgttgctgt aaaatttccc agaaattact tattctttgg 1500
atcagaagct tcattccatt ataaactacc ctaaagtttc tgcttctgtc caccagtata 1560
ataaagagca gtttatcaag tggaaacaaa gtataggaca gaattattca aacgttatag 1620
caaattttag gtggcaccaa gactggcaga aggaaccaag gaagtatgaa aatgcaattg 1680
atcagtggct taaaacccat atgaatccaa gagcagtttg aacaaaaagt ttaaaaatag 1740
tgttctagag atacatataa atatattaca agatcataat tatgtatttt aaatgaaaca 1800
gttttaataa ttaccaagtt ttggccgggc acagtggctc acacctgtaa tcccaggact 1860
ttgggaggct gaggaaagca gatcacaagg tcaagagatt gagaccatcc tggccaacat 1920
ggtgaaaccc tgtctctact aaaaatacaa aaattagctg ggcgcggtgg tgcacaccta 1980
tagtctcagc tactcagagg ctgaggcagg aggatcgctt gaacccggga ggcagcagtt 2040
gcagtgagct gagattgcgc cactgtactc cagcctggca acagagtgag actgtgtcgc 2100
aaaaaaaaaa aaaaaaaaa 2119

<210> 49
<211> 1188
<212> DNA
<213> Homo sapiens

<220>
<221> misc feature
<222> (577)
<223> n equals a,t,g, or c

<220>
<221> misc feature
<222> (1022)
<223> n equals a,t,g, or c

<220>
<221> misc feature
<222> (1052)
<223> n equals a,t,g, or c

<400> 49
gaattcggca cgagtaattt tgtattttta gtagagacag ggtttctccg tgttggtcag 60
actggtctcg aactcccgac ctcaggtgat ctgcccacct cggcctccca aagtgctggg 120
attacaggcg tgagccaccg agcctagccc tgtttaggct ttttatagcc tatgttctta 180
tgagcagtaa acattatgaa tggtttagtt agacctgttg aattgaattc acttcttctg 240
cctgtggtca ggtatcaggt agcacagcca cagaagttac tgaatgtctt tgttggtgga 300
ctttaggaaa gtggtttaat ttatgtggta ttcctatctg ggaattgcaa cagtattgtt 360
agattgcatt ttgtcacagg gaggaaatta cctggtaact ccctgattag gaacaaaatg 420
aagcttcccc tttttacaaa tcctggctaa cattccattt ggatctcttc tgttgaacac 480
ctctctctct cccctccctc ctcactccat tttctcagtt attttattgt ttactattgg 540
aagtcacctc ccaactcagg atacttgtta gtccatntta ggaaaaatat caccattctt 600
tcactattat tctctgttga agttgaagaa cagaatatta ctttttttct ttccattatt 660
ggttacacca gctagttaga gacttggggt aatactgtgg gcatgggttg gatcctgata 720
tctgtgtcag ttagtgagag ttggttctat gaccctagag ctctttgtgt ccttcaaacg 780
agggtgctga aacaagacga acatagaact gtctatacca agcaaaaaac tcctgaaagc 840
acatgcccac tgcaggtgaa ttggtagcat agtgtggaga taagtgggca gtgcttggtc 900

ctgtttctgc ctcctagaga gtacctctca gcatccaggg atgctttagt aactcttagt 960
taaaacgaaa tgaactataa ttaattacct ttttttgggg ggggacacag agagtttcca 1020
cngcatttac catgcttttt tttttttttt gnaaaggaaa tatgatagga tattaagatt 1080
gacagagctg gggatgggtt ggaggctgaa ttatgatgtg tgtatttctt tatgcttgga 1140
ttatttcata attaaaaacc aaacatataa aaaaaaaaaa gaaaaaaa 1188

<210> 50
<211> 478
<212> DNA
<213> Homo sapiens

<400> 50
tttttagca tttcacgcta tttattcccc aaaaccttct gccatagaag acagccacca 60
tacagattgg aaaatgtgga cgaggagaaa aggggtgtat ggtaagcaaa ataaattgta 120
ttttccatcc ttggggagga taaaggaact ctttgcactg ctataatgaa cagcccccaa 180
atgccagtgg tttaattcag tggagttcag acctcattcc tatatcattg cagtgtggat 240
gctcctggat gaaggctctt gtaggtaact ctcctccagt cggtgattca gggacccagc 300
ctccttctgc cttgcggctt tgccttttaa aggtcctcag ggtgctctcc atgtatcttg 360
ccaatgggga acgagtgtgg aggactcaca agcgggtcyc acatcacgtc ctccggggct 420
aatacacatc ccttctcccc acactctgtt ggtcagaagt cactgcttgg cgccctgc 478

<210> 51
<211> 1333
<212> DNA
<213> Homo sapiens

<220>
<221> misc feature
<222> (485)
<223> n equals a,t,g, or c

<220>
<221> misc feature
<222> (486)
<223> n equals a,t,g, or c

<220>
<221> misc feature
<222> (493)
<223> n equals a,t,g, or c

<220>
<221> misc feature
<222> (496)
<223> n equals a,t,g, or c

<220>
<221> misc feature
<222> (587)
<223> n equals a,t,g, or c

<220>
<221> misc feature
<222> (633)
<223> n equals a,t,g, or c

<220>
<221> misc feature

<222> (1330)
<223> n equals a,t,g, or c

<400> 51
agctggtacc aaagcaagtt tttcactgag ctctcatgaa agatcctcag tctcttgtgg 60
atttagaatc ctgcagcagc ccaccatcta agagcaagar ccaaagatgt ttgtcttgct 120
ctatgttaca agttttgcca tttgtgccag tggacaaccc cggggtaatc agttgaaagg 180
agagaactac tcccccaggt atatctgcag cattcctggc ttgcctggac ctccagggcc 240
ccctggagca aatggttccc ctgggcccca tggtcgcatc ggccttccag gaagagatgg 300
tagagacggc aggaaaggag agaaaggtga aaagggaact gcaggtttga gaggtaagac 360
tggaccgcta ggtcttgccg gtgagaaagg ggaccaagga gagactggga agaaaggacc 420
cataggacca gagggagaga aaggagaagt aggtccaatt ggtcctcctg gaccaaaggg 480
agacnnatga tanctntggg acccggggct gcctggagtt tgcagatgtg gaagcatcgt 540
gctcaaatcc gccttttctg ttggcatcac aaccagctac ccagaanaaa gactacctat 600
tatatttaac aaggtcctcc ttccacgagg ganagcacta caaccctgcc acaggggaag 660
ttcatctgtg ctttcccagg ggatctatta cttttcttat gatatcacat tggctaataa 720
gcatctggca atcggactgg tacacaatgg gcaataccgg ataaagacct tcgacgccaa 780
cacaggaaac catgatgtgg cttcggggtc cacagtcatc tatctgcagc cagaagatga 840
agtctggctg gagattttct tcacagacca gaatggcctc ttctcagacc caggttgggc 900
agacagctta ttctccgggt ttctcttata cgttgacaca gattacctag attccatatc 960
agaagatgat gaattgtgat caggaccaag atccctgtgg taaacactct gattgaatct 1020
ggggttccag aaggtggaac aagcaggaat gggatccaaa gagactccca ctcagattct 1080
aaagcattta aagacaattc tagcagaatt tatcaaaaca agatgaaaca cagaaaagtt 1140
gaaaccacaa caaaatgaat tctattaaag aatagcccca gatataaatt ctcttgaaag 1200
caatgttcat aaatatttaa gcaaattaaa gacaatgtta acaaattttc tattaaatgc 1260
cctgagtgat aaaaccagtt ggcaataata ttgccttatt aaatcttcaa aaaataaaaa 1320
aaattaaaan aaa 1333

<210> 52
<211> 1255
<212> DNA
<213> Homo sapiens

<220>
<221> misc feature
<222> (541)
<223> n equals a,t,g, or c

<220>
<221> misc feature
<222> (542)
<223> n equals a,t,g, or c

<220>
<221> misc feature
<222> (1156)
<223> n equals a,t,g, or c

<220>
<221> misc feature
<222> (1162)
<223> n equals a,t,g, or c

<220>
<221> misc feature
<222> (1223)
<223> n equals a,t,g, or c

<400> 52

gaattcggca cgagcacatt taataatcta attcacacac acacacacac gtgaaatcat 60
tcttgagaat gaaatttatc atgcttttac ttcttccttc aatcttccca actactgttg 120
aaatgatctg agatttagaa tctacattat tgttactttt taacattatg tatcttctgt 180
ttcaagaagg cttttgatgt ttgagttaag tttcataagc ttttaaacaa gcatttagac 240
atttacacct gcttaactga tttcattgat cacttttatt tcatttgcac tgtatatccc 300
cattatttca actcatttca cagttgtctt tggtacttct tttagtactt ttttaaggaa 360
cagatgggtg atacagtatt atatgttctt gccttcctga agatacttgt gttcaataga 420
gcgtaacatt tttttcccac agtgactttt ccctcagaat actaaagtca cagaaagtta 480
tcacatcaac ttaatgttgc ccaagagaag tccaaactct ttgcgcttct tttgtaggtt 540
nntttgggtt atctccccac aatgatgttt atagattctt tattctttct tcttggaaca 600
aagaaatttc attgggatat gttttaaaaa atagatctct ttttattatt tttgcatggt 660
actagatgag acattttagt gcatagatgc aagtcttttt tcaactctgg gaattttact 720
tctatggaat ttttttttct ttccctaata ttttttcact ctttttctta tcctttagaa 780
atttttatgt tgatccccta gatctgctct ctgttctgac tagtttttgc tcattatatc 840
tttttatcct tttcccttag aatcagtact tcttgaaata aactgcttct atgattctga 900
ggtatagcca aattggggaa gccctcttgt gaagggtcag cagtgtttac ctggaagaag 960
aacccatttc agttgtgctt cttgctgttt ggctgcctga ttcaatcagt ggcagaaaat 1020
catattaaat atatttagag tactcccttt aaaagratta cctctctttg aaattcagta 1080
aatttacatt gagratattt gacaaatttg tatatacatt tgcaggcaat aatttttatg 1140
agctgatctg ccatgnttaa angttttcct ttgtaaacca tttggtgtgg gtatttttta 1200
aatttcctca gtatgatccc agngggcatt aactgtccaa aaaaaaaaaa aaaaa 1255

<210> 53
<211> 1140
<212> DNA
<213> Homo sapiens

<400> 53
ctaggagcct cctaatgcag tgttctgcac agtcctgggg actgactgac tgaatcacac 60
ctctggggct gggggctgct gacatgtgtg cctttccttg gctgcttctt ctcctgctgc 120
tccaggargg cagccaaagg agactctgga gatggtgtgg atccgaggaa gtggttgcgg 180
tccttcagga gtccatcagc ctccccctgg aaataccacc agatgaagag gttgagaaca 240
tcatctggtc ctctcacaaa agtcttgcca ctgtggtgcc agggaaagag ggacatccag 300
ctaccatcat ggtgaccaat ccacactacc agggccaagt gagcttcctg gaccccarct 360
attccctgca tatcagcaat ctgagctggg aggattcagg gctttaccaa gctcaagtca 420
acctgagaac atcccagatc tctaccatgc agcagtacaa tctatgtgtc taccgatggc 480
tgtcagagdc cccasatcac tgtgaacttt gagagttctg gggaaggtgc ctgcagtatg 540
tccctggtgt gctctgtgga graaggcagg catggatatg acctacagct ggctctcccg 600
gggggatagc acttatacat tccatgaagg ccctgtcctc agcacatcct ggaggccggg 660
ggacagtgcc ctctcctaca cctgcagagc caacaacccc atcagcaacg tcagttcttg 720
ccccatccct gatgggccct tctatgcaga tcctaactat gcttctgaga agccttcaac 780
agccttctgc ctcctggcca agggattgct catcttcttg ctcttggtaa ttctggccat 840
gggactctgg gtcatccgag tccagaaaag acacaaaatg ccaaggatga agaaactcat 900
gagaaacaga atgaaattga ggaaggaggc aaagcctggc tccagccctg cctgactgct 960
ccttgggaac cccagtcctg agcttggttt cttcccagca cccagagaat ccttcctcag 1020
ctctcttctt tccaggggaa ggaggtgctc aggggtgggt atccagagag ccatacttct 1080
gagggaagac tggctggcaa taaagtcaaa ttaagtgacc accaaaaaaa aaaaaaaaaa 1140

<210> 54
<211> 1220
<212> DNA
<213> Homo sapiens

<220>
<221> misc feature
<222> (1197)
<223> n equals a,t,g, or c

<220>
<221> misc feature
<222> (1208)
<223> n equals a,t,g, or c

<220>
<221> misc feature
<222> (1209)
<223> n equals a,t,g, or c

<400> 54
ccacgcgtcc gcaaataggt acctaaggca tgtgatttta tttttaaata acaaaaaata 60
acccaagttt cttgcttctc caaagtattc ttctcatagc ttataaaaga aagtccacat 120
tgaatagcat ggtctgggaa cattccttct ttattgtgtt tatttgaaca tgatatgagt 180
ttccaagatg aaatgatcaa aaaagataag taccacaaga aagttttttt gtttggttgg 240
tttttttgtt tgtttgtttt tttcttgaga ctgagtctct ccctgttgcc caagttggag 300
tgcaatcttg gctcactgca gcctccacct ccccggttcc agcgattctc ctgcctcagc 360
ctcttgaata gctgggatta caggcgcccg ccaccacacc tggctaattt ttgtgttgtt 420
agtagaggcg gggtttcatc atgttggcca ggctggtctc gaactcctga tctcatgatc 480
cgtctgcctc ggcctcccag agtgctggga ttacaggcat gagccactgc gcccggccaa 540
gaaagtatgt ttttagaggt gtgtgtaagt gcatttgtat tacctatgaa caaaattacc 600
tgactcttgt cccaggaaag ctgtttcgca ttttcgcttt ttgattggta ttatccagtt 660
ctatgtagtt catattattg ttctgtctga ctctcagaaa ttacttcttc acgccagtgt 720
cttgttgcat gactttgatg tcacctatag gaatacacct cactgcacgt aagtgggtat 780
cttactgtat aaaaggtcta catggcttta ggttttagga caaatgtgta gatttataga 840
ccatttctgt tggccaggac acagattttg agagctgtgt gtatatatat ataatcatgt 900
ttgtattttt ttcctgaaag ttatcaattg cttttgttta aaacagtttg ttttagaggt 960
ggggtgggga tgtatataac gaggaaaagt tatatgtact ttaaagtatg tcaagttctt 1020
actagtttcc tgtactgaag gttcaatttt ttttatataa gtttactttt cacctgctct 1080
attctttgtg gggaaaaaat gcatctagaa aaacatagtt taaatactgt atataagata 1140
atgaaagtta gtaatgtcca ttatttaata aagtttgtaa agtacaaggt aaaaaanaaa 1200
aaaaaaanna aaaaaaaagg 1220

<210> 55
<211> 694
<212> DNA
<213> Homo sapiens

<220>
<221> misc feature
<222> (621)
<223> n equals a,t,g, or c

<220>
<221> misc feature
<222> (651)
<223> n equals a,t,g, or c

<400> 55
ataactggag agacatcaaa ctcatgctga gaacaactag aagagttaga attgaagaaa 60
aaggatttca ttaagatatt agagagtgtt caaggcaact ggaggcagaa cgargattct 120
ggaaaggggc cacagagaag ttgtctgcat tcaaaagagc attctattaa agctacctta 180
atttggcgct tatttttctt aatcatgttt ctgacaatca tagtgtgtgg aatggttgct 240
gctttaagyg caataagagc taactgccat caagagccat cagtatgtct tcaagctgca 300
tgcccagaaa gctggattgg ttttcaaaga aagtgtttct atttttctga tgacaccaag 360
aactggacat caagtcagag gttttgtgac tcacaagatg ctgatcttgc tcaggttgaa 420
agmttccagg aactgktaag aaaatagttc tggccagaat caaagattca gccctacaag 480
gatatgtttt cctgtgaaat tatctaagag aatttcctgt tgagatataa aggcccatct 540
gatcactgga ttgggctgas caragaacaa ggccaaccat ggaaatggat aaatggtact 600

gaatggacaa gacagtaagt nctaaaaatc tggcagtaat atttgtattt naatttactt 660
tgcattaaat ctgaagtgtt ctctagttac atgc 694

<210> 56
<211> 988
<212> DNA
<213> Homo sapiens

<400> 56
cggcacgagc cagaccctat gatgtgtcca ctctggaggc tcctcatctt cctcgggttg 60
ctggccttgc ccttggcacc acacaagcag ccttggcctg gcctggccca agcccacaga 120
gacaacaaat ccaccctggc aagaattatt gctcagggcc tcataaagca caacgcagaa 180
agccgaattc agaacatcca ctttggggac agactgaatg cctcagcaca agtggcccca 240
gggctggtgg gctggctaat cagcggcagg aaacaccagc agcagcaaga gagcagcatc 300
aacatcacca acattcagct ggactgtggt gggatccaga tatcattcca taaggagtgg 360
ttctcggcaa atatctcact tgaatttgac cttgaattga gaccgtcctt cgataacaac 420
atcataaaga tgtgtgcaca tatgagcatc gttgtggagt tctggctgga gaaagacgag 480
tttggccgga gggatctggt gataggcaaa tgcgatgcag agcccagcag tgtccatgtg 540
gccatcctca ctgaggctat cccaccaaag atgaatcagt ttctctacaa cctcaaagag 600
aatctgcaaa aagttctccc acacatggta gaaagtcagc ccctggcctg atccttctct 660
ctgtgctgat ggtccaggta tgtcctctga tcggtgaaat cctcgggcag ctggatgtga 720
aactgttgaa aagcctcata gaacaggagg ctgctcatga accaacccac catgaaacca 780
gccaaccctc tgcatgccag gctggagagt cccccagctg acttctgctg atcagaagga 840
aagtccacat cttgcaacct taagtctccc ttagagtggg gcttctgcta ccctaaaaac 900
tttaccccag gctctgtgga cataccatcc tctcctacaa taaactctag ctctgaaggg 960
tgaaaaaaaa aaaaaaaaaa cggcacga 988

<210> 57
<211> 1500
<212> DNA
<213> Homo sapiens

<220>
<221> misc feature
<222> (71)
<223> n equals a,t,g, or c

<220>
<221> misc feature
<222> (73)
<223> n equals a,t,g, or c

<220>
<221> misc feature
<222> (755)
<223> n equals a,t,g, or c

<400> 57
gcctgaaggg tcgtgaggct ggggcgggac ccggcaccgc tggggcgcca ggccgtgagg 60
acgccaatgg nangcakcgt ggacgaggag gcrctcacca gctgtacctg tgggtagaca 120
acatccctct gtcccggccc aagcgaaacc tctcccggga ctttagcgat ggagtccttg 180
ttgcagaggt catcaagttt tacttcccca agatggtgga gatgcacaat tatgtcggca 240
cgagctctct ccagcagaag ctcagcaact ggggtcatct gaacaggaag gtactgaaga 300
ggctgaactt ttcagtaccg gatgacgtga tgcgcaagat cgcgcagtgc gccccaggcg 360
tggtggagct ggtgctcatc ccgctgaggc agcgcctgga ggagaggcag aggcgcagga 420
agcagggcgc cggctcctta caggagctgg ctccccagga tggcagtggc tacatggatg 480
tgggtgtatc ccagaaggcc cgaggtgaar gtgtcccgga cccccaggga gggggtcagc 540
tcagctggga ccggccgccg gcgcctcggc ctccagcgta taaccgggcg ttgcagggcg 600

accccagctt cgtcctccag atcgctgaaa aggagcagga gctgttggcc tctcaagaga 660
ccgtgcaggt cctgcagatg aaggtaaggc gcctggagca cctgctccag ctcaagaatg 720
tgcggatcga aaacctctcc cggcggctcc agcangcgga rcgtaagcag cggtgagcgg 780
cggcccgggc cgcgcgggga cgcccgggta cccgccagag ccccgacgcc gcgccggacc 840
cacccaccga tggatagacc attgggaggg cggagcccgc tgctctcacg agcctgctgg 900
ggcccgagtg ccctccttcc ttgggatggg tgagcgtgga ggagatggga caggaactct 960
aggagcgcag gcccgggact gagccgcctc ctaccactcc ggagatccgg gtcaggagaa 1020
tggaccgctt tccagagccc agaagccacg tgcagagacc tagcctgtcc cccaaagcag 1080
tgtccaacac cttgggcccg gccttgcatc tcccggcgct gggccttggg gggcggtccc 1140
ttggctctgt ccacaccccc agaatcaggt ccccgcccag ctccgaggac ggcggcgtct 1200
ccatccaggc tagttcccca tgccctcagc catgggggaa tctgtcccgg gccgctgagg 1260
ggctcccctg cccctcctgg gagcttacct gggacccacc tcggcgacgg agaccgcagc 1320
agctggagag gaaggggtga ggcgtgggat cgccaggagt agggaggaca tcgacgatgt 1380
gcccgtagca gtcgcccctc cctcctcgcg cacggggtac tgaggcggaa ggtttgaagg 1440
ttacggctca gggctgcccc attaaagtca gtgttgtgtt ctaaaaaaaa aaaaaaaaaa 1500

<210> 58
<211> 1391
<212> DNA
<213> Homo sapiens

<400> 58
   ggtaaatacc aaggtaatta aatttttaag ttctgagtat tagaggtaat ggttactgta 60
   gctcctaaaa tgcacatcac atctctggta ggtgctggaa ccctcatggt actgctgctt 120
   ttaattttgc ttttggaatg tttctttgta gctgaagctt tagtgatgag aagttagaaa 180
   tactctcatt gacctttagt gttttgtcct gttgatatat atcaagttcg cttagtttga 240
   cattgtttga acttatttcc ctaagcaaaa aacagccaga aagaagaaaa tccagaacat 300
   gtagaaattc agaagatgat ggattccctc ttcttaaaat tggatgccct ctcaaacttc 360
   cactttatcc ctaaaccgcc tgtaccagag attaaagttg tgtcaaatct gccagccata 420
   accatggagg aagtagcccc agtgagtgtt agtgatgcag ctctcctggc cccagaggag 480
   atcaaggaga aaaataaagc tggagatata aaaacagctg ctgaaaaaac agctacagac 540
   aagaaacgag agcgaaggaa aaagaaatat caaaagcgta tgaaaataaa agagaaggag 600
   aagcggagaa aactgcttga aaagagcagt gtagatcaag cagggaaata cagcaaaaca 660
   gtagcttcgg agaagttaaa acagctgacc aaaactggca aagcttcctt cataaaggta 720
   aggacaaggg aaagaaaact gctcaagggg acctttgtgg gggaagtgga tagcaagtgc 780
   tgggtgactg gaatgtctga gccagctgac agcccacctg tgggatagag atgcatgatg 840
   ctgactggct ggaatcgcaa cctttaatgt tctagaattt ttcacgtagg gtcctcacaa 900
   taacctgggt cctggcagca gcttgtcttc cactcctttc tctcttagat tataagaaca 960
   ttgtagcagt gcagaatacg tctatgctaa ctgattccag ttttctgtaa ttctagtccc 1020
   tttttcatat ttatggttgc atacattgtt gtaatggtga tgtactattt ttggcttttt 1080
   tcacttataa gtacatttta cagcataagc atgtggtgtt tttaattgca ggatgaaggt 1140
   aaagacaagg ccttaaagtc ctctcaagca ttcttttcta aattacaaga tcaagtaaaa 1200
   atgcaaatca atgatgcaaa gaaaacagaa aagaaaaaga agaaaagaca ggatatttct 1260
   gttcataaat taaagctgta atatattttg aatataatgt aaatattaat gtgtaagctt 1320
   atattgtgtc attgttctgt tttataataa aattcttgag aaccttcaaa aaaaaaaaaa 1380
   aaaaaactcg a 1391

<210> 59
<211> 1579
<212> DNA
<213> Homo sapiens

<400> 59
ggcacgaggc agcgcaggga gctgtctgca gaggccaggg tgcgcctgcc acgaatcccc 60
aggcaccggt ggccgccgcg gcccgagtag ctcggcgggt aaacatggcc gcactgacga 120
cggttgtggt agcggctgcg gccaccgcgg tagccggggc tgtggcaggg gcgggcgcgg 180
ccaccgggac cggcgtggga gcgacgccag cgcctcaaca gagtgatggc tgttttagta 240
cttcaggtgg aattcgtcct tttcatcttc agaactggaa gcagaaagtt aatcagacta 300

agaaagcaga atttgtacgc acagcagaaa aatttaaaaa tcaagtaatt aacatggaaa 360
aagataaaca cagtcatttc tacaaccaaa aaagtgactt cagatttgag catagtatgc 420
tagaagaatt ggaaaataaa ttgattcaca gcaggaaaac agaaagagca aaattccagc 480
aacaattggc caaaatacat aataatgtaa agaaacttca gcatcaatta aaagatgtga 540
agcctacacc tgattttgtt gagaagctca gagaaatgat ggaagaaatt gaaaatgcaa 600
ttaacacttt taaagaagag cagaggttga tatatgaaga gctaattaaa gaagagaaga 660
caactaataa tgagttgagt gccatatcaa gaaaaattga cacatgggct ttgggtaatt 720
cagaaacaga gaaagctttc agagcaatct caagcaaagt tcctgtagac aaagtaacac 780
caagtactct tccagaagag gtactagatt ttgaaaaatt ccttcagcaa acaggagggc 840
gacaaggtgc ctgggatgtg atcaccagaa ctttgtaaag gtgagaaaca aacataaagg 900
gaagccaaca tttatggaag aagttctaga acaccttcct ggaaaaacac aagatgaagt 960
tcaacagcat gaaaaatggt atcaaaagtt tctggctcta gaagaaagaa aaaaagagtc 1020
aattcagatt tggaaaacta aaaagcagca aaaaagggag gaaattttca agttaaagga 1080
aaaggcagac aacacacctg tgctttttca taataaacaa gaggataatc aaaagcaaaa 1140
agaggaacaa agaaagaaac agaaattggc agttgaagct tggaagaaac agaaaagtat 1200
agaaatgtca atgaaatgtg cttcccagtt aaaaaaaaaa aaaaaaaaaa aaaaaaaaaa 1260
tcagaaagaa cgccagcgcc agtttaagtt aaaattacta ctagaaagtt atacccagca 1320
gaagaaagaa caggaagaat ttttgaggct tgaaaaggag ataagggaaa aggcagaaaa 1380
ggcagaaaaa aggaaaaatg ctgctgatga aatttccaga tttcaagaaa gagatttaca 1440
taaacttgaa ctgaaaattc tagatagaca ggcaaaggaa gatgaaaagt cacaaaaaca 1500
aagaagactg gcaaaattaa aagaaaaggt tgaaaacaat gttagtagag atccctctag 1560
gctttacaaa cccaccaaa 1579

<210> 60
<211> 1241
<212> DNA
<213> Homo sapiens

<220>
<221> misc feature
<222> (8)
<223> n equals a,t,g, or c

<220>
<221> misc feature
<222> (59)
<223> n equals a,t,g, or c

<220>
<221> misc feature
<222> (78)
<223> n equals a,t,g, or c

<220>
<221> misc feature
<222> (84)
<223> n equals a,t,g, or c

<220>
<221> misc feature
<222> (86)
<223> n equals a,t,g, or c

<220>
<221> misc feature
<222> (104)
<223> n equals a,t,g, or c

<220>

<221> misc feature
<222> (128)
<223> n equals a,t,g, or c

<400> 60
ggcacganct gttatcctac ttcctgctgg ctgcctttaa acatggagct gctacacana 60
caggtcctgg ctctgcanac acananggtc ctgctggaga agangcggaa ggcttcagcc 120
tgggaacnga acctgggcta ccccctggct atgctgtgct tgctggtgct gacsggcctg 180
tytgtgctca ttgtgggcat ccacatcctg gagctgctca tcgatgaggc tgccatgccc 240
cgaggcatgc agggtacctc cttaggccag gtctccttct ccaagctggg ctcctttgcc 300
tcttcagcct ccctttctgc aagatagggg caagggcggc tagatggatg tgtctgctgg 360
gcaagtcata taacatttct gatcctcagt ttcatcctac aaaatggcgg taacaatgtc 420
tacctactcc attgtgtgga ccaaaggaga tggttaatgt gaaagccctt tgtgaacctg 480
aagtgagcaa ctgctggatg aatgtcatta cgggcacagg ctctgtgtca tctcctctcc 540
tagtgcttcc acagccagga ccagagacct ccctgatgac tggggaacct ggtgatggtg 600
gcctttctct ttatggggag cctgagtatg ctcagatcgc agctttcctt ccctagacat 660
tgtgtaattg gggtgggggg cacacttgcc ccacwkccta gctccagcct ttcctcctct 720
taggatggct caggatgagt cccccctcaa caaggcagct acccaagagt aattcccctg 780
gggactttct gtgtgaatct ccccttcccc ctcctctctt ttccctttcc tggacccagc 840
cactgatgta accaacctca cagactagtt gtttattata ttaatagttt gagcatataa 900
agaggaactt gtgatgggag agatctaggg aggagtaaag aagtatagga atgtctggcc 960
tgtattctct tcacctggga ccactgattt ttaagctgcc acattggctg gagaacaggc 1020
tatggagttc ataatgtgtg gtctcctgga gctcctgttc agctctgcct tctttgaggg 1080
ggcagggatg gggcagggag cacatygtaa tactaacggc ctcagagmtg ccccctgatg 1140
tcctcctgcc tgttaccccg tgcctctgtc tcttaacagt gggatgatga agatgccacc 1200
gtcaacaagr ktgcgctcga gctgtgcamc tttaacctgg g 1241

<210> 61
<211> 930
<212> DNA
<213> Homo sapiens

<400> 61
gaattcggca cgagctaagt cctgatatcc catgatgttt tttgttttac tttgtttttg 60
gctatttcct ttttctaaaa atagccctct ctggggaatg ctgagatctt cattctttat 120
tagtatcaat ttataattat ctacatctgt aagcagttat tcgaaagtct ccagatctta 180
ttctatcctg gcacccatgg tgactaaaaa aatcaaagac gttaaatctt tgaaagcagc 240
cttcaaacca catactccaa ccaacttacc ttatatgtcg gggagttatg gagcaaatac 300
attaattaac ttgacagaag ttgcacactt tctgtacttc tgaaccaaaa tttggatgca 360
tgtttttctt tatcatgagt cacacctgat taggatttcc ttagcttttg ttggggtcag 420
acaggattgt gaccaaaggc aagatttctc tgtcatctct tttgacagaa tttccacaat 480
catggatttt gtaatagtcc tggacattca tcagaaagta acctgtagtg gggctgccta 540
cataggattc ttcctttgaa aagccttaaa catttttcta atggttggtc tctcttaact 600
aacaataaaa aacagcaaca atgcasctgg gcacagtggc ttttgcctgt aatcccagca 660
ctttgggagg cccaggcagg tggatcaact gaggtcagga gtttaagacc agcctggcca 720
acatgtgaaa ccctgtctct acgaaaaata caaaaattag ccggatgttg tgttgcacac 780
ccgtagtccc acctactggg gaggctgagg caggagaatt gcttaaaccc aggaggcaga 840
gcttgcattg agctgaaatc gtaccacagc actccagcct gggcaacaga gtgagactcc 900
atatccaaaa aaaaaaaaaa aaaaactcga 930

<210> 62
<211> 998
<212> DNA
<213> Homo sapiens

<400> 62
ggcacgaggc ttaagtcaag ccacctgatc agtcttgtaa ccactggaga gatgagcagt 60
gtttagtcat gtccctaata ctgttattgt cagtcaccct tttacatctg tctttttctg 120

ttggcttctt tctttttagg ttgtagggga gacccattgt ctagagagaa tatacgcttt 180
gacttgatga aatcccagtt taatctagaa aggtccattt tgaggttaag aacatttcgg 240
agatgtggag gtgaagatat aaagtaggtc tcagctttgg ctggccaata tgggatccta 300
cttatctcct caggggactg gacaattcgt gtcaagactc tgtgcttcag gagcctctgc 360
ttcttcctcc ttcatggtcc aactttcctg ccccttcttc atctcattag cttaaccctc 420
agttgcctga cccaagtcaa ggtgtgtgac ctggtcctga tcaccacctc ttttgggggg 480
cttctgcaac tgtgctctgt cctggcaacc tgcttctgta atctgtttat ccccaaattt 540
gaatgagtaa taggaattgc ctaaattttg gataaattat cctacaaaat aaaagcattc 600
tcacattgcc ctctcaaatc acatgatctt tgtagaaaat ggccggtccc tatgaagcta 660
attgatcttt ggcatcaata gggaaattca gctgggcgca gtggctcaca cctgtaatcc 720
cagcactttg ggaggccgag gtgggagggt catttgaggt caagcattca agaccagcct 780
ggccaacgtg gtgaaacccc gcctctacta aaaatacaaa aaaattagct gggcgtggtg 840
gtgtgtgcct gtaatcccag ctactcagga ggctgaggca ggagaattgc ttgaaccagg 900
gagatggagc ttgcagtgag ccgggattgc gccactgcac tacagccagg atgacagagt 960
gaggctccat ctcaaaaaaa aaaaaaaaaa aaaaaaaa 998

<210> 63
<211> 1193
<212> DNA
<213> Homo sapiens

<220>
<221> misc feature
<222> (1080)
<223> n equals a,t,g, or c

<220>
<221> misc feature
<222> (1186)
<223> n equals a,t,g, or c

<400> 63
ggtcgaccca cgcgtccgca ccgaagccca gagggtctgg gggcacaaga ctgacgccag 60
ggtatgaaga gtgttatttt cattcaaagt gttattttgt ttttccttcc aatgtctgga 120
gaccaccagg gcatctctgg gctggatgag ctcccacaag cctgagggaa aggccagcac 180
tcgctagcag tggcaggcag aggcccaggc tgccgtcccc tagagtccca ggttggctct 240
gccagtgcct gtcctttacc aaagatgaat gaagcaaatg tcatgctgcc ttattcaggg 300
aaggaggagc ctgtcctgcc tgtggccatg accctgcctc tcccaggcag gggcccgcga 360
tgtggaactg ctgccactga ggggggatcc agttttgtca atgcagttgt ctctgtttta 420
caagttggag tcactcttat gctgtaccca gtttctaaac tggagactgt gtgtgccctc 480
tggctctgag tacccctgct ttgggcttgg gcctaggctg cattgaaaag agctgaaggt 540
tgtggccttt gcgctcctgg cccagccttt gttccccact ggagcagaag gggagatgga 600
cgacacggts ggggcatctg gcctggccag tgccctgatc ccagagagcc cgaggaggtg 660
tctcaggctg cctgagtcgt gacctgctag gccagagccc actccatctg gtagaaggga 720
aagcccatat gctaccacca gctgtgtcca aaaccgccag ctctgttctt cctcagccag 780
cctcgcccat ccccttgagg tctcagcccc tttcccttgt agctcctccc ctggaggggg 840
aatggcagca ggggttgggg aaacagcatc tccaagcagc ttagagttgg ccatatttac 900
ctcagcctgg gcgctggtcc tttcttccgg cccctcccct ccaaaatgtg cctattgcta 960
gagctcctcc ctctcaacac ccagtttcct tgggagttgt cattaaagga aaaaaaaaaa 1020
aaaaaaaaag ccagtgccca gggatgggca tctccaggga gctggggatt agtgccaggn 1080
agccctgcca gccatgccta catccccatg ggcacagaac aagccaaagc cttcgttgta 1140
tgttgacgat gcacttttat gaaatgtagt ttctatcgct gttttnagcc ttt 1193

<210> 64
<211> 830
<212> DNA
<213> Homo sapiens

<400> 64
ggtcgaccca cgcgtccgct gaaaggaaaa gcactgtttg gagaatgatc cacctttcaa 60
gattttactt attgttgata atgctcccac atgtcctctt ttttacgggt gatcttcatt 120
cctaatatca aagtgatatt tcttcctcca ggcaccacct ctttgatcca cacaatggat 180
caaggagtta tagcagcttt taagttctac tacctgagaa gggaggactt ttgcccagtc 240
ccatactgca gtggaggaag acactgagaa gactctgatg aaattctgaa cagcatcaag 300
aaccttgttt aggcttggat tatgtcgcta aggactgtag gaatggcacc tggaagaaga 360
cacgcaagag gtttgtcaat aayttcaaag gatttgccaa ggatgasgaa gttgcaaaaa 420
tcaagaargc tgtggttgag atggcaaaca actttaacct gggtgtggat gtggatgaca 480
ttgagtaatt cctagagggg gttcctgagg aattgactaa tgggttgctg ttggaactgg 540
aataggagtg catagctgaa gaagaggtaa agaaaaagaa agtgcaggag aagggaaaaa 600
agaactccca agaatactca cagtgatggg tttagcagaa gcttcttcag actccaacaa 660
gctccttaag aagtctgaaa acatggaccc caaaactgaa aggttttcac taatagagag 720
gaaagttcat ggtgcattat ctgcctacaa gcaaaaccag gattcaaaaa accctttgag 780
ctggagcttc aaagcacaaa aaaaaaaaaa aaaaaaaaaa aagggcggcc 830

<210> 65
<211> 867
<212> DNA
<213> Homo sapiens

<220>
<221> misc feature
<222> (457)
<223> n equals a,t,g, or c

<400> 65
ggccgccctt tttttttttt tttttttttt ttttttattg gttaaagtgc tgatgccaga 60
tgacccttga gatcccttat tagtgaaatg ttctgataat aaagaagagt ttggctcacc 120
tgctggtctc caccacacag gtttataacc aagagcccta cagctcttgt cccaccctga 180
gggcctgact gacctgtgga gggccccacc tttcgcctcc attcactcac ccctgttccc 240
aagaaccact gacttcttta catgaagcct actttgagta agtttttagg tacagatgct 300
gaattaccca agctgtatcc accctcactc caggcacccc gaggagagac tcaactgctt 360
ggcccagggt tagagaggcc cacacgggaa ggcagagtgg agcagatgtt atttaaccaa 420
aagtctgtat cctggggctc ccagctacca cagtcangaa acacattttt aaaaaatcma 480
gacccttgaa ctagcagcag tagtcaccca taccgtatac gataaataaa agtaagccaa 540
tgtttattct tctttgcata aaatcaccta taccaacact tatacattac agcatcattc 600
agttaattca agtctgaatc ccagaaactc tcctgaaatc aagccacagt tcagccctat 660
tcttcctagt ttttcctgac atacttttgc ttactctata aatccacgga tattcttctt 720
gcctactccc accaaagccc aaatacacgt gaaaaaagtt aatcatgaag tttttcttat 780
tcccttacat ttagaaaatc agcatctact ctcatagact acttgtaaga agacaaattt 840
ctgctactcc ggacgcgtgg gtcgacc 867

<210> 66
<211> 685
<212> DNA
<213> Homo sapiens

<220>
<221> misc feature
<222> (7)
<223> n equals a,t,g, or c

<400> 66
gggcccnttt gggggccccc cctttttttt tttttttttt tttttttttt tttttttttt 60
tttttttttt tttggacagg aagtagaatt tattggtgag tattaagagg ggggcagcac 120
attggaagcc ctcatgagtg cagggcccgc cacttgtcca gagggccacg actggggatg 180
tacttgaccc cacagccatc tgggatgagc cgcttttcag ccaccatgtc ttcaaattca 240

tcagcattga acttggtgaa gccccacttc tttgagatgt ggatcttctg gcggccagga 300
aacttgaact tggccctgcg cagggcctca atcacatgct ccttgttctg cagcttggtg 360
cggatggaca tgataacttg gccaatgtga accctggcca cagtgccctg ggctttccca 420
aaggcacctc gcatgcctgt ttggagcctg tcagccccag cacaggacaa catcttgttg 480
atgcggatga cgtggaaggg gtggagccgc acccggatat ggaagccatc tttgccacaa 540
ctttttacca tgtacttatt ggcacaaatt cgggcagcct ccagggcttc agaggacagc 600
tgctcatatt catctgacac catgtggcca caaagcggaa actcatccac ttttgccttt 660
ttccgcccca ggtyaaaaat gcgaa 685

<210> 67
<211> 801
<212> DNA
<213> Homo sapiens

<400> 67
gaattcggca cgagtggaga tgcactgacc ttccttgcaa gagcctttcc ctgaagttgg 60
gctcctgaga gaagttctga acatggctat ccctgccttt tcatcttgtc agcagatttc 120
ttcagcagct gctctacaaa tatgcaatgg accctttaag catttctcct ttacagtgag 180
cacaatgcta agctttgtca gcagatgcca ctggagcagc attgcagaag aaagcgagtt 240
tctcttcctg attttggtgt gctacttttc ttcttcttgc tccagctgca ttatccatca 300
gtggtactat gtataagacc atcccgctgt gccctgccct accacctgcc cagaggcaca 360
tccctcactg actatttggc ctgattctga gcctgtggcc accttctcac agccctgcaa 420
cacaggcact gtgtgctcca ggcctcacgt ccccagcagt ggcctgactg tgcacttagc 480
cacagcctca gtttgcctgt gctccaagaa attgcatcct atttgcccag cagctatgga 540
ccagctctct ggtcctggaa aacagcaggc ttctctgaca tctagtggac tgcaaacaca 600
ccttctccaa caaggcctga ccccagcctt aaggagagaa ccgtctttcc gagttgtctt 660
tccttgggta ctctccctca atcctcggat acccttgaaa gttctcttta cattgttata 720
gttattcttc tatcactgtc gaataatttt ttatattaaa cttctcttgc tttacattaa 780
aaaaaaaaaa aaaaaactcg a 801

<210> 68
<211> 908
<212> DNA
<213> Homo sapiens

<400> 68
gaattcggca cgagatttta ttaaaaataa gtgcttttct ctgcttacct tttactatga 60
tctaactatg atactttcaa tatgctgcag actctcattc ttatctttct tttgttgtta 120
ccttgttacc tagaactctt atgtttcagc ctaatttctt catctgcaaa gacctaatag 180
gaagaaattt ttactttggt ttagtgtgta taaaatctgg gaacagctaa atttcagttt 240
taatataaaa ttttgacttt tatatattac ccaatattgt taaaaggaga attctatgta 300
tacctatctc ttaaaaatat tgctctatat attacccgct taaaacaaca acagcaacaa 360
caaaaactta gaaggtaaac aaaaagtaat ctcataaaac atagaagggg aatacacctt 420
ggtttcagat atgcacagaa agtatgtaag ctgtacccca gaagcatcct tataaatttt 480
gcagtcagtt tctctgacct ttctttacac aggagggatt tgttgtayca atctttaatc 540
taagtgtgat acaccaactt cctattgaat tgccttagag cagaagaaaa ggtataaaga 600
tgatgcatct tacttagaaa tgaaaatata acaaaacaag tcatgttaaa caaggaaaga 660
tatggatctt taatcacgaa cccaaaccaa gttggtggct gaacagagaa gaactgtggg 720
agccaggcca gttggcatga cagtatgtgt tcagctggtg tggagtaagc ccctggactg 780
agggtgttca gtgtggcttc agccagggga ttcagtggtg aagaaccctc ttgctactgt 840
actctttgtc tttattacaa tactagtcaa gaaaaaattc tttctaaaaa gaaaaaaaaa 900
aaactcga 908

<210> 69
<211> 696
<212> DNA
<213> Homo sapiens

<220>
<221> misc feature
<222> (605)
<223> n equals a,t,g, or c

<220>
<221> misc feature
<222> (648)
<223> n equals a,t,g, or c

<220>
<221> misc feature
<222> (655)
<223> n equals a,t,g, or c

<400> 69
ggaaaatttt taaaaaatag attataatga tacatattgg tatcattaag acaacagatt 60
tgagcaaata caattaaggt gtcttatttt ttgcatcaag taattattgc tgtggtcttt 120
ctactccaca aaataatttt ttctttttgc agttgaaaat taactgcatt attaactaat 180
taataaaata aatcaagtgg tataagggat tagtttaccc tcaagccgat gactccatgg 240
ctactgatat tagttagttt wggattttta aaaagcatat cagaccccca gtttcaggaa 300
ttgagtataa atattgcttc ttgtcaccct gggacagtaa tgccttatag tggcactagt 360
caccttaagt agattacaca tggttgaggt gaataaagct gcatgggaat ttgctttcgt 420
gatatatttc atttgcaaac ttctacataa tcaagtttta tgtttaaaac catcggttct 480
atatatctag ctttaggaag ttgcccttac aggtgggacc ttttgtgtta atctgttttc 540
tccccagtca tcttattggc tatgttaaaa aaaaaaaaaa aaaaaagggs ggccgctcta 600
kaggntccaa gcttacgtac gcgtgcatgc gacgtcatag ctcttctnta agggncacct 660
aaattcaatt cactgggcgg ccgtttacaa cgtcgg 696

<210> 70
<211> 455
<212> DNA
<213> Homo sapiens

<220>
<221> misc feature
<222> (431)
<223> n equals a,t,g, or c

<220>
<221> misc feature
<222> (432)
<223> n equals a,t,g, or c

<400> 70
gtgctcaggg agctgaatac acggctgcgg gatgacaggg acgcctgcct gggcccacct 60
gctgctgctg ctgttgctgg gctcggcccc ccagacgcgg ctctggccac cttcccagtg 120
cccggtgacc agccccgagt gactcacgga ccatgagcta gaagctgccc ttgcaggagg 180
cttgtcatgg gtcggggrtg cccactcagg atgcaggctc tccccagggg gccccaggct 240
cgcctgactg aagacatgaa ggacctagcc taggagtggt cagggtcccg ggagtggcca 300
gggtcccgtg tgtkccctct gccagtcttc gctctgtccc cgttcaatca accccatctc 360
agttcagcag aaaacccccct cgtcaaataa aacccactga ctgcaaaaaa aaaaaaaaaa 420
aaaaaaactc nngggggggc ccggtaccca atttg 455

<210> 71
<211> 413
<212> DNA

<213> Homo sapiens

<220>
<221> misc feature
<222> (343)
<223> n equals a,t,g, or c

<220>
<221> misc feature
<222> (385)
<223> n equals a,t,g, or c

<220>
<221> misc feature
<222> (410)
<223> n equals a,t,g, or c

<400> 71
gaattcggca cgagcggctt tgggcggaac tggctttgtt gaccgggaga aacgagatgg 60
gggtgaagct ggagatattt cggatgataa tctacctcac tttccctgtg gctatgttct 120
gggtttccaa tcaggccgag tggtttgagg acgatgtcat acagcgcaag agggagctgt 180
ggccacctga gaagcttcaa gagatagagg aattcaaaga gaggttacgg aagcggcggg 240
aggagaagct ccttcgcgac gcccagcaga actcctgagg cctccaagtg ggagtcctag 300
cccctcccct gatgaaatat acatatactc agttccttgt tanaaaaaaa aaaaaaaaaa 360
aaaaaaaaaa aaaaaaaaaa aaaanaaaaa aaaaaaaaaa aaaaaaaaan aaa 413

<210> 72
<211> 849
<212> DNA
<213> Homo sapiens

<400> 72
gaattcggca cgaggtataa tgccattctc ttcctctgtg aagtgcctgt tcggggtgtt 60
gctacgtttt tgttttgttg tgttttctgt tgtagtgttt acatttttct tgtcgattcc 120
taagaggact ttagggtact gagtcaccca tggtcatgtg ttgcagagaa gtgtcacaga 180
gtgaaaactg tcttttcctt gatactacct ttagattcat atttgggaag accttcacta 240
atcatgacta cataagtatt cacttttact ttcttaaggc ctttttgttt tcattctttt 300
atagtaatgt ctaagccatc tggaattagt ttgttgatta tgcaagaaag ggatcgaagt 360
gctttttctg agtcattatc cacatgccga aacatttatt gaatagccct ttccttattg 420
atctgaaaac accttcttat aaaaccttgc attggttttt ggacttgctg tgctttcagg 480
agtcagaaga acattctttt gattatkgta gctttacatw aataatacat ttkggccggg 540
tgcggtggct cacgtatgta atcctagcat tttgggagac tgaggcaggc ggaacacctg 600
aggtcagggg ttcaagacca gactggccaa catggcaaaa ccccgtctct acaaaaaaaa 660
aaaaaaaaaa aattagctgg gcatggtggt gcctgcctga aatcccagct actttgggag 720
gctgaggcag gagaacctct tgagcctggg aggtagaggc tgcagtgagc cgagcttgca 780
ccactgcact ccaacttggg taacagagtg agactccatc tcaaaaaaaa aaaaaaaaaa 840
aaaactcga 849

<210> 73
<211> 505
<212> DNA
<213> Homo sapiens

<220>
<221> misc feature
<222> (12)
<223> n equals a,t,g, or c

<220>
<221> misc feature
<222> (501)
<223> n equals a,t,g, or c

<400> 73
gctccaccgc gntggcggcc cctctagaac tagtggatcc cccgggttgc caggaattcg 60
gcacgagttc atctattgaa gggtgtttga gtttttcac tttttggctt ttgtaagtga 120
tatagtttgg atctgggtcc ccattcaaat ctcatgtcaa gttgcagtcc ctagtgttgg 180
aggtgggcct ggtgggaggt gatgggatgg tagggttggc ttctcatgaa tggttaacac 240
catccccttt ggtactgtct ttggcatagt gagtttgttc tcctgagatc tcatttttta 300
aaagcatgtg gcacctctcc tttcactgtc tcttgctcct gctcccacta tgtgaggtga 360
ctcactcttt gtttgctttc taccataatt ggaagctttt tgaggcctct ctagaaacag 420
aagctgctat gcttcctgta cagcctgcag aaccacgagc caattaaacc tttttctaaa 480
aaaaaaaaaa aaaaactcga ngggg 505

<210> 74
<211> 719
<212> DNA
<213> Homo sapiens

<400> 74
   gaattcggca cgaggagaaa ggagggaagg cacagcgctg ggcagagatg ccagaaaacc 60
   tagttctaat cttggccttg ctgctgtcag tgtgtggcct taagcaagtc atttttctct 120
   cggcctcaat ttactctaaa atgtgtaccc tcatagctac taagaaagtt gttgcaaaaa 180
   ctagaaatga tgcttactgg tatttaatta gtctcaaaca catagtaggc ttttaacaat 240
   tagtggctgt cattttcatt attattaggc gcttcaattt ttacatgttg gcaatctcaa 300
   acataccatt ttcttttttt taaaaccctt ttttttkttt ttttttttga gacagaatct 360
   ccagcctggg agacagagca agaccgtgtc tcagaaaaaa gtggggccgg gtgcagtggc 420
   tcatgcctgt aatcccagca ctttgggagg ccagggcggg cggatcacaa gatcaggaga 480
   tcgagaccat cctggctaat gcggtgaaaa catgtctcta ctaaaaatac aaaaaattgg 540
   ctgggcttgg tggtgggcgc ctgtagtccc agctactcag gaggctgagg caggagaatg 600
   gcgtgagccc gggaggcgga gcttgcagtg agcagaaatt gcgccactgc actccagcct 660
   gggcaacaga gcgagactct gtctccaaaa aaaaaaaaaa aaaaaaaaaa aaaactcga 719

<210> 75
<211> 1274
<212> DNA
<213> Homo sapiens

<220>
<221> misc feature
<222> (1243)
<223> n equals a,t,g, or c

<220>
<221> misc feature
<222> (1270)
<223> n equals a,t,g, or c

<400> 75
cctgccgttg gcggcctgag tccgcagcgc cctgckccac ccgccccgga cgtggggccc 60
aagcccccgt gaagatggtg tcctggatga tctccagagc cgtggtgctg gtgtttggaa 120
tgctttatcc tgcatattat tcatacaaag ctgtgaaaac aaaaaacgtg aaggaatatg 180
ttcgatggat gatgtactgg attgtttttg ctctctatac tgtgattgaa acagtagccg 240
atcaaacagt tgcttggttt cccctgtact atgagctgaa gattgctttt gtcatatggc 300
tgctttctcc ctataccaaa ggagcaagtt taatatatag aaaattcctt catccacttc 360
tttcttcaaa ggaaagggag attgatgatt atattgtaca agcaaaggaa cgaggctatg 420

aaaccatggt aaactttgga cggcaaggtt taaaccttgc agckactgct gctgttactg 480
cagcagtaaa gagccaagga gcaataactg aacgtttaag aagcttcagt atgcatgatt 540
taacaactat ccaaggtgat gagcctgtgg gacaaagacc ataccaacct ctaccagaag 600
cmaaaaagaa aagtarccag cccccagtga atcagcmggt tatggaattc cactgraaga 660
cggrgatgwg raaacagatk aagaagcaga ggggccatat tcagataatg agatgttaac 720
acacaaaggg cttcgaagat cgcaaagcat gaaatctgtg aaaaccacca aaggccgcaa 780
agaggtgcgg tacgggtcac taaaatacaa agtgaagaaa cgaccacaag tgtattttta 840
gtcatctaca cgtcaaatat cccaagacag attatgctaa atacatcgac ttcatcttct 900
aacatgatat attcaggatt tacacattaa aatgattatt taaattgtgg cagtgatggg 960
gtttactttc atgaatttaa attgttttta tttcctgtaa caattgcttc caaatattga 1020
ctactaaagg cagttctgca agatgtacta aatatgtata ttagaaatta tagaaaatca 1080
tgttgtccgt tttcaaattc atcaacagcc tagagtgcct gagatataag atgaaacaca 1140
aatccacagt atacttgaaa ggagcctttt tacggttcag gataaatcag cctttgtgat 1200
gtactgtgtt tacctccttt tgtgttgtat ctggtaatta aantagggcc cagattcagc 1260
aagtgacatn acaa 1274

<210> 76
<211> 519
<212> DNA
<213> Homo sapiens

<220>
<221> misc feature
<222> (13)
<223> n equals a,t,g, or c

<220>
<221> misc feature
<222> (24)
<223> n equals a,t,g, or c

<220>
<221> misc feature
<222> (35)
<223> n equals a,t,g, or c

<220>
<221> misc feature
<222> (44)
<223> n equals a,t,g, or c

<400> 76
accaaaagct ggnagctccc accncggttg gccgnccgct ctangaacta gtggaatccc 60
cccggggctg caggaattcg gcacgaggtt ttgttttgtt tttttctaat cctgctttca 120
tactagccag tgtggggaaa aggtacaata tgtcaaagag atgagagagt gttatttctt 180
gggcaatttt ctattagtgt ttcttatttt ggccagttct tttatttatg tccttgtgac 240
ccaggtactt ggggggccag ctacccttct ggccttttag cgtctttgaa ggagaccaga 300
catgagtgaa tacctaggag agtgtcagca tgtttctgga aaattggcag agaccaagcc 360
ctgctgcaga ttcgtcaggc caggtgaaag ggccaggcag ttgcagctga tgatgtaaat 420
attttgtaca gtagataaat aaatgtttaa aaaaaaaaaa aaaaaaaaaa aaaaraaaaa 480
aaarwaaaaa aaaactcgag ggggggcccg gtacccaat 519

<210> 77
<211> 389
<212> DNA
<213> Homo sapiens

<400> 77

ggtcgaccca cgcgtccggt ttgccatata atgagcattt tgtatacata aatttatagt 60
ttaattaaat taggacattt gtaaaaaatt ggatacaatt ttattttcaa ataccttttt 120
ttagctacac tcaaacactt attgaattga aattatgcac atgtttgatt tagtgatatg 180
gtattacaaa acaccaatac cctgttaatt gtttctgcct ttcttctttc catgctgttt 240
ttcaaatttt ctattgctat atttctagtc actaatctgt cttttgaaag gtctaatctg 300
ttgttagggc catccagtga tttgttttta aattttaagt aatttatctc tataagttct 360
agatcgcgag cggccgctct agagggatc 389

<210> 78
<211> 823
<212> DNA
<213> Homo sapiens

<400> 78
cccacgcgtc cgcccacgcg tccggtaact ttatgaatat aaatttacag tttgatacag 60
gaattattag gagtaattct tttctgtttc tgtttataat gtagctacag tgttcttcat 120
tttcagaagt taacatcaag ccatcaaacc tgggtatagt gcagaaaacg tggcacacac 180
tgaccacaca ttaggctgtg tcaccattgt gtggtgtacc tgctggaaga attctagcat 240
gctacttggg gacataattt cagtgggaaa tatgccactg accgattttt tttttttcct 300
ctttgcagtg gggctaggac agttgattca acaaagtatt tttttctttt ttctcagtcc 360
taatttgaac aggtcaaaga tgtgttcagg cattccaggt aacaggtgtg tatgtaaagt 420
taaaaatagg ctttttagga actcactctt tagatattta catccagctt ctcatgttaa 480
atatttgtcc ttaaagggtt tgagatgtac atctttcatt tcgtatttct cataggctat 540
gccatgtgcg gaattcaagt taccaatgta acactggcca gcgggcccag caatctccat 600
gtgtacttat tacagtctta tttaaccagg ggtcctaacc actaacattg tgactttgct 660
ttgagacctt tcctctcctg ggtactgagg tgctatgaag ccaactgaca aagatgcatc 720
acgtgtctta ggctgatgcc actacccgat ttgtttattt gcaatttgag ccatttaaag 780
accaataaac ttcctttttt aaaaaaaaaa aaaaaaaact cga 823

<210> 79
<211> 2455
<212> DNA
<213> Homo sapiens

<220>
<221> misc feature
<222> (2277)
<223> n equals a,t,g, or c

<400> 79
ggcctcgctc ccggaagtgg agggtctaca cgaagcgccg ctgggtctgg gtgcccggag 60
gcagcagcgt tcgcggagtt cgcccgctgg cccccgatca ccatgtcggc tttcgacacc 120
aaccccttcg cggacccagt ggatgtaaac cccttccagg atccctctgt gacccagctg 180
accaacgccc cgcagsggcc ctggcggaat tcaacccctt ctcagagaca aatgcagcga 240
caacagttcc tgtcacccaa ctccctgggt cctcacagcc agcggttctc cagccatcag 300
tggaaccaac ccagccgacc ccccaggccg tggtgtctgc agcccaggca ggcctgctcc 360
ggcagcagga agaactggac aggaaagctg ccgagctgga acgcaaggag cgggagctgc 420
agaacactgt agccaacttg catgtgagac agaacaactg gccccctctg ccctcgtggt 480
gccctgtgaa gccctgcttc tatcaggatt tctccacaga gatccctgcc gactaccagc 540
ggatatgcaa gatgctctac tatctgtgga tgttgcattc agtgactctg tttctgaacc 600
tgcttgcctg cctggcctgg ttctcgggca acagctccaa gggagtggac tttggcctct 660
ccatcctgtg gtttctgatc ttcactccct gtgccttcct ttgttggtac cgacccatct 720
ataaggcctt taggtccgac aactctttca gcttctttgt gttcttcttt gtattttttt 780
gtcaaatagg gatctacatc atccagttgg ttggcatccc tggcctgggg gacagcggtt 840
ggattgcagc cctgtctaca ctggataatc attccctggc catatcagtc atcatgatgg 900
tggtggctgg cttcttcacc ctctgtgccg tgctctcagt cttcctcctg cagcgggtgc 960
actccctcta ccgacggaca ggggccagct tccagcaggc ccaggaggag ttttcccagg 1020
gcatcttcag cagcagaacc ttccacagag ctgcttcatc tgctgcccaa ggagccttcc 1080

aggggaatta gtcctcctct cttctctccc cctcagcctt tctctcgcct gccttctgag 1140
ctgcactttc cgtgggtgcc ttatgtggtg gtggttgtgc ccagcacaga cctggcaggg 1200
ttcttgccgt ggctcttcct cctccctcag cgaccagctc tccctggaac gggagggaca 1260
gggaattttt tccccctcta tgtacaaaaa aaaacaaagc tctctttcct tctctggtga 1320
tggtttggta ggattctttt gtctctggaa gcagtgggac tgaagttctc ttcgtcctgt 1380
gcacacacag acacccccac acagttggga tcacaggctg acctgggccc atcccagctg 1440
gagctttctg ccagggtcct gggccttgac tcccccaccc tgcaggcctg gcctgaatct 1500
ggcttcttag acacagccca gtccttcctg cctgggctgg gaataagcct ctcacaggtt 1560
ctggtggaca gatctgttcc ccaggtcact ccagtggtct ccaggcttcc agagaaggct 1620
ggttgcctca agctcttctc tgcctcataa acggatccag agaaggctgg ttgccttaag 1680
ctcttccctg cctcgtgttc ctgagaaacg gattaatagc cctttatccc cctgcaccct 1740
cctgcagggg atggcacttt gagccctctg gagccctccc cttgctgagc cttactctct 1800
tcagactttc tgaatgtaca gtgccgttgg ttgggatttg gggactggaa gggaccaagg 1860
acactgaccc caagctgtcc tgcctagcgt ccagcgtctt ctaggagggt ggggtctgcc 1920
tgtcctggtg tggttggttt ggccctgttt gctgtgacta cccccccccc tccccgaacc 1980
gagggacggc tgcctttgtc tctgcctcag atgccacctg ccccgcccat gctccccatc 2040
agcagcatcc agactttcag gaagggcagg gccagccagt ccagaaccgc atccctcagc 2100
agggactgat aagccatctc tcggagggcc ccctaatacc cagtggagtc tggtttcama 2160
ccctgggggg tgtgtcactg tgatgggaca cgtaggagtc cacccttaaa accagcaccc 2220
tgtccctcga ggctgccgag tgggtgtgtg gactcggggg ccttcccaca aaaactnstc 2280
cggctctggg cccgagacag ccgcaggccc cagccactga atgatactgg cagcggctgg 2340
ggttttatga actcctttct ggtatttttt cccctctatg tacaaatgta tatgttacgt 2400
ctcaattttt gtgcttaagt aaaaataaaa acattttcag acaaaaaaaa aaaaa 2455

<210> 80
<211> 921
<212> DNA
<213> Homo sapiens

<220>
<221> misc feature
<222> (111)
<223> n equals a,t,g, or c

<400> 80
ttttcttaag ggaaaaatca cgctgtgttc ttttaaaatc cctcaggttt tatgttttat 60
tgctaccaga gtctgcctcc ctgaggttct tgtatagact agttatttcc ntctgtaaag 120
aagctgttct attcgttctc gcctggtttg gaacaaactg aacacttcca aaggaggcag 180
tccttgcagc cttgtctcct tccactcccc tcctccccac agtcctgggc tggagcagcg 240
agtctgtcga tcccagggcc agagacaagg cagacaaagg ttcatttgta aagaagctcc 300
ttccagcacc tcctctcttc tccttttgcc caaactcacc cagtgagtgt gagcatttaa 360
gaagcatcct ctgccaagac caaaaggaaa gaagaaaaag ggccaaaagc caaaatgaaa 420
ctgatggtac ttgttttcac cattgggcta actttgctgc taggagttca agccatgcct 480
gcaaatcgcc tctcttgcta cagaaagata ctaaaagatc acaactgtca caaccttccg 540
gaaggagtag ctgacctgac acagattgat gtcaatgtcc aggatcattt ctgggatggg 600
aagggatgtg agatgatctg ttactgcaac ttcagcgaat tgctctgctg cccaaaagac 660
gttttctttg gaccaaagat ctctttcgtg attccttgca acaatcaatg agaatcttca 720
tgtattctgg agaacaccat tcctgatttc ccacaaactg cactacatca gtataactgc 780
atttctagtt tctatatagt gcaatagagc atagattcta taaattctta cttgtctaag 840
acaagtaaat ctgtgttaaa caagtagtaa taaaagttaa ttcaatctaa tttttctctg 900
tggaaaaaaa aaaaaaaaaa t 921

<210> 81
<211> 678
<212> DNA
<213> Homo sapiens

<400> 81

gaattcggca cgctcttggg ggtagtggat gcgggttgag gggtttcagg tgccctgggc 60
tgtcactttg taaaggcttg ccaacctaga ttgagatggg tggtaaagga atcaattaca 120
caatgccaca catttgcttg cttctgctga atgccttagt agtttcatgt ttattgctgg 180
aagccattct cttacagcat ctagtgctgt gtaacgagct accttaaaat gtaaaggctt 240
aaaacagcca tctttgatgt ctttgcaggt ctagaagtca ggaagggtaa ttattcagct 300
ccaagtggca ttggctctag ttactacctg atattccagg gtggtagctg gagtggtctc 360
aagggtccaa gctgacctca cttacaagct gggtgccttg gcagggacag ttaggaggct 420
gtgtgtagca gagcctcact cggtctttgt attctccagg cctcttcagt ggtttctttg 480
gcacttctta aatgatgtca gggttccagg agttaatgtt ccaagagaca ggaagtggat 540
gctgcccatc tctttttttt tgtttgtttg tttgtttgtt tttttgagat ggagtcttac 600
tctgtcacca ctgcactcca gcctgggcaa cagagcgaga ctctgtctca aaaaaaaaaa 660
aaaaaaaaaa aaactcga 678

<210> 82
<211> 857
<212> DNA
<213> Homo sapiens

<220>
<221> misc feature
<222> (493)
<223> n equals a,t,g, or c

<220>
<221> misc feature
<222> (562)
<223> n equals a,t,g, or c

<400> 82
gaattcggca cgaggggaaa taatgtttgt ggaaaattgc ttagaggaaa tggagtatat 60
tactggtata ggtactctaa aatgtctttt gaattaagtc agagttagag ggttgtgtct 120
ctaaaccgca tcttactggt attatgctat cagcctgtat tgagagactt tataggtaaa 180
gtccaattta ggctgtttgg tattatctat taaaattaga atgttcatgc tctgtaacct 240
gctacttcca cttctagaat ttatctttgg aagcacatat ctgtccacag acctatattt 300
acacacatgt atgaagaatg tkttccttca cattcattca ttttaacaaa tgttttgatg 360
tgtagggcct aagctgattt gaatgcagct gaaatgcaca tatctggttg agtcmtggga 420
actgatttgc atgtgtcttt ctcttttatg gcttgaagag gagagaaatt tgtgcttagc 480
acattgaagg gcntacgaga tacaaggagt ctgtccttag ctctgccctt tggactgttg 540
tctgaaggct aaagaagaga gnacaaagaa agcttgcatt gggaggctga ggtgggagga 600
tcacttgagc ttaggagttt gagaccagcc tgggcaacat agggagactg cacctctata 660
agaaatttta aaaattagcc gggttggcag cgtgctcttg tggtcccagc cgcttgaaaa 720
gctgaggtgg gagaatcgcg tgagcctggg aggtcgaggc tgcagtgcac cgtgattatg 780
ccactgcact ccagcttggc aacattgact gtctcaaaaa gattatatat ctctaaaaaa 840
aaaaaaaaaa aactcga 857

<210> 83
<211> 1977
<212> DNA
<213> Homo sapiens

<220>
<221> misc feature
<222> (664)
<223> n equals a,t,g, or c

<220>
<221> misc feature
<222> (716)

<223> n equals a,t,g, or c

<220>
<221> misc feature
<222> (1319)
<223> n equals a,t,g, or c

<400> 83
   gcaaaaaccc aaaaggggac agcagtagtg ggagaggcca gcatctgtac accccatcag 60
   ggtccccgct gtgtgtgccc ctcaggcggc caccagccct accaggtcct cccctcccgg 120
   caggtcttcg ccttgatcgt gttctcctgc atctatggtg agggctacag caatgcccac 180
   gagtctaagc agatgtactg cgtgttcaac cgcaacgagg atgcctgccg ctatggcagt 240
   gccatcgggg tgctggcctt cctggcctcg gccttcttct tggtggtcga cgcgtatttc 300
   ccccagatca gcaacgccac tgaccgcaag tacctggtca ttggtgacct gctcttctca 360
   ggtatctgcc tgtggcacct ccatttgatc ttgggggagg cattaactct agggttccgc 420
   agctgggagg gtctcggcct ctctgggagg ggcagggagc agctcactcc tccagggcat 480
   ttttaggaaa gggttttcag ctagtgtttt tccgtgcttg aatggcacca gccctgcctg 540
   gggtagctag aagctgagtg gacctgcagc acacccgagc agatgggctt tgcctctgcc 600
   ccttttgtcc cctaggctgt ctgctgtggc ccaccctgcc aaggcccgag tgtgggggac 660
   tttngaggtg gctcccggcc cggcttccaa gtcctcccct ccatagtgtg gaagcntccc 720
   ccgggaggtc cctgccctac ctgcccgcgt cccctcccag agtcctggaa agcccctccc 780
   tttccatgga actgacgctt cacccgtcct cttctcagct ctctggacct tcctgtggtt 840
   tgttggtttc tgcttcctca ccaaccagtg ggcagtcacc aacccgaaga cgtgctggtg 900
   ggggccgact ctgtgagggc agccatcacc ttcagcttct tttccatctt ctcctggcgc 960
   tacaaggctg gcgtggacga cttcatccag aattacgttg accccactcc ggaccccaac 1020
   actgcctacg cctcctaccc aggtgcatct gtggacaact accaacagcc acccttcacc 1080
   cagaacgcgg agaccaccga gggctaccag ccgccccctg tgtactgagc ggcggttagc 1140
   gtgggaaggg ggacagagag ggccctcccc tctgccctgg actttcccat gagcctcctg 1200
   gaactgccag cccctctctt tcacctgttc catcctgtgc agctgacaca cagctaagga 1260
   gcctcatagc ctggcggggg ctggcagagc cacaccccaa gtgcctgtgc ccagagggnt 1320
   tcagtcagcy gctcactcct ccagggcact tttaggaaag ggtttttagc tagtgttttt 1380
   cctcgctttt aatgacctca gccccgcctg cagtggctag aagccagcag gtgcccatgt 1440
   gctactgaca agtgcctcag cttccccccg gcccgggtca ggccgtggga gccgctatta 1500
   tctgcgttct ctgccaaaga ctcgtggggg ccatcacacc tgccctgtgc agcggagccg 1560
   gaccaggctc ttgtgtcctc actcaggttt gcttcccctg tgcccactgc tgtatgatct 1620
   gggggccacc accctgtgcc ggtggcctct gggctgcctc ccgtggtgtg agggcggggc 1680
   tggtgctcat ggcacttcct ccttgctccc acccctggca gcagggaagg ctttgcctga 1740
   caacacccag ctttatgtaa atattctgca gttgttactt aggaagcctg gggagggcag 1800
   gggtgcccca tggctcccag actctgtctg tgccgagtgt attataaaat cgtgggggag 1860
   atgcccggcc tgggatgctg tttggagacg gaataaatgt tttctcattc aaaaaaaaaa 1920
   aaaaaaaaaa aaaaaaaaaa aaaaaaaaaa aaagggcggc cgctcgcgat ctagaac 1977

<210> 84
<211> 1149
<212> DNA
<213> Homo sapiens

<220>
<221> misc feature
<222> (837)
<223> n equals a,t,g, or c

<400> 84
acccactgac aggcattatg acctaacagg aggttggtag cagtagatcc aagcatgcat 60
gttgcctggc ctgtagattg gccttatcag gtttctgggt gcctctgcct taagatcctg 120
aaggmaaatt ttgtttcaac agtttggaag tcatctgtgg gtccagcttg actttggagg 180
aataagaaga tacttctaga gtatgggaat gattccagat aatttctggg atttgaatct 240
acttgagttt aagggcctgg gacctaattt ggtttagtat agaatttgaa gaattaattt 300
ataggcagct gaatacccaa aacttgggtg gtggtcctgt ggtttggctg agctgtccgg 360

gcataacctg gttctctgtt atgttaaggc tttctgggaa gccagccact ctgcgcagga 420
gtgaaacatg aagttgtttt ctgaggacct gttttggtgg gattgtttgg gcagaggact 480
gtgtttatgc agggcaaatc ccagaaagat aagaggaagc tagagaaact taatgtacct 540
gaattcttca tggtgtattt gcaaactaac ttaacataga ttcttttgac tatggtaagt 600
ttgaatctct ccttgccaaa caacattata agtttagttt tcttcttcct cttgcagccg 660
gtacagaaag gtgtaagtgg tggctgaaaa ttgaggaagc ttcatctgac caatgtgggt 720
gctggtttct tgtgaaatgt gtccctaagc ctccttctcc ttgcaggcag ccacccaccc 780
aggtgtctaa gataggacat gctcctttct ttctctaatc csatcctgag gttgccngca 840
aagccaatat gaccactact gagaaatagt aatgacttct acaaatgcaa gggtcttacc 900
ctcctctttc ccttaaamac cctccctttt ccttagaccc cgtttttgcc atcccccaaa 960
tgtgtggtat ggtgaaacta atcccctgaa tgtgaattgc tatccttatt gccctattaa 1020
agaagagcca gctggtatat tgtcaggaag cactatttaa aatgtgaact gttatagagt 1080
aaataaataa atactctaca ggaaaaaaaa aaaaaaaaaa aaaaaaaaaa aaaaaaaaaa 1140
agggcggcc 1149

<210> 85
<211> 767
<212> DNA
<213> Homo sapiens

<400> 85
catgaaaaca cattctctta tagtttttaa attcatcatc caagagttcc tgctctttga 60
tgatgagaca tacctggtag actccaaaac agagagcaga cgcctagtat ctttgttctg 120
gggtgtgcat taagagtaca ttgacctgtc tgtctccagt cttgactctt ttggaagaga 180
gatgctagta ctgatgacaa cctgcattct ggctgcggtg tgygtccaca ctgcacagtg 240
tgcaccagac tctcgtatgg acaatgactg tccctcacat caggcgcaga tccattttag 300
agcctcagaa gtcaggagag ggtggacttt caaccacgac tgaaaacact gtctttctta 360
ggacatgctg tgtgtatgac acacttacag atgtctgtgc tcactgatgc ttgttgatgt 420
gtcatcgcac atcagtgaca aacatttgtc atgtttttgc ctttggtgga acttctttat 480
tatactcact ttcctcccaa accatttttc tcaacttcat catgaagcaa atgtcatgtg 540
gtcattctgt gatggggctc agggctaggt taggtgatga tttctgaaag ctcagagacg 600
tgaaggaaaa aggacatcag tgcttggatc ttagctctta taagcctcac gtgcaacaat 660
aaacccgagt tcaagaatca gattcttaga tagattggtt tggtagcaaa tgacaaaaaa 720
ccaacgtaaa tatgcttcgg caaaaaaaaa aaaaaaaaag ggcggcc 767

<210> 86
<211> 728
<212> DNA
<213> Homo sapiens

<400> 86
   aaatgattta gtgacctata caagtagcct gcagtaccgg atccgaattc ccggtcgacc 60
   cacgcgtccg gtgaaaacag cagagtgcta ctccatacca ctgggatctt gtccagtaaa 120
   catccagaga gtgaggttag gaaataaaaa gtatataaat attagatgcc tagaaatgca 180
   agtcacttta aagattttat gtgaaataga aaaaaaagag aggagaggga ctcattgtct 240
   tgtaatgggt ccttcccaga gagaggtgac tgtccagtgg caccgggccc ttttcctcct 300
   tcccctttta ctcttatcaa ctaggacaga aactaagaat tttggcttca agtggctaaa 360
   agactgatgg gggaaaaaag aaaatagaaa aaaataacag agagactgac gctctaggca 420
   gttacaagtc caagaaaaaa gacagaaact tttaagtatt gagccaaaac caggtctagc 480
   aamcataatg ctggccctag attatttatt aatttatgaa gaaacttcta gatatggggg 540
   tgacaaaagg aaattaaatc cattatatat gcatatattt taatgtaaat atataataga 600
   taaattatgt atacataata tataaccaaa ttgaaacagt tttacaattt ggtttgactg 660
   gaaattcaaa atccatatat taatttttgt agtaaaagtt tatgtaaaaa aaaaaaaaaa 720
   gggcggcc 728

<210> 87
<211> 735

<212> DNA
<213> Homo sapiens

<220>
<221> misc feature
<222> (376)
<223> n equals a,t,g, or c

<400> 87
   gaattcggca cgagtagcag cttgattttc tgttagccta tgaaatgtta ttgtcctata 60
   aaaataactt taaactgatt taatatttca tatttacatt atatgaaaat caattacatt 120
   ataaaaggaa tccctaatgc agaaacaaag atgcaacttt caaaattctt attattccta 180
   tttgtatata cacgagagaa cccaaccagt gcctgtgttt ggggggaaaa gtcaacagtg 240
   tagttctaaa ccttatccca aacagaaaat gtggktaatg atgtcacttt ccttgctggk 300
   catcattagg cttaaattaa atgctgaagc tgtcatcaaa gagtttacac taaaatcttc 360
   agggctttaa ataaanggtt aagtccagct tccaaacaca attttccaca ttagcagctc 420
   caatcttctt aaataaagct ctgttttcct atatttttat gactgctgag accccacagg 480
   gaccaatatt tgtattcaaa ttacatttca tggtttccca ttgtttcaca atgagttcta 540
   ataaatggga tttactataa taatccaagt atgacatagc cggtatgctt tcatgaatgt 600
   ttttatgtag attttcctcc catgaacatg agtaaataaa tctgtttcct gaatggattg 660
   tggttgcatt taaagctctg taataattct aataaattta ctctatagaa aaaaaaaaaa 720
   aaaaaaaaaa ctcga 735

<210> 88
<211> 889
<212> DNA
<213> Homo sapiens

<220>
<221> misc feature
<222> (117)
<223> n equals a,t,g, or c

<220>
<221> misc feature
<222> (292)
<223> n equals a,t,g, or c

<220>
<221> misc feature
<222> (341)
<223> n equals a,t,g, or c

<400> 88
   tgttaggtta attattgctt cacatgtggt cacggtttga aaacttattt tggggggagt 60
   ataaagtaga atacagagat tccttgctca tagctcctac tgctatcggg gaacaancct 120
   tgagggtgag aacgtggatt gattcttgat tgatagtggg gattccatta tctgtatttg 180
   gcagttatgg cctgctgcgg tgtatagaag cttctttcca ttcattttcc cgaattttca 240
   tactgctcaa ggaacagttg ggggggaatg ggcagaaggt tgggcacttg angtatttga 300
   gctatcggta ataactgact ttttagggcg cacagatttg nagtagagcc atggtagtag 360
   ttagtaccaa tgggtttttg ctgcttctac tctttcttaa cagaaaaagt ggattgtgtt 420
   catataggaa agcagttcac agactgtctt cctgcccctc ccgccaccaa gctggaccta 480
   gaatcaagtg tgactttaaa tggggaaagc tgtgttacag ttgtgcttaa gccactgctg 540
   tggcttaacc tcacctatgc ataagaattt gctcgtggct ggccgggcgc ggtggctcga 600
   gcctgtaatc ccagcacttt gggaggctga ggcgggcgga tcacgaggtc aggagattgg 660
   gaccatcgtg gctaacacgg tgaagccccg tctctactaa aaatacaaaa aaaattagcc 720
   gggcgtggtg gcgggcgccg ctagtcccac tactgagtcc caggctgaag caggagaatg 780
   gtgtgaaccc aggaggcgga sttgcarcga gccgagatcc tgtcactgca ctccagcctg 840
   ggcgaagcga gactctgtct caaaaaaaaa aaaaaaaaaa aaaactcga 889

<210> 89
<211> 569
<212> DNA
<213> Homo sapiens

<220>
<221> misc feature
<222> (1)
<223> n equals a,t,g, or c

<400> 89
ntaaggtgtt gattctggat cacgggatac cattcctgtc macaccccga ccaggggcta 60
gaaaatttgt ttgagatttt tatatcatct tgtcaaattg cttcagttgt aaatgtgaaa 120
aatgggctgg ggaaaggagg tggtgtccct aattgtttta cttgttaact tgttcttgtg 180
cccctgggca cttggccttt gtctgctctc agtgtcttcc ctttgacatg ggaaaggagt 240
tgtggccaaa atccccatct tcttgcacct caacgtctgt ggctcagggc tggggtggca 300
gagggaggcc ttcaccttat atctgtgttg ttatccaggg ctccagactt cctcctctgc 360
ctgccccact gcaccctctc cccttatct atctccttct cggctcccca gcccagtctt 420
ggcttcttgt cccctcctgg ggtcatccct ccactctgac tctgactatg gcagcagaac 480
accaggcctg gcccagtgga tttcatggtg atcattaaaa aagaaaaatc gcaaccaaaa 540
aaaaaaaaaa aaaaaaaaaa aaaactcga 569

<210> 90
<211> 334
<212> DNA
<213> Homo sapiens

<220>
<221> misc feature
<222> (321)
<223> n equals a,t,g, or c

<400> 90
agaaaatgaa caaactagtg agaaacattg taaacatata gtgtagatga taactctgaa 60
cttaagtaca agataatgat gaatattctg ctgcttaagt atatcttaga aatattaatt 120
cttagtgaaa atcttaacct attcaacatc acttatggta agtataactt attttcctta 180
tacaggtatt aaatatataa tttatatgcc agtcacattt cctcacacta aataaggcag 240
cagacacata tatttaatat catgggtatg cattttaggt tctaaaacct aaggtatgtg 300
gatttcttaa agccatatct naaatatttt cacc 334

<210> 91
<211> 795
<212> DNA
<213> Homo sapiens

<220>
<221> misc feature
<222> (3)
<223> n equals a,t,g, or c

<400> 91
cgnaccattt tttttttttt gaatatcatc agcttacttg actggcaagg gcagaagctg 60
gggttggcct gaactctgcc aaacaaatat caaagtgtat ttaatagtta aatttgtgcc 120
ctttcccttc ttgctgcacc catgttgtca cttaaccccc aggagttatt tattatcttt 180
ttgttaaagt caggctcatt tggggtaatg tgatgactgt ttaggtttac atgaccctcc 240
tctcctttcc ctacccccaa atatgtatat atacatatat aaaatatgta tatattttac 300

ctatataaaa tatatatata tacacatata tgtatctata ttcctttgtt tctttgcctg 360
cttatactgg ccataaaaga gggagctgcc ttcaatgtat aaagtataag aagagtgcca 420
gggaatgcca taatggaggc ttttggatct gaatttggac catttcacta aagagaacat 480
gagtttgctc agccctttcc tcacaagagg gagggccccg gttccccaga cttctccacg 540
cgctggctcc ataaaggcca gctttggccr ggctgccaca ggggcctgag gagctcactc 600
tgggcctacc tggtttcagt tagagggtcc tcctgttatt tttccattta aaaagtatgt 660
cctcagaaaa ctgtactgga aggatgggtg gcaggaactt gtatagttca gcttccaaca 720
ctttggaaca gattaaaaag ggaatctttt aaataaaaac gtataaaaat aaaaaaaaaa 780
aaaaaaaggg cggcc 795

<210> 92
<211> 577
<212> DNA
<213> Homo sapiens

<400> 92
tagtggatcc cccgggctgc aggaattcgg cacgaggctg cacgaggttg ttgagaggat 60
caagtaagat aatgaatgaa agtgtctatg acgacagtac tagttcttac acaccatccc 120
tccacatttt gggatgtctg ttgctgctct tccttggggt ggaaagagca ctggagccct 180
tctctggtct ttgtgcttct ttacatgatg tgagacctat agtaaacccc ttaacctcct 240
tcagcctcat ttattagaga gagagagaaa aaaaaaggtg attttaaaaa aatctgtttt 300
cggccaggtg cagtggctca tgcctgtaat cccagcactt tgggaggccg aggcaggtgg 360
atcacctgag gtcaggagtt cgagaccagt ctggctaaca tggtgaaacc ctgtcactac 420
taaaaatacm aaaaaatcag ctactcggga ggctgaggca ggagaatcct atgaaaacgg 480
gaggcagagg ttgcagtgag ccgagatcgt gccattgcac tctagcctgg gcaatgagca 540
aaactttgtc tcaaaaaaaa aaaaaaaaaa actcgta 577

<210> 93
<211> 968
<212> DNA
<213> Homo sapiens

<220>
<221> misc feature
<222> (904)
<223> n equals a,t,g, or c

<220>
<221> misc feature
<222> (907)
<223> n equals a,t,g, or c

<400> 93
gaattcggca cgagcttact ttcactcacc gcctgtcctt cctgacacct caccatgtgt 60
acgggaaaat gtgcccgctg tgtggggctc tccctcatta ccctctgcct cgtctgcatt 120
gtggccaacg ccctcctgct ggtacctaat ggggagacct cctggaccaa caccaaccat 180
ctcagcttgc aagtctggct catgggcggc ttcattggcg gggcctaat ggtactgtgt 240
ccagggattg cagccgttcg ggcagggggc aagggctgct gtggtgctgg gtgctgtgga 300
aaccgctgca ggatgctgcg ctcggtcttc tcctcggcgt tcggggtgct tggtgccatc 360
tactgcctct cggtgtctgg agctgggctc cgaaatggac ccagatgctt aatgaacggc 420
gagtggggct accacttcga agacaccgcg ggagcttact tgctcaaccg cactctatgg 480
gatcggtgcg aggcgccccc tcgcgtggtc ccctggaatg tgacgctctt ctcgctgctg 540
gtggccgcct cctgcctgga gatagtactg tgtgggatcc agctggtgaa cgcgaccatt 600
ggtgtcttct gcggcgattg caggaaaaaa caggacacac ctcactgagg ctccactgac 660
cgccgggtta cacctgctcc ttcctggacg ctcactccct tgctcgctag aataaactgc 720
tttgcgctct caaaaaaaaa aaaaaaaaac tcgagggggg gcccggtacc caattcgccc 780
tatagtgagt cgtattacaa ttcactggcc gtcgttttac aacgtcgtga ctgggaaaac 840
cctggcgtta cccaacttaa tcgccttgca gcacatcccc ctttcgccag ctggcgtaat 900

aacnaanaag cccgcaccga tcgcccttcc caacagttgc gcagcctgaa tggcgaatgg 960
caaattgt 968

<210> 94
<211> 553
<212> DNA
<213> Homo sapiens

<400> 94
gaattcggca cgagtcccta aacagttaaa atgtcacagc tgtttcttat aatgcttaca 60
ttcatatttc taaataacat gtttataatg catctaactt ccttccatgg aaaaagagta 120
tttggctttt taaaccaatc gagtcacatg catgctttcc cccttccacg ttggactaca 180
tcaatattta gtgttagtat ttttataaat agataaatat tgttcgcaaa ttttatttgc 240
tgtctattgc tgtgtaacaa attcctccaa aattattggc tttaaacaac atttattatc 300
ccatagtttc tatgagttga gaatctaagc aggcttagct gggtccacta gctcggggtc 360
tctcacaagg ccacagatca aggtgttggt cagtggtttg tgcccttagt cccagctact 420
tgggaggctg aggcaggagg atcacttgaa cccagtagtt caaggctgca gtgagcwakg 480
gttacaccac tgcactccar cctgggtgac agagcaagat gccatctctt aaaaaaaaaa 540
aaaaaaaact cga 553

<210> 95
<211> 968
<212> DNA
<213> Homo sapiens

<400> 95
ggcacagcaa ccgtcactgc ctatcagaat cagcagatta ctcgcctgaa gatagatagg 60
aatccatttg ctaaaggctt ccgagactcc gggcgcaaca gaatgggttt ggaagccttg 120
gtggaatcat atgcattctg gcgaccatca ctacggactc tgacctttga agatatccct 180
ggaattccca agcaaggcaa tgcaagttcc tccaccttgc tccaagtact gggaatggcg 240
ttcctgccac tcaccctcac cttttgtctg gctcctcttg ctcctctcct gccttccatc 300
tggggcccaa caccagccag ctgtgtagtc tggcccctgc tgactattct gcctgtgccc 360
gctcaggcct caccctcaac cgatacagca catctttggc agagacctac aacaggctca 420
ccaaccaggc tggtgagacc tttgccccgc ccaggactcc ctcctatgtg ggcgtgagca 480
gcagcacctc cgtgaacatg tccatgggtg gcactgatgg ggacaccttc agctgcccam 540
agaccagctt atccatgcag atttcgggaa tgtcccccca gctccagtat atcatgccat 600
caccctccag caatgccttc gccactaacc agacccatca gggttcctat aatactttta 660
gattacacag cccctgtgca ctatatggat ataacttctc cacatcyccc aaactggctg 720
ccagtcctga gaaaattgtt tcttcccaag gaagtttctt ggggtcctca ccgagtggga 780
ccatgacgga tcggcagatg ttgccccctg tggaaggagt gcacctgctt agcatggggg 840
tcagcagagt ttctttgact ctaggaccct aggaagctta actctgtcat catctcaagt 900
atctgcacat atggtctgat gaagccttta aagttaaatg aacatttggg atctgtctaa 960
acatattt 968

<210> 96
<211> 697
<212> DNA
<213> Homo sapiens

<220>
<221> misc feature
<222> (19)
<223> n equals a,t,g, or c

<220>
<221> misc feature
<222> (50)

<223> n equals a,t,g, or c

<220>
<221> misc feature
<222> (57)
<223> n equals a,t,g, or c

<220>
<221> misc feature
<222> (662)
<223> n equals a,t,g, or c

<220>
<221> misc feature
<222> (680)
<223> n equals a,t,g, or c

<220>
<221> misc feature
<222> (690)
<223> n equals a,t,g, or c

<400> 96
aagaaaatta ccctcactna aaaaaaacaa aaactaaaag ctcgcacgcn tgcaggnacg 60
acactagtgg atccaaagaa ttcggcacga ggccacatcc caccggccct tacactgtgg 120
tgtccagcag catccggctt catgggggga cttgaaccct gcagcaggct cctgctcctg 180
cctctcctgc tggctgtagg tctccgtcct gtccaggccc aggcccagag cgattgcagt 240
tgctctacgg tgagcccggg cgtgctggca gggatcgtga tgggagacct ggtgctgaca 300
gtgctcattg ccctggccgt gtacttcctg ggccggctgg tccctcgggg gcgaggggct 360
gcggaggcga cccggaaaca gcgtatcact gagaccgagt cgccttatca ggagctccag 420
ggtcagaggt cggatgtcta cagcgacctc aacacacaga ggccgtatta caaatgagcc 480
cgaatcatga cagtcagcaa catgatacct ggatccagcc attcctgaag cccaccctgc 540
acctcattcc aactcctacc gcgatacaga cccacagagt gccatccctg agagaccaga 600
ccgctcccca atactctcct aaaataaaca tgaagcacaa aaaaaaaaaa aaaaaaaact 660
cngggggggg gcccggttan ccaatttggn cctaaag 697

<210> 97
<211> 866
<212> DNA
<213> Homo sapiens

<400> 97
ttttagttca ttattctctt ctattaagag aaattcactg ttaaaaaatt gtttcccatt 60
tccgtatctg aaataatgac tgtagttgag gtgatcttgc cctgggtctg aaatcatact 120
tccaaaccaa aaaggacttt gaatacaaaa cttttaagaa atcttgtatg aatacaagct 180
atatctgaaa aattgtgttt tataatattg atgcctagtt ttgccccagg ccatctgcag 240
tgtggttact atgcaaagaa tgctggtgtt gctgtttttt tttttttctt tgttggctat 300
taacccagcg gagacaatat gtggctatgg tagtacttgg aagttctagc attacacaga 360
ctagcttcca tttctctcat agaggtcatt ttggcattta aaacacatac ttttagaaaa 420
cagatttgga tgtatgtaaa cacagggtta atccaccaca ctctggatgc tagagctgtt 480
gacaaagtca tgctttgcag attttaaaat aaactttttg ttactcttac agcttggtat 540
tttcccctcc tatttttttt acctcctcta aataaacctc tttgttaaat aattgatgtt 600
tctggatcat agaaaatagt aagtttaaaa tacagaatat ttccaagcta actacaaatc 660
tgatgacagt tttttgagtg tgcacttttc cttttatttc ttaggtcctt tttggtcctt 720
tgcaaacata gtaagattcc atatttgtgt cccaactgtg gtaatattgc tgacttctta 780
ctggaaaaca gtcagctcta ggtagcattt cttctgtgtg gtatttaagt taaattatta 840
ccaaaaaaaa aaaaaaaagg gcggcc 866

<210> 98
<211> 1368
<212> DNA
<213> Homo sapiens

<220>
<221> misc feature
<222> (637)
<223> n equals a,t,g, or c

<220>
<221> misc feature
<222> (1140)
<223> n equals a,t,g, or c

<220>
<221> misc feature
<222> (1170)
<223> n equals a,t,g, or c

<220>
<221> misc feature
<222> (1286)
<223> n equals a,t,g, or c

<400> 98
ttcctgtgtg ccctgagccc gctggggcag ctgctgcagg accgctacgg ctggcggggc 60
ggcttcctca tcctgggcgg cctgctgctc aactgctgcg tgtgtgccgc actcatgagg 120
cccctggtgg tcacggccca gccgggcycg gggccgccgc gaccctcccg gcgcctgcwa 180
gacctgagcg tcttccggga ccgcggcttt gtgctttacg ccgtggccgc ctcggtcatg 240
gtgctggggc tcttcgtccc gcccgtgttc gtggtgagct acgccaagga cctgggcgtg 300
cccgacacca aggccgcctt cctgctcacc atcctgggct tcattgacat cttcgcgcgg 360
ccggccgcgg gcttcgtggc ggggcttggg aaggtgcggc cctactccgt ctacctcttc 420
agcttctcca tgttcttcaa cggcctcgcg gacctggcgg gctctacggc gggcgactac 480
ggcggcctcg tggtcttctg catcttcttt ggcatctcct acggcatggt gggggccctg 540
cagttcgagg tgctcatggc catcgtgggc acccacaagt tctccagtgc cattggcctg 600
gtgctgctga tggaggcggt ggccgtgctc gtcgggnccc cttcgggagg caaactcctg 660
gatgcgaccc acgtctacat gtacgtgttc atcctggcgg gggccgaggt gctcacctcc 720
tccctgattt tgctgctggg caacttcttc tgcattagga agaagcccaa agagccacag 780
cctgaggtgg cggccgcgga ggaggagaag ctccacaagc ctcctgcaga ctcgggggtg 840
gacttgcggg aggtggagca tttcctgaag gctgagcctg agaaaaacgg ggaggtggtt 900
cacaccccgg aaacaagtgt ctgagtggct gggcggggcc ggcagcacag gggaggaggt 960
acagaagccg gcaacgcttg ctatttattt tacaaactgg actggctcag gcagggccac 1020
ggctgggctc cagctgccgg cccagcggat cgtcgcccga tcagtgtttt gagggggaag 1080
gtggcggggt gggaaccgtg tcattccaga gtggatctgc ggtgaagcca agccgcaagn 1140
ttacaaggca tcctcaccag gggccccgcn tgctgctccc aggtggcctg cgcatggctt 1200
atgctcaagg acctggaaac ccatgcttcg agacaacgtg actttaatgg gaagggtggg 1260
tgggccgcag acaggctggc agggcnggtg ctgcgtgggg ccctctccag cccgtcctac 1320
cctgggctca catggggcct gtgcccaccc ctcttgagtg tcttgggg 1368

<210> 99
<211> 613
<212> DNA
<213> Homo sapiens

<220>
<221> misc feature
<222> (25)
<223> n equals a,t,g, or c

<400> 99
gaattcggca cgagcgggac gcggntgaag atagcctgcg gagtgtccgg gcggaacacg 60
gttgcagcac tcccagtaga ccaggagctc cgggaggcag ggccggcccc acgtcctctg 120
cgcaccaccc tgagttggat cctctgtgcg ccacccctga gttggatcca gggctagctg 180
ctgttgacct ccccactccc acgctgccct cctgcctgca gccatgacgc ccctgctcac 240
cctgatcctg gtggtcctca tgggcttacc tctggcccag gccttggact gccacgtgtg 300
tgcctacaac ggagacaact gcttcaaccc catgcgctgc ccggctatgg ttgcctactg 360
catgaccacg cgcacctact acacccccac caggatgaag gtcagtaagt cctgcgtgcc 420
ccgctgcttc gagactgtgt atgatggcta ctccaagcac gcgtccacca cctcctgctg 480
ccagtacgac ctctgcaacg gcaccggcct tgccaccccg gccaccctgg ccctggcccc 540
catcctcctg gccaccctct ggggtctcct ctaaagcccc cgaggcagac ccactcaaga 600
acaaagctct cga 613

<210> 100
<211> 685
<212> DNA
<213> Homo sapiens

<400> 100
gaattcggca cgagcctcag ccaccccagt agctaggact atagacacaa gctagccttt 60
ttatacttac tgttttcatc aaatgtcttt ttccaactag tttccaacct gtctttgtat 120
ttgaagtgca tctattgtag atagttcagt gttgctttta aagtgcttac tccatttgtg 180
tttagtatgt tgacatggtt ggatttagat ctactatttt gctttctgtt tttattcctg 240
tttatccttt tttacttctt acagcttaat gaattttggg gggggaatcc attttaattc 300
tctcttgggt ttttagctac atcttcttta ggattgcact agagattaca atatacattc 360
ttaacgtctc acccttttgc ctggggcggt ggctcatgcc tgtaatccca gcactttggg 420
aggctgaggt gggtggattg cctgagctca ggagttccag accggcttag gcaacatggt 480
gaaaccctgt ctctatgaaa aatacagaaa cattagctgg ttgtggtggc acacacctgt 540
agtcccagct acttgggagg ctgaggtggg aggatccctt gagcctggga ggttgaggct 600
gcagtgagct gagatcatac cactgcattc tagcctgggt gacagagtga gatgctgtct 660
ccaaaaaaaa aaaaaaaaaa ctcga 685

<210> 101
<211> 646
<212> DNA
<213> Homo sapiens

<400> 101
tcgacccacg cgtccgataa ctttttcaag caatatcagt gagtgggtcc catcgacagg 60
gttccaggac ctggaacact ttaacagaag gaaatgccga agcagcttgc acagttgctt 120
tacagacttc caagaggctg attctggctt caagatggag ccttggagtt ggtttttttt 180
tttttttttt ttcttccctc aaagaacctg cggttgcgct ttgtgtgttt tgtttttgtt 240
ttccatttgg gggccccatg ggaaagagct tctgaactct ttcctttatg aactcccact 300
gtgttcctat aaaggccctt ttctttctta gtgttgtaag ttacattttc attatgcccc 360
atcacatctt ctttactgta aaaatattaa aaagctgttt ccaagtggga cagctaatga 420
agctctaatt attgcagaca tatttttgag atgtaaaaaa aaaaatttaa agttaaatga 480
taagtcttag aggcgagtga ggaataaaat ggatgtaaac atttacatgg gatgcattag 540
aattctgctg tgtgtactgt cttttggttg aaacaaatta tgaacagtga ctaataataa 600
aaagtcaata cccaawraaa aaaaaaaaaa aaaaaaaagg gcggcc 646

<210> 102
<211> 826
<212> DNA
<213> Homo sapiens

<220>

<221> misc feature
<222> (726)
<223> n equals a,t,g, or c

<400> 102
ggtcgaccca cgcgtccggc tccctttgtt ttggtggcag ccttcttgtg ctgtatactt 60
gttccctagg gtgtataata atatgtgcac tagagtgcta ggtaccctac cacattgctg 120
ggaccttgcc acactgctgc agccttccag taggatatgg gggaatgtca gtgaggctcc 180
agggatgtag atatgtaggg aatgttggac cccagggcaa catgcaatct ggtaggagtt 240
gggctctcaa aatggtgctg ctgtgtaaca gctgcttggg tcttggggta gggagtgtag 300
gacccagcat gagctccctc tttggagcag tgctgtctga gactccaggc agctccgtgt 360
attagtctca ggacctgcaa aggcctaggg gctctttttg ggtaggactg caggagtctc 420
catggtggga atgtgaacca ctggaaatct ctcatttacc atttccctgt actggagatg 480
ctttctgggc tcccagatga tactarctgg gctggttgcc tcamttcctt ctccctctgt 540
gcataaggca ttttctgtca cttctctgct gaactctagt gttctttctt agaggctgta 600
ctcaaagttt cattatccat tcagtatttt tattcttctt tgtggaggtg gcaagtgcta 660
ggtgcctcta gtcaatcatc ttgaagcccc ctgttatgtt aaagtcttta atggaaaaag 720
aagacnacat gcatgaccag gcagatactt tgagcagagt cataggaact gctaaaaaaa 780
aaaaaaaaaa aaaaaaaaaa aaaaaaaaaa aaaaaaaagg gcggcc 826

<210> 103
<211> 586
<212> DNA
<213> Homo sapiens

<400> 103
gaattcggca cgaggtggct atcagatttg gggttctact ctatgagact tttaagtcat 60
tatgcaattt ctttattttw atttttttga caagaagtct ggagcatgat tacattatgc 120
attttcttac tctttaaagt atttgtgggg ataatccttc attatttgat tggcaaaaat 180
atatatgttt atagtgtgta acatggtgat tggatatatg tacacattgt ggaacagcta 240
aatcaagcta ataacaaatc agttacctca catacttatt ttgtggtgaa aacatgtaaa 300
atccactctc ttagcaattt tcaagcatcc aatacattgt tawtaactgt agtcaccatg 360
ttatacaata gatctcttga acttattctt cctgtctaac taaaattttg tattccttga 420
tcaacatcta cccaatccct cactgttctc cagcctkgat aactaccatt ctactctctg 480
cttctatgaa tttgactttt ttttttttta gattccacat atgtgagatc gcgcagtatt 540
tgtctttctg tgcctggctt atttcactta atataaagtc cctcga 586

<210> 104
<211> 628
<212> DNA
<213> Homo sapiens

<400> 104
tacagcagtt taaaaagcag tgtctttctt tgagagacag gaagtctagt gaagagccag 60
tattttaggg atagataatg aaagaggctg tcatttcaga cattttaatc ctctgaaaga 120
atacaaaaga aaaaaaaaag aaaacaaatc tttcagaatt gtttgaagta agaacaagac 180
aagaggaggt gattggtgtg ttactgttct acgaaaaagg agaaaaagct tcatgaaatc 240
gccattcagc aaggacagaa ctggagatgg cttctctttt acaaagaaat ctctgtccca 300
ggctttcagt ctgtttggtg ttcatacaag tgtttgtgtg ttgtgtggaa ggcgggggaa 360
ggcgggtgaa ggcggtcctg ttcagggccc cctttggtga acacagcagg caaaatactc 420
tcgtcatccc cagccaaact ggcctgcaag cgcactgact tccacatccc tagcatttag 480
gcctttgaat agaagctgac acgtagcagc cagctgaaca agtatttaat gaggagcaac 540
acaactccaa gaagggctcc ttagtgtatt gtcaagttgc tgcagccttg tgagatggaa 600
aaaaaaaaaa aaaaaaaaaa gggcggcc 628

<210> 105
<211> 558

<212> DNA
<213> Homo sapiens

<400> 105
agctctaata ttactcactw tgaaggsaaa gctggatacg cctggcaggt accggttccg 60
ggrattcccg ggccccatca caccctatgg gggagagcga atgttacagg aggctttctg 120
gtgcctcgtg cacatggact gtgcatgtgg attttgccta aggtcagcct tatatgcatt 180
gtggaactag ggtatggaaa accatgaaac atgattattt tcttctagca tgcctgtcta 240
tgacttcaac tggtggtatt ctttgtactt tataatctac attatcatta atacctacat 300
cttcaagtct gtctttctgg ccatggtgta cagcaattat aggaagcatt ttcacatact 360
gtgtgtgtgt gtgtgtgtgt tttgtagtga tgaacagaac ttgttattta cccaattcta 420
ttatctatca taatagtaaa ttagctacta taatagacaa aagtatgact ctcagttaaa 480
taagagattt tttaaaaact tgttacaaaa aaaaaaaaaa aaararaaar aaaaaaaaaa 540
aaaaaaaaaa gggcggcc 558

<210> 106
<211> 756
<212> DNA
<213> Homo sapiens

<220>
<221> misc feature
<222> (230)
<223> n equals a,t,g, or c

<220>
<221> misc feature
<222> (755)
<223> n equals a,t,g, or c

<400> 106
gaattccaat gtccacaggt gatgggagag atgctgagaa agggtggcca gtgagtgagg 60
aggaaaacca gaggagtgtg tatcctgggt accctgaatg tgatgagcga caagctgtcc 120
cccagcactg tgccattgct tctcccagtt ctcttcaaag tcaccatcct gcttcagcgt 180
gtgtgcccag aagatagccc ttcctcttct gtgcttccag aatccgtagn cagggaatag 240
gaatacatgg acaagtagca tgcagtgcag tgagaatgta taacaacaga tgactctggg 300
gaccaaaatc aaatggggcc agctacaaag agggcaggaa atccccacag gtgattttac 360
tgtgaggaat tttatgaggt tcagcatcat atattgttag gagaaaatgc tgttttgata 420
agcagagata tgagaaaagt aaacgggaac tatgatttag agatctcatc tgrttacttt 480
gtcctattcy cagtttwatt actaaagagc agtaaagcca aggagaaagt agtaaagatt 540
agatgaatgg ttagcatgtg aaacctgaaa ggaaccagag tgatttccct cgaggaacaa 600
atgcacttct cttacatatg aaagatgatg tgttctgtgt tcccatagaa tctagggaaa 660
gaaaaagtga gcagatactc tgatatgagc aatataactt aggtgtaaaa aaaaaaggaa 720
ttcgatatca agcttatcga taccgtcgac ctcgna 756

<210> 107
<211> 1146
<212> DNA
<213> Homo sapiens

<400> 107
cccgtccaca atgcagcaga ctcttcccaa ggccacctag caagcaaggt tgatcggatc 60
atctaaactg gccgcctcct gaatatttca ctgaatcctg gcgttcatgt tgaagcagac 120
aaaatgagaa aggaggaggg cattgctcac ctctcaatag cttttttcgt tcaagttcta 180
tgtctttatc agctcttgcc tgtgatttta ccccaattca accttgggag tgggaagaat 240
atgaacagat aacccttggc ctaacagctc catcaaacct ccttgagagc aactacctag 300
gccaggctag tgagtgcttt gtgaggaagc tggtcagaag gttccctcaa ctccttcctg 360
gtcctcctgg acactgcaga aaagacttag ggatccccca gcagaggcca attgctctcc 420

ttccttccct gccccaccag gaaaggaata acgtccacag acttgaagca gatagtgaag 480
tagatctgtg agaggttcta ggtacttagt gtgtagactt tgacgaatat ttctcaagtt 540
gggagccctt gttaaaaatg atgtttaagg gagtggttgg ggggaagatg aaggcatgga 600
ggaggaagaa gagaaggaag cccttgccat ataaaattca tgcagactaa acagtttccc 660
tgacagaata aataaagtgg atgctacccc actccagaat caaaagcaat ttaattaaag 720
tctcttaagt tgtaaagagt tttaaatgat ccgtgttgaa ggcgaatsct gcyaaatgca 780
gtgggtctga cgtcagctgc cgggcctggg ctgggaggcc atttgctatt ctgtttaagg 840
caggctggat tgtcttattt tggaaccagc ttggtggggg gtttgctttg ctactgcttc 900
tgagccctga gcttcaaagg ctgaaattaa tggtgaacaa aattgtgcgg ctctggccat 960
cccatgcggg caagcccatt gagggttatc attaagtaaa gaaataaaga ggggaaaaaa 1020
agcctgcctg ttccaaaaac ctcatcagat aatgacctca gtgattgggt tttcattacc 1080
aaacagcatc cagagattat caacccatag aagaagggag gggaaaaaaa aaaaaaaaaa 1140
aaattc 1146

<210> 108
<211> 775
<212> DNA
<213> Homo sapiens

<400> 108
   tcgacccacg cgtccgaaaa aggaaatgat acatgtcttg acatttctat tgcagtwtta 60
   catcttaatt tctaagggca aaggtgatgt ttcccagttc gtaaagtctc gagagtacta 120
   atgctatcaa aagtaattaa tttcaagtgt aaataagacc aaacaaaaac gatcagatgc 180
   gacattgtct cataaacatg atagactatt aaatcacttt gtgtttttgg gaaacagcta 240
   taactattaa tatatacagt aatctagtaa atttccttca gatatgctat tgcggataca 300
   acagatcatc tattgtcaca agctaaccat tatcctaaca aaatggcgga atacagcaag 360
   acataagagt aaaaagaaag aagatgagct gatattaaaa catgaacttc aattgaaaaa 420
   atggaaaaat aggttaatac tcaaaagagc tgctgcagaa gaatccaatt ttcctgaacg 480
   aagttcttct gaagtctttc ttgtagatga gactctaaaa tgtgacattt cactgttacc 540
   kgaargrgca atattacagg tttgtatgaa ttcagtatac attatatact ataatctgcc 600
   aagtgtggtg gtgcatgcct gtaatcccag ctgcttggga ggctgagaca ggagaattgc 660
   ttgaacccag gaggcagagg ttgcagtgag ccgagatcac accattgcac tccagcctgg 720
   gcgacaatag caaaactcca tctcaaaaaa aaaaaaaaaa aaaaaaaggg cggcc 775

<210> 109
<211> 911
<212> DNA
<213> Homo sapiens

<400> 109
gaattcggca cgagacgaca atggggaacg cggtgtttcc cacctcttgt gggtagaaag 60
cagtctgctt tgaggaggcg agaaggcaaa gccagggcag ggcgttgctg tgggaagcgt 120
tcggtgaaag crggtttcga cgcttaggag ggccgaggga gaagattcca ccagcattgt 180
ccttgcttca agttttagga tgtctgaact ttcagctttc atgttttcaa ccatcatttt 240
tttaatggca caacctacat cttgttttta aaagaagtag cctcaaatta aactcctaaa 300
ctctgatgcc ctggggatga gaacaactag ctkggatctc gtgccgtgta atcaatgttt 360
cattccgctg cctccatcat gtaatagaat cgcttccaga aaggcagtta actggaagca 420
gcagaggctc ccagccgtga gaggactgct caacaatgcc ccccatcgcc gcccccccac 480
ccctcgcacc ccttgtgttt tcccctctga ggggcccaag ggttatggct ttcatgtcta 540
ggtgtgggga cagaggaggg agaggcagat ccygggccgg gagaggatgg ccctggtctg 600
aatctggagt aattaatgcc cacccaaaga aaaggccctg cccaggtcca atgttgtctt 660
agatctgatg atgctgctat ttacaaaaca ctgatcgtcc gaaagcttga atctgttcct 720
cctcgaatga ccctgtagat gcctgacctc caccgtacct ccacatcact attcatgtcc 780
ttctaggaaa atgtgcacat gcctcacgca ctatgtggga agggcgtgtt tttaaattaa 840
taaagtgtgt caccattagc catamraaaa aaaaaaaaaa aaaaaaaaaa aaaaaaaaaa 900
aaaactcgta g 911

<210> 110
<211> 456
<212> DNA
<213> Homo sapiens

<220>
<221> misc feature
<222> (456)
<223> n equals a,t,g, or c

<400> 110
   gaattcggca tgagctttct ttctcctgca ggcattggaa atacagtccc agctggcaac 60
   accagccagc agcacagccc ggaatcctgc tcctgacctg caccatcccc accagcccac 120
   gatagaacgt ttttgtaggc attcctcctc atgggagagg atagagtaca tgcgagtttt 180
   tgctctcctc ccaccctttc acaagagcac tgtgctttct tttcttctct ttttcctttc 240
   tttttttttt tttaggcagg gtcttgctgt gtcasccagg ctggaatgca gtggtgcaat 300
   catagctcac tgcagccttg acctcctgga ctcaagcaat cctcctgcct taacctccca 360
   gctactcagg agaccgagac aggaggacca cttgagccca ggaggttgag gctgcagtga 420
   gccgagattg caccactgsa mtccagcctg gggaan 456

<210> 111
<211> 554
<212> DNA
<213> Homo sapiens

<400> 111
gaattcggca cgagcctcca cctcccaggt tcaagagatt ctcctgcctc agcctcctga 60
gtagctggga ttacaggcgt gcaccaccac acgttgctat tttttgtact ttaagtagag 120
acggagtttt gccacattgg ccaggctggt ctcaaactcc tgacctcaag tgatccaccc 180
accttggcct cccaaggtgc tgggattaca ggcatgagcc actgtgcctg gctccattta 240
caactatttc tatcattata atgcaggggc tctcaaacct gagcatgcct cagaatcccc 300
cagagggctg tgcgcacaga ctgctggacc tttccccagc ttctgattcc gtccctccag 360
agtggggctc gaagattgcc tttgaggtga rgctgcgggt cgggggcacg tctgagaact 420
gctgcagagg tgartgctgt ggctctgtct gcattccccc tggaagactg argcaccagg 480
tgtgctggtg ctaacagacc acaagtccct cctggacact gcccttctct gaagggagct 540
gcctcctcac tcga 554

<210> 112
<211> 722
<212> DNA
<213> Homo sapiens

<220>
<221> misc feature
<222> (2)
<223> n equals a,t,g, or c

<400> 112
gnaattcggc acgagaaaaa tttacgggta acactgaggg gtggggtgga aagttttgat 60
cataaagtgg tcaccaacaa gggcacttct gaggtgctaa tgatgttctg ttttctgatc 120
tgggtcgtgg tgacattcac atattcatta aattgtacat ttgttttaca taagtttatt 180
atatttccta attttaaaaa agttaaaagg aggaggaaaa agttggttat gaaagtgtaa 240
ccattcttcc aaaatatcaa ttaaaacaca tctgaattaa gaggtaaaat atatcaaaga 300
ttgacagaaa acaaaagctc tgaaatgata tttccagcct aagaacagtc gttgcttttg 360
ttggtttagg aagttttgtt ctcctgaact aatgttcaaa atgaaaaaaa gtcacctggg 420
ccaggagcag aggcccacac ctgtaatccc agcactttgg gaggccgarg tgggtggatc 480
acaaggtcag gagatcgaga ccatcctggt taacgtggtg aaaccccatc tctacaaaaa 540
tacaaaaaat tagctgggct tagcggtggg catctgtagc cccagctact cgggagattg 600

aggcaggaga atggcatgaa cctgggaggt agagcttgca gtgagccgag attgcgccac 660
tgtaccagcc taggtgacag agcgagactc cgtctcaaaa aaaaaaaaaa aaaaaaactc 720
ga 722

<210> 113
<211> 931
<212> DNA
<213> Homo sapiens

<220>
<221> misc feature
<222> (930)
<223> n equals a,t,g, or c

<400> 113
gaattcggca cgagaaccag atgtttttcc acacagaatg ctagttcttt aagacacagg 60
ctgggtgaca tgtttcctta gagtgacaat atttccttat agtgacattt tccttgactg 120
gctccatgca gaataggagg atatagaata ggaggagaag gtttctgctg tggcacctgg 180
agtggtactt ggtgcacgcc aggtgctaga caatgtgtgt gacaaggatg cacgtgaaat 240
gccccccccc gagtgcctca gtgactgcag taaagtggcc cttgtcatgg tcctcttcct 300
ctttctgcat cagtcttcat gctgggcggc atgaagagag aaacaaaaac cacctttctt 360
gccagggtct tagtaccatt tgctgctctt atctttcaag taagggagaa catctaagaa 420
acttatcacc gtattcattc tagactgtta gggrtttaac tcttcaccta cttccctgag 480
tggtctgggc tggargttca gagctaartg ggctgggtgt aaatcaggat tccgtccctc 540
amtagctgtg aggctgtggg taattcactt catctctctg agccttcatt ttctcacctg 600
aaaattgggc atgctaatac ttttccatct ccttcccagg gttcacagga ttaaatgaaa 660
ttattaacac aaagttcttg gcctggtagg gggcatgtac gtggccaccg tcctggtgct 720
ggacactggg gtaagagttt ggaagctatt ggctgggcaa ggtggctcac gcctgtaatc 780
ctagcacttt gggaggctga ggcaggtgga tcacgaggtc aggagattga gaccatcttg 840
gctaacacgg tgaaacaccg tctctactaa aaatacaaaa aaaaatttag ctgggcgtgg 900
tggcatgcgc ctgtagtccc atctactcgn a 931

<210> 114
<211> 588
<212> DNA
<213> Homo sapiens

<400> 114
gattcggcac gagatcaaaa tggccagttc tgtgacagta aaagaggttt gtgtcttatt 60
taatcttttg ataataataa cagctatggt gtatcacagc tttaccaagt accagacact 120
gttctaaggg ctttgcatgg ttcactcact ccttacgtca tccctcggtg gcaggtgctg 180
taattatcct tatattgcag acaaggacat tgagacagag gtcaagccac cttcccaagg 240
gcacacatgg catctgcact gctcctgacc gaccgacaga gagagctgct gtcacgatcc 300
tcaaatgagc tatgcatgtc aaaagtttaa aaataaaaaa gataaaaaca tgcacaaaat 360
ttaaaaagta aaccatttca agctggacag actaaaactg agagatggcc agagaagagt 420
atgaaagata aatctatgga cagagtaaac cctgactggc ttgaaattag ggcccttact 480
cctccacact cctgacgggt tggttcaaga ccargaawta gaagcmcmtt gtgagttcta 540
cgstgctgcc ctgggaaaca cacaggctaa acacacccac aggctcga 588

<210> 115
<211> 812
<212> DNA
<213> Homo sapiens

<220>
<221> misc feature
<222> (443)

<223> n equals a,t,g, or c

<400> 115
gaattcggca cgagtatggc ccttctttgg cttctgggta tttaaaaaga gctcttggga      60
ctcttctgag gtcttcctgg gagcagaaca gtacacatgg tctggaattg ggttgcatgg     120
aataactttc aaggaaagcc actgaataaa gtgccctgca ttcctgtcca ttggatactg     180
ataatgctat aagatgatct ttctcttctt tattttgttt gagattattg tgactctctg     240
gctaactcct acttatcctc aggccttttc tgaactcaca attcaaatta cagctccctt     300
tggttctctt ccacagcagt tgtacttaca tatgtctatt atataattat gaattgtttc     360
atattgtcgc ccttacaggt aaactaatga atttggggct ccatctgttt gctcaccact     420
tgatcctggc agtagcacac aanggctgct caatacctat ttactgaatg agcaaakgga     480
ctggaccact tttagagact ggagtatttc cttawaccak gtgagattga wttttgagga     540
cagtttacca ctggaagctt ttgcagaact aaggtcattt ttacagtata cataacctct     600
gctgtgtttg ttgatactgt aagtttacat tttcttatga ctctttttaa gtagagcacc     660
cctgtgttta ggaaagctag agctattgtg atgcctttga gtttgcttgg ctgattgctg     720
ggacttgaac tactgagctt atctaaaagc ctcagaggcc ttgtagcctc tgtcttttag     780
agagtgtagg taaaggcttg ttttccctca aa                                   812

<210> 116
<211> 506
<212> DNA
<213> Homo sapiens

<220>
<221> misc feature
<222> (13)
<223> n equals a,t,g, or c

<400> 116
gaccatgatt acnccaagct cgaattaccc ctcactaaag ggaacaaaac tggactccaa      60
cgcgttggcg gccgctctag aactagtgga tcccccgggc tgcaggaatt cggcacgagc     120
acctcctgag gaatatggtg taggaaagcc acccgcgtgc tttctggctg ggatggctct     180
cttccttggc tgctggaggc actggagaga ggtctgataa ggatggctgt atggatcagt     240
gggtcttatt cctcattctg cagcagaagc aactgggatg tttttctcc taatattgtg     300
ctggcttctc tgcctttctc tttccggtct gtatccaagg ctgctaaacc ctggtggctg     360
gctctccctg ctctctttcc agatggatta tggctggatt ctgccatggg gagcttgtac     420
agtcagacat ggaaagccag gaatgggaaa gaggtcaggt ggttctctcc cacacctcac     480
tgccttggtg ctatgtctca cctcga                                          506

<210> 117
<211> 751
<212> DNA
<213> Homo sapiens

<400> 117
gaattcggca cgagagcctc gcaggtggat tagacccacc cgaggctcgg gagaaaccac      60
ggcaccttgt tgttttgagc cactaaatgg cgggacgctt gttcacgctg ctgctatggc     120
aagagctagc gaggcggctg gtaccgggtg atgcttcacc acggctttcc agaaagcgct     180
ccgtgacccc aggcccaccc ttcccgacac tcacggttcc ctcagaaatg ctcctctcaa     240
atctctcact ctccctgcag cctttgttgt ttcttttttc tttctttctc ttttgcaaga     300
tgggatcaag gaaaggtctc agacacaaaa cgcaacattt ttcttccatg acagatcaga     360
tattgaaggg ctcagtgagg agccctgctc tgggacaact ccatgattag cgctccaaga     420
ggcagtcaca gggaagcagg tgctctgttc ccctcctggc tcagcaatcc cgcagtcctc     480
ccgtcccgct ccaggcccag ccagcctggc tgcttggatc cgagacaata gcttggtctg     540
gaggcggctc agggtgggag ggacccaggg acccgggcac cagtacagca gctgggaatt     600
caggcccagg gatagggatg gggcacagga caccaccccc atctcacaca gggagatgaa     660
ggtgggatcc agcatgggga ctggacatcc ctgagtccag ctgccccgtt acaatggggg     720
aactgagatc cggggatggg atagttctcg a                                    751

<210> 118
<211> 960
<212> DNA
<213> Homo sapiens

<220>
<221> misc feature
<222> (460)
<223> n equals a,t,g, or c

<400> 118
ttttgtctag tacatatatg taaatatatt aatgttgttt ttgtgtttgt gatgtagtaa 60
ggagatgtac atagaaattc attgaggtat atagatactc atctgtctag gcagttccca 120
attttctgaa gaatgtttta cagcaaaatt ttctattttc ttttattaaa tagtgacacg 180
tcaaacaatg tcacatccaa aacactagtt tcatcaattt ctagcagtaa taatagactt 240
gctgtaagta ttgttttctg atgccatacc cttgtcatac atattattaa atgaccaata 300
ttatgtatga agtagacaaa aaaatttact caaacttcat tcaaatccta attgtgataa 360
tttttgtttt atatttaatt ataaaccaaa atacatttgc atttttaagc taatttgtct 420
caaaattttg ctttatattt ttggatcagg ttaaagtccn gtggatcccc tgaatgttat 480
tgtccctctt gatggttttt acttctgagc tatacgtcaa aagacacata agcttcaaaa 540
gtcmagacaa acctcattgc cataaaaatc aagatataga tgttctgttc cgtaaactcc 600
ttgaaaaaca ttttaaagtc atcaatatga tctgtttccc atgaaactta agttagcttt 660
cttattggag twattycttt tctgtaagtc tgaaaagtag agatttgtt ttacgcattt 720
tagtaacctg caacaaccaa ctctaaaaaa gatttggctt gtaatgacgg tctctgcttt 780
tttgggtttg gagtacacaa ttgtaatatt tacttagtta tttgtgtttt tctttgttca 840
aggtattgac tagtttcata aatttttgm aagtttttct ttcattggtt ggaaagcaga 900
ttacattttg cactattaaa ataagtttat tactttaaaa aaaaaaaaaa aaaaactcga 960

<210> 119
<211> 1442
<212> DNA
<213> Homo sapiens

<220>
<221> misc feature
<222> (1377)
<223> n equals a,t,g, or c

<220>
<221> misc feature
<222> (1419)
<223> n equals a,t,g, or c

<400> 119
cttctatatt agatggacag atttatatac ttttccatgg aggattaagt aaactgaaac 60
ctaagacaca cgaagaaatt ctaagtggaa aggccactta ttagttagtt tacagcagta 120
tcgtaagtga caggatgata ggagtgtggt aagtgatcag gataataatc tgcttagtaa 180
gagaaacaat ttgaatttta gaaggaaatt gccttaccat ttgcaaatta aggtaattaa 240
aatacagtga atttcaaaat gcctttttaa tgacaatgtg tgaacttaat ttgttttaat 300
aaaccaaaat trttgttatt gtgttaaggc tattttacat tgaatgtgta tcttgccact 360
gatgttaact tatcccatct tacccaaggt tgtaggtaac aatatactat tgggtgacag 420
tggactaaca tctctagtga tccctttgtc agtggtcttt aacttaaaat aatttagaga 480
atatggtttc tacaacttac attttgttt wcttgtaact acagattatt atgatggttg 540
taatgaagat tatgagtata attggagcta tatgtttctg aattctgaac aactatttat 600
aaaattttat cctacttttt tctgttgaac atatgacttc tctggtctgc taaacacata 660
cagaccttta gttttggttt acatggattt aaatatatag atatatcact gtaaaataaa 720
cttcaggtgt aacagattta tagagaaagt aatcatattt gtttatggtt gtgtacctac 780

tttgagaaga aaagaaaaat attagaatga acagataatt ttacaagtgt tgatcactta 840
ccagcaaacc agaaacttca gagattttga aagcaaatct attttctctg ctgtgtatta 900
aattcattta tctaaaatgt tattgctcct ggcttagaat catcttgtgc aaattctctt 960
tttttgttgt ttgtctgttt gcctgttgct caccatagac ataattttct tttcataaaa 1020
cattctttgt ataatcacct cagagattat gaaagtgact ttgataaaat ttaatggtgt 1080
tcacaaaata attttcacgt gagtaatttc acagtgcgtg tattgtatgt tatttagtgt 1140
attttatatt ttgtttcaat tagagaatgc tattgaatcc agtttttgtt tagttactgt 1200
tcattttact ttataaaatt gacataattg agtttattaa atttattggg ccaatttaag 1260
taaacagttg aacgtttcat aagtcatgag gtctttttgg gcatatacat gaagtaaaca 1320
aagacaatac taggctatgt aataggragg ctaccttaat taggaggtaa atattcnttt 1380
tggaaattgg gcccgtgggc ctcgggtgga aaatggggna atatccctag gtaaaaaaat 1440
gg 1442

<210> 120
<211> 845
<212> DNA
<213> Homo sapiens

<400> 120
gattttacac agaacatatt ctctgcatga tttcagaaaa gaaaatctaa aaaggtaata 60
cgggtatttc aaataaaatc ctttctggta tgaaaggctc cattgatttt attaagcctt 120
cctttacctt gtagtacaag gtgctttaat gggatagaac taagcatatc aatatctata 180
actgcatttt gtgctagaca attactgttc ttttctctaa aatgtatatg tcaatttaca 240
aggccaggga tagaaaacac tccataattg ctttccttga ttttgctgag gatttggtat 300
gattttagta agcaaactgt tttttggttt ttccttaatg tttttaattt tttttcctct 360
tgcaacaatg acggtgcatg ttcttataaa tataggaagg tccagatata aatagtaacc 420
taaagttctt gctgtgctta aaaaaaaaaa tcatgtggcc ctttcaatat ttgaactgct 480
aagcaatgac atctgtagtt ttatctcctt ttttatgtca tagaaattaa tatgatactt 540
taaatatgta aatataatac attaggtaat gctattattt atatctgtct taacataatt 600
taagttgtag ctgtgtcttg gaaatatttt taaggtaatc tatattcaca ttgcctgtgt 660
taatgctttt taaagtttgt atacatcaga tgtatatttt tggtttggca taagctacga 720
ttgtaatttt tcttggcttt ttgttcataa agaatttttt gaaggaatgg taacaaatgg 780
taatttacaa atggttgtga ataaacacat ttttacactt aaaggwaaaa aaaaaaaaaa 840
ctcga 845

<210> 121
<211> 360
<212> DNA
<213> Homo sapiens

<220>
<221> misc feature
<222> (340)
<223> n equals a,t,g, or c

<400> 121
gaattcggca gagaatcagc atgtctatca cctcaaatac ttatttcttt ttattgggag 60
cattcaaaat cctctcttct agctattgga aaatacacac taaattactg ttaactatag 120
tccccctgca gtgctgcgga atgccacaac ttatccctcc tctccagctg tagtttagta 180
tccagtaaca tactcttttc atttcctttc tttgggcaga aggctagatg ttgcctgttt 240
ttgttttatt tttctgcttc acatatagcg cacgaaagca gagtgtattc aaaaaaggaa 300
atgtgtttga aaaaaaaaaa aaaaaaactc gagggggggn ccggtaccca attcgcccta 360

<210> 122
<211> 944
<212> DNA
<213> Homo sapiens

<220>
<221> misc feature
<222> (932)
<223> n equals a,t,g, or c

<220>
<221> misc feature
<222> (942)
<223> n equals a,t,g, or c

<220>
<221> misc feature
<222> (944)
<223> n equals a,t,g, or c

<400> 122
tcgacccacg cgtccgccca cgcgtccgga cagacccagc ctggagctgg cccctggcct 60
gtgtgctgac ttcttggggt cctcaaacca ctgtattttt ctgttgagcc tgtacttggg 120
gagagatcag tagcatttga ggaagtaaga gaaaagaatc atggtacctc agggtttctt 180
tccctttact cgctggcagc cattgtctgt gggcacctca tgtttttcca cactctactg 240
ggccgtggag gtaacgatca cccaggccag tctcctctgc ctgggatgcg ccctctgaga 300
ggaggcctag cagggcaggc tccctctggg catccctgga tgcagcctct ggacacatgc 360
ctcctttaaa gtgtccgggt gcagctcagg ttgagtggag gtagaaggag aaacagacat 420
gtttaccacg cgttttccaa agctcctgat ctttcccaag attgtaactg aaaactgctg 480
tctcttgttt tgttcgtttt gggggtggtg gtgctggctg ggccatgctt gtgaagtgat 540
gtgtgtctct gatttaacgg attcactgtt ttctctgcta attgagagag cgttatttac 600
attatttatt tgttttgaca caagtgcttt cagtgtttta tcctagctaa tggcttctta 660
aaggtaataa aacccttcca acgtaattgg tcagataaaa ctttttttct tgtatgctta 720
aataaagcaa ttagtgaagc acttctatcc aaaatgactt ttttgtcctt ttttaaaacc 780
aatttactgt tactggaaac tttttgtaca ataawgcaat cacgcagatt aaagaaaaaa 840
aaaaaaaaaa aaaaaaaaaa aagggcggcc gctctagagg atccaagctt acgtacgcgt 900
gcatgcgacg tcatagctct tctactacgt gnaccctaac tncn 944

<210> 123
<211> 914
<212> DNA
<213> Homo sapiens

<220>
<221> misc feature
<222> (909)
<223> n equals a,t,g, or c

<400> 123
ggcagagcaa gagatgactt tagatgagtg gaaaaatctt caagaacaga ccagaccaaa 60
gcctgagttt aacatccgga aaccagaatc cactgttcct tccaaagccg tggtgattcg 120
agagtcaaaa tacagagatg atatggtaaa agatgactat gaggacgatt cccatgtttt 180
ccggaaaccc gccaatgaca tcacatccca gctggagatt aattttggta acctccctcg 240
tcctgggcgt ggagccagag gaggcacccg gggaggccgg ggaaggatca ggagggcaga 300
gaactatgga cccagagcag aagtggtgat gcaagatgtt gcccccaacc cagatgaccc 360
ggaagatttc cctgcgctgt cttgaaagag ccctgtttcc cagcaccgcg gagctgcact 420
gcacacctgt ggggagactt ttccagctgg gccaagggag tcagactcta agaacaatag 480
atgttgcttt tcccgtgtca tgtaaatttg ttgcactttt ttgggctgag ctgttagagg 540
ggcttctcca gaggctcgag agcaggccat ttcccaagaa gatgaagaat ggtgactgtg 600
tttttattga aggaatttca aatgaagaat aatgtttaaa atgtgtatat agagatagta 660
tagactcctc cgcggaagca tggagggaaa ggaggttgta aaatagactc catggagact 720
cttaggaagc agtagattcc cgggggctgt gcctttagcg ttagaggaaa cacatagagc 780
tggaactgtt aatggaaagc agtcacagct gagttttcgg agaccaagaa attaaaatac 840

aattgcactt acaaaaaaaa aaaaaaaaaa aaaaactcga gggggggccc gtacccaatc 900
gccttgtgnt gcat 914

<210> 124
<211> 462
<212> DNA
<213> Homo sapiens

<400> 124
gaattcggca cgagctgggc tcaagtgatc ctcctgccga ggcctcccaa attgctggga 60
ctgcagctgt gagccaccat gcccagcctt aacttggttt taagacctct gatttgcctt 120
gcctcaatta cctcctttct tattttcttt cctttgttga ctctcatact ctgttctcct 180
aattctcccc cttttccact ccctgcccac cctgaaagac acacacacac acaataagtg 240
ggtggagtaa gaagtcaacg gagttggata taagcattcc tgcttttctg acatctccag 300
tgtcttggag aacaaggatt ctagaatgag ggctcctcat tatgcttcct ttcaacattt 360
tttctctgtg ttacttaagc tttcacccca agcatgtttg acagagagcc agtgcattcc 420
ccttactttt tacaaaaata aaaaaaaaaa aaaaaaactc ga 462

<210> 125
<211> 545
<212> DNA
<213> Homo sapiens

<220>
<221> misc feature
<222> (7)
<223> n equals a,t,g, or c

<220>
<221> misc feature
<222> (16)
<223> n equals a,t,g, or c

<220>
<221> misc feature
<222> (41)
<223> n equals a,t,g, or c

<220>
<221> misc feature
<222> (42)
<223> n equals a,t,g, or c

<220>
<221> misc feature
<222> (87)
<223> n equals a,t,g, or c

<400> 125
tcacttncgg ttccgntcga tgtggtgtgg attgtgagcg nntacaattt cacacaggaa 60
acagctatga ccatgattac gccaagncga aattaaccct cactaaaggg aacaaaagct 120
ggagctccac cgcggtggcg gccgctctag aactagtgga tcccccgggc tgcaggaatt 180
cggcacgaga ttcgctgcct aattccacca tgatgtttta ctatgcatgc tttatcttat 240
actcatctct ctctcctctc tctctttctc tttctccctc cctcctttct ctattataat 300
ttagtcatct tattttttga ggcatttcag aatatatcac acttgtccta aatacttcag 360
tatgaacatc attaactaga atttattctt tgttttactt ctgatgtgaa ayttatataa 420
atacaacatg ctatgaattt gttttccmaa aaaccaatca acaatttawt aagcatggka 480
acaaaaaacc tgaaggcttt atcttttaga gtagtagttt ttaaaaaaaa aaaaaaaaac 540

tcgta                                                                 545

<210> 126
<211> 912
<212> DNA
<213> Homo sapiens

<220>
<221> misc feature
<222> (906)
<223> n equals a,t,g, or c

<400> 126
   gaattcggca cagagaaaca tttcatcccc agtaagattc ctcatcgtca ttcacaggtg      60
   atctctgttc ccaccctagc cttggacaat tctgcatcta ctttgtagct ctataaattt     120
   gccttttctg gacatttcat gtaagtcgat cacacagtat gtgttccttt gtgactggct     180
   gcttttgctt agcatgacgt tcttggggct cgcaacgcag cttgtgtctg ttgttcattc     240
   cttttgcagc agaatcgtat tctgttgttt ggatgggcca cctgtttgtt gtctgtttac     300
   tctccagctg gtggacattt aggccgtttg cactggcggt tactgtgaat catgtcgctg     360
   tgaacattgt gtgtgtgtct gcgtggactt gtgtgtcctg ttctctggga aggagttgcg     420
   ggttagargg tagttttttg tttcccctgg agactctctg gtttccacat atggtagttt     480
   tatgcttaac cttttgagaa attgccaaat ggctttctga agtggccacg tcattttgct     540
   ccctccagcc gtttgtaatg ttcccatttc tcctatgtgt aattttaata caaagcagta     600
   aaaagttgcc attatggacc tagtaaattc tgaggtaaca taagagagaa ataatgatgc     660
   agccgtcatt actgtgctgg taatgtaagt ttcctttttt tttgttttta aatggagctt     720
   tgcagagatc aagtcgagag aagaacactg ggccagcctg actccaaagc ctactctctt     780
   aagcgctttg ctgacttgtg atgttttaaa atctagcatt attttcaaat gctgtgagag     840
   cactgaagat aaaggatttg attctttttt tcaggcatcc aaggatggtt catcatcaag     900
   aatcanttta at                                                         912

<210> 127
<211> 1048
<212> DNA
<213> Homo sapiens

<220>
<221> misc feature
<222> (13)
<223> n equals a,t,g, or c

<220>
<221> misc feature
<222> (16)
<223> n equals a,t,g, or c

<220>
<221> misc feature
<222> (17)
<223> n equals a,t,g, or c

<400> 127
gatcccccgg gcncgnngaa ttcggcacga gggacagagt agttccagag gcagttctca      60
ctgtgacagc ccttcgccac aagaagatgg gcagatcatg tttgatgtgg aaatgcacac     120
cagcagggac catagctctc agtcagaaga agaagttgta gaaggagaga aggaagtcga     180
ggctttgaag aaaagtgcgg actgggtatc agactggtcc agtagacccg aaaacattcc     240
acccaaggag ttccacttca gacaccctaa acgttctgtg tctttaagca tgaggaaaag     300
tggagccatg aagaaagggg gtattttctc cgcagaattt ctgaaggtgt tcattccatc     360
tctcttcctt tctcatgttt tggctttggg gctaggcatc tatattggaa agcgactgag     420

cacaccctct gccagcacct actgagggaa aggaaaagcc cctggaaatg cgtgtgacct 480
gtgaagtggt gtattgtcac agtagcttat ttgaacttga gaccattgta agcatgaccc 540
aacctaccac cctgttttta catatccaat tccagtaact ctcaaattca atattttatt 600
caaactctgt tgaggcattt tactaacctt ataccctttt tggcctgaag acattttaga 660
atttcctaac agagtttact gttgtttaga aatttgcaag ggcttctttt ccgcaaatgc 720
caccagcaga ttataatttt gtcagcaatg ctattatctc taattagtgc caccagacta 780
gacctgtatc attcatggta taaattttac tcttgcaaca taactaccat ctctctctta 840
aaacgagatc aggttagcaa atgatgtaaa agaagcttta ttgtctagtt gtttttttc 900
ccccaagaca aaggcaagtt tccctaagtt tgagttgata gttattaaaa agaaaacaaa 960
acaaaaaaaa aaggcaaggc acaacaaaaa aatatcctgg gcaataaaaa aaatatttta 1020
aaccaaaaaa aaaaaaaaaa aagggggt 1048

<210> 128
<211> 722
<212> DNA
<213> Homo sapiens

<220>
<221> misc feature
<222> (251)
<223> n equals a,t,g, or c

<400> 128
gaattcggca cgaggaaagt ttcaaccctc tgacatgtgg gttcagctta tttttttctt 60
tgttcagtat ggagactctc ttacttctgc tttttttcct ttctcttcta atttttcgct 120
tcagaattct ggtttctcaa tgcataaact gaagtaattt cttccattct acttttctct 180
gccccaggct tgagatagaa ctagggagcc cagtgaggcc ttttctttcc taaattaaca 240
ggcatctgtg ncataaatgc tacctttgaa ctatgtgatt taagataatg tgcagaagta 300
cttctctggt ctttcaggtt gcytgcataa ctawgtactt ggttgaactt gtaattcttg 360
ctgacaacag tcctgctgtt ttccagtaag gttcgtgatc ctcgggccaa ttttgatcag 420
tccctacgtg tactgaaaca tgccaagaag gttcagcctg atgttatttc taaaacatct 480
ataatgttgg gtttaggcga gaatgatgag caagtatatg caacaatgaa aggtaaagaa 540
attgaaaaat gaaaaatctt tcccatgtaa tttgagtaat agccaggaac ccactcactt 600
tgaaggccct tctaagaaca aagaaaagta tatggttata gatggcagca tgaaaaggaa 660
accaacttgc acatgcaccc tcaaatctaa aatacaagtt aaaaaaaaaa aaaaaaactc 720
ga 722

<210> 129
<211> 477
<212> DNA
<213> Homo sapiens

<400> 129
   gaattcggca cgaggtgagt atggcttttg tcttccatct tgctcagggt actttggaac 60
   cgctatacat tgcaggagct tagcttctgg ttaccatggt ttgcttccag agcaacaagc 120
   ctagtacttc aacatggaga caattatctt ttgtttttgt tttgttttgt ttgttttgtc 180
   ttggccatgc ctttttgagt ttaccttttt atattttgtc catcattgcc atgtgtttgg 240
   agcagtgggc gttccataac atgaactcac tgtaccatca cgaatgggaa gtaaggggaa 300
   accttatcca tgtggatttt actcttccct gattccctaa attgggtttg caaaatacta 360
   ctgtgcactt tcttgatgat tcgggcttat ctttatgact gtctgtkttt gtgtcagact 420
   gtaaagaagt ataaaagtct ttagcttgaa aaaaaaaaaa aaaaaaaaaa aactcga 477

<210> 130
<211> 1296
<212> DNA
<213> Homo sapiens

<400> 130
ggcacgaggc cactggaatc tgatcctgat tgtcttccac tactaccagg ccatcaccac 60
tccgcctggg tacccacccc agggcaggaa tgatatcgcc accgtctcca tctgtragaa 120
gtgcatttac cccaagccag cccgaacaca ccactgcagc atctgcaaca ggtgtgtgct 180
gaagatggat caccactgcc cctggctaaa caattgtgtg ggccactata accatcggta 240
cttcttctct ttctgctttt tcatgactct gggctgtgtc tactgcagct atggaagttg 300
ggaccttttc cgggaggctt atgctgccat tgagaaaatg aaacagctcg acaagaacaa 360
actacaggcg gttgccaacc agacttatca ccagacccca ccacccacct tctcctttcg 420
agaaaggatg actcacaaga gtcttgtcta cctctggttc ctgtgcagtt ctgtggcact 480
tgccctgggt gccctaactg tatggcatgc tgttctcatc agtcgaggtg agactagcat 540
cgaaaggcac atcaacaaga aggagagacg tcggctacag gccaagggca gagtatttag 600
gaatccttac aactacggct gcttggacaa ctggaaggta ttcctgggtg tggatacagg 660
aaggcactgg cttactcggg tgctcttacc ttctagtcac ttgccccatg ggaatggaat 720
gagctgggag ccccctccct gggtgactgc tcactcagcc tctgtgatgg cagtgtgagc 780
tggactgtgt cagccacgac tcgagcactc attctgctcc ctatgttatt tcaagggcct 840
ccaagggcag cttttctcag aatccttgat caaaaagagc cagtgggcct gccttagggt 900
accatgcagg acaattcaag gaccagcctt tttaccactg cagaagaaag acacaatgtg 960
gagaaatctt aggactgaca tccctttact caggcaaaca gaagttccaa ccccagacta 1020
ggggtcaggc agctagctac ctaccttgcc cagtgctgac ccggacctcc tccaggatac 1080
agcactggag ttggccacca cctcttctac ttgctgtctg aaaaaacacc tgactagtac 1140
agctgagatc ttggcttctc aacagggcaa agataccagg cctgctgctg aggtcactgc 1200
cacttctcac atgctgctta agggagcaca aataaaggta ttcgattttt aaagataaaa 1260
aaaaaaaaaa aaaatttggg gggggggcc ccgtta 1296

<210> 131
<211> 738
<212> DNA
<213> Homo sapiens

<400> 131
gaattcggca cgagtgacaa gaaagacggt gtcagatgca cattaatctt tagcctgatg 60
tccttcatga tgtccaacct ccagtttcat ctcctgccac actcatcccc catacttcca 120
ctcttcacac tggccttact caaaatgcag attccaggac tcaggctatc tcactgcctt 180
cttacttaca attcttatac cagaacaccc ttcctcctcc cctcatctga atcttacctg 240
gtttttgaaa tttaagtcag ggccttctta ggaagatttc cctgattcag atccaagttg 300
aattatgata accctccttt ggctcccata aaatcttata acttcctaac tgtgttttat 360
gaatagttgt ctagtttagc actatgtcag gagctattga cagcagggct gggcacagtg 420
actcacagct gtaatcctag ccctttgaga ggacaaggtg ggaggactgt ttgaggacac 480
ctcaagccca tccagcctag gcaacagaat gagatcttgt ctgtacaaaa aaacaaaaga 540
ttaattgggc gtggtgacgt gcacctgtag tcccaactac ttgagaggct gaggcaggag 600
gattgcttga ccccaggaga tcgaggctgc agtgatccat gatggtgtca ctgcactcca 660
gtctgagcaa cagagcaaga ccccaccccc caaaaaagct attgagggta gcagtttact 720
ttcattgctc tacctcga 738

<210> 132
<211> 442
<212> DNA
<213> Homo sapiens

<220>
<221> misc feature
<222> (306)
<223> n equals a,t,g, or c

<400> 132
gaattcggca cgagtgaccc agaagggtga gtcagttggt agtgtggggt gcatgagggc 60
cattgcaggt tttgataatt accctttatt ttaatttgat catacttttt tgtttataac 120
cttattctaa aaataattca aggtgaccat gcttccatta tacttcttgc aaccatacct 180

atctttggtg atatttatta tgttaaggga caattggcat cttttggccc ttacctgtag 240
ctattctatc atctggagat tatctccaga cacaaatcca tcgcccattg ctccatcgag 300
gcacantcag ctckttgtag ttgccattgc ccctctcgag ccttctccac atagccacat 360
gcaatccatt cccaaaaacc tagctcaatt ttcctcatca cagatgtttt ccctgaccct 420
ccagttggta tatatctcct cc 442

<210> 133
<211> 882
<212> DNA
<213> Homo sapiens

<220>
<221> misc feature
<222> (881)
<223> n equals a,t,g, or c

<400> 133
gaattcggca cgagatgttt tcttcactca aaaaatttta tattctcaaa catgtatatt 60
ctttccctgt cttgttccat tttcttttct tttttctttt ttctttttcc tttctttcgt 120
gggctgagaa aggggcaggc aaaatgaagc tggccactga aaactgtaag atggtcaaaa 180
gctgacagcc tgtgtatgtg aaaagggaat tgtaaatgga ctgcaatgta atgtacactg 240
taatttgaat acaattactg tatctaaaag gagctgctat gaagtacctt tcttatgttg 300
ctaggctact gtttctgaaa gccctggatc tctttgcacc aaaaatggtc cagatagact 360
ctttttaagg atcttggctg ctttttacta gaaggttgct tttatgagca tatttatact 420
gctgaaggat gagtgttaat tttaattaac tttgccgttt tgtagagaaa actattccac 480
aagataaatt ccaagtcttt tcacctgtca ggcatgcata ttttaatatc tgtttggata 540
gtcagaagta gaatcataaa ggtaaaatat gagttgttac tttgtttctt cgatgtcata 600
ttttatgtgt aatatatatg taaagggcca ttcttaagtt ctctccttaa acttaatgct 660
gtcaagtgtt agatgtgtgc atgtgaactt gttgcactgc agaaacatat tcagagttta 720
tctatgtaac ttattcactc tgtaaataca tttaaagttt ttgtgatgta agcttaattg 780
atattctgtt cagaacttty tttagwctaa araaagttct gaacagaata tcaattaagc 840
ttacattgat attctgttca gaactttctt tagctagaaa na 882

<210> 134
<211> 1032
<212> DNA
<213> Homo sapiens

<220>
<221> misc feature
<222> (5)
<223> n equals a,t,g, or c

<220>
<221> misc feature
<222> (593)
<223> n equals a,t,g, or c

<400> 134
ggcanaggga accaccttct gtagaacatt caaccaggcc cagatccaga aggcttgagg 60
ccctgtggtc cccatccttg gggagaagtc agctccagca ccmatgaagg gcatcctcgt 120
tgctggtatc actgcagtgc ttgttgcagc tgtagaatyt ytgagctgcg tgcagtgtaa 180
ttcatgggaa aaatcctgtg tcaacagcat tgcctctgaa tgtccctcac atgccaacac 240
cagctgtatc agctcctcag ccagctcctc tctagagaca ccagtcagat tataccagaa 300
tatgttctgc tcagcggaga actgcagtga ggagacacac attacagcct tcactgtcca 360
cgtgtctgct gaagaacact ttcattttgt aagccagtgc tgccaaggaa aggaatgcag 420
caacaccagc gatgccctgg accctccccc tgaagaacgt gtccagcaac gcagagtgcc 480
ctgcttgtta tgaatctaat ggaactttcc tgtcatggga agccctggaa atgctatgaa 540

gaagaacagt gtgtccttcy tagttgcaga acttaagaat gacattgagt ctnaagagtc 600
tcgtgctgaa aggctgttcc caacgtcagt aacgccacct gtcagttcct gtctggtgaa 660
aacaagactc ttggaggagt catctttcga aagtttgagt gtgcaaatgt aaacagctta 720
acccccacgt ctgcaccaac cacttcccac aacgtgggct ccaaagcttc cctctacctc 780
ttggcccttg ccagcctcct tcttcgggga ctgctgccct gaggtcctgg ggctgcactt 840
tgcccagcac cccatttctg cttctctgag gtccagagca tcccctgcgg tgctgacacc 900
ctctttccct gctctgcccc gtttaactgc ccagtaagtg ggagtcacag gtctccaggc 960
aatgccgaca gctgccttgt tcttcattat taaagcactg gttcattcac tgaaaaaaaa 1020
aaaaaaaaaa aa 1032

<210> 135
<211> 1766
<212> DNA
<213> Homo sapiens

<400> 135
gtkattcaaa gccatcacaa aacactataa gactgaccaa aatttagata acctttgaac 60
cacgattttt ttccacatct gtctgtgaga cacagcgcaa tgctactgcc cttccagaaa 120
ctgtgctaaa aagagaaagt ccaaaagact ctaaacaaaa acctcgacgc cgttgaggat 180
gtgtttcatt ctggtggtct gttttgcaag cttgataaca gaatgtccgt gccattgtaa 240
atgttgtaga gatgtgggcc gtggcccaac cgtcctatat gagatgtagc atggtacaga 300
acaaactgct tacacaggtc tcactagtta gaaacctgtg ggccatggag gtcagacatc 360
catcttgtcc atctataggc aagaagtgtt tccagatcct ttggaaaggt gggcatgggg 420
caggtgcttg gagagtggcg tttgagccag agcgacccca tttcccgtgt gaaccatagg 480
cacaacccag gaagtttccc cacttgtagg agtgtgggta ttccagagca agactgtggc 540
caccatcttc ccctcttggt gttttccgaa agtgacagtg ttggtcatcc catgaccact 600
gaagcttagt aaccagcgcc aaaaagtaga ttcatcaaac tagagacccc agctcccctt 660
ctcgccatct tctttctcaa gttgaccgtg gtgctgtttc tggaaggcat ctgcaactcc 720
aagtccatgc agaactctgg aaggccaagt tcatcgcagc atgttcacca tatcccagcc 780
tccaaatcta tcctcctacc ttccaacgca tgacctgttg gggagcagag acttaacccc 840
caactcagag gaacccttcc tccagcgtct ttggcatggt ttctagggtg agagttccca 900
atttggatag aacggccacc atattggtta ctgaatctct ctcccttgtt tttattacgt 960
ttcctttttc aaactgtcca tgggaaggct gaattgagtg actccccaga atgaagatga 1020
gaaggtgaat ataatcaatg ccaatgtaat gccagcgggg tgagatgccc gatggagrtt 1080
tcaaagatgt agctagcatt ttgaaaccat atgggcaaaa cccggcaacc agaaggggac 1140
agataaggac cgttccagaa atcccaactc tcacacccag cccaggctgc agtctccaca 1200
ccaaacagtc aacaaaacac aaaccctgaa ggaaaacctt ttccatacac ccaggctatg 1260
cattgaagag ttttccactg tatacatttt tatccagatg aaggtatttt tatattttga 1320
caataggaaa cagtgaccat tttcagagta atcaaatctg gaacaaatga aacatctttt 1380
agccaccacc accctgttgc aattaagaca accgtggggg aacacaccac tttttactgt 1440
tgaaaccaac acaacgttga aatccaggct tatacgcaga ctccgattcc ctagagaact 1500
aaatttggct ttagtgtgac gggatttgat taagcactta gtatagtctt ttgaacacgg 1560
aaatcctgtt gtacttaaag ctagcggacc cgtgaacaac tttgtcaggt tcacgtccta 1620
taacggttma aaracacaca cacacataca caaaccgttt ctatgagaga ttgatgaact 1680
ttgtttaaaa ttttaaaaaa aggaacacgt tctgtaaacg agtcgctaaa tacagaattg 1740
tataataaaa aaaaaaaaaa aaaawt 1766

<210> 136
<211> 470
<212> DNA
<213> Homo sapiens

<220>
<221> misc feature
<222> (315)
<223> n equals a,t,g, or c

<400> 136

ccgccgccgc cgctacagcg accctgaccg ccgtccgagc cgccagacac ccagagagac 60
gccagaggcc gcggaggggc gaagacccgg agtaactctc ccttccaccc caacccggat 120
cgccagccct cgagagctct gtgctccacg ccgaggatgc accgtctctg gattggtccg 180
gccttcttcc taatgacatc gctcagcgtc tctggagccg tcatcccgcg gaatgggggc 240
ccagggggtg tcagytcggg gccttgcctc ttgcagctac tctgtggtca ggccgggtcc 300
tccaccatca ggaanatccc atcctgagct ctgtctcctg cccctcctgc tgtgggatgc 360
tgagcacaga gcccacagcc catctgcctc ttcacctccc tgaatccgtg tccatctgca 420
ataaacgaca gcctcggctg cctcgtgctg aaaaaaaaaa aaaaaaaaaa 470

<210> 137
<211> 1168
<212> DNA
<213> Homo sapiens

<220>
<221> misc feature
<222> (1163)
<223> n equals a,t,g, or c

<400> 137
ctggatctat agactcttct tgccctaaag aatggcatgt ttgcactcct tcccccaaca 60
tgtacagatg cctgtcactc ttggtgactt tgctgggctt ctagtccctg cagatgttta 120
agggagcaat gaatggggag tgtggatgca aactacggcc tccttggcac tgtttcagat 180
gggggatttc ccttctctag gagaacctgt gctggaaaag gtgtggcacc cacactgaaa 240
tggggcaagc tcttcccagc tttgtggggg cccttggaaa acatccactg agatggaggc 300
agtcttcttc ctcttcttcc tcctgctcct cttgacctgg accagcaaga tagcaccaat 360
ccttttctcc tgatggcagt atctgaatga ctttcacagc tgaaggccag agaccagcct 420
acagctggga ttcaggcttc aaagctttgg tgaggatgac tccagaacca ggcaggtagt 480
ccccctccag gatgccatgg cctaaagcat ttcactcctc agtcactagg ctgtgaactc 540
attgtggctg acacttttat tcgctgctat gttttttagc aatgcccggc acacagacct 600
gcttactatg cttttgctga gtgagtgaag ggataagtcc ctttctgcct ttttgatact 660
cactttggtg ccccttgagg tcacagagac ctggatttga cttctggctc tgccacacaa 720
gagcacggat gctttgggtc agttacttca gctctgagag gctcaattgc ctcacctgtg 780
aaatgggtta gtgattccag gaatcttacc aggccccatg gacagcatgt acataaagag 840
cctagccctt ccctctcctc cctctccagg ggccaggcct gactcccctg aagccatttc 900
cttaccattt tgatccctaa gcctgttatc agatcttctt tctgatctac caccatggct 960
caaatcttgc ccttcatcct tgcctttctc aaagacaaaa acacccttcc tctgctccac 1020
tcagagtgta gcggggaggc ttatactgca gtggttaaga gcatatccct ggaattggaa 1080
ggaacagggt ctaagattat gtagatatag cacaaagcct tgctcctgct cgtgccgaat 1140
tcgatatcaa gcttatcgat acngtcga 1168

<210> 138
<211> 1294
<212> DNA
<213> Homo sapiens

<400> 138
gatcttgtcc aagcagtcgg ggctacttcc aagaatgtca gctcctgtta gcaaccagtg 60
gagtctggcc ttgggctcta agttgacctc tctatagctc caaatcctac caatctcaga 120
aaactgtaag aggcacagat gactccacca gctgcagagt gactctgaag agagtcttca 180
cttactgcac aggcaaagaa aggcacagga atatttccta cctctggcac gaggtgagtc 240
ccacctcccc ccacccccat ctccaggagg caggtagagc agttctgacc gagaggatag 300
actgctgttg ctgtctttcc ccagctctga actagtttta aggtagctta ggatgaaaaa 360
tggagaatga ttgggggttc caaaccactt tcttctccct tggcttatat ctcttcacca 420
tttggtggtc aactgtgggc ctaccctgga cctcatctac tcagcgagaa ttggacatga 480
agctagaggc agctgccttg gaagggaart tcaggctcac ttggacagcc caggccatgg 540
caggaagaat cccttcctct tggggtcctt gatgggcatg tgtgatgggg aaggagcagt 600
ctcccagccc tgggtctgct ccccacatct ctcctaattc cacttcacct tttgccaccc 660

cctccccacc agaggcctag cccttttgtc accgaaggcc cccagagtgt ttctgtgtga 720
aaccctctca tttacactgt ggcmwcaaaa atccacaaaa gatggattaa ttgcactctg 780
gttaatagca gcagcacaat gattaaaatc tatattccta tcttctctag caccctggtg 840
tggggatggg gcggaagggt gtcttgaggg gcagggagga ccccataaaa caatccctcc 900
tgcattctca ggctaaatag ggcccccagt gactacctgt tcttggctgt cccctctgaa 960
gagctctgcc ttctcacagc caccaccagt tgccccactc ccaggaaaac agcacatgtt 1020
cttcttctcc tgccttgaga ctgcgtgtta gtcttccatt cataactcat cagcagctca 1080
gtccttctta tgtctagtct cagttcattc agccaaagct catttttgtc ctatccaaag 1140
tagaaagggt tcttttagaa aacttgaaga atgtgcctcc tcttagcatc tgtttctgac 1200
tcccagttat ttttaaaata aatgatgaat aaaatgcctg ccctgaaggg ttctggagga 1260
aaaaaaaaaa aaaaaaaaaa aaaaaaaact cgta 1294

<210> 139
<211> 1720
<212> DNA
<213> Homo sapiens

<400> 139
aaccagaagt ggacgtgcat gacagtggac ctagaggctg acaaacagga ctacccgcag 60
ccctcggacc tgtccacctt tgtaaacgag accaaattca gttcacccac tgaggagttg 120
gattacagaa actcctatga aattgaatat atggagaaaa ttggctcctc cttacctcag 180
gacgacgatg ccccgaagaa gcaggccttg taccttatgt ttgacacttc tcaggagagc 240
cctgtcaagt catctcccgt ccgcatgtca gagtccccga cgccgtgttc agggtcaagt 300
tttgaagaga ctgaagccct tgtgaacact gctgcgaaaa accagcatcc tgtcccacga 360
ggactggccc ctacccaaga gtcacacttg caggtgccag agaaatcctc ccagaaggag 420
ctggaggcca tgggcttggg caccccttca gaagcgattg aaattagaga ggctgctcac 480
ccaacagacg tctccatctc caaaacagcc ttgtwctccc gcatcaggac cactgaggtg 540
gagaaacctg caggccttct gttccagcag cccgaacttg gactctgccc tccagatcgc 600
cagagcagag atcataacca aggasagaga ggtctcagaa tggaaagata aatatgaaga 660
aagcaggcgg gaagtgatgg aaatgaggaa aatcagtggc cgagtatgag aagaccatcg 720
ctcagatgat agaggacgaa cagagagaga agtcagtctc ccaccagacg gtgcagcagc 780
tggttctgga gaaggagcaa gccctggccg acctgaactc cgtggagaag tctctggccg 840
acctcttcag aagatatgag aagatgaagg aggtcctaga aggcttccgc aagaatgaag 900
aggtgttgaa gagatgtgcg caggagtacc tgtcccgggt gaagaaggag gagcagaggt 960
accaggccct gaaggtgcac gcggaggaga aactggacag ggccaatgct gagattgctc 1020
aggttcgagg caaggcccag caggagcaag ccgcccacca ggccagcctg cggaaggagc 1080
agctgcgagt ggagcgccct ggaaaggacg ctggagcaga agaataaaga aatagaagaa 1140
ctcaccaaga tttgtgacga actgattgcc aaaatgggga aaagctaact ctgaaccgaa 1200
tgttttggac ttaactgttg cgtgcaatat gaccgtcggc acactgctgt tcctccagtt 1260
ccatggacag gttctgtttt cacttttttg tatgcactac tgtatttcct ttctaaataa 1320
aattgatttg attgtatgca gtactaagga gactatcaga atttcttgct attggtttgc 1380
attttcctag tataattcat agcaagttga cctcagagtt cctgtatcag ggagattgtc 1440
tgattctcta ataaaagaca cattgctgac cttggccttg ccctttgtac acaagttccc 1500
cagggtgagc agcttttgga tttaatatga acatgtacag cgtgcatagg gactcttgcc 1560
ttaaggagtg taaacttgat ctgcatttgc tgatttgttt ttaaaaaaac aagaaatgca 1620
tgtttcaaat aaaattctct attgtaaata aaattttttc tttggatctt ggcaaaaaaa 1680
aaaaaaaaaa aaaaaaaaaa aaaaaaaaaa aaaaaaattc 1720

<210> 140
<211> 774
<212> DNA
<213> Homo sapiens

<220>
<221> misc feature
<222> (697)
<223> n equals a,t,g, or c

<220>
<221> misc feature
<222> (709)
<223> n equals a,t,g, or c

<220>
<221> misc feature
<222> (716)
<223> n equals a,t,g, or c

<220>
<221> misc feature
<222> (733)
<223> n equals a,t,g, or c

<400> 140
cggcacgagt tttgggatgc ctcttactct gccaagccgc ttagcgggag ggaacgtgtt 60
cctgatcatc tttaccccag gcttctgtcc ggggcgtgtc aatgtagaaa tcccccagcg 120
aatgttggat gaatgaatga agttgaagag agggtaggcg gggaacgagg atgaggggga 180
cggctggaga agaggtatgg gaggttcgat gtttcaggga tggcacccaa ggggggacat 240
tcgaggcagc accggtagca cttcctttgc gatgaggggc gtctctttgg acttcttgga 300
aaagaggtgg gcattggaaa ccagggtctg ggaacaaacc gtggtttgga cataacattt 360
gttaccttca cttttctggg agttggagaa gtagaggagg aagttcagac aatttcataa 420
gtgtctaaaa agagacagtt atgcgaccat tgacgaggag taaaagtcgt ctattgagca 480
tcttattcac tacaaataga agaaagaaat accagtttcc tgacaagccc caccccatgc 540
ttggccagtt cctgagtaca cttaatatat tttaggtact gtcatcaaac tcaaagctcg 600
ctgtcagcct caaaggtctg aaccctagta tagattcttg tagcttgctt gaagttacag 660
tgggtcatga tcaggaattg atgctttgtt tttgttntga aacggagtnt cgccantgca 720
ctccagcctg ggngacagag cccgagactc cttctcaaaa aaaaaaaaaa aaaa 774

<210> 141
<211> 1566
<212> DNA
<213> Homo sapiens

<220>
<221> misc feature
<222> (415)
<223> n equals a,t,g, or c

<220>
<221> misc feature
<222> (718)
<223> n equals a,t,g, or c

<220>
<221> misc feature
<222> (1116)
<223> n equals a,t,g, or c

<220>
<221> misc feature
<222> (1122)
<223> n equals a,t,g, or c

<220>
<221> misc feature
<222> (1127)
<223> n equals a,t,g, or c

<220>
<221> misc feature
<222> (1312)
<223> n equals a,t,g, or c

<220>
<221> misc feature
<222> (1373)
<223> n equals a,t,g, or c

<220>
<221> misc feature
<222> (1455)
<223> n equals a,t,g, or c

<220>
<221> misc feature
<222> (1456)
<223> n equals a,t,g, or c

<220>
<221> misc feature
<222> (1540)
<223> n equals a,t,g, or c

<400> 141
ggcacgagac tatcctcaag gagcttacat atcagtaaat aaattattaa aggtggaaaa 60
tgtggtaaaa gagacataat gtctcggaga gagaacaaat ttctgcttta ggagtgttct 120
tagttaaggt aacattagct tctataatac gcacactccc aaatctcagt atttcaacat 180
gagtttctct cttgctcatg taaagactgg tcagggaccc aggttgacag aggctcttca 240
gtacatagct tccaagattg ctgtgggtgt gacatccagc cagaaatctg gtgaagagag 300
agcaatgatt acacaggaac ttttaatgga ccaggcctgg gacagcgtat gtcacttcca 360
ccaacatccc actcaccaga atttggtcac agggccatag ctatctgcag agaangctgg 420
gaaatggaac ttagctatgt gctcaagagg aaaagtaaaa cagttattga ataattagta 480
ataattagca agtaactacc taggggtcac agaggacctc tcaggtagaa tttagactta 540
aagatgatgg gggagtgtgt ggaagatggg tgcagaatag ggaaaggggg gattgaagga 600
agaacaagct ctagcttcac ctgcatgggt agagcccaca gtgttggtag ggacatgtta 660
gctttcaaca tcagcttctt aacagtatta ttctttcatc ggaggaaatt agtctatntc 720
tgaggaaaaa aaaatctgca atacgtagca atttacttac ttggatattg aatgttaaag 780
cagagagaga ctttgtcctc aaaaccctcc catttcagaa gtgaggagcc tggggaggtc 840
atgctctctg gatgtcacac agtgagtcac tgtcaaagcc agaatagaac ccagacctct 900
cagtttccca ttccagtgct ctttctatga ggaaagtata agtttgagca tttttaaacc 960
ttaattatgt agaaataacc atgatatttt atcgtaaatt atttcagtca tctcatttta 1020
aattttactc caaactaaag gaaaacggta ctgatttaaa acatctatca taattcaata 1080
tagcccatat ttcttcttta ggaaaaattt tttttngttt tntatcntga agacccgtgc 1140
cctcttcctg tgtctcatgt agacatttca cagtccaaat atacagagca agaatagatg 1200
aaatcaacat gtttaccatt attctatcta aattttcaaa gaaaaaggga acaaaaggtg 1260
agtgatgact gagttgcatg gctataattg agtttttgtt gcttttattt tnataatatt 1320
ttaattgaca tagatgctta aatgtatatc aaaatgcatg tcacagctct tgnacaaaga 1380
taaatttgac tctagagcac attttcttta gtgagaatga taaattatct cagagcttgt 1440
gattctctac ttttnnaaat cataaggtca gttctttaat taaaagataa agaaaagtag 1500
gcattgtcca tgtagtgaaa tcacttttat caggataatn tagtaaccaa aaaaaaaaaa 1560
aaaaaa 1566

<210> 142
<211> 1384
<212> DNA
<213> Homo sapiens

<400> 142
tcgacccacg cgtccggccg gactaaccag ctcctccagg cgctgggggc gggtgtggca 60
ggaggaagcc cgatcagccc caggctgtgg atgtgggaga agggcgagct caggggggcca 120
tcatggggtt cccccagagg caacctggcc tatcagggct gctcctcctc gtgtgggcac 180
tggcctggcc cctgccttgt atgagcttgg agctgatccc ctacacacca cagataacag 240
cttgggacct agaagggaag gtcacagcca ccacgttctc cctggagcag cctcgctgtg 300
tcctggacgg gcttgmcggc gttgccagca ccatctggct ggtggtggcc ttcagcaacg 360
cctccagaga cttccagaac ccacagacgc gagctgagat cccagccttc ccacggctgc 420
tgacggaggg gcactatatg acactgcccc tgtccctgga ccagctgccc tgtcaggacc 480
ccgcaggcgg cggcagggac gtccccttgc tgcgggtggg caatgacccc ggctgccttg 540
ctgacctcct ccagccgccc tactgcaaca gccccctccc cagccccgga ccttacaggg 600
tgaagttcct cctgatggac gccaggggct caccccaggc cgagaccagg tggtccgacc 660
ccatcgctct tcaccaaggg aagtcgccag cctccatcga cacgtggcca gggcgamgca 720
gtggtggtat gatcgtcatc acctctatcc tctcctccct ggccagcctc ctgctcctgg 780
ccttcctggc agcgtccacc scacgcttct ccagcctgtg gtggccggag gargccccgg 840
agcagctgag aattggctcc ttcatgggga agcgctacat gacccaccac atcccaccca 900
gcgaagccgc caccctgccc gtgggctgtg agcctggcyt ggaccccytc cccagcctca 960
gcccctagcc tggcccttgt ggctggggcg tgtgtggctg tggccagtgt gggggcaagg 1020
acgtggtagt tattcccagc ccctgcaccc tcctcctcac ccctgccama gtcccactga 1080
tgtaggacag atgtcagggt tctagacgtc tttggtgcaa aaggggggtt ttattcaagc 1140
acagggacag gacccatggg cagggagagc ggcaccgggg tggtgaggag tggcccgtta 1200
tatatacttt cgagttggga gggcttagag agagcgtaag tctctaagga attttggaag 1260
caaggtctcc agggtcctga gggggctagc tgttgttagg aaaaggtcat ttattactgt 1320
ttagtaaaaa ctttcacgag aaaaaaaaaa aaaaaaaaaa aaaaaaaaaa aaaaaagggc 1380
ggcc 1384

<210> 143
<211> 537
<212> DNA
<213> Homo sapiens

<220>
<221> misc feature
<222> (429)
<223> n equals a,t,g, or c

<220>
<221> misc feature
<222> (502)
<223> n equals a,t,g, or c

<220>
<221> misc feature
<222> (520)
<223> n equals a,t,g, or c

<400> 143
gccccccaaa aagaaaggta attaattata gttcatttcg ttttaactaa gagttactaa 60
agcatccctg gatgctgaga ggtactctct aggaggcaga aacaggacca agcactgccc 120
acttatctcc acactatgct accaattcac ctgcagtggg catgtgcttt caggagtttt 180
ttgcttggta tagacagttc tatgttcgtc ttgtttcagc accctcgttt gaaggacaca 240
aagagctcta gggtcataga accaactctc actaactgac acagatatca ggatccaacc 300
catgcccaca gtattacccc aagtctctaa ctagctggtg taaccaataa tggaaagaaa 360
aaaagtaata ttctgttctt caacttcaac agagaataat agtgaaagaa tggtgatatt 420
tttcctaana tggactaaca agtatcctga gttgggaggt gacttccaat agtaaacaat 480
aaaataactg agaaaatgga gngaggaggg aggggagagn gagagtgggc acagaag 537

<210> 144
<211> 680
<212> DNA
<213> Homo sapiens

<400> 144
aatttttgt atttttagt agagacaggg tttcaccatg ttagccagga tggtctcgat 60
ctcctgacct cgtgatccac ctgccttggg ctcccaaagt gctgggatta caggcgtgag 120
cmaccacacc cggccaatca tattttttct tgttactaat tagaatcatg attctcctgg 180
cattcttcat tttgttatac ctcacttcct tttccttagc aagatctttg ccatagagta 240
tggaaaccag gttccttgcc agttaatctg tattgtgctt tgtcatgtat tgttactaaa 300
cagctcaaga tcaaggggaa gaaatgtata tgaggctcag ttcatgttca gttttttttt 360
tttcagcatt gcaacattgc cactcatcat catgagtgta gccctgtgtc aggtactgaa 420
ggtaatggaa aaggtatata aggttgatcc ctgtactctt gttgggaact tgagtggtat 480
gaatagagaa ggtgagttct tggggacaga ggctacagtt tagcaagctt tcctatgcgg 540
accttggtaa tttctttaca ttttatagac caaagaacaa tcttaacttg cccttttttc 600
taaaggcatt gtttaaaaac tgtcatcaaa tcattgcagt ttatggcaaa tggccttttt 660
ttaaaaaaaa aaaaaaaaaa 680

<210> 145
<211> 1048
<212> DNA
<213> Homo sapiens

<220>
<221> misc feature
<222> (79)
<223> n equals a,t,g, or c

<220>
<221> misc feature
<222> (117)
<223> n equals a,t,g, or c

<220>
<221> misc feature
<222> (138)
<223> n equals a,t,g, or c

<220>
<221> misc feature
<222> (144)
<223> n equals a,t,g, or c

<220>
<221> misc feature
<222> (147)
<223> n equals a,t,g, or c

<220>
<221> misc feature
<222> (625)
<223> n equals a,t,g, or c

<400> 145
   ggcacgagag aaagtgggcc cttaccaggc accaaatctg ccagcactct gatcttggac 60
   ttccagcctc ctgaactgnt gtctgcattc aaaagaacat tctattaaag ctacctnaat 120
   ttggcgctta tttttctnaa tcangtntct gacaatcata ttgtgtggaa tggttgctgc 180
   tttaagtgca ataagagcta actgccatca agagccatca gtatgttctt caagctgcat 240
   gcccagaaag ctggattggt tttcaaagaa agtgtttcta tttttctgat gacaccaaga 300
   actggacatc aagtcagagg ttttgtgact cacaagatgc tgatcttgct caggttgaaa 360
   gcttccagga actggtaaga aaatagttct ggccagaatc aaagattcag ccctacaagg 420
   atatgttttc ctgtgaaatt atctaagaga atttcctgtt gagatataaa ggcccatctg 480
   atcactggat tgggctgagc agagaacaag gccaaccatg gaaatggata aatggtactg 540
   aatggacaag acagttagtc atgaaagaag atggtgccaa cttgtatgtt gcaaaggttt 600
   cacaagttcc tcgaatgaat ccaanactgt catgggtctt actctgttac ccaggctgga 660
   gtgcagtart taccatcgtg gctcactgca gccttgactt ccctggctcc aagtgagcct 720
   cccatctcag gctcctgagt agctgggact acaggtttcc tatcctggga gcaggagagt 780
   gtgcctattt gaatgacaaa ggtgccagta gtgccaggca ctacacagag aggaagtgga 840
   tttgttccaa atcagatata catgtctaga tgttacagca aagccccaac taatctttag 900
   aagcatattg gaactgataa ctccatttta aaatgagcaa agaatttatt tcttatacca 960
   acaggtatat gaaaatatgc tcaatatcac taataactgg gaaaatacaa atcaaaatca 1020
   tagtaaaata aaaaaaaaaa aaaaaaaa 1048

<210> 146
<211> 930
<212> DNA
<213> Homo sapiens

<400> 146
tcgagttttt tttttttttt ttttggatat ggagtctcac tctgttgccc aggctggagt 60
gctgtggtac gatttcagct caatgcaagc tctgcctcct gggtttaagc aattctcctg 120
cctcagcctc cccagtaggt gggactacgg gtgtgcaaca caacatccgg ctaatttttg 180
tatttttcgt agagacaggg tttcacatgt tggccaggct ggtcttaaac tcctgacctc 240
agttgatcca cctgcctggg cctcccaaag tgctgggatt acaggcaaaa gccactgtgc 300
ccagctgcat tgttgctgtt ttttattgtt agttaagaga gaccaaccat tagaaaaatg 360
tttaaggctt ttcaaaggaa gaatcctatg taggcagccc cactacaggt tactttctga 420
tgaatgtcca ggactattac aaaatccatg attgtggaaa ttctgtcaaa agagatgaca 480
gagaaatctt gcctttggtc acaatcctgt ctgaccccaa caaaagctaa ggaaatccta 540
atcaggtgtg actcatgata aagaaaaaca tgcatccaaa ttttggttca gaagtacaga 600
aagtgtgcaa cttctgtcaa gttaattaat gtatttgctc cataactccc cgacatataa 660
ggtaagttgg ttggagtatg tggtttgaag gctgctttca aagatttaac gtctttgatt 720
tttttagtca ccatgggtgc caggatagaa taagatctgg agactttcga ataactgctt 780
acagatgtag ataattataa attgatacta ataaagaatg aagatctcag cattccccag 840
agagggctat ttttagaaaa aggaaatagc caaaaacaaa gtaaaacaaa aaacatcatg 900
ggatatcagg acttagctcg tgccgaattc 930

<210> 147
<211> 830
<212> DNA
<213> Homo sapiens

<400> 147
ggtcgaccca cgcgtccgct gaaaggaaaa gcactgtttg gagaatgatc cacctttcaa 60
gattttactt attgttgata atgctcccac atgtcctctt ttttacgggt gatcttcatt 120
cctaatatca aagtgatatt tcttcctcca ggcaccacct ctttgatcca cacaatggat 180
caaggagtta tagcagcttt taagttctac tacctgagaa gggaggactt ttgcccagtc 240
ccatactgca gtggaggaag acactgagaa gactctgatg aaattctgaa cagcatcaag 300
aaccttgttt aggcttggat tatgtcgcta aggactgtag gaatggcacc tggaagaaga 360
cacgcaagag gtttgtcaat aacttcaaag gatttgccaa ggatgaggaa gttgcaaaaa 420
tcaagaaggc tgtggttgag atggcaaaca actttaacct gggtgtggat gtggatgaca 480
ttgagtaatt cctagagggg gttcctgagg aattgactaa tgggttgctg ttggaactgg 540
aataggagtg catagctgaa gaagaggtaa agaaaaagaa agtgcaggag aagggaaaaa 600
agaactccca agaatactca cagtgatggg tttagcagaa gcttcttcag actccaacaa 660
gctccttaag aagtctgaaa acatggaccc caaaactgaa aggttttcac taatagagag 720
gaaagttcat ggtgcattat ctgcctacaa gcaaaaccag gattcaaaaa accctttgag 780
ctggagcttc aaagcacaaa aaaaaaaaaa aaaaaaaaaa aagggcggcc 830

<210> 148
<211> 865
<212> DNA
<213> Homo sapiens

<220>
<221> misc feature
<222> (321)
<223> n equals a,t,g, or c

<220>
<221> misc feature
<222> (409)
<223> n equals a,t,g, or c

<400> 148
   ggtcgaccca cgcgtccgga gtagcagaaa tttgtcttct tacaagtagt ctatgagagt 60
   agatgctgat tttctaaatg taagggaata agaaaaactt catgattaac ttttttcacg 120
   tgtatttggg ctttggtggg agtaggcaag aagaatatcc gtggatttat agagtaagca 180
   aaagtatgtc aggaaaaact aggaagaata gggctgaact gtggcttgat ttcaggagag 240
   tttctgggat tcagacttga attaactgaa tgatgctgta atgtataagt gttggtatag 300
   gtgattttat gcaaagaaga ntaaacattg gcttactttt attatcgtat acggtatggg 360
   tgactactgc tgctagttca aggtctkgat tttttaaaaa tgtgtttcnt gactgtggta 420
   gctgggagcc ccaggataca gacttttggt taaataacat ctgctccact ctgccttccc 480
   gtgtgggcct ctctaaccct gggccaagca gttgagtctc tcctcggggt gcctggagtg 540
   agggtggata cagcttgggt aattcagcat ctgtacctaa aaacttactc aaagtaggct 600
   tcatgtaaag aagtcagtgg ttcttgggaa caggggtgag tgaatggagg cgaaaggtgg 660
   ggccctccac aggtcagtca ggccctcagg gtgggacaag agctgtaggg ctcttggtta 720
   taaacctgtg tggtggagac cagcaggtga gccaaactct tctttattat cagaacattt 780
   cactaataag ggatctcaag ggtcatctgg catcagcact ttaaccaata aaaaaaaaaa 840
   aaaaaaaaaa aaaaaaaggg cggcc 865

<210> 149
<211> 545
<212> DNA
<213> Homo sapiens

<400> 149
agccagggtt ctagtcattt aagatgyacc tgaataaaac aaagagcctt actctcctag 60
aacttgtgtt tctacctggg gagactgtca gtaaaccatc cacaaaataa atacagcaga 120
tgctgttaga agatgatggt gctatggtgt gctgtggaaa atagagaaag tagagggaag 180
tgagagggat tgcgtacact aggattgtga ctttacacag aagggtcagt ggtgccattt 240
tagcaaagat ctgagagagg taaaggaata agctttgcag aagtgtggga gacaaatgtt 300
ccaggtacag gaaatgacca acgccaagac cctagggtgg caatgtgtct gcttkgagtt 360
ctagagaarg ggtatattat acatcgcttt ttgtgactca ctttttggca aacattatgc 420
tctaaaatga acctgtattt tggaatawat ckgtggttca ttaattctca tctttgtaca 480
gtattctatt atataaatac accatgattt atttagtcaa ttaaaaaaaa aaaaaaaaaa 540
ctcga 545

<210> 150
<211> 54
<212> PRT
<213> Homo sapiens

<220>
<221> misc feature

<222> (54)
<223> Xaa equals stop translation

<400> 150
Met Gln Leu Leu Cys Ser Pro Tyr Pro Glu Glu Lys Pro Lys Gly Ser
1               5                   10                  15

Asn Arg Asn Phe Cys Asn Trp Phe Leu Ser Glu Arg Ser Ser Cys Leu
            20                  25                  30

Gln Met Leu Leu Lys Gly His Lys Lys Leu Glu Leu Glu Lys Ile Asp
        35                  40                  45

Glu Ser Ala Gly Val Xaa
    50


<210> 151
<211> 46
<212> PRT
<213> Homo sapiens

<220>
<221> misc feature
<222> (46)
<223> Xaa equals stop translation

<400> 151
Met Ser Asn Leu Met Val Ala Met Ile Ala Val Ile Thr Ile Ala Val
1               5                   10                  15

Ser Ile Pro Ser Thr Arg Ala Asp Thr Glu Ile Ser Tyr Thr Tyr Trp
            20                  25                  30

Ala Tyr Leu Ser Ile Leu Ala Gly Asn Asn Ala Trp Ile Xaa
        35                  40                  45


<210> 152
<211> 25
<212> PRT
<213> Homo sapiens

<220>
<221> misc feature
<222> (25)
<223> Xaa equals stop translation

<400> 152
Met Ile Met Glu Glu Ile Phe Leu Asn Leu Ile Lys Asn Ile Tyr Lys
1               5                   10                  15

Ser Pro Tyr Ser Gln Cys Asn Thr Xaa
            20                  25


<210> 153
<211> 265
<212> PRT
<213> Homo sapiens

<220>
<221> misc feature
<222> (71)
<223> Xaa equals any one of the naturally occurring L-amino acids

<220>
<221> misc feature
<222> (80)
<223> Xaa equals any one of the naturally occurring L-amino acids

<220>
<221> misc feature
<222> (86)
<223> Xaa equals any one of the naturally occurring L-amino acids

<220>
<221> misc feature
<222> (93)
<223> Xaa equals any one of the naturally occurring L-amino acids

<220>
<221> misc feature
<222> (95)
<223> Xaa equals any one of the naturally occurring L-amino acids

<220>
<221> misc feature
<222> (133)
<223> Xaa equals any one of the naturally occurring L-amino acids

<220>
<221> misc feature
<222> (157)
<223> Xaa equals any one of the naturally occurring L-amino acids

<220>
<221> misc feature
<222> (183)
<223> Xaa equals any one of the naturally occurring L-amino acids

<220>
<221> misc feature
<222> (204)
<223> Xaa equals any one of the naturally occurring L-amino acids

<220>
<221> misc feature
<222> (265)
<223> Xaa equals stop translation

<400> 153
Met Ala Thr Pro Leu Pro Pro Pro Ser Pro Arg His Leu Arg Leu Leu
  1               5                  10                  15

Arg Leu Leu Leu Ser Gly Leu Val Leu Gly Ala Ala Leu Arg Gly Ala
             20                  25                  30

Ala Ala Gly His Pro Glu Cys Cys Arg Leu Ser Arg Glu Pro Gly Leu
         35                  40                  45

Cys Pro Glu Glu Ala Gly Lys Cys Pro Pro Gly Ala His Ala Cys Gly
    50                  55                  60

Pro Ala Phe Ser Pro Ser Xaa Arg Asn Ser Lys Gly Leu Phe Cys Xaa
65                  70                  75                  80

Asp Ala Pro Gly Phe Xaa Arg Gly Pro Gly Pro Thr Xaa Thr Xaa Asn
                85                  90                  95

Glu Ile Asp Ser Trp Pro Lys Gly Ala Cys Pro Glu Arg Asn Leu Asp
            100                 105                 110

Ile Asn Ser Ala Leu Thr Gln Gly Arg Thr Ala Val Pro Gly Ala Cys
        115                 120                 125

His Leu Gly Ile Xaa Gly Thr Gly Ala Gly Ala Gly Ala Gly Leu Pro
    130                 135                 140

Phe His Ser Arg Asn Pro His Ala His Ala Pro His Xaa Pro Trp Val
145                 150                 155                 160

Thr Pro Val Ser Ser Asp Pro Val His Met Ser Pro Leu Glu Pro Arg
                165                 170                 175

Gly Gly Gln Gly Asp Gly Xaa Ala Leu Val Leu Ile Leu Ala Phe Cys
            180                 185                 190

Val Ala Gly Ala Ala Ala Leu Ser Val Ala Ser Xaa Cys Trp Cys Arg
        195                 200                 205

Leu Gln Arg Glu Ile Arg Leu Thr Gln Lys Ala Glu Tyr Ala Thr Ala
    210                 215                 220

Lys Ala Leu Ala Thr Pro Ala Ala Thr Pro Asp Leu Ala Trp Gly Pro
225                 230                 235                 240

Ala Pro Gly Thr Glu Arg Gly Asp Val Pro Leu Pro Ala Pro Thr Ala
                245                 250                 255

Thr Asp Val Val Pro Gly Ala Ala Xaa
            260                 265

<210> 154
<211> 237
<212> PRT
<213> Homo sapiens

<220>
<221> misc feature
<222> (137)
<223> Xaa equals any one of the naturally occurring L-amino acids

<220>
<221> misc feature
<222> (151)
<223> Xaa equals any one of the naturally occurring L-amino acids

<400> 154

Met Lys Gly Ile Leu Val Ala Gly Ile Thr Ala Val Leu Val Ala Ala
1               5                   10                  15

Val Glu Ser Leu Ser Cys Val Gln Cys Asn Ser Trp Glu Lys Ser Cys
            20                  25                  30

Val Asn Ser Ile Ala Ser Glu Cys Pro Ser His Ala Asn Thr Ser Cys
        35                  40                  45

Ile Ser Ser Ser Ala Ser Ser Ser Leu Glu Thr Pro Val Arg Leu Tyr
    50                  55                  60

Gln Asn Met Phe Cys Ser Ala Glu Asn Cys Ser Glu Glu Thr His Ile
65                  70                  75                  80

Thr Ala Phe Thr Val His Val Ser Ala Glu Glu His Phe His Phe Val
                85                  90                  95

Ser Gln Cys Cys Gln Gly Lys Glu Cys Ser Asn Thr Ser Asp Ala Leu
            100                 105                 110

Asp Pro Pro Leu Lys Asn Val Ser Ser Asn Ala Glu Cys Pro Ala Cys
        115                 120                 125

Tyr Glu Ser Asn Gly Thr Ser Cys Xaa Gly Lys Pro Trp Lys Cys Tyr
    130                 135                 140

Glu Glu Glu Gln Cys Val Xaa Leu Val Ala Glu Leu Lys Asn Asp Ile
145                 150                 155                 160

Glu Ser Lys Ser Leu Val Leu Lys Gly Cys Ser Asn Val Ser Asn Ala
                165                 170                 175

Thr Cys Gln Phe Leu Ser Gly Glu Asn Lys Thr Leu Gly Gly Val Ile
            180                 185                 190

Phe Arg Lys Phe Glu Cys Ala Asn Val Asn Ser Leu Thr Pro Thr Ser
        195                 200                 205

Ala Pro Thr Thr Ser His Asn Val Gly Ser Lys Ala Ser Leu Tyr Leu
    210                 215                 220

Leu Ala Leu Ala Ser Leu Leu Leu Arg Gly Leu Leu Pro
225                 230                 235

<210> 155
<211> 314
<212> PRT
<213> Homo sapiens

<220>
<221> misc feature
<222> (49)
<223> Xaa equals any one of the naturally occurring L-amino acids

<220>
<221> misc feature
<222> (167)
<223> Xaa equals any one of the naturally occurring L-amino acids

<220>
<221> misc feature
<222> (314)
<223> Xaa equals stop translation

<400> 155
Met Asn Gln Leu Ser Phe Leu Leu Phe Leu Ile Ala Thr Thr Arg Gly
1               5                   10                  15

Trp Ser Thr Asp Glu Ala Asn Thr Tyr Phe Lys Glu Trp Thr Cys Ser
            20                  25                  30

Ser Ser Pro Ser Leu Pro Arg Ser Cys Lys Glu Ile Lys Asp Glu Cys
        35                  40                  45

Xaa Ser Ala Phe Asp Gly Leu Tyr Phe Leu Arg Thr Glu Asn Gly Val
    50                  55                  60

Ile Tyr Gln Thr Phe Cys Asp Met Thr Ser Gly Gly Gly Gly Trp Thr
65                  70                  75                  80

Leu Val Ala Ser Val His Glu Asn Asp Met Arg Gly Lys Cys Thr Val
                85                  90                  95

Gly Asp Arg Trp Ser Ser Gln Gln Gly Ser Lys Ala Asp Tyr Pro Glu
            100                 105                 110

Gly Asp Gly Asn Trp Ala Asn Tyr Asn Thr Phe Gly Ser Ala Glu Ala
        115                 120                 125

Ala Thr Ser Asp Asp Tyr Lys Asn Pro Gly Tyr Tyr Asp Ile Gln Ala
    130                 135                 140

Lys Asp Leu Gly Ile Trp His Val Pro Asn Lys Ser Pro Met Gln His
145                 150                 155                 160

Trp Arg Asn Ser Ser Leu Xaa Arg Tyr Arg Thr Asp Thr Gly Phe Leu
                165                 170                 175

Gln Thr Leu Gly His Asn Leu Phe Gly Ile Tyr Gln Lys Tyr Pro Val
            180                 185                 190

Lys Tyr Gly Glu Gly Lys Cys Trp Thr Asp Asn Gly Pro Val Ile Pro
        195                 200                 205

Val Val Tyr Asp Phe Gly Asp Ala Gln Lys Thr Ala Ser Tyr Tyr Ser
    210                 215                 220

Pro Tyr Gly Gln Arg Glu Phe Thr Ala Gly Phe Val Gln Phe Arg Val
225                 230                 235                 240

Phe Asn Asn Glu Arg Ala Ala Asn Ala Leu Cys Ala Gly Met Arg Val
                245                 250                 255

Thr Gly Cys Asn Thr Glu His His Cys Ile Gly Gly Gly Gly Tyr Phe
            260                 265                 270

Pro Glu Ala Ser Pro Gln Gln Cys Gly Asp Phe Ser Gly Phe Asp Trp
        275                 280                 285

Ser Gly Tyr Gly Thr His Val Gly Tyr Ser Ser Ser Arg Glu Ile Thr
290                 295                 300

Glu Ala Ala Val Leu Leu Phe Tyr Arg Xaa
305                 310

<210> 156
<211> 99
<212> PRT
<213> Homo sapiens

<220>
<221> misc feature
<222> (17)
<223> Xaa equals any one of the naturally occurring L-amino acids

<220>
<221> misc feature
<222> (24)
<223> Xaa equals any one of the naturally occurring L-amino acids

<220>
<221> misc feature
<222> (99)
<223> Xaa equals stop translation

<400> 156
Met Leu Ala Phe Pro Val Leu Leu Glu Val Ser Trp Ser Val Leu Phe
1               5                   10                  15

Xaa Phe Ser Phe Phe Ser Pro Xaa Pro Ser Ala Pro Gln Pro Pro Thr
            20                  25                  30

Pro Ser Arg Ser Val Leu His Ala Arg Cys Ser Asn Val Arg Ser Glu
        35                  40                  45

Met Ala Gly Thr Arg Glu Lys Leu Leu Val Ser Phe Val Ser Gly Ser
    50                  55                  60

Gly Met Ala Leu Ser Ser Leu Ala Ser Leu Phe Val Leu Phe Glu Leu
65                  70                  75                  80

Cys Arg Ser Leu Phe Ser Gln Ala Glu Leu Pro Thr Arg Ser Ile Leu
                85                  90                  95

Asp Gln Xaa

<210> 157
<211> 37
<212> PRT
<213> Homo sapiens

<220>
<221> misc feature
<222> (8)
<223> Xaa equals any one of the naturally occurring L-amino acids

<220>
<221> misc feature
<222> (19)
<223> Xaa equals any one of the naturally occurring L-amino acids

<220>
<221> misc feature
<222> (28)
<223> Xaa equals any one of the naturally occurring L-amino acids

<220>
<221> misc feature
<222> (37)
<223> Xaa equals stop translation

<400> 157
Met Asn Pro Phe Ser Val Phe Xaa Ser Leu Cys Leu Lys Gln Phe Glu
1               5                   10                  15

Asp Val Xaa Leu Phe Leu Gly Leu Met Phe Gly Xaa Ser Leu Asn Gly
            20                  25                  30

Gln Glu Gly Thr Xaa
        35


<210> 158
<211> 23
<212> PRT
<213> Homo sapiens

<220>
<221> misc feature
<222> (23)
<223> Xaa equals stop translation

<400> 158
Met Val Ile Phe Ile Ile Leu Leu Thr Cys Phe Gly Phe Ser Asn Gly
1               5                   10                  15

Ser Phe Ser Phe Ser Leu Xaa
            20


<210> 159
<211> 96
<212> PRT
<213> Homo sapiens

<220>
<221> misc feature
<222> (30)
<223> Xaa equals any one of the naturally occurring L-amino acids

<220>
<221> misc feature
<222> (35)
<223> Xaa equals any one of the naturally occurring L-amino acids

<220>
<221> misc feature
<222> (64)
<223> Xaa equals any one of the naturally occurring L-amino acids

<220>
<221> misc feature
<222> (83)
<223> Xaa equals any one of the naturally occurring L-amino acids

<400> 159
Met Cys Phe Ile Leu Val Val Cys Phe Ala Ser Leu Ile Thr Glu Cys
1               5                   10                  15

Pro Cys His Cys Lys Cys Cys Arg Asp Val Gly Arg Gly Xaa Thr Val
            20                  25                  30

Leu Tyr Xaa Cys Ser Met Val Gln Asn Lys Leu Leu Thr Gln Val Ser
        35                  40                  45

Leu Val Arg Asn Leu Trp Ala Met Glu Val Arg His Pro Ser Cys Xaa
    50                  55                  60

Ser Ile Gly Lys Lys Cys Phe Gln Ile Leu Trp Lys Gly Gly His Gly
65                  70                  75                  80

Ala Gly Xaa Trp Arg Val Ala Phe Glu Gln Ser Asp Pro Ile Ser Val
                85                  90                  95

<210> 160
<211> 66
<212> PRT
<213> Homo sapiens

<220>
<221> misc feature
<222> (66)
<223> Xaa equals stop translation

<400> 160
Met Val Glu Asn Trp Val Leu Glu Glu Ser Pro Gly Arg Leu Leu Ala
1               5                   10                  15

Leu Phe Val Val Arg Arg Ala Leu Ala Gln Gly Gln Arg Glu Glu Lys
            20                  25                  30

Gly Gln Pro Ala Ala Val Glu Ser Ala Gly Trp Leu Pro Thr Arg Phe
        35                  40                  45

Leu Ser Ser Gln Asp Ser Leu Pro Leu Ser Ser Arg Ile Ser Asn Gly
    50                  55                  60

Leu Xaa
65

<210> 161
<211> 222
<212> PRT
<213> Homo sapiens

<220>
<221> misc feature
<222> (86)
<223> Xaa equals any one of the naturally occurring L-amino acids

<400> 161
Met His Phe Gln Arg Gln Lys Leu Met Ala Val Thr Glu Tyr Ile Pro
1               5                   10                  15

Pro Lys Pro Ala Ile His Pro Ser Cys Leu Pro Ser Pro Pro Ser Pro
            20                  25                  30

Pro Gln Glu Glu Ile Gly Leu Ile Arg Leu Leu Arg Arg Glu Ile Ala
        35                  40                  45

Ala Val Phe Gln Asp Asn Arg Met Ile Ala Val Cys Gln Asn Val Ala
    50                  55                  60

Leu Ser Ala Glu Asp Lys Leu Leu Met Arg His Gln Leu Arg Lys His
65                  70                  75                  80

Lys Ile Leu Met Lys Xaa Phe Pro Asn Gln Val Leu Lys Pro Phe Leu
                85                  90                  95

Glu Asp Ser Lys Tyr Gln Asn Leu Leu Pro Leu Phe Val Gly His Asn
            100                 105                 110

Met Leu Leu Val Ser Glu Glu Pro Lys Val Lys Glu Met Val Arg Ile
        115                 120                 125

Leu Arg Thr Val Pro Phe Leu Pro Leu Leu Gly Gly Cys Ile Asp Asp
    130                 135                 140

Thr Ile Leu Ser Arg Gln Gly Phe Ile Asn Tyr Ser Lys Leu Pro Ser
145                 150                 155                 160

Leu Pro Leu Val Gln Gly Glu Leu Val Gly Gly Leu Thr Cys Leu Thr
                165                 170                 175

Ala Gln Thr His Ser Leu Leu Gln His Gln Pro Leu Gln Leu Thr Thr
            180                 185                 190

Leu Leu Asp Gln Tyr Ile Arg Glu Gln Arg Glu Lys Asp Ser Val Met
        195                 200                 205

Ser Ala Asn Gly Lys Pro Asp Pro Asp Thr Val Pro Asp Ser
    210                 215                 220

<210> 162
<211> 91
<212> PRT
<213> Homo sapiens

<220>

<221> misc feature
<222> (53)
<223> Xaa equals any one of the naturally occurring L-amino acids

<400> 162
Met Val Val Asp Gln Lys Glu Asp Leu Ile Thr Gly Leu Gly Ile Lys
1               5                   10                  15

Met Val Arg Lys Trp Leu Gln Gly Ser Gln Ala Trp Pro Leu Glu Arg
            20                  25                  30

Glu Glu Arg Glu Gly Leu Gly Ser Leu Cys Thr Cys Cys Pro Trp Gly
        35                  40                  45

Leu Val Arg Phe Xaa Glu Ser Leu Thr His Phe Thr Gly Glu Ala Ile
    50                  55                  60

Glu Pro Leu Arg Ala Glu Val Thr Asp Pro Lys His Pro Cys Ser Cys
65                  70                  75                  80

Val Ala Glu Pro Glu Val Lys Ser Arg Ser Leu
                85                  90


<210> 163
<211> 74
<212> PRT
<213> Homo sapiens

<220>
<221> misc feature
<222> (51)
<223> Xaa equals any one of the naturally occurring L-amino acids

<400> 163
Met Glu Asn Asp Trp Gly Phe Gln Thr Thr Phe Phe Ser Leu Gly Leu
1               5                   10                  15

Tyr Leu Phe Thr Ile Trp Trp Ser Thr Val Gly Leu Pro Trp Thr Ser
            20                  25                  30

Ser Thr Gln Arg Glu Leu Asp Met Lys Leu Glu Ala Ala Ala Leu Glu
        35                  40                  45

Gly Lys Xaa Gly Ser Leu Gly Gln Pro Arg Pro Trp Gln Glu Glu Ser
    50                  55                  60

Leu Pro Leu Gly Val Leu Asp Gly His Val
65                  70


<210> 164
<211> 78
<212> PRT
<213> Homo sapiens

<220>
<221> misc feature
<222> (69)
<223> Xaa equals any one of the naturally occurring L-amino acids

<220>
<221> misc feature
<222> (78)
<223> Xaa equals stop translation

<400> 164
Met Thr Gly Gln Ile Pro Arg Leu Ser Lys Val Asn Leu Phe Thr Leu
1               5                   10                  15

Leu Ser Leu Trp Met Glu Leu Phe Pro Ala Glu Ala Gln Arg Gln Lys
            20                  25                  30

Ser Gln Lys Asn Glu Glu Gly Lys His Gly Pro Leu Gly Asp Asn Glu
        35                  40                  45

Glu Arg Thr Arg Val Ser Thr Asp Lys Arg Gln Asp Tyr Trp Glu Gln
    50                  55                  60

Leu Arg Cys Leu Xaa Glu Arg Phe Thr Ile Thr Ala Gly Xaa
65                  70                  75


<210> 165
<211> 38
<212> PRT
<213> Homo sapiens

<220>
<221> misc feature
<222> (38)
<223> Xaa equals stop translation

<400> 165
Met Ala Phe Leu Leu Thr Leu Val Pro Leu Leu Pro Ser Arg Cys Leu
1               5                   10                  15

Gly Leu Glu Glu Met Ala Val Pro Asn Ser Thr Cys Ile Ser Pro Phe
            20                  25                  30

Ser Cys Cys Tyr Gly Xaa
        35


<210> 166
<211> 45
<212> PRT
<213> Homo sapiens

<220>
<221> misc feature
<222> (45)
<223> Xaa equals stop translation

<400> 166
Met Phe His Val Phe Val Leu Leu Leu Thr Phe Ile Ala Leu Ser Pro
1               5                   10                  15

Ser Gly Ile Arg Leu Leu Phe Gly Phe Ile Gln Lys Gly Leu Asn Leu
            20                  25                  30

Asn Ser Phe Met Phe Arg Leu Glu Leu Leu His Phe Xaa
        35                  40                  45

<210> 167
<211> 39
<212> PRT
<213> Homo sapiens

<220>
<221> misc feature
<222> (39)
<223> Xaa equals stop translation

<400> 167
Met Thr Ser Leu Pro Ile Leu Ala Phe Gly Ala Val Tyr Trp Pro Asp
  1               5                  10                  15

Leu Ala Ser His Ser Phe Ser Pro Ser Arg Ser Leu Ala Gln Thr Pro
             20                  25                  30

His Met Ser Val Ser Gly Xaa
        35

<210> 168
<211> 174
<212> PRT
<213> Homo sapiens

<220>
<221> misc feature
<222> (83)
<223> Xaa equals any one of the naturally occurring L-amino acids

<220>
<221> misc feature
<222> (110)
<223> Xaa equals any one of the naturally occurring L-amino acids

<220>
<221> misc feature
<222> (115)
<223> Xaa equals any one of the naturally occurring L-amino acids

<220>
<221> misc feature
<222> (118)
<223> Xaa equals any one of the naturally occurring L-amino acids

<220>
<221> misc feature
<222> (168)
<223> Xaa equals any one of the naturally occurring L-amino acids

<220>
<221> misc feature
<222> (174)
<223> Xaa equals stop translation

<400> 168
Met Gln Leu Ile Pro Leu Glu Gln Leu Cys Met Leu Leu Leu Met Ser
1               5                   10                  15

Asp Asn Val Asp Arg Cys Phe Glu Thr Cys Pro Pro Arg Thr Phe Leu
            20                  25                  30

Pro Ala Leu Cys Lys Ile Phe Leu Asp Glu Ser Ala Pro Asp Asn Val
        35                  40                  45

Leu Glu Val Thr Ala Arg Ala Ile Thr Tyr Tyr Leu Asp Val Ser Ala
    50                  55                  60

Glu Cys Thr Arg Arg Ile Val Gly Val Asp Gly Ala Ile Lys Ala Leu
65                  70                  75                  80

Cys Asn Xaa Leu Val Val Val Glu Leu Asn Asn Arg Thr Ser Arg Asp
                85                  90                  95

Leu Ala Glu Gln Cys Val Lys Val Leu Glu Leu Ile Cys Xaa Pro Glu
            100                 105                 110

Ser Gly Xaa Val Phe Xaa Ala Gly Gly Leu Asn Arg Val Ala Tyr Leu
        115                 120                 125

Pro Ser Val Asn Ser Gly His Leu Val His Lys Asp Thr Leu His Ser
    130                 135                 140

Ala Met Ala Val Val Ser Arg Leu Cys Gly Lys Met Glu Pro Gln Asp
145                 150                 155                 160

Ser Ser Leu Glu Ile Cys Val Xaa Ser Leu Ser Ser Leu Xaa
                165                 170


<210> 169
<211> 55
<212> PRT
<213> Homo sapiens

<220>
<221> misc feature
<222> (55)
<223> Xaa equals stop translation

<400> 169
Met Phe Glu Asp Thr Leu Arg Thr Leu Tyr Ile Leu Leu Phe Tyr Leu
1               5                   10                  15

Arg Tyr Ile Cys Leu Leu Ser Pro His Ile Ala Leu Met Thr Leu Ile
            20                  25                  30

Leu Ile Asp Gly Phe Leu Gln Cys Tyr Tyr Cys Ala Leu His Val Pro
        35                  40                  45

Cys Ile Ile Ala Phe Leu Xaa
    50                  55

<210> 170
<211> 344
<212> PRT
<213> Homo sapiens

<220>
<221> misc feature
<222> (126)
<223> Xaa equals any one of the naturally occurring L-amino acids

<220>
<221> misc feature
<222> (128)
<223> Xaa equals any one of the naturally occurring L-amino acids

<400> 170
Met Glu Lys Ile Gly Ser Ser Leu Pro Gln Asp Asp Asp Ala Pro Lys
1               5                   10                  15

Lys Gln Ala Leu Tyr Leu Met Phe Asp Thr Ser Gln Glu Ser Pro Val
            20                  25                  30

Lys Ser Ser Pro Val Arg Met Ser Glu Ser Pro Thr Pro Cys Ser Gly
        35                  40                  45

Ser Ser Phe Glu Glu Thr Glu Ala Leu Val Asn Thr Ala Ala Lys Asn
    50                  55                  60

Gln His Pro Val Pro Arg Gly Leu Ala Pro Asn Gln Glu Ser His Leu
65                  70                  75                  80

Gln Val Pro Glu Lys Ser Ser Gln Lys Glu Leu Glu Ala Met Gly Leu
                85                  90                  95

Gly Thr Pro Ser Glu Ala Ile Glu Ile Arg Glu Ala Ala His Pro Thr
            100                 105                 110

Asp Val Ser Ile Ser Lys Thr Ala Leu Tyr Ser Arg Ile Xaa Thr Xaa
        115                 120                 125

Glu Val Glu Lys Pro Ala Gly Leu Leu Phe Gln Gln Pro Asp Leu Asp
    130                 135                 140

Ser Ala Leu Gln Ile Ala Arg Ala Glu Ile Ile Thr Lys Glu Arg Glu
145                 150                 155                 160

Val Ser Glu Trp Lys Asp Lys Tyr Glu Glu Ser Arg Arg Glu Val Met
                165                 170                 175

Glu Met Arg Lys Ile Val Ala Glu Tyr Glu Lys Thr Ile Ala Gln Met
            180                 185                 190

Ile Glu Asp Glu Gln Arg Glu Lys Ser Val Ser His Gln Thr Val Gln
        195                 200                 205

Gln Leu Val Leu Glu Lys Glu Gln Ala Leu Ala Asp Leu Asn Ser Val
    210                 215                 220

Glu Lys Ser Leu Ala Asp Leu Phe Arg Arg Tyr Glu Lys Met Lys Glu
225                 230                 235                 240

Val Leu Glu Gly Phe Arg Lys Asn Glu Glu Val Leu Lys Arg Cys Ala
                245                 250                 255

Gln Glu Tyr Leu Ser Arg Val Lys Lys Glu Glu Gln Arg Tyr Gln Ala
            260                 265                 270

Leu Lys Val His Ala Glu Glu Lys Leu Asp Arg Ala Asn Ala Glu Ile
        275                 280                 285

Ala Gln Val Arg Gly Lys Ala Gln Gln Glu Gln Ala Ala His Gln Ala
    290                 295                 300

Ser Leu Arg Lys Glu Gln Leu Arg Val Asp Ala Leu Glu Arg Thr Leu
305                 310                 315                 320

Glu Gln Lys Asn Lys Glu Ile Glu Glu Leu Thr Lys Ile Cys Asp Glu
                325                 330                 335

Leu Ile Ala Lys Met Gly Lys Ser
            340

<210> 171
<211> 90
<212> PRT
<213> Homo sapiens

<220>
<221> misc feature
<222> (90)
<223> Xaa equals stop translation

<400> 171
Met Tyr His Tyr Ala Trp Leu Ile Phe Val Phe Leu Val Glu Met Gly
  1               5                  10                  15

Phe Cys His Val Gly Gln Ala Gly Leu Lys Leu Leu Thr Ser Ser Asp
             20                  25                  30

Pro Pro Ala Ser Ala Ser Gln Ser Ala Gly Ile Thr Gly Val Ser His
         35                  40                  45

His Ala Trp Gly Lys Arg Tyr Phe Gln Asn Ile Val Asn Asn Phe Ser
     50                  55                  60

Pro Lys Pro Arg Gln Gly Leu Ile Leu Leu Pro Arg Leu Glu Trp Gln
 65                  70                  75                  80

Gly His His Arg Ser Ser Leu Gln Pro Xaa
                 85                  90

<210> 172
<211> 104
<212> PRT
<213> Homo sapiens

<400> 172
Met Leu Cys Pro Asn His Gly Leu Phe Pro Asp Pro Gly Phe Gln Cys

1                   5                   10                  15

Pro Pro Leu Phe Gln Glu Val Gln Arg Asp Ala Pro His Arg Lys Gly
            20                  25                  30

Ser Ala Thr Val Leu Pro Arg Cys Pro Pro Trp Val Pro Ser Leu Lys
        35                  40                  45

His Arg Thr Ser His Thr Ser Ser Pro Ala Val Pro Leu Ile Leu Val
    50                  55                  60

Pro Arg Leu Pro Ser Leu Gln Leu His Ser Phe Ile Gln His Ser Leu
65                  70                  75                  80

Gly Asp Phe Tyr Ile Asp Thr Pro Arg Thr Glu Ala Trp Gly Lys Asp
                85                  90                  95

Asp Gln Glu His Val Pro Ser Arg
            100


<210> 173
<211> 42
<212> PRT
<213> Homo sapiens

<220>
<221> misc feature
<222> (42)
<223> Xaa equals stop translation

<400> 173
Met Ser Val Leu Phe Val Ala Val Ser Leu Leu Ser Ser Ile Val Pro
1               5                   10                  15

Asp Ile Gln Tyr Arg Leu Lys Thr Tyr Leu His Ile Asp Leu Trp Lys
            20                  25                  30

Thr Asp Thr Gln Val Leu Lys Asn Lys Xaa
        35                  40


<210> 174
<211> 47
<212> PRT
<213> Homo sapiens

<220>
<221> misc feature
<222> (47)
<223> Xaa equals stop translation

<400> 174
Met Met Leu Gly Leu Phe Ser Pro Leu Cys Leu Val Thr Gly Ile Ala
1               5                   10                  15

Glu Gly Arg Ala Glu Asp Ala Ser Leu His Asp Ile Cys Thr Thr Gln
            20                  25                  30

His Thr Leu Thr Phe Thr Pro Ser Tyr Pro Val Gly Gly Ser Xaa

35                  40                  45

<210> 175
<211> 73
<212> PRT
<213> Homo sapiens

<220>
<221> misc feature
<222> (44)
<223> Xaa equals any one of the naturally occurring L-amino acids

<220>
<221> misc feature
<222> (69)
<223> Xaa equals any one of the naturally occurring L-amino acids

<400> 175
Met Ser Phe Ser Leu Ala His Val Lys Thr Gly Gln Gly Pro Arg Leu
1               5                   10                  15

Thr Glu Ala Leu Gln Tyr Ile Ala Ser Lys Ile Ala Val Gly Val Thr
            20                  25                  30

Ser Ser Gln Lys Ser Gly Glu Glu Arg Ala Met Xaa Thr Gln Glu Leu
        35                  40                  45

Leu Met Asp Gln Ala Trp Asp Ser Val Cys His Phe His Gln His Pro
    50                  55                  60

Thr His Gln Asn Xaa Val Thr Gly Pro
65                  70


<210> 176
<211> 29
<212> PRT
<213> Homo sapiens

<220>
<221> misc feature
<222> (29)
<223> Xaa equals stop translation

<400> 176
Met Leu Ser Leu Asp Phe Pro Leu Ile Leu Leu Gly Leu Asn Leu His
1               5                   10                  15

Ile Ala Leu Leu Ser Leu Leu Val Pro Arg Leu Ser Xaa
            20                  25


<210> 177
<211> 67
<212> PRT
<213> Homo sapiens

<400> 177
Met Ile Phe Arg Asn Gly Val Arg Leu Val Phe Val Phe Val Leu Phe

1               5                   10                  15

Tyr Thr Ser Thr Gln Ser Leu Phe Asn Ser Leu Gln Thr Ala Glu Tyr
            20                  25                  30

Val Leu Phe Cys Gln Gln Arg Leu Ser Leu Tyr Glu Pro Ser His Val
        35                  40                  45

Leu Cys Leu Cys Met Ser Pro His Arg Lys His Thr Arg Glu Ser Asp
    50                  55                  60

Thr Ser Gly
65

<210> 178
<211> 24
<212> PRT
<213> Homo sapiens

<220>
<221> misc feature
<222> (24)
<223> Xaa equals stop translation

<400> 178
Met Asn Phe Leu Leu Leu Ile Phe Pro Tyr Phe Ser Ser Leu Leu Gly
1               5                   10                  15

Glu Val Glu Val Val Lys Cys Xaa
            20

<210> 179
<211> 31
<212> PRT
<213> Homo sapiens

<220>
<221> misc feature
<222> (31)
<223> Xaa equals stop translation

<400> 179
Met Ser Pro Gly Arg Val Ser Val Val Ser Leu Gln Gly Ser Gln Leu
1               5                   10                  15

Cys Leu Leu Val Ser Ile Ala Ile Met Gly Leu Leu Leu Phe Xaa
            20                  25                  30

<210> 180
<211> 11
<212> PRT
<213> Homo sapiens

<220>
<221> misc feature
<222> (11)
<223> Xaa equals stop translation

<400> 180
Met Ala Tyr Ala Phe His Arg Thr Ser Thr Xaa
1               5                   10

<210> 181
<211> 32
<212> PRT
<213> Homo sapiens

<220>
<221> misc feature
<222> (32)
<223> Xaa equals stop translation

<400> 181
Met Ser Val Lys Val Gly Ser Leu Leu Val Leu Val Tyr Phe Thr Leu
1               5                   10                  15

Gly Pro Val Val Ala Glu Leu Glu Val Thr Leu Pro Ser His Ser Xaa
            20                  25                  30

<210> 182
<211> 36
<212> PRT
<213> Homo sapiens

<220>
<221> misc feature
<222> (36)
<223> Xaa equals stop translation

<400> 182
Met Ile Val Ile Thr Ser Ile Leu Ser Ser Leu Ala Ser Leu Leu Leu
1               5                   10                  15

Leu Ala Phe Leu Ala Ala Ser Thr Ala Arg Leu Ser Pro Gln Ser Leu
            20                  25                  30

Pro Glu Thr Xaa
        35

<210> 183
<211> 35
<212> PRT
<213> Homo sapiens

<220>
<221> misc feature
<222> (35)
<223> Xaa equals stop translation

<400> 183
Met Ser Gly Leu Glu Ser Ala Arg Val Leu Leu Cys Ala Leu Gly Ser

1                   5                   10                  15

Phe Leu Leu Asn Ser Leu Leu Ser Thr Phe Arg Leu Asn Ser Ser Ala
            20                  25                  30

Pro Ser Xaa
        35


<210> 184
<211> 29
<212> PRT
<213> Homo sapiens

<220>
<221> misc feature
<222> (29)
<223> Xaa equals stop translation

<400> 184
Met His Ser Ile Ile Val Lys Glu Leu Ile Val Thr Phe Phe Leu Gly
 1               5                  10                  15

Ile Thr Val Leu Leu Leu Leu Met Gln Arg Ser Leu Xaa
            20                  25


<210> 185
<211> 6
<212> PRT
<213> Homo sapiens

<220>
<221> misc feature
<222> (6)
<223> Xaa equals stop translation

<400> 185
Met Gly Tyr Leu Asn Xaa
 1               5


<210> 186
<211> 53
<212> PRT
<213> Homo sapiens

<220>
<221> misc feature
<222> (53)
<223> Xaa equals stop translation

<400> 186
Asp Leu Thr Ser Leu Leu Phe Tyr Leu Ala Gly Cys Phe Ser Ser Cys
 1               5                  10                  15

Arg Leu Gly Gln Gly Thr Pro Gly Ser Leu Pro Trp Thr Ser Asn Glu
            20                  25                  30

Glu Gly Ile Ile Gln Gly Pro Thr Pro Met Phe Trp Asn Leu Thr Pro

35                  40                  45

Phe Ser Gly Thr Xaa
    50


<210> 187
<211> 406
<212> PRT
<213> Homo sapiens

<220>
<221> misc feature
<222> (273)
<223> Xaa equals any one of the naturally occurring L-amino acids

<220>
<221> misc feature
<222> (406)
<223> Xaa equals stop translation

<400> 187
Met Leu Leu Leu Trp Val Ser Val Val Ala Ala Leu Ala Leu Ala Val
 1               5                  10                  15

Leu Ala Pro Gly Ala Gly Glu Gln Arg Arg Arg Ala Ala Lys Ala Pro
            20                  25                  30

Asn Val Val Leu Val Val Ser Asp Ser Tyr Asp Gly Arg Leu Thr Phe
        35                  40                  45

His Pro Gly Ser Gln Val Val Lys Leu Pro Phe Ile Asn Phe Met Lys
    50                  55                  60

Thr Arg Gly Thr Ser Phe Leu Asn Ala Tyr Thr Asn Ser Pro Ile Cys
65                  70                  75                  80

Cys Pro Ser Arg Ala Ala Met Trp Ser Gly Leu Phe Thr His Leu Thr
                85                  90                  95

Glu Ser Trp Asn Asn Phe Lys Gly Leu Asp Pro Asn Tyr Thr Thr Trp
            100                 105                 110

Met Asp Val Met Glu Arg His Gly Tyr Arg Thr Gln Lys Phe Gly Lys
        115                 120                 125

Leu Asp Tyr Thr Ser Gly His His Ser Ile Ser Asn Arg Val Glu Ala
    130                 135                 140

Trp Thr Arg Asp Val Ala Phe Leu Leu Arg Gln Glu Gly Arg Pro Met
145                 150                 155                 160

Val Asn Leu Ile Arg Asn Arg Thr Lys Val Arg Val Met Glu Arg Asp
                165                 170                 175

Trp Gln Asn Thr Asp Lys Ala Val Asn Trp Leu Arg Lys Glu Ala Ile
            180                 185                 190

Asn Tyr Thr Glu Pro Phe Val Ile Tyr Leu Gly Leu Asn Leu Pro His
        195                 200                 205

Pro Tyr Pro Ser Pro Ser Ser Gly Glu Asn Phe Gly Ser Ser Thr Phe
    210                 215                 220

His Thr Ser Leu Tyr Trp Leu Glu Lys Val Ser His Asp Ala Ile Lys
225                 230                 235                 240

Ile Pro Lys Trp Ser Pro Leu Ser Glu Met His Pro Val Asp Tyr Tyr
                245                 250                 255

Ser Ser Tyr Thr Lys Asn Cys Thr Gly Arg Phe Thr Lys Lys Glu Ile
            260                 265                 270

Xaa Asn Ile Arg Ala Phe Tyr Tyr Ala Met Cys Ala Glu Thr Asp Ala
        275                 280                 285

Met Leu Gly Glu Ile Ile Leu Ala Leu His Gln Leu Asp Leu Leu Gln
    290                 295                 300

Lys Thr Ile Val Ile Tyr Ser Ser Asp His Gly Glu Leu Ala Met Glu
305                 310                 315                 320

His Arg Gln Phe Tyr Lys Met Ser Met Tyr Glu Ala Ser Ala His Val
                325                 330                 335

Pro Leu Leu Met Met Gly Pro Gly Ile Lys Ala Gly Leu Gln Val Ser
            340                 345                 350

Asn Val Val Ser Leu Val Asp Ile Tyr Pro Thr Met Leu Asp Ile Ala
        355                 360                 365

Gly Ile Pro Leu Pro Gln Asn Leu Ser Gly Tyr Ser Ser Leu Pro Leu
    370                 375                 380

Ser Ser Glu Thr Phe Lys Asn Glu His Lys Val Lys Asn Leu His Pro
385                 390                 395                 400

Pro Trp Ile Thr Glu Xaa
                405

<210> 188
<211> 37
<212> PRT
<213> Homo sapiens

<220>
<221> misc feature
<222> (37)
<223> Xaa equals stop translation

<400> 188
Met Asn Gly Leu Val Arg Pro Val Glu Leu Asn Ser Leu Leu Leu Pro
  1               5                  10                  15

Val Val Arg Tyr Gln Val Ala Gln Pro Gln Lys Leu Leu Asn Val Phe
             20                  25                  30

Val Gly Gly Leu Xaa
        35

<210> 189
<211> 57
<212> PRT
<213> Homo sapiens

<220>
<221> misc feature
<222> (51)
<223> Xaa equals any one of the naturally occurring L-amino acids

<400> 189
Met Lys Ala Leu Val Gly Asn Ser Pro Pro Val Gly Asp Ser Gly Thr
1               5                   10                  15

Gln Pro Pro Ser Ala Leu Arg Leu Cys Leu Leu Lys Val Leu Arg Val
            20                  25                  30

Leu Ser Met Tyr Leu Ala Asn Gly Glu Arg Val Trp Arg Thr His Lys
        35                  40                  45

Arg Val Xaa His His Val Leu Arg Gly
    50                  55


<210> 190
<211> 128
<212> PRT
<213> Homo sapiens

<220>
<221> misc feature
<222> (127)
<223> Xaa equals any one of the naturally occurring L-amino acids

<220>
<221> misc feature
<222> (128)
<223> Xaa equals stop translation

<400> 190
Met Phe Val Leu Leu Tyr Val Thr Ser Phe Ala Ile Cys Ala Ser Gly
1               5                   10                  15

Gln Pro Arg Gly Asn Gln Leu Lys Gly Glu Asn Tyr Ser Pro Arg Tyr
            20                  25                  30

Ile Cys Ser Ile Pro Gly Leu Pro Gly Pro Pro Gly Pro Pro Gly Ala
        35                  40                  45

Asn Gly Ser Pro Gly Pro His Gly Arg Ile Gly Leu Pro Gly Arg Asp
    50                  55                  60

Gly Arg Asp Gly Arg Lys Gly Glu Lys Gly Glu Lys Gly Thr Ala Gly
65                  70                  75                  80

Leu Arg Gly Lys Thr Gly Pro Leu Gly Leu Ala Gly Glu Lys Gly Asp
                85                  90                  95

Gln Gly Glu Thr Gly Lys Lys Gly Pro Ile Gly Pro Glu Gly Glu Lys
            100                 105                 110

Gly Glu Val Gly Pro Ile Gly Pro Pro Gly Pro Lys Gly Asp Xaa Xaa
        115                 120                 125

<210> 191
<211> 21
<212> PRT
<213> Homo sapiens

<220>
<221> misc feature
<222> (21)
<223> Xaa equals stop translation

<400> 191
Met Lys Phe Ile Met Leu Leu Leu Leu Pro Ser Ile Phe Pro Thr Thr
  1               5                  10                  15

Val Glu Met Ile Xaa
            20

<210> 192
<211> 143
<212> PRT
<213> Homo sapiens

<220>
<221> misc feature
<222> (92)
<223> Xaa equals any one of the naturally occurring L-amino acids

<220>
<221> misc feature
<222> (136)
<223> Xaa equals any one of the naturally occurring L-amino acids

<220>
<221> misc feature
<222> (138)
<223> Xaa equals any one of the naturally occurring L-amino acids

<220>
<221> misc feature
<222> (143)
<223> Xaa equals stop translation

<400> 192
Met Cys Ala Phe Pro Trp Leu Leu Leu Leu Leu Leu Leu Gln Glu Gly
  1               5                  10                  15

Ser Gln Arg Arg Leu Trp Arg Trp Cys Gly Ser Glu Glu Val Val Ala
            20                  25                  30

Val Leu Gln Glu Ser Ile Ser Leu Pro Leu Glu Ile Pro Pro Asp Glu
        35                  40                  45

Glu Val Glu Asn Ile Ile Trp Ser Ser His Lys Ser Leu Ala Thr Val
    50                  55                  60

Val Pro Gly Lys Glu Gly His Pro Ala Thr Ile Met Val Thr Asn Pro
65                  70                  75                  80

His Tyr Gln Gly Gln Val Ser Phe Leu Asp Pro Xaa Tyr Ser Leu His
                85                  90                  95

Ile Ser Asn Leu Ser Trp Glu Asp Ser Gly Leu Tyr Gln Ala Gln Val
            100                 105                 110

Asn Leu Arg Thr Ser Gln Ile Ser Thr Met Gln Gln Tyr Asn Leu Cys
        115                 120                 125

Val Tyr Arg Trp Leu Ser Glu Xaa Pro Xaa His Cys Glu Leu Xaa
    130                 135                 140


<210> 193
<211> 110
<212> PRT
<213> Homo sapiens

<220>
<221> misc feature
<222> (110)
<223> Xaa equals stop translation

<400> 193
Met Ile Lys Lys Asp Lys Tyr His Lys Lys Val Phe Leu Phe Gly Trp
1               5                   10                  15

Phe Phe Cys Leu Phe Val Phe Phe Leu Arg Leu Ser Leu Ser Leu Leu
            20                  25                  30

Pro Lys Leu Glu Cys Asn Leu Gly Ser Leu Gln Pro Pro Pro Pro Arg
        35                  40                  45

Phe Gln Arg Phe Ser Cys Leu Ser Leu Leu Asn Ser Trp Asp Tyr Arg
    50                  55                  60

Arg Pro Pro Pro His Leu Ala Asn Phe Cys Val Val Ser Arg Gly Gly
65                  70                  75                  80

Val Ser Ser Cys Trp Pro Gly Trp Ser Arg Thr Pro Asp Leu Met Ile
                85                  90                  95

Arg Leu Pro Arg Pro Pro Arg Val Leu Gly Leu Gln Ala Xaa
            100                 105                 110


<210> 194
<211> 80
<212> PRT
<213> Homo sapiens

<220>
<221> misc feature
<222> (73)
<223> Xaa equals any one of the naturally occurring L-amino acids

<220>
<221> misc feature
<222> (78)
<223> Xaa equals any one of the naturally occurring L-amino acids

<400> 194
Met Phe Leu Thr Ile Ile Val Cys Gly Met Val Ala Ala Leu Ser Ala
1               5                   10                  15

Ile Arg Ala Asn Cys His Gln Glu Pro Ser Val Cys Leu Gln Ala Ala
            20                  25                  30

Cys Pro Glu Ser Trp Ile Gly Phe Gln Arg Lys Cys Phe Tyr Phe Ser
        35                  40                  45

Asp Asp Thr Lys Asn Trp Thr Ser Ser Gln Arg Phe Cys Asp Ser Gln
    50                  55                  60

Asp Ala Asp Leu Ala Gln Val Glu Xaa Phe Gln Glu Leu Xaa Arg Lys
65                  70                  75                  80

<210> 195
<211> 210
<212> PRT
<213> Homo sapiens

<220>
<221> misc feature
<222> (210)
<223> Xaa equals stop translation

<400> 195
Met Cys Pro Leu Trp Arg Leu Leu Ile Phe Leu Gly Leu Leu Ala Leu
1               5                   10                  15

Pro Leu Ala Pro His Lys Gln Pro Trp Pro Gly Leu Ala Gln Ala His
            20                  25                  30

Arg Asp Asn Lys Ser Thr Leu Ala Arg Ile Ile Ala Gln Gly Leu Ile
        35                  40                  45

Lys His Asn Ala Glu Ser Arg Ile Gln Asn Ile His Phe Gly Asp Arg
    50                  55                  60

Leu Asn Ala Ser Ala Gln Val Ala Pro Gly Leu Val Gly Trp Leu Ile
65                  70                  75                  80

Ser Gly Arg Lys His Gln Gln Gln Gln Glu Ser Ser Ile Asn Ile Thr
                85                  90                  95

Asn Ile Gln Leu Asp Cys Gly Gly Ile Gln Ile Ser Phe His Lys Glu

100                 105                 110

Trp Phe Ser Ala Asn Ile Ser Leu Glu Phe Asp Leu Glu Leu Arg Pro
        115                 120                 125

Ser Phe Asp Asn Asn Ile Ile Lys Met Cys Ala His Met Ser Ile Val
    130                 135                 140

Val Glu Phe Trp Leu Glu Lys Asp Glu Phe Gly Arg Arg Asp Leu Val
145                 150                 155                 160

Ile Gly Lys Cys Asp Ala Glu Pro Ser Ser Val His Val Ala Ile Leu
                165                 170                 175

Thr Glu Ala Ile Pro Pro Lys Met Asn Gln Phe Leu Tyr Asn Leu Lys
            180                 185                 190

Glu Asn Leu Gln Lys Val Leu Pro His Met Val Glu Ser Gln Pro Leu
        195                 200                 205

Ala Xaa
    210


<210> 196
<211> 149
<212> PRT
<213> Homo sapiens

<220>
<221> misc feature
<222> (61)
<223> Xaa equals any one of the naturally occurring L-amino acids

<220>
<221> misc feature
<222> (142)
<223> Xaa equals any one of the naturally occurring L-amino acids

<220>
<221> misc feature
<222> (149)
<223> Xaa equals stop translation

<400> 196
Met Arg Lys Ile Ala Gln Cys Ala Pro Gly Val Val Glu Leu Val Leu
  1               5                  10                  15

Ile Pro Leu Arg Gln Arg Leu Glu Glu Arg Gln Arg Arg Arg Lys Gln
             20                  25                  30

Gly Ala Gly Ser Leu Gln Glu Leu Ala Pro Gln Asp Gly Ser Gly Tyr
         35                  40                  45

Met Asp Val Gly Val Ser Gln Lys Ala Arg Gly Glu Xaa Val Pro Asp
     50                  55                  60

Pro Gln Gly Gly Gly Gln Leu Ser Trp Asp Arg Pro Pro Ala Pro Arg
 65                  70                  75                  80

Pro Pro Ala Tyr Asn Arg Ala Leu Gln Gly Asp Pro Ser Phe Val Leu
                85                  90                  95

Gln Ile Ala Glu Lys Glu Gln Glu Leu Leu Ala Ser Gln Glu Thr Val
            100                 105                 110

Gln Val Leu Gln Met Lys Val Arg Arg Leu Glu His Leu Leu Gln Leu
        115                 120                 125

Lys Asn Val Arg Ile Glu Asn Leu Ser Arg Arg Leu Gln Xaa Ala Glu
    130                 135                 140

Arg Lys Gln Arg Xaa
145


<210> 197
<211> 36
<212> PRT
<213> Homo sapiens

<220>
<221> misc feature
<222> (36)
<223> Xaa equals stop translation

<400> 197
Met His Ile Thr Ser Leu Val Gly Ala Gly Thr Leu Met Val Leu Leu
  1               5                  10                  15

Leu Leu Ile Leu Leu Leu Glu Cys Phe Phe Val Ala Glu Ala Leu Val
            20                  25                  30

Met Arg Ser Xaa
        35


<210> 198
<211> 258
<212> PRT
<213> Homo sapiens

<220>
<221> misc feature
<222> (258)
<223> Xaa equals stop translation

<400> 198
Met Ala Ala Leu Thr Thr Val Val Val Ala Ala Ala Ala Thr Ala Val
  1               5                  10                  15

Ala Gly Ala Val Ala Gly Ala Gly Ala Ala Thr Gly Thr Gly Val Gly
            20                  25                  30

Ala Thr Pro Ala Pro Gln Gln Ser Asp Gly Cys Phe Ser Thr Ser Gly
        35                  40                  45

Gly Ile Arg Pro Phe His Leu Gln Asn Trp Lys Gln Lys Val Asn Gln
    50                  55                  60

Thr Lys Lys Ala Glu Phe Val Arg Thr Ala Glu Lys Phe Lys Asn Gln
65                  70                  75                  80

Val Ile Asn Met Glu Lys Asp Lys His Ser His Phe Tyr Asn Gln Lys
                85                  90                  95

Ser Asp Phe Arg Phe Glu His Ser Met Leu Glu Glu Leu Glu Asn Lys
            100                 105                 110

Leu Ile His Ser Arg Lys Thr Glu Arg Ala Lys Phe Gln Gln Gln Leu
        115                 120                 125

Ala Lys Ile His Asn Asn Val Lys Lys Leu Gln His Gln Leu Lys Asp
    130                 135                 140

Val Lys Pro Thr Pro Asp Phe Val Glu Lys Leu Arg Glu Met Met Glu
145                 150                 155                 160

Glu Ile Glu Asn Ala Ile Asn Thr Phe Lys Glu Glu Gln Arg Leu Ile
                165                 170                 175

Tyr Glu Glu Leu Ile Lys Glu Glu Lys Thr Thr Asn Asn Glu Leu Ser
            180                 185                 190

Ala Ile Ser Arg Lys Ile Asp Thr Trp Ala Leu Gly Asn Ser Glu Thr
        195                 200                 205

Glu Lys Ala Phe Arg Ala Ile Ser Ser Lys Val Pro Val Asp Lys Val
    210                 215                 220

Thr Pro Ser Thr Leu Pro Glu Glu Val Leu Asp Phe Glu Lys Phe Leu
225                 230                 235                 240

Gln Gln Thr Gly Gly Arg Gln Gly Ala Trp Asp Val Ile Thr Arg Thr
                245                 250                 255

Leu Xaa

<210> 199
<211> 59
<212> PRT
<213> Homo sapiens

<220>
<221> misc feature
<222> (11)
<223> Xaa equals any one of the naturally occurring L-amino acids

<220>
<221> misc feature
<222> (59)
<223> Xaa equals stop translation

<400> 199
Met Leu Cys Leu Leu Val Leu Thr Gly Leu Xaa Val Leu Ile Val Gly
1               5                   10                  15

Ile His Ile Leu Glu Leu Leu Ile Asp Glu Ala Ala Met Pro Arg Gly

20                  25                  30

Met Gln Gly Thr Ser Leu Gly Gln Val Ser Phe Ser Lys Leu Gly Ser
        35                  40                  45

Phe Ala Ser Ser Ala Ser Leu Ser Ala Arg Xaa
    50                  55


<210> 200
<211> 34
<212> PRT
<213> Homo sapiens

<220>
<221> misc feature
<222> (34)
<223> Xaa equals stop translation

<400> 200
Met Phe Phe Val Leu Leu Cys Phe Trp Leu Phe Pro Phe Ser Lys Asn
  1               5                  10                  15

Ser Pro Leu Trp Gly Met Leu Arg Ser Ser Phe Phe Ile Ser Ile Asn
             20                  25                  30

Leu Xaa



<210> 201
<211> 26
<212> PRT
<213> Homo sapiens

<220>
<221> misc feature
<222> (26)
<223> Xaa equals stop translation

<400> 201
Met Ser Leu Ile Leu Leu Leu Ser Val Thr Leu Leu His Leu Ser Phe
  1               5                  10                  15

Ser Val Gly Phe Phe Leu Phe Arg Leu Xaa
             20                  25


<210> 202
<211> 34
<212> PRT
<213> Homo sapiens

<220>
<221> misc feature
<222> (34)
<223> Xaa equals stop translation

<400> 202
Met Lys Ser Val Ile Phe Ile Gln Ser Val Ile Leu Phe Phe Leu Pro

1                   5                   10                  15

Met Ser Gly Asp His Gln Gly Ile Ser Gly Leu Asp Glu Leu Pro Gln
                20                  25                  30

Ala Xaa



<210> 203
<211> 58
<212> PRT
<213> Homo sapiens

<400> 203
Met Ser Ser Phe Leu Arg Val Ile Phe Ile Pro Asn Ile Lys Val Ile
1               5                   10                  15

Phe Leu Pro Pro Gly Thr Thr Ser Leu Ile His Thr Met Asp Gln Gly
                20                  25                  30

Val Ile Ala Ala Phe Lys Phe Tyr Tyr Leu Arg Arg Glu Asp Phe Cys
        35                  40                  45

Pro Val Pro Tyr Cys Ser Gly Gly Arg His
    50                  55


<210> 204
<211> 75
<212> PRT
<213> Homo sapiens

<220>
<221> misc feature
<222> (66)
<223> Xaa equals any one of the naturally occurring L-amino acids

<220>
<221> misc feature
<222> (73)
<223> Xaa equals any one of the naturally occurring L-amino acids

<400> 204
Met Lys Pro Thr Leu Ser Lys Phe Leu Gly Thr Asp Ala Glu Leu Pro
1               5                   10                  15

Lys Leu Tyr Pro Pro Ser Leu Gln Ala Pro Arg Gly Glu Thr Gln Leu
                20                  25                  30

Leu Gly Pro Gly Leu Glu Arg Pro Thr Arg Glu Gly Arg Val Glu Gln
        35                  40                  45

Met Leu Phe Asn Gln Lys Ser Val Ser Trp Gly Ser Gln Leu Pro Gln
    50                  55                  60

Ser Xaa Asn Thr Phe Leu Lys Asn Xaa Asp Pro
65                  70                  75

<210> 205
<211> 66
<212> PRT
<213> Homo sapiens

<220>
<221> misc feature
<222> (63)
<223> Xaa equals any one of the naturally occurring L-amino acids

<400> 205
Met Thr Trp Lys Gly Trp Ser Arg Thr Arg Ile Trp Lys Pro Ser Leu
1               5                   10                  15

Pro Gln Leu Phe Thr Met Tyr Leu Leu Ala Gln Ile Arg Ala Ala Ser
            20                  25                  30

Arg Ala Ser Glu Asp Ser Cys Ser Tyr Ser Ser Asp Thr Met Trp Pro
        35                  40                  45

Gln Ser Gly Asn Ser Ser Thr Phe Ala Phe Phe Arg Pro Arg Xaa Lys
    50                  55                  60

Met Arg
65


<210> 206
<211> 44
<212> PRT
<213> Homo sapiens

<220>
<221> misc feature
<222> (44)
<223> Xaa equals stop translation

<400> 206
Met Leu Ser Phe Val Ser Arg Cys His Trp Ser Ser Ile Ala Glu Glu
1               5                   10                  15

Ser Glu Phe Leu Phe Leu Ile Leu Val Cys Tyr Phe Ser Ser Ser Cys
            20                  25                  30

Ser Ser Cys Ile Ile His Gln Trp Tyr Tyr Val Xaa
        35                  40


<210> 207
<211> 32
<212> PRT
<213> Homo sapiens

<220>
<221> misc feature
<222> (32)
<223> Xaa equals stop translation

<400> 207
Met Leu Gln Thr Leu Ile Leu Ile Phe Leu Leu Leu Leu Pro Cys Tyr

1                   5                   10                  15

Leu Glu Leu Leu Cys Phe Ser Leu Ile Ser Ser Ser Ala Lys Thr Xaa
                20                  25                  30

<210> 208
<211> 48
<212> PRT
<213> Homo sapiens

<220>
<221> misc feature
<222> (11)
<223> Xaa equals any one of the naturally occurring L-amino acids

<220>
<221> misc feature
<222> (48)
<223> Xaa equals stop translation

<400> 208
Met Thr Pro Trp Leu Leu Ile Leu Val Ser Xaa Gly Phe Leu Lys Ser
 1               5                  10                  15

Ile Ser Asp Pro Gln Phe Gln Glu Leu Ser Ile Asn Ile Ala Ser Cys
                20                  25                  30

His Pro Gly Thr Val Met Pro Tyr Ser Gly Thr Ser His Leu Lys Xaa
            35                  40                  45

<210> 209
<211> 37
<212> PRT
<213> Homo sapiens

<220>
<221> misc feature
<222> (37)
<223> Xaa equals stop translation

<400> 209
Met Thr Gly Thr Pro Ala Trp Ala His Leu Leu Leu Leu Leu Leu Leu
 1               5                  10                  15

Gly Ser Ala Pro Gln Thr Arg Leu Trp Pro Pro Ser Gln Cys Pro Val
                20                  25                  30

Thr Ser Pro Glu Xaa
            35

<210> 210

<211> 74
<212> PRT
<213> Homo sapiens

<220>
<221> misc feature
<222> (74)
<223> Xaa equals stop translation

<400> 210
Met Gly Val Lys Leu Glu Ile Phe Arg Met Ile Ile Tyr Leu Thr Phe
1               5                   10                  15

Pro Val Ala Met Phe Trp Val Ser Asn Gln Ala Glu Trp Phe Glu Asp
            20                  25                  30

Asp Val Ile Gln Arg Lys Arg Glu Leu Trp Pro Pro Glu Lys Leu Gln
        35                  40                  45

Glu Ile Glu Glu Phe Lys Glu Arg Leu Arg Lys Arg Arg Glu Glu Lys
    50                  55                  60

Leu Leu Arg Asp Ala Gln Gln Asn Ser Xaa
65                  70


<210> 211
<211> 41
<212> PRT
<213> Homo sapiens

<220>
<221> misc feature
<222> (41)
<223> Xaa equals stop translation

<400> 211
Met Pro Phe Ser Ser Ser Val Lys Cys Leu Phe Gly Val Leu Leu Arg
1               5                   10                  15

Phe Cys Phe Val Val Phe Ser Val Val Val Phe Thr Phe Phe Leu Ser
            20                  25                  30

Ile Pro Lys Arg Thr Leu Gly Tyr Xaa
        35                  40


<210> 212
<211> 54
<212> PRT
<213> Homo sapiens

<220>
<221> misc feature
<222> (54)
<223> Xaa equals stop translation

<400> 212
Met Trp His Leu Ser Phe His Cys Leu Leu Leu Leu Leu Pro Leu Cys
1               5                   10                  15

Glu Val Thr His Ser Leu Phe Ala Phe Tyr His Asn Trp Lys Leu Phe
            20                  25                  30

Glu Ala Ser Leu Glu Thr Glu Ala Ala Met Leu Pro Val Gln Pro Ala
        35                  40                  45

Glu Pro Arg Ala Asn Xaa
    50


<210> 213
<211> 63
<212> PRT
<213> Homo sapiens

<220>
<221> misc feature
<222> (63)
<223> Xaa equals stop translation

<400> 213
Met Pro Glu Asn Leu Val Leu Ile Leu Ala Leu Leu Leu Ser Val Cys
 1               5                  10                  15

Gly Leu Lys Gln Val Ile Phe Leu Ser Ala Ser Ile Tyr Ser Lys Met
            20                  25                  30

Cys Thr Leu Ile Ala Thr Lys Lys Val Val Ala Lys Thr Arg Asn Asp
        35                  40                  45

Ala Tyr Trp Tyr Leu Ile Ser Leu Lys His Ile Val Gly Phe Xaa
    50                  55                  60


<210> 214
<211> 176
<212> PRT
<213> Homo sapiens

<220>
<221> misc feature
<222> (142)
<223> Xaa equals any one of the naturally occurring L-amino acids

<220>
<221> misc feature
<222> (149)
<223> Xaa equals any one of the naturally occurring L-amino acids

<220>
<221> misc feature
<222> (155)
<223> Xaa equals any one of the naturally occurring L-amino acids

<220>
<221> misc feature
<222> (158)
<223> Xaa equals any one of the naturally occurring L-amino acids

<220>
<221> misc feature
<222> (160)
<223> Xaa equals any one of the naturally occurring L-amino acids

<220>
<221> misc feature
<222> (163)
<223> Xaa equals any one of the naturally occurring L-amino acids

<220>
<221> misc feature
<222> (176)
<223> Xaa equals stop translation

<400> 214
Met Tyr Trp Ile Val Phe Ala Leu Tyr Thr Val Ile Glu Thr Val Ala
1               5                   10                  15

Asp Gln Thr Val Ala Trp Phe Pro Leu Tyr Tyr Glu Leu Lys Ile Ala
            20                  25                  30

Phe Val Ile Trp Leu Leu Ser Pro Tyr Thr Lys Gly Ala Ser Leu Ile
        35                  40                  45

Tyr Arg Lys Phe Leu His Pro Leu Leu Ser Ser Lys Glu Arg Glu Ile
    50                  55                  60

Asp Asp Tyr Ile Val Gln Ala Lys Glu Arg Gly Tyr Glu Thr Met Val
65                  70                  75                  80

Asn Phe Gly Arg Gln Gly Leu Asn Leu Ala Ala Thr Ala Ala Val Thr
                85                  90                  95

Ala Ala Val Lys Ser Gln Gly Ala Ile Thr Glu Arg Leu Arg Ser Phe
            100                 105                 110

Ser Met His Asp Leu Thr Thr Ile Gln Gly Asp Glu Pro Val Gly Gln
        115                 120                 125

Arg Pro Tyr Gln Pro Leu Pro Glu Ala Lys Lys Lys Ser Xaa Gln Pro
    130                 135                 140

Pro Val Asn Gln Xaa Val Met Glu Phe His Xaa Lys Thr Xaa Met Xaa
145                 150                 155                 160

Lys Gln Xaa Lys Lys Gln Arg Gly His Ile Gln Ile Met Arg Cys Xaa
                165                 170                 175

<210> 215
<211> 40
<212> PRT
<213> Homo sapiens

<220>
<221> misc feature

<222> (40)
<223> Xaa equals stop translation

<400> 215
Met Arg Glu Cys Tyr Phe Leu Gly Asn Phe Leu Leu Val Phe Leu Ile
1               5                   10                  15

Leu Ala Ser Ser Phe Ile Tyr Val Leu Val Thr Gln Val Leu Gly Gly
            20                  25                  30

Pro Ala Thr Leu Leu Ala Phe Xaa
        35                  40


<210> 216
<211> 55
<212> PRT
<213> Homo sapiens

<220>
<221> misc feature
<222> (55)
<223> Xaa equals stop translation

<400> 216
Met Val Leu Gln Asn Thr Asn Thr Leu Leu Ile Val Ser Ala Phe Leu
1               5                   10                  15

Leu Ser Met Leu Phe Phe Lys Phe Ser Ile Ala Ile Phe Leu Val Thr
            20                  25                  30

Asn Leu Ser Phe Glu Arg Ser Asn Leu Leu Leu Gly Pro Ser Ser Asp
        35                  40                  45

Leu Phe Leu Asn Phe Lys Xaa
    50                  55


<210> 217
<211> 47
<212> PRT
<213> Homo sapiens

<220>
<221> misc feature
<222> (47)
<223> Xaa equals stop translation

<400> 217
Met Tyr Ile Phe His Phe Val Phe Leu Ile Gly Tyr Ala Met Cys Gly
1               5                   10                  15

Ile Gln Val Thr Asn Val Thr Leu Ala Ser Gly Pro Ser Asn Leu His
            20                  25                  30

Val Tyr Leu Leu Gln Ser Tyr Leu Thr Arg Gly Pro Asn His Xaa
        35                  40                  45


<210> 218

<211> 180
<212> PRT
<213> Homo sapiens

<220>
<221> misc feature
<222> (180)
<223> Xaa equals stop translation

<400> 218
Met Leu Tyr Tyr Leu Trp Met Leu His Ser Val Thr Leu Phe Leu Asn
1               5                   10                  15

Leu Leu Ala Cys Leu Ala Trp Phe Ser Gly Asn Ser Ser Lys Gly Val
            20                  25                  30

Asp Phe Gly Leu Ser Ile Leu Trp Phe Leu Ile Phe Thr Pro Cys Ala
        35                  40                  45

Phe Leu Cys Trp Tyr Arg Pro Ile Tyr Lys Ala Phe Arg Ser Asp Asn
    50                  55                  60

Ser Phe Ser Phe Phe Val Phe Phe Phe Val Phe Phe Cys Gln Ile Gly
65                  70                  75                  80

Ile Tyr Ile Ile Gln Leu Val Gly Ile Pro Gly Leu Gly Asp Ser Gly
                85                  90                  95

Trp Ile Ala Ala Leu Ser Thr Leu Asp Asn His Ser Leu Ala Ile Ser
            100                 105                 110

Val Ile Met Met Val Val Ala Gly Phe Phe Thr Leu Cys Ala Val Leu
        115                 120                 125

Ser Val Phe Leu Leu Gln Arg Val His Ser Leu Tyr Arg Arg Thr Gly
    130                 135                 140

Ala Ser Phe Gln Gln Ala Gln Glu Glu Phe Ser Gln Gly Ile Phe Ser
145                 150                 155                 160

Ser Arg Thr Phe His Arg Ala Ala Ser Ser Ala Ala Gln Gly Ala Phe
                165                 170                 175

Gln Gly Asn Xaa
            180


<210> 219
<211> 99
<212> PRT
<213> Homo sapiens

<220>
<221> misc feature
<222> (99)
<223> Xaa equals stop translation

<400> 219
Met Lys Leu Met Val Leu Val Phe Thr Ile Gly Leu Thr Leu Leu Leu
1               5                   10                  15

Gly Val Gln Ala Met Pro Ala Asn Arg Leu Ser Cys Tyr Arg Lys Ile
            20                  25                  30

Leu Lys Asp His Asn Cys His Asn Leu Pro Glu Gly Val Ala Asp Leu
        35                  40                  45

Thr Gln Ile Asp Val Asn Val Gln Asp His Phe Trp Asp Gly Lys Gly
    50                  55                  60

Cys Glu Met Ile Cys Tyr Cys Asn Phe Ser Glu Leu Leu Cys Cys Pro
65                  70                  75                  80

Lys Asp Val Phe Phe Gly Pro Lys Ile Ser Phe Val Ile Pro Cys Asn
                85                  90                  95

Asn Gln Xaa

<210> 220
<211> 44
<212> PRT
<213> Homo sapiens

<220>
<221> misc feature
<222> (44)
<223> Xaa equals stop translation

<400> 220
Met Gly Gly Lys Gly Ile Asn Tyr Thr Met Pro His Ile Cys Leu Leu
  1               5                  10                  15

Leu Leu Asn Ala Leu Val Val Ser Cys Leu Leu Leu Glu Ala Ile Leu
            20                  25                  30

Leu Gln His Leu Val Leu Cys Asn Glu Leu Pro Xaa
        35                  40

<210> 221
<211> 42
<212> PRT
<213> Homo sapiens

<220>
<221> misc feature
<222> (42)
<223> Xaa equals stop translation

<400> 221
Met Phe Met Leu Cys Asn Leu Leu Leu Pro Leu Leu Glu Phe Ile Phe
  1               5                  10                  15

Gly Ser Thr Tyr Leu Ser Thr Asp Leu Tyr Leu His Thr Cys Met Lys
            20                  25                  30

Asn Val Phe Leu His Ile His Ser Phe Xaa
        35                  40

<210> 222
<211> 52
<212> PRT
<213> Homo sapiens

<220>
<221> misc feature
<222> (52)
<223> Xaa equals stop translation

<400> 222
Met Ala Val Pro Ser Gly Cys Trp Pro Ser Trp Pro Arg Pro Ser Ser
1               5                   10                  15

Trp Trp Ser Thr Arg Ile Ser Pro Arg Ser Ala Thr Pro Leu Thr Ala
            20                  25                  30

Ser Thr Trp Ser Leu Val Thr Cys Ser Ser Gln Val Ser Ala Cys Gly
        35                  40                  45

Thr Ser Ile Xaa
    50


<210> 223
<211> 32
<212> PRT
<213> Homo sapiens

<220>
<221> misc feature
<222> (32)
<223> Xaa equals stop translation

<400> 223
Met Val Ser Leu Asn Leu Ser Leu Pro Asn Asn Ile Ile Ser Leu Val
1               5                   10                  15

Phe Phe Phe Leu Leu Gln Pro Val Gln Lys Gly Val Ser Gly Gly Xaa
            20                  25                  30


<210> 224
<211> 54
<212> PRT
<213> Homo sapiens

<220>
<221> misc feature
<222> (54)
<223> Xaa equals stop translation

<400> 224
Met Leu Val Leu Met Thr Thr Cys Ile Leu Ala Ala Val Cys Val His
1               5                   10                  15

Thr Ala Gln Cys Ala Pro Asp Ser Arg Met Asp Asn Asp Cys Pro Ser
            20                  25                  30

His Gln Ala Gln Ile His Phe Arg Ala Ser Glu Val Arg Arg Gly Trp
        35                  40                  45

Thr Phe Asn His Asp Xaa
    50


<210> 225
<211> 41
<212> PRT
<213> Homo sapiens

<220>
<221> misc feature
<222> (41)
<223> Xaa equals stop translation

<400> 225
Met Gly Pro Ser Gln Arg Glu Val Thr Val Gln Trp His Arg Ala Leu
 1               5                  10                  15

Phe Leu Leu Pro Leu Leu Leu Leu Ser Thr Arg Thr Glu Thr Lys Asn
            20                  25                  30

Phe Gly Phe Lys Trp Leu Lys Asp Xaa
        35                  40


<210> 226
<211> 31
<212> PRT
<213> Homo sapiens

<220>
<221> misc feature
<222> (31)
<223> Xaa equals stop translation

<400> 226
Met Gln Leu Ser Lys Phe Leu Leu Phe Leu Phe Val Tyr Thr Arg Glu
 1               5                  10                  15

Asn Pro Thr Ser Ala Cys Val Trp Gly Glu Lys Ser Thr Val Xaa
            20                  25                  30


<210> 227
<211> 60
<212> PRT
<213> Homo sapiens

<220>
<221> misc feature
<222> (60)
<223> Xaa equals stop translation

<400> 227
Met Val Val Val Ser Thr Asn Gly Phe Leu Leu Leu Leu Leu Phe Leu
1               5                   10                  15

Asn Arg Lys Ser Gly Leu Cys Ser Tyr Arg Lys Ala Val His Arg Leu
            20                  25                  30

Ser Ser Cys Pro Ser Arg His Gln Ala Gly Pro Arg Ile Lys Cys Asp
        35                  40                  45

Phe Lys Trp Gly Lys Leu Cys Tyr Ser Cys Ala Xaa
    50                  55                  60


<210> 228
<211> 35
<212> PRT
<213> Homo sapiens

<220>
<221> misc feature
<222> (35)
<223> Xaa equals stop translation

<400> 228
Met Gly Trp Gly Lys Glu Val Val Ser Leu Ile Val Leu Leu Val Asn
1               5                   10                  15

Leu Phe Leu Cys Pro Trp Ala Leu Gly Leu Cys Leu Leu Ser Val Ser
            20                  25                  30

Ser Leu Xaa
        35


<210> 229
<211> 39
<212> PRT
<213> Homo sapiens

<220>
<221> misc feature
<222> (39)
<223> Xaa equals stop translation

<400> 229
Met Met Asn Ile Leu Leu Leu Lys Tyr Ile Leu Glu Ile Leu Ile Leu
1               5                   10                  15

Ser Glu Asn Leu Asn Leu Phe Asn Ile Thr Tyr Gly Lys Tyr Asn Leu
            20                  25                  30

Phe Phe Leu Tyr Arg Tyr Xaa
        35


<210> 230
<211> 39
<212> PRT
<213> Homo sapiens

<220>
<221> misc feature
<222> (39)
<223> Xaa equals stop translation

<400> 230
Met Tyr Ile Phe Tyr Leu Tyr Lys Ile Tyr Ile Tyr Thr His Ile Cys
1               5                   10                  15

Ile Tyr Ile Pro Leu Phe Leu Cys Leu Leu Ile Leu Ala Ile Lys Glu
            20                  25                  30

Gly Ala Ala Phe Asn Val Xaa
        35


<210> 231
<211> 62
<212> PRT
<213> Homo sapiens

<220>
<221> misc feature
<222> (62)
<223> Xaa equals stop translation

<400> 231
Met Asn Glu Ser Val Tyr Asp Asp Ser Thr Ser Ser Tyr Thr Pro Ser
1               5                   10                  15

Leu His Ile Leu Gly Cys Leu Leu Leu Leu Phe Leu Gly Val Glu Arg
            20                  25                  30

Ala Leu Glu Pro Phe Ser Gly Leu Cys Ala Ser Leu His Asp Val Arg
        35                  40                  45

Pro Ile Val Asn Pro Leu Thr Ser Phe Ser Leu Ile Tyr Xaa
    50                  55                  60


<210> 232
<211> 198
<212> PRT
<213> Homo sapiens

<220>
<221> misc feature
<222> (198)
<223> Xaa equals stop translation

<400> 232
Met Cys Thr Gly Lys Cys Ala Arg Cys Val Gly Leu Ser Leu Ile Thr
1               5                   10                  15

Leu Cys Leu Val Cys Ile Val Ala Asn Ala Leu Leu Leu Val Pro Asn
            20                  25                  30

Gly Glu Thr Ser Trp Thr Asn Thr Asn His Leu Ser Leu Gln Val Trp
        35                  40                  45

Leu Met Gly Gly Phe Ile Gly Gly Gly Leu Met Val Leu Cys Pro Gly
50                  55                  60

Ile Ala Ala Val Arg Ala Gly Gly Lys Gly Cys Cys Gly Ala Gly Cys
65                  70                  75                  80

Cys Gly Asn Arg Cys Arg Met Leu Arg Ser Val Phe Ser Ser Ala Phe
                85                  90                  95

Gly Val Leu Gly Ala Ile Tyr Cys Leu Ser Val Ser Gly Ala Gly Leu
            100                 105                 110

Arg Asn Gly Pro Arg Cys Leu Met Asn Gly Glu Trp Gly Tyr His Phe
        115                 120                 125

Glu Asp Thr Ala Gly Ala Tyr Leu Leu Asn Arg Thr Leu Trp Asp Arg
    130                 135                 140

Cys Glu Ala Pro Pro Arg Val Val Pro Trp Asn Val Thr Leu Phe Ser
145                 150                 155                 160

Leu Leu Val Ala Ala Ser Cys Leu Glu Ile Val Leu Cys Gly Ile Gln
                165                 170                 175

Leu Val Asn Ala Thr Ile Gly Val Phe Cys Gly Asp Cys Arg Lys Lys
            180                 185                 190

Gln Asp Thr Pro His Xaa
        195

<210> 233
<211> 62
<212> PRT
<213> Homo sapiens

<220>
<221> misc feature
<222> (62)
<223> Xaa equals stop translation

<400> 233
Met Ser Gln Leu Phe Leu Ile Met Leu Thr Phe Ile Phe Leu Asn Asn
1               5                   10                  15

Met Phe Ile Met His Leu Thr Ser Phe His Gly Lys Arg Val Phe Gly
            20                  25                  30

Phe Leu Asn Gln Ser Ser His Met His Ala Phe Pro Leu Pro Arg Trp
        35                  40                  45

Thr Thr Ser Ile Phe Ser Val Ser Ile Phe Ile Asn Arg Xaa
    50                  55                  60

<210> 234
<211> 81
<212> PRT
<213> Homo sapiens

<220>
<221> misc feature
<222> (81)
<223> Xaa equals stop translation

<400> 234
Met Ala Phe Leu Pro Leu Thr Leu Thr Phe Cys Leu Ala Pro Leu Ala
1               5                   10                  15

Pro Leu Leu Pro Ser Ile Trp Gly Pro Thr Pro Ala Ser Cys Val Val
            20                  25                  30

Trp Pro Leu Leu Thr Ile Leu Pro Val Pro Ala Gln Ala Ser Pro Ser
        35                  40                  45

Thr Asp Thr Ala His Leu Trp Gln Arg Pro Thr Thr Gly Ser Pro Thr
    50                  55                  60

Arg Leu Val Arg Pro Leu Pro Arg Pro Gly Leu Pro Pro Met Trp Ala
65                  70                  75                  80

Xaa


<210> 235
<211> 111
<212> PRT
<213> Homo sapiens

<400> 235
Met Gly Gly Leu Glu Pro Cys Ser Arg Leu Leu Leu Leu Pro Leu Leu
1               5                   10                  15

Leu Ala Val Gly Leu Arg Pro Val Gln Ala Gln Ala Gln Ser Asp Cys
            20                  25                  30

Ser Cys Ser Thr Val Ser Pro Gly Val Leu Ala Gly Ile Val Met Gly
        35                  40                  45

Asp Leu Val Leu Thr Val Leu Ile Ala Leu Ala Val Tyr Phe Leu Gly
    50                  55                  60

Arg Leu Val Pro Arg Gly Arg Gly Ala Ala Glu Ala Thr Arg Lys Gln
65                  70                  75                  80

Arg Ile Thr Glu Thr Glu Ser Pro Tyr Gln Glu Leu Gln Gly Gln Arg
                85                  90                  95

Ser Asp Val Tyr Ser Asp Leu Asn Thr Gln Arg Pro Tyr Tyr Lys
            100                 105                 110


<210> 236
<211> 33
<212> PRT
<213> Homo sapiens

<220>

<221> misc feature
<222> (33)
<223> Xaa equals stop translation

<400> 236
Met Gln Arg Met Leu Val Leu Leu Phe Phe Phe Phe Ser Leu Leu Ala
1               5                   10                  15

Ile Asn Pro Ala Glu Thr Ile Cys Gly Tyr Gly Ser Thr Trp Lys Phe
            20                  25                  30

Xaa


<210> 237
<211> 229
<212> PRT
<213> Homo sapiens

<220>
<221> misc feature
<222> (134)
<223> Xaa equals any one of the naturally occurring L-amino acids

<220>
<221> misc feature
<222> (229)
<223> Xaa equals stop translation

<400> 237
Met Val Leu Gly Leu Phe Val Pro Pro Val Phe Val Val Ser Tyr Ala
1               5                   10                  15

Lys Asp Leu Gly Val Pro Asp Thr Lys Ala Ala Phe Leu Leu Thr Ile
            20                  25                  30

Leu Gly Phe Ile Asp Ile Phe Ala Arg Pro Ala Ala Gly Phe Val Ala
        35                  40                  45

Gly Leu Gly Lys Val Arg Pro Tyr Ser Val Tyr Leu Phe Ser Phe Ser
    50                  55                  60

Met Phe Phe Asn Gly Leu Ala Asp Leu Ala Gly Ser Thr Ala Gly Asp
65                  70                  75                  80

Tyr Gly Gly Leu Val Val Phe Cys Ile Phe Phe Gly Ile Ser Tyr Gly
                85                  90                  95

Met Val Gly Ala Leu Gln Phe Glu Val Leu Met Ala Ile Val Gly Thr
            100                 105                 110

His Lys Phe Ser Ser Ala Ile Gly Leu Val Leu Leu Met Glu Ala Val
        115                 120                 125

Ala Val Leu Val Gly Xaa Pro Ser Gly Gly Lys Leu Leu Asp Ala Thr
    130                 135                 140

His Val Tyr Met Tyr Val Phe Ile Leu Ala Gly Ala Glu Val Leu Thr
145                 150                 155                 160

Ser Ser Leu Ile Leu Leu Leu Gly Asn Phe Phe Cys Ile Arg Lys Lys
                165                 170                 175

Pro Lys Glu Pro Gln Pro Glu Val Ala Ala Ala Glu Glu Glu Lys Leu
            180                 185                 190

His Lys Pro Pro Ala Asp Ser Gly Val Asp Leu Arg Glu Val Glu His
        195                 200                 205

Phe Leu Lys Ala Glu Pro Glu Lys Asn Gly Glu Val Val His Thr Pro
    210                 215                 220

Glu Thr Ser Val Xaa
225


<210> 238
<211> 117
<212> PRT
<213> Homo sapiens

<220>
<221> misc feature
<222> (117)
<223> Xaa equals stop translation

<400> 238
Met Thr Pro Leu Leu Thr Leu Ile Leu Val Val Leu Met Gly Leu Pro
  1               5                  10                  15

Leu Ala Gln Ala Leu Asp Cys His Val Cys Ala Tyr Asn Gly Asp Asn
             20                  25                  30

Cys Phe Asn Pro Met Arg Cys Pro Ala Met Val Ala Tyr Cys Met Thr
         35                  40                  45

Thr Arg Thr Tyr Tyr Thr Pro Thr Arg Met Lys Val Ser Lys Ser Cys
     50                  55                  60

Val Pro Arg Cys Phe Glu Thr Val Tyr Asp Gly Tyr Ser Lys His Ala
 65                  70                  75                  80

Ser Thr Thr Ser Cys Cys Gln Tyr Asp Leu Cys Asn Gly Thr Gly Leu
                 85                  90                  95

Ala Thr Pro Ala Thr Leu Ala Leu Ala Pro Ile Leu Leu Ala Thr Leu
            100                 105                 110

Trp Gly Leu Leu Xaa
        115


<210> 239
<211> 37
<212> PRT
<213> Homo sapiens

<220>
<221> misc feature

<222> (37)
<223> Xaa equals stop translation

<400> 239
Met Leu Thr Trp Leu Asp Leu Asp Leu Leu Phe Cys Phe Leu Phe Leu
1               5                   10                  15

Phe Leu Phe Ile Leu Phe Tyr Phe Leu Gln Leu Asn Glu Phe Trp Gly
            20                  25                  30

Gly Asn Pro Phe Xaa
        35


<210> 240
<211> 39
<212> PRT
<213> Homo sapiens

<220>
<221> misc feature
<222> (39)
<223> Xaa equals stop translation

<400> 240
Ser Leu Gly Val Gly Phe Phe Phe Phe Phe Phe Ser Ser Leu Lys Glu
1               5                   10                  15

Pro Ala Val Ala Leu Cys Val Phe Cys Phe Cys Phe Pro Phe Gly Gly
            20                  25                  30

Pro Met Gly Lys Ser Phe Xaa
        35


<210> 241
<211> 48
<212> PRT
<213> Homo sapiens

<220>
<221> misc feature
<222> (48)
<223> Xaa equals stop translation

<400> 241
Met Gln Ser Gly Arg Ser Trp Ala Leu Lys Met Val Leu Leu Cys Asn
1               5                   10                  15

Ser Cys Leu Gly Leu Gly Val Gly Ser Val Gly Pro Ser Met Ser Ser
            20                  25                  30

Leu Phe Gly Ala Val Leu Ser Glu Thr Pro Gly Ser Ser Val Tyr Xaa
        35                  40                  45


<210> 242

<211> 32
<212> PRT
<213> Homo sapiens

<220>
<221> misc feature
<222> (32)
<223> Xaa equals stop translation

<400> 242
Met Ile Thr Leu Cys Ile Phe Leu Leu Phe Lys Val Phe Val Gly Ile
1               5                   10                  15

Ile Leu His Tyr Leu Ile Gly Lys Asn Ile Tyr Val Tyr Ser Val Xaa
            20                  25                  30

<210> 243
<211> 64
<212> PRT
<213> Homo sapiens

<220>
<221> misc feature
<222> (64)
<223> Xaa equals stop translation

<400> 243
Met Ala Ser Leu Leu Gln Arg Asn Leu Cys Pro Arg Leu Ser Val Cys
1               5                   10                  15

Leu Val Phe Ile Gln Val Phe Val Cys Cys Val Glu Gly Gly Gly Arg
            20                  25                  30

Arg Val Lys Ala Val Leu Phe Arg Ala Pro Phe Gly Glu His Ser Arg
        35                  40                  45

Gln Asn Thr Leu Val Ile Pro Ser Gln Thr Gly Leu Gln Ala His Xaa
    50                  55                  60

<210> 244
<211> 68
<212> PRT
<213> Homo sapiens

<220>
<221> misc feature
<222> (68)
<223> Xaa equals stop translation

<400> 244
Met Pro Val Tyr Asp Phe Asn Trp Trp Tyr Ser Leu Tyr Phe Ile Ile
1               5                   10                  15

Tyr Ile Ile Ile Asn Thr Tyr Ile Phe Lys Ser Val Phe Leu Ala Met
            20                  25                  30

Val Tyr Ser Asn Tyr Arg Lys His Phe His Ile Leu Cys Val Cys Val
        35                  40                  45

Cys Val Phe Cys Ser Asp Glu Gln Asn Leu Leu Phe Thr Gln Phe Tyr
    50                  55                  60

Tyr Leu Ser Xaa
65


<210> 245
<211> 46
<212> PRT
<213> Homo sapiens

<220>
<221> misc feature
<222> (43)
<223> Xaa equals any one of the naturally occurring L-amino acids

<220>
<221> misc feature
<222> (46)
<223> Xaa equals stop translation

<400> 245
Met Ser Asp Lys Leu Ser Pro Ser Thr Val Pro Leu Leu Leu Pro Val
 1               5                  10                  15

Leu Phe Lys Val Thr Ile Leu Leu Gln Arg Val Cys Pro Glu Asp Ser
            20                  25                  30

Pro Ser Ser Ser Val Leu Pro Glu Ser Val Xaa Arg Glu Xaa
        35                  40                  45


<210> 246
<211> 43
<212> PRT
<213> Homo sapiens

<220>
<221> misc feature
<222> (43)
<223> Xaa equals stop translation

<400> 246
Met Arg Lys Glu Glu Gly Ile Ala His Leu Ser Ile Ala Phe Phe Val
 1               5                  10                  15

Gln Val Leu Cys Leu Tyr Gln Leu Leu Pro Val Ile Leu Pro Gln Phe
            20                  25                  30

Asn Leu Gly Ser Gly Lys Asn Met Asn Arg Xaa
        35                  40

<210> 247
<211> 32
<212> PRT
<213> Homo sapiens

<220>
<221> misc feature
<222> (11)
<223> Xaa equals any one of the naturally occurring L-amino acids

<220>
<221> misc feature
<222> (32)
<223> Xaa equals stop translation

<400> 247
Met Ile His Val Leu Thr Phe Leu Leu Gln Xaa Tyr Ile Leu Ile Ser
1               5                   10                  15

Lys Gly Lys Gly Asp Val Ser Gln Phe Val Lys Ser Arg Glu Tyr Xaa
            20                  25                  30

<210> 248
<211> 24
<212> PRT
<213> Homo sapiens

<220>
<221> misc feature
<222> (24)
<223> Xaa equals stop translation

<400> 248
Met Ser Glu Leu Ser Ala Phe Met Phe Ser Thr Ile Ile Phe Leu Met
1               5                   10                  15

Ala Gln Pro Thr Ser Cys Phe Xaa
            20

<210> 249
<211> 80
<212> PRT
<213> Homo sapiens

<220>
<221> misc feature
<222> (36)
<223> Xaa equals any one of the naturally occurring L-amino acids

<220>
<221> misc feature
<222> (80)
<223> Xaa equals stop translation

<400> 249
Met Arg Val Phe Ala Leu Leu Pro Pro Phe His Lys Ser Thr Val Leu
1               5                   10                  15

Ser Phe Leu Leu Phe Phe Leu Ser Phe Phe Phe Phe Arg Gln Gly Leu
            20                  25                  30

Ala Val Ser Xaa Arg Leu Glu Cys Ser Gly Ala Ile Ile Ala His Cys
        35                  40                  45

Ser Leu Asp Leu Leu Asp Ser Ser Asn Pro Pro Ala Leu Thr Ser Gln
    50                  55                  60

Leu Leu Arg Arg Pro Arg Gln Glu Asp His Leu Ser Pro Gly Gly Xaa
65                  70                  75                  80

<210> 250
<211> 16
<212> PRT
<213> Homo sapiens

<220>
<221> misc feature
<222> (16)
<223> Xaa equals stop translation

<400> 250
Met Ser His Cys Ala Trp Leu His Leu Gln Leu Phe Leu Ser Leu Xaa
1               5                   10                  15

<210> 251
<211> 47
<212> PRT
<213> Homo sapiens

<220>
<221> misc feature
<222> (47)
<223> Xaa equals stop translation

<400> 251
Met Met Phe Cys Phe Leu Ile Trp Val Val Val Thr Phe Thr Tyr Ser
1               5                   10                  15

Leu Asn Cys Thr Phe Val Leu His Lys Phe Ile Ile Phe Pro Asn Phe
            20                  25                  30

Lys Lys Val Lys Arg Arg Arg Lys Lys Leu Val Met Lys Val Xaa
        35                  40                  45

<210> 252

<211> 32
<212> PRT
<213> Homo sapiens

<220>
<221> misc feature
<222> (32)
<223> Xaa equals stop translation

<400> 252
Met Pro Pro Pro Glu Cys Leu Ser Asp Cys Ser Lys Val Ala Leu Val
1               5                   10                  15

Met Val Leu Phe Leu Phe Leu His Gln Ser Ser Cys Trp Ala Ala Xaa
            20                  25                  30

<210> 253
<211> 36
<212> PRT
<213> Homo sapiens

<220>
<221> misc feature
<222> (36)
<223> Xaa equals stop translation

<400> 253
Met Ala Ser Ser Val Thr Val Lys Glu Val Cys Val Leu Phe Asn Leu
1               5                   10                  15

Leu Ile Ile Ile Thr Ala Met Val Tyr His Ser Phe Thr Lys Tyr Gln
            20                  25                  30

Thr Leu Phe Xaa
        35

<210> 254
<211> 51
<212> PRT
<213> Homo sapiens

<220>
<221> misc feature
<222> (51)
<223> Xaa equals stop translation

<400> 254
Met Ile Phe Leu Phe Phe Ile Leu Phe Glu Ile Ile Val Thr Leu Trp
1               5                   10                  15

Leu Thr Pro Thr Tyr Pro Gln Ala Phe Ser Glu Leu Thr Ile Gln Ile
            20                  25                  30

Thr Ala Pro Phe Gly Ser Leu Pro Gln Gln Leu Tyr Leu His Met Ser
        35                  40                  45

Ile Ile Xaa
    50

<210> 255
<211> 76
<212> PRT
<213> Homo sapiens

<400> 255
Met Phe Phe Leu Leu Ile Leu Cys Trp Leu Leu Cys Leu Ser Leu Ser
 1               5                  10                  15

Gly Leu Tyr Pro Arg Leu Leu Asn Pro Gly Gly Trp Leu Ser Leu Leu
            20                  25                  30

Ser Phe Gln Met Asp Tyr Gly Trp Ile Leu Pro Trp Gly Ala Cys Thr
        35                  40                  45

Val Arg His Gly Lys Pro Gly Met Gly Lys Arg Ser Gly Gly Ser Leu
    50                  55                  60

Pro His Leu Thr Ala Leu Val Leu Cys Leu Thr Ser
65                  70                  75

<210> 256
<211> 61
<212> PRT
<213> Homo sapiens

<220>
<221> misc feature
<222> (61)
<223> Xaa equals stop translation

<400> 256
Met Leu Leu Ser Asn Leu Ser Leu Ser Leu Gln Pro Leu Leu Phe Leu
 1               5                  10                  15

Phe Ser Phe Phe Leu Phe Cys Lys Met Gly Ser Arg Lys Gly Leu Arg
            20                  25                  30

His Lys Thr Gln His Phe Ser Ser Met Thr Asp Gln Ile Leu Lys Gly
        35                  40                  45

Ser Val Arg Ser Pro Ala Leu Gly Gln Leu His Asp Xaa
    50                  55                  60

<210> 257
<211> 37
<212> PRT
<213> Homo sapiens

<220>
<221> misc feature
<222> (37)
<223> Xaa equals stop translation

<400> 257
Met Tyr Glu Val Asp Lys Lys Ile Tyr Ser Asn Phe Ile Gln Ile Leu
1               5                   10                  15

Ile Val Ile Ile Phe Val Leu Tyr Leu Ile Ile Asn Gln Asn Thr Phe
            20                  25                  30

Ala Phe Leu Ser Xaa
        35


<210> 258
<211> 43
<212> PRT
<213> Homo sapiens

<220>
<221> misc feature
<222> (43)
<223> Xaa equals stop translation

<400> 258
Met Cys Ile Leu Pro Leu Met Leu Thr Tyr Pro Ile Leu Pro Lys Val
1               5                   10                  15

Val Gly Asn Asn Ile Leu Leu Gly Asp Ser Gly Leu Thr Ser Leu Val
            20                  25                  30

Ile Pro Leu Ser Val Val Phe Asn Leu Lys Xaa
        35                  40


<210> 259
<211> 39
<212> PRT
<213> Homo sapiens

<220>
<221> misc feature
<222> (39)
<223> Xaa equals stop translation

<400> 259
Met Ile Leu Val Ser Lys Leu Phe Phe Gly Phe Ser Leu Met Phe Leu
1               5                   10                  15

Ile Phe Phe Pro Leu Ala Thr Met Thr Val His Val Leu Ile Asn Ile
            20                  25                  30

Gly Arg Ser Arg Tyr Lys Xaa
        35


<210> 260
<211> 51
<212> PRT
<213> Homo sapiens

<220>

<221> misc feature
<222> (51)
<223> Xaa equals stop translation

<400> 260
Met Ser Ile Thr Ser Asn Thr Tyr Phe Phe Leu Leu Gly Ala Phe Lys
1               5                   10                  15

Ile Leu Ser Ser Ser Tyr Trp Lys Ile His Thr Lys Leu Leu Leu Thr
            20                  25                  30

Ile Val Pro Leu Gln Cys Cys Gly Met Pro Gln Leu Ile Pro Pro Leu
        35                  40                  45

Gln Leu Xaa
    50


<210> 261
<211> 76
<212> PRT
<213> Homo sapiens

<220>
<221> misc feature
<222> (76)
<223> Xaa equals stop translation

<400> 261
Met Phe Thr Thr Arg Phe Pro Lys Leu Leu Ile Phe Pro Lys Ile Val
1               5                   10                  15

Thr Glu Asn Cys Cys Leu Leu Phe Cys Ser Phe Trp Gly Trp Trp Cys
            20                  25                  30

Trp Leu Gly His Ala Cys Glu Val Met Cys Val Ser Asp Leu Thr Asp
        35                  40                  45

Ser Leu Phe Ser Leu Leu Ile Glu Arg Ala Leu Phe Thr Leu Phe Ile
    50                  55                  60

Cys Phe Asp Thr Ser Ala Phe Ser Val Leu Ser Xaa
65                  70                  75


<210> 262
<211> 45
<212> PRT
<213> Homo sapiens

<220>
<221> misc feature
<222> (45)
<223> Xaa equals stop translation

<400> 262
Met Thr Ser His Pro Ser Trp Arg Leu Ile Leu Val Thr Ser Leu Val
1               5                   10                  15

Leu Gly Val Glu Pro Glu Glu Ala Pro Gly Glu Ala Gly Glu Gly Ser

20                  25                  30

Gly Gly Gln Arg Thr Met Asp Pro Glu Gln Lys Trp Xaa
        35                  40                  45


<210> 263
<211> 53
<212> PRT
<213> Homo sapiens

<220>
<221> misc feature
<222> (53)
<223> Xaa equals stop translation

<400> 263
Met Pro Ser Leu Asn Leu Val Leu Arg Pro Leu Ile Cys Leu Ala Ser
  1               5                  10                  15

Ile Thr Ser Phe Leu Ile Phe Phe Pro Leu Leu Thr Leu Ile Leu Cys
             20                  25                  30

Ser Pro Asn Ser Pro Pro Phe Pro Leu Pro Ala His Pro Glu Arg His
        35                  40                  45

Thr His Thr Gln Xaa
    50


<210> 264
<211> 43
<212> PRT
<213> Homo sapiens

<220>
<221> misc feature
<222> (43)
<223> Xaa equals stop translation

<400> 264
Met His Ala Leu Ser Tyr Thr His Leu Ser Leu Leu Ser Leu Phe Leu
  1               5                  10                  15

Phe Leu Pro Pro Ser Phe Leu Tyr Tyr Asn Leu Val Ile Leu Phe Phe
             20                  25                  30

Glu Ala Phe Gln Asn Ile Ser His Leu Ser Xaa
        35                  40


<210> 265
<211> 50
<212> PRT
<213> Homo sapiens

<220>
<221> misc feature
<222> (50)
<223> Xaa equals stop translation

<400> 265
Asp Trp Leu Leu Leu Leu Ser Met Thr Phe Leu Gly Leu Ala Thr Gln
1               5                   10                  15

Leu Val Ser Val Val His Ser Phe Cys Ser Arg Ile Val Phe Cys Cys
            20                  25                  30

Leu Asp Gly Pro Pro Val Cys Cys Leu Phe Thr Leu Gln Leu Val Asp
        35                  40                  45

Ile Xaa
    50


<210> 266
<211> 52
<212> PRT
<213> Homo sapiens

<220>
<221> misc feature
<222> (52)
<223> Xaa equals stop translation

<400> 266
Met Arg Lys Ser Gly Ala Met Lys Lys Gly Gly Ile Phe Ser Ala Glu
1               5                   10                  15

Phe Leu Lys Val Phe Ile Pro Ser Leu Phe Leu Ser His Val Leu Ala
            20                  25                  30

Leu Gly Leu Gly Ile Tyr Ile Gly Lys Arg Leu Ser Thr Pro Ser Ala
        35                  40                  45

Ser Thr Tyr Xaa
    50


<210> 267
<211> 41
<212> PRT
<213> Homo sapiens

<220>
<221> misc feature
<222> (41)
<223> Xaa equals stop translation

<400> 267
Met Trp Val Gln Leu Ile Phe Phe Phe Val Gln Tyr Gly Asp Ser Leu
1               5                   10                  15

Thr Ser Ala Phe Phe Pro Phe Ser Ser Asn Phe Ser Leu Gln Asn Ser
            20                  25                  30

Gly Phe Ser Met His Lys Leu Lys Xaa
        35                  40

<210> 268
<211> 79
<212> PRT
<213> Homo sapiens

<220>
<221> misc feature
<222> (79)
<223> Xaa equals stop translation

<400> 268
Met Val Cys Phe Gln Ser Asn Lys Pro Ser Thr Ser Thr Trp Arg Gln
1               5                   10                  15

Leu Ser Phe Val Phe Val Leu Phe Cys Leu Phe Cys Leu Gly His Ala
            20                  25                  30

Phe Leu Ser Leu Pro Phe Tyr Ile Leu Ser Ile Ile Ala Met Cys Leu
        35                  40                  45

Glu Gln Trp Ala Phe His Asn Met Asn Ser Leu Tyr His His Glu Trp
    50                  55                  60

Glu Val Arg Gly Asn Leu Ile His Val Asp Phe Thr Leu Pro Xaa
65                  70                  75


<210> 269
<211> 117
<212> PRT
<213> Homo sapiens

<220>
<221> misc feature
<222> (117)
<223> Xaa equals stop translation

<400> 269
Met Thr His Lys Ser Leu Val Tyr Leu Trp Phe Leu Cys Ser Ser Val
1               5                   10                  15

Ala Leu Ala Leu Gly Ala Leu Thr Val Trp His Ala Val Leu Ile Ser
            20                  25                  30

Arg Gly Glu Thr Ser Ile Glu Arg His Ile Asn Lys Lys Glu Arg Arg
        35                  40                  45

Arg Leu Gln Ala Lys Gly Arg Val Phe Arg Asn Pro Tyr Asn Tyr Gly
    50                  55                  60

Cys Leu Asp Asn Trp Lys Val Phe Leu Gly Val Asp Thr Gly Arg His
65                  70                  75                  80

Trp Leu Thr Arg Val Leu Leu Pro Ser Ser His Leu Pro His Gly Asn
                85                  90                  95

Gly Met Ser Trp Glu Pro Pro Pro Trp Val Thr Ala His Ser Ala Ser
            100                 105                 110

Val Met Ala Val Xaa

        115

<210> 270
<211> 62
<212> PRT
<213> Homo sapiens

<220>
<221> misc feature
<222> (62)
<223> Xaa equals stop translation

<400> 270
Met Ser Asn Leu Gln Phe His Leu Leu Pro His Ser Ser Pro Ile Leu
1               5                   10                  15

Pro Leu Phe Thr Leu Ala Leu Leu Lys Met Gln Ile Pro Gly Leu Arg
            20                  25                  30

Leu Ser His Cys Leu Leu Thr Tyr Asn Ser Tyr Thr Arg Thr Pro Phe
        35                  40                  45

Leu Leu Pro Ser Ser Glu Ser Tyr Leu Val Phe Glu Ile Xaa
    50                  55                  60


<210> 271
<211> 98
<212> PRT
<213> Homo sapiens

<220>
<221> misc feature
<222> (53)
<223> Xaa equals any one of the naturally occurring L-amino acids

<220>
<221> misc feature
<222> (56)
<223> Xaa equals any one of the naturally occurring L-amino acids

<400> 271
Met Leu Pro Leu Tyr Phe Leu Gln Pro Tyr Leu Ser Leu Val Ile Phe
1               5                   10                  15

Ile Met Leu Arg Asp Asn Trp His Leu Leu Ala Leu Thr Cys Ser Tyr
            20                  25                  30

Ser Ile Ile Trp Arg Leu Ser Pro Asp Thr Asn Pro Ser Pro Ile Ala
        35                  40                  45

Pro Ser Arg His Xaa Gln Leu Xaa Val Val Ala Ile Ala Pro Leu Glu
    50                  55                  60

Pro Ser Pro His Ser His Met Gln Ser Ile Pro Lys Asn Leu Ala Gln
65                  70                  75                  80

Phe Ser Ser Ser Gln Met Phe Ser Leu Thr Leu Gln Leu Val Tyr Ile
                85                  90                  95

Ser Ser

<210> 272
<211> 32
<212> PRT
<213> Homo sapiens

<220>
<221> misc feature
<222> (32)
<223> Xaa equals stop translation

<400> 272
Met Tyr Ile Leu Ser Leu Ser Cys Ser Ile Phe Phe Ser Phe Phe Phe
1               5                   10                  15

Phe Leu Phe Pro Phe Phe Arg Gly Leu Arg Lys Gly Gln Ala Lys Xaa
            20                  25                  30

<210> 273
<211> 15
<212> PRT
<213> Homo sapiens

<220>
<221> misc feature
<222> (15)
<223> Xaa equals stop translation

<400> 273
Ala Ser Ser Leu Leu Val Ser Leu Gln Cys Leu Leu Gln Leu Xaa
1               5                   10                  15

<210> 274
<211> 37
<212> PRT
<213> Homo sapiens

<220>
<221> misc feature
<222> (37)
<223> Xaa equals stop translation

<400> 274
Met Cys Phe Ile Leu Val Val Cys Phe Ala Ser Leu Ile Thr Glu Cys
1               5                   10                  15

Pro Cys His Cys Lys Cys Cys Arg Asp Val Gly Arg Gly Pro Thr Val
            20                  25                  30

Leu Tyr Glu Met Xaa
        35

<210> 275
<211> 57
<212> PRT
<213> Homo sapiens

<220>
<221> misc feature
<222> (53)
<223> Xaa equals any one of the naturally occurring L-amino acids

<220>
<221> misc feature
<222> (57)
<223> Xaa equals stop translation

<400> 275
Met His Arg Leu Trp Ile Gly Pro Ala Phe Phe Leu Met Thr Ser Leu
1               5                   10                  15

Ser Val Ser Gly Ala Val Ile Pro Arg Asn Gly Gly Pro Gly Gly Val
            20                  25                  30

Ser Ser Gly Pro Cys Leu Leu Gln Leu Leu Cys Gly Gln Ala Gly Ser
        35                  40                  45

Ser Thr Ile Arg Xaa Ile Pro Ser Xaa
    50                  55


<210> 276
<211> 27
<212> PRT
<213> Homo sapiens

<220>
<221> misc feature
<222> (27)
<223> Xaa equals stop translation

<400> 276
Met Glu Ala Val Phe Phe Leu Phe Phe Leu Leu Leu Leu Leu Thr Trp
1               5                   10                  15

Thr Ser Lys Ile Ala Pro Ile Leu Phe Ser Xaa
            20                  25


<210> 277
<211> 68
<212> PRT
<213> Homo sapiens

<220>
<221> misc feature
<222> (68)
<223> Xaa equals stop translation

<400> 277

Asp Trp Gly Phe Gln Thr Thr Phe Phe Ser Leu Gly Leu Tyr Leu Phe
1               5                   10                  15

Thr Ile Trp Trp Ser Thr Val Gly Leu Pro Trp Thr Ser Ser Thr Gln
            20                  25                  30

Arg Glu Leu Asp Met Lys Leu Glu Ala Ala Ala Leu Glu Gly Lys Phe
        35                  40                  45

Arg Leu Thr Trp Thr Ala Gln Ala Met Ala Gly Arg Ile Pro Ser Ser
    50                  55                  60

Trp Gly Pro Xaa
65


<210> 278
<211> 46
<212> PRT
<213> Homo sapiens

<220>
<221> misc feature
<222> (46)
<223> Xaa equals stop translation

<400> 278
Met Pro Arg Arg Ser Arg Pro Cys Thr Leu Cys Leu Thr Leu Leu Arg
1               5                   10                  15

Arg Ala Leu Ser Ser His Leu Pro Ser Ala Cys Gln Ser Pro Arg Arg
            20                  25                  30

Arg Val Gln Gly Gln Val Leu Lys Arg Leu Lys Pro Leu Xaa
        35                  40                  45


<210> 279
<211> 40
<212> PRT
<213> Homo sapiens

<220>
<221> misc feature
<222> (40)
<223> Xaa equals stop translation

<400> 279
Met Pro Leu Thr Leu Pro Ser Arg Leu Ala Gly Gly Asn Val Phe Leu
1               5                   10                  15

Ile Ile Phe Thr Pro Gly Phe Cys Pro Gly Arg Val Asn Val Glu Ile
            20                  25                  30

Pro Gln Arg Met Leu Asp Glu Xaa
        35                  40


<210> 280
<211> 11

<212> PRT
<213> Homo sapiens

<220>
<221> misc feature
<222> (11)
<223> Xaa equals stop translation

<400> 280
Met Ser Arg Arg Glu Asn Lys Phe Leu Leu Xaa
1               5                   10


<210> 281
<211> 282
<212> PRT
<213> Homo sapiens

<220>
<221> misc feature
<222> (65)
<223> Xaa equals any one of the naturally occurring L-amino acids

<220>
<221> misc feature
<222> (199)
<223> Xaa equals any one of the naturally occurring L-amino acids

<220>
<221> misc feature
<222> (227)
<223> Xaa equals any one of the naturally occurring L-amino acids

<220>
<221> misc feature
<222> (276)
<223> Xaa equals any one of the naturally occurring L-amino acids

<220>
<221> misc feature
<222> (282)
<223> Xaa equals stop translation

<400> 281
Met Gly Phe Pro Gln Arg Gln Pro Gly Leu Ser Gly Leu Leu Leu Leu
1               5                   10                  15

Val Trp Ala Leu Ala Trp Pro Leu Pro Cys Met Ser Leu Glu Leu Ile
            20                  25                  30

Pro Tyr Thr Pro Gln Ile Thr Ala Trp Asp Leu Glu Gly Lys Val Thr
        35                  40                  45

Ala Thr Thr Phe Ser Leu Glu Gln Pro Arg Cys Val Leu Asp Gly Leu
    50                  55                  60

Xaa Gly Val Ala Ser Thr Ile Trp Leu Val Val Ala Phe Ser Asn Ala
65                  70                  75                  80

Ser Arg Asp Phe Gln Asn Pro Gln Thr Arg Ala Glu Ile Pro Ala Phe

85                  90                  95

Pro Arg Leu Leu Thr Glu Gly His Tyr Met Thr Leu Pro Leu Ser Leu
            100                 105                 110

Asp Gln Leu Pro Cys Gln Asp Pro Ala Gly Gly Gly Arg Asp Val Pro
        115                 120                 125

Leu Leu Arg Val Gly Asn Asp Pro Gly Cys Leu Ala Asp Leu Leu Gln
    130                 135                 140

Pro Pro Tyr Cys Asn Ser Pro Leu Pro Ser Pro Gly Pro Tyr Arg Val
145                 150                 155                 160

Lys Phe Leu Leu Met Asp Ala Arg Gly Ser Pro Gln Ala Glu Thr Arg
                165                 170                 175

Trp Ser Asp Pro Ile Ala Leu His Gln Gly Lys Ser Pro Ala Ser Ile
            180                 185                 190

Asp Thr Trp Pro Gly Arg Xaa Ser Gly Gly Met Ile Val Ile Thr Ser
        195                 200                 205

Ile Leu Ser Ser Leu Ala Ser Leu Leu Leu Leu Ala Phe Leu Ala Ala
    210                 215                 220

Ser Thr Xaa Arg Phe Ser Ser Leu Trp Trp Pro Glu Glu Ala Pro Glu
225                 230                 235                 240

Gln Leu Arg Ile Gly Ser Phe Met Gly Lys Arg Tyr Met Thr His His
                245                 250                 255

Ile Pro Pro Ser Glu Ala Ala Thr Leu Pro Val Gly Cys Glu Pro Gly
            260                 265                 270

Leu Asp Pro Xaa Pro Ser Leu Ser Pro Xaa
        275                 280


<210> 282
<211> 47
<212> PRT
<213> Homo sapiens

<400> 282
Met Leu Pro Ile His Leu Gln Trp Ala Cys Ala Phe Arg Ser Phe Leu
 1               5                  10                  15

Leu Gly Ile Asp Ser Ser Met Phe Val Leu Phe Gln His Pro Arg Leu
            20                  25                  30

Lys Asp Thr Lys Ser Ser Arg Val Ile Glu Pro Thr Leu Thr Asn
        35                  40                  45


<210> 283
<211> 23
<212> PRT
<213> Homo sapiens

<220>
<221> misc feature
<222> (23)
<223> Xaa equals stop translation

<400> 283
Met Ile Leu Leu Ala Phe Phe Ile Leu Leu Tyr Leu Thr Ser Phe Ser
1               5                   10                  15

Leu Ala Arg Ser Leu Pro Xaa
            20


<210> 284
<211> 21
<212> PRT
<213> Homo sapiens

<220>
<221> misc feature
<222> (21)
<223> Xaa equals stop translation

<400> 284
Ser Ser Ser Cys Met Pro Arg Lys Leu Asp Trp Phe Ser Lys Lys Val
1               5                   10                  15

Phe Leu Phe Phe Xaa
            20


<210> 285
<211> 122
<212> PRT
<213> Homo sapiens

<400> 285

<210> 286
<211> 27
<212> PRT
<213> Homo sapiens

<220>
<221> misc feature
<222> (27)
<223> Xaa equals stop translation

<400> 286
Met Ile His Leu Ser Arg Phe Tyr Leu Leu Leu Ile Met Leu Pro His
1               5                   10                  15

Val Leu Phe Phe Thr Gly Asp Leu His Ser Xaa
            20                  25


<210> 287
<211> 8
<212> PRT
<213> Homo sapiens

<220>
<221> misc feature
<222> (8)
<223> Xaa equals stop translation

<400> 287
Met Tyr Lys Cys Trp Tyr Arg Xaa
1               5


<210> 288
<211> 29
<212> PRT
<213> Homo sapiens

<220>
<221> misc feature
<222> (2)
<223> Xaa equals any one of the naturally occurring L-amino acids

<220>
<221> misc feature
<222> (29)
<223> Xaa equals stop translation

<400> 288
Met Xaa Leu Asn Lys Thr Lys Ser Leu Thr Leu Leu Glu Leu Val Phe
1               5                   10                  15

Leu Pro Gly Glu Thr Val Ser Lys Pro Ser Thr Lys Xaa
            20                  25


<210> 289
<211> 52

<212> PRT
<213> Homo sapiens

<400> 289


<210> 290
<211> 17
<212> PRT
<213> Homo sapiens

<400> 290
Val Arg Ser Glu Lys Gly Phe Asp Lys Ile Gln Cys Pro Phe Met Val
1               5                   10                  15

Lys


<210> 291
<211> 46
<212> PRT
<213> Homo sapiens

<400> 291


<210> 292
<211> 14
<212> PRT
<213> Homo sapiens

<400> 292
Lys Lys Glu Thr Lys Leu Ser Leu Phe Ala Asn Asp Met Ile
1               5                   10


<210> 293
<211> 23
<212> PRT
<213> Homo sapiens

<400> 293
Ser Pro Leu Leu Phe Asn Ile Leu Leu Glu Val Leu Ser Ser Ala Val
1               5                   10                  15

Arg Lys Glu Lys Glu Leu Lys
            20


<210> 294
<211> 122
<212> PRT
<213> Homo sapiens

<400> 294


<210> 295
<211> 60
<212> PRT
<213> Homo sapiens

<400> 295
Arg Ala Thr Gln Gly Ala Gly His Gly Ser Ser Asp Glu Glu Asn Glu
1               5                   10                  15

Asp Gly Asp Phe Thr Val Tyr Glu Cys Pro Gly Met Ala Pro Thr Gly
            20                  25                  30

Glu Met Glu Val Arg Asn His Leu Phe Asp His Ala Ala Leu Ser Ala
        35                  40                  45

Pro Leu Pro Ala Pro Ser Ser Pro Leu Ala Leu Pro
    50                  55                  60


<210> 296

<211> 47
<212> PRT
<213> Homo sapiens

<400> 296
Lys Ala Glu Tyr Ala Thr Ala Lys Ala Leu Ala Thr Pro Ala Ala Thr
1               5                   10                  15

Pro Asp Leu Ala Trp Gly Pro Ala Pro Gly Thr Glu Arg Gly Asp Val
            20                  25                  30

Pro Leu Pro Ala Pro Thr Ala Thr Asp Val Val Pro Gly Ala Ala
        35                  40                  45


<210> 297
<211> 15
<212> PRT
<213> Homo sapiens

<400> 297
Ser Ala Glu Met Tyr His Tyr Gln His Gln Arg Gln Gln Met Leu
1               5                   10                  15


<210> 298
<211> 11
<212> PRT
<213> Homo sapiens

<400> 298
Leu Glu Arg His Lys Glu Pro Pro Lys Glu Leu
1               5                   10


<210> 299
<211> 12
<212> PRT
<213> Homo sapiens

<400> 299
Ala Lys Cys Pro Pro Gly Ala His Ala Cys Gly Pro
1               5                   10


<210> 300
<211> 9
<212> PRT
<213> Homo sapiens

<400> 300
Pro Val His Met Ser Pro Leu Glu Pro
1               5


<210> 301
<211> 12
<212> PRT
<213> Homo sapiens

<400> 301
Trp Cys Arg Leu Gln Arg Glu Ile Arg Leu Thr Gln
1               5                   10

<210> 302
<211> 18
<212> PRT
<213> Homo sapiens

<400> 302
Ser Ser Asp Glu Glu Asn Glu Asp Gly Asp Phe Thr Val Tyr Glu Cys
1               5                   10                  15

Pro Gly

<210> 303
<211> 10
<212> PRT
<213> Homo sapiens

<400> 303
Ala Pro Thr Gly Glu Met Glu Val Arg Asn
1               5                   10

<210> 304
<211> 10
<212> PRT
<213> Homo sapiens

<400> 304
Cys Pro Gly Ser Leu Asp Cys Ala Leu Lys
1               5                   10

<210> 305
<211> 8
<212> PRT
<213> Homo sapiens

<400> 305
Arg Ser Cys Lys Glu Ile Lys Asp
1               5

<210> 306
<211> 13
<212> PRT
<213> Homo sapiens

<400> 306
Gly Gly Gly Trp Thr Leu Val Ala Ser Val His Glu Asn
1               5                   10

<210> 307
<211> 19

<212> PRT
<213> Homo sapiens

<400> 307
Ala Asp Tyr Pro Glu Gly Asp Gly Asn Trp Ala Asn Tyr Asn Thr Phe
1               5                   10                  15

Gly Ser Ala


<210> 308
<211> 14
<212> PRT
<213> Homo sapiens

<400> 308
Ala Thr Ser Asp Asp Tyr Lys Asn Pro Gly Tyr Tyr Asp Ile
1               5                   10


<210> 309
<211> 11
<212> PRT
<213> Homo sapiens

<400> 309
Cys Ile Gly Gly Gly Gly Tyr Phe Pro Glu Ala
1               5                   10


<210> 310
<211> 11
<212> PRT
<213> Homo sapiens

<400> 310
Glu Ile Thr Glu Ala Ala Val Leu Leu Phe Tyr
1               5                   10


<210> 311
<211> 300
<212> PRT
<213> Homo sapiens

<220>
<221> misc feature
<222> (4)
<223> Xaa equals any one of the naturally occurring L-amino acids

<220>
<221> misc feature
<222> (62)
<223> Xaa equals any one of the naturally occurring L-amino acids

<400> 311
Lys His Glu Xaa His Gln Val Ser Asp Gly Ala Leu Arg Cys Phe Ala
1               5                   10                  15

Ser Leu Ala Asp Arg Phe Thr Arg Arg Gly Val Asp Pro Ala Pro Leu
            20                  25                  30

Ala Lys His Gly Leu Thr Glu Glu Leu Leu Ser Arg Met Ala Ala Ala
        35                  40                  45

Gly Gly Thr Val Ser Gly Pro Ser Ser Ala Cys Lys Pro Xaa Arg Ser
    50                  55                  60

Thr Thr Gly Ala Pro Ser Thr Thr Ala Asp Ser Lys Leu Ser Asn Gln
65                  70                  75                  80

Val Ser Thr Ile Val Ser Leu Leu Ser Thr Leu Cys Arg Gly Ser Pro
                85                  90                  95

Val Val Thr His Asp Leu Leu Arg Ser Glu Leu Pro Asp Ser Ile Glu
            100                 105                 110

Ser Ala Leu Gln Gly Asp Glu Arg Cys Val Leu Asp Thr Met Arg Leu
        115                 120                 125

Val Asp Phe Leu Leu Val Leu Leu Phe Glu Gly Arg Lys Ala Leu Pro
    130                 135                 140

Lys Ser Ser Ala Gly Ser Thr Gly Arg Ile Pro Gly Leu Arg Arg Leu
145                 150                 155                 160

Asp Ser Ser Gly Glu Arg Ser His Arg Gln Leu Ile Asp Cys Ile Arg
                165                 170                 175

Ser Lys Asp Thr Asp Ala Leu Ile Asp Ala Ile Asp Thr Gly Ala Phe
            180                 185                 190

Glu Val Asn Phe Met Asp Asp Val Gly Gln Thr Leu Leu Asn Trp Ala
        195                 200                 205

Ser Ala Phe Gly Thr Gln Glu Met Val Glu Phe Leu Cys Glu Arg Gly
    210                 215                 220

Ala Asp Val Asn Arg Gly Gln Arg Ser Ser Ser Leu His Tyr Ala Ala
225                 230                 235                 240

Cys Phe Gly Arg Pro Gln Val Ala Lys Thr Leu Leu Arg His Gly Ala
                245                 250                 255

Asn Pro Asp Leu Arg Asp Glu Asp Gly Lys Thr Pro Leu Asp Lys Ala
            260                 265                 270

Arg Glu Arg Gly His Ser Glu Val Val Ala Ile Leu Gln Ser Pro Gly
        275                 280                 285

Asp Trp Met Cys Pro Val Asn Lys Gly Asp Asp Lys
    290                 295                 300

<210> 312
<211> 17
<212> PRT
<213> Homo sapiens

<400> 312
Pro Leu Asp Lys Ala Arg Glu Arg Gly His Ser Glu Val Val Ala Ile
1               5                   10                  15

Leu

<210> 313
<211> 15
<212> PRT
<213> Homo sapiens

<400> 313
Ala Lys Thr Leu Leu Arg His Gly Ala Asn Pro Asp Leu Arg Asp
1               5                   10                  15

<210> 314
<211> 52
<212> PRT
<213> Homo sapiens

<220>
<221> misc feature
<222> (26)
<223> Xaa equals any one of the naturally occurring L-amino acids

<220>
<221> misc feature
<222> (29)
<223> Xaa equals any one of the naturally occurring L-amino acids

<400> 314
Asp Cys Asn Arg Asp Tyr His Lys Ala Phe Gly Asn Leu Arg Ser Pro
1               5                   10                  15

Gly Trp Pro Asp Asn Tyr Asp Asn Asp Xaa Asp Cys Xaa Val Thr Leu
            20                  25                  30

Thr Ala Pro Gln Asn His His Ser Gly Ile Val Glu Asn Ala Glu Thr
        35                  40                  45

Ile Ser Trp Arg
    50

<210> 315
<211> 15
<212> PRT
<213> Homo sapiens

<400> 315
Phe Gly Asn Leu Arg Ser Pro Gly Trp Pro Asp Asn Tyr Asp Asn
1               5                   10                  15

<210> 316
<211> 16
<212> PRT

<213> Homo sapiens

<220>
<221> misc feature
<222> (6)
<223> Xaa equals any one of the naturally occurring L-amino acids

<400> 316
Ala Pro Gln Asn His Xaa Leu Lys Cys Arg Asn Asp Phe Leu Glu Val
1               5                   10                  15

<210> 317
<211> 20
<212> PRT
<213> Homo sapiens

<400> 317
Ala Ser Ile Asp Thr Trp Pro Gly Arg Arg Ser Gly Gly Met Ile Val
1               5                   10                  15

Ile Thr Ser Ile
            20

<210> 318
<211> 41
<212> PRT
<213> Homo sapiens

<400> 318
Gly Ser Pro Gln Ala Glu Thr Arg Trp Ser Asp Pro Ile Ala Leu His
1               5                   10                  15

Gln Gly Lys Ser Pro Ala Ser Ile Asp Thr Trp Pro Gly Arg Arg Ser
            20                  25                  30

Gly Gly Met Ile Val Ile Thr Ser Ile
        35                  40

<210> 319
<211> 43
<212> PRT
<213> Homo sapiens

<220>
<221> misc feature
<222> (2)
<223> Xaa equals any one of the naturally occurring L-amino acids

<400> 319
Val Xaa Asp Ile Thr Phe Asp Pro Asp Thr Ala His Lys Tyr Leu Arg
1               5                   10                  15

Leu Gln Glu Glu Asn Arg Lys Val Thr Asn Thr Thr Pro Trp Glu His
            20                  25                  30

Pro Tyr Pro Asp Leu Pro Ser Arg Phe Leu His
        35                  40

<210> 320
<211> 19
<212> PRT
<213> Homo sapiens

<400> 320
Leu Tyr Leu His Arg Tyr Tyr Phe Glu Val Glu Ile Phe Gly Ala Gly
1               5                   10                  15

Thr Tyr Val

<210> 321
<211> 22
<212> PRT
<213> Homo sapiens

<400> 321
Ser Cys Ile Ser Gly Asn Asn Phe Ser Trp Ser Leu Gln Trp Asn Gly
1               5                   10                  15

Lys Glu Phe Thr Ala Trp
            20

<210> 322
<211> 17
<212> PRT
<213> Homo sapiens

<400> 322
Thr Pro Leu Lys Ala Gly Pro Phe Trp Ser Ser Gly Ser Ile Leu Thr
1               5                   10                  15

Ser

<210> 323
<211> 39
<212> PRT
<213> Homo sapiens

<220>
<221> misc feature
<222> (32)
<223> Xaa equals any one of the naturally occurring L-amino acids

<400> 323
Ser Val Ser Glu Val Lys Ala Val Ala Glu Met Gln Phe Gly Glu Leu
1               5                   10                  15

Leu Ala Ala Val Arg Lys Ala Gln Ala Asn Val Met Leu Phe Leu Xaa
            20                  25                  30

Glu Lys Glu Gln Ala Ala Leu
        35

<210> 324
<211> 43
<212> PRT
<213> Homo sapiens

<400> 324
Glu Lys Ser Lys Gln Glu Leu Glu Thr Met Ala Ala Ile Ser Asn Thr
 1               5                  10                  15

Val Gln Phe Leu Glu Glu Tyr Cys Lys Phe Lys Asn Thr Glu Asp Ile
            20                  25                  30

Thr Phe Pro Ser Val Tyr Ile Gly Leu Lys Asp
        35                  40

<210> 325
<211> 29
<212> PRT
<213> Homo sapiens

<220>
<221> misc feature
<222> (26)
<223> Xaa equals any one of the naturally occurring L-amino acids

<400> 325
Leu Glu Asn Tyr Lys Lys Lys Leu Gln Glu Phe Ser Lys Glu Glu Glu
 1               5                  10                  15

Tyr Asp Ile Arg Thr Gln Val Ser Ala Xaa Val Gln Arg
            20                  25

<210> 326
<211> 38
<212> PRT
<213> Homo sapiens

<400> 326
Gly Val Tyr Ile Asp Phe Pro Gly Gly Ile Leu Ser Phe Tyr Gly Val
 1               5                  10                  15

Glu Tyr Asp Ser Met Thr Leu Val His Lys Phe Ala Cys Lys Phe Ser
            20                  25                  30

Glu Pro Val Tyr Ala Ala
        35

<210> 327
<211> 196
<212> PRT
<213> Homo sapiens

<400> 327
Ser Lys Ile Lys Tyr Asp Trp Tyr Gln Thr Glu Ser Gln Val Val Ile
1               5                   10                  15

Thr Leu Met Ile Lys Asn Val Gln Lys Asn Asp Val Asn Val Glu Phe
            20                  25                  30

Ser Glu Lys Glu Leu Ser Ala Leu Val Lys Leu Pro Ser Gly Glu Asp
        35                  40                  45

Tyr Asn Leu Lys Leu Glu Leu Leu His Pro Ile Ile Pro Glu Gln Ser
    50                  55                  60

Thr Phe Lys Val Leu Ser Thr Lys Ile Glu Ile Lys Leu Lys Lys Pro
65                  70                  75                  80

Glu Ala Val Arg Trp Glu Lys Leu Glu Gly Gln Gly Asp Val Pro Thr
                85                  90                  95

Pro Lys Gln Phe Val Ala Asp Val Lys Asn Leu Tyr Pro Ser Ser Ser
            100                 105                 110

Pro Tyr Thr Arg Asn Trp Asp Lys Leu Val Gly Glu Ile Lys Glu Glu
        115                 120                 125

Glu Lys Asn Glu Lys Leu Glu Gly Asp Ala Ala Leu Asn Arg Leu Phe
    130                 135                 140

Gln Gln Ile Tyr Ser Asp Gly Ser Asp Glu Val Lys Arg Ala Met Asn
145                 150                 155                 160

Lys Ser Phe Met Glu Ser Gly Gly Thr Val Leu Ser Thr Asn Trp Ser
                165                 170                 175

Asp Val Gly Lys Arg Lys Val Glu Ile Asn Pro Pro Asp Asp Met Glu
            180                 185                 190

Trp Lys Lys Tyr
        195


<210> 328
<211> 39
<212> PRT
<213> Homo sapiens

<400> 328
Gly Asp Ala Ala Leu Asn Arg Leu Phe Gln Gln Ile Tyr Ser Asp Gly
1               5                   10                  15

Ser Asp Glu Val Lys Arg Ala Met Asn Lys Ser Phe Met Glu Ser Gly
            20                  25                  30

Gly Thr Val Leu Ser Thr Asn
        35


<210> 329
<211> 23
<212> PRT

<213> Homo sapiens

<400> 329
Asp Trp Tyr Gln Thr Glu Ser Gln Val Val Ile Thr Leu Met Ile Lys
1               5                   10                  15

Asn Val Gln Lys Asn Asp Val
            20


<210> 330
<211> 162
<212> PRT
<213> Homo sapiens

<220>
<221> misc feature
<222> (1)
<223> Xaa equals any one of the naturally occurring L-amino acids

<220>
<221> misc feature
<222> (33)
<223> Xaa equals any one of the naturally occurring L-amino acids

<220>
<221> misc feature
<222> (48)
<223> Xaa equals any one of the naturally occurring L-amino acids

<400> 330
Xaa Leu Trp Asp Pro Gly Leu Pro Gly Val Cys Arg Cys Gly Ser Ile
1               5                   10                  15

Val Leu Lys Ser Ala Phe Ser Val Gly Ile Thr Thr Ser Tyr Pro Glu
            20                  25                  30

Xaa Arg Leu Pro Ile Ile Phe Asn Lys Val Leu Leu Pro Arg Gly Xaa
        35                  40                  45

Ala Leu Gln Pro Cys His Arg Gly Ser Ser Ser Val Leu Ser Gln Gly
    50                  55                  60

Ile Tyr Tyr Phe Ser Tyr Asp Ile Thr Leu Ala Asn Lys His Leu Ala
65                  70                  75                  80

Ile Gly Leu Val His Asn Gly Gln Tyr Arg Ile Lys Thr Phe Asp Ala
                85                  90                  95

Asn Thr Gly Asn His Asp Val Ala Ser Gly Ser Thr Val Ile Tyr Leu
            100                 105                 110

Gln Pro Glu Asp Glu Val Trp Leu Glu Ile Phe Phe Thr Asp Gln Asn
        115                 120                 125

Gly Leu Phe Ser Asp Pro Gly Trp Ala Asp Ser Leu Phe Ser Gly Phe
    130                 135                 140

Leu Leu Tyr Val Asp Thr Asp Tyr Leu Asp Ser Ile Ser Glu Asp Asp
145                 150                 155                 160

Glu Leu

<210> 331
<211> 15
<212> PRT
<213> Homo sapiens

<400> 331
Gly Ser Ile Val Leu Lys Ser Ala Phe Ser Val Gly Ile Thr Thr
1               5                   10                  15

<210> 332
<211> 14
<212> PRT
<213> Homo sapiens

<400> 332
Gly Ile Tyr Tyr Phe Ser Tyr Asp Ile Thr Leu Ala Asn Lys
1               5                   10

<210> 333
<211> 13
<212> PRT
<213> Homo sapiens

<400> 333
Asp Ser Leu Phe Ser Gly Phe Leu Leu Tyr Val Asp Thr
1               5                   10

<210> 334
<211> 13
<212> PRT
<213> Homo sapiens

<400> 334
Asn His Asp Val Ala Ser Gly Ser Thr Val Ile Tyr Leu
1               5                   10

<210> 335
<211> 89
<212> PRT
<213> Homo sapiens

<220>
<221> misc feature
<222> (60)
<223> Xaa equals any one of the naturally occurring L-amino acids

<220>
<221> misc feature
<222> (75)
<223> Xaa equals any one of the naturally occurring L-amino acids

<400> 335
Glu Asn Phe Leu Leu Arg Tyr Lys Gly Pro Ser Asp His Trp Ile Gly
1               5                   10                  15

Leu Ser Arg Glu Gln Gly Gln Pro Trp Lys Trp Ile Asn Gly Thr Glu
            20                  25                  30

Trp Thr Arg Gln Leu Val Met Lys Glu Asp Gly Ala Asn Leu Tyr Val
        35                  40                  45

Ala Lys Val Ser Gln Val Pro Arg Met Asn Pro Xaa Leu Ser Trp Val
    50                  55                  60

Leu Leu Cys Tyr Pro Gly Trp Ser Ala Val Xaa Thr Ile Val Ala His
65                  70                  75                  80

Cys Ser Leu Asp Phe Pro Gly Ser Lys
                85


<210> 336
<211> 63
<212> PRT
<213> Homo sapiens

<400> 336
Glu Leu Thr Ala Ile Lys Ser His Gln Tyr Val Leu Gln Ala Ala Cys
1               5                   10                  15

Pro Glu Ser Trp Ile Gly Phe Gln Arg Lys Cys Phe Tyr Phe Ser Asp
            20                  25                  30

Asp Thr Lys Asn Trp Thr Ser Ser Gln Arg Phe Cys Asp Ser Gln Asp
        35                  40                  45

Ala Asp Leu Ala Gln Val Glu Ser Phe Gln Glu Leu Val Arg Lys
    50                  55                  60


<210> 337
<211> 17
<212> PRT
<213> Homo sapiens

<400> 337
Trp Ile Gly Leu Ser Arg Glu Gln Gly Gln Pro Trp Lys Trp Ile Asn
1               5                   10                  15

Gly


<210> 338
<211> 12
<212> PRT
<213> Homo sapiens

<400> 338
Cys Pro Glu Ser Trp Ile Gly Phe Gln Arg Lys Cys
1               5                   10

<210> 339
<211> 16
<212> PRT
<213> Homo sapiens

<400> 339
Asn Phe Leu Leu Arg Tyr Lys Gly Pro Ser Asp His Trp Ile Gly Leu
1               5                   10                  15

<210> 340
<211> 50
<212> PRT
<213> Homo sapiens

<400> 340
Ala Ser His Leu Arg Leu Leu Ser Ser Trp Asp Tyr Arg Phe Pro Ile
1               5                   10                  15

Leu Gly Ala Gly Glu Cys Ala Tyr Leu Asn Asp Lys Gly Ala Ser Ser
            20                  25                  30

Ala Arg His Tyr Thr Glu Arg Lys Trp Ile Cys Ser Lys Ser Asp Ile
        35                  40                  45

His Val
    50

<210> 341
<211> 89
<212> PRT
<213> Homo sapiens

<220>
<221> misc feature
<222> (60)
<223> Xaa equals any one of the naturally occurring L-amino acids

<220>
<221> misc feature
<222> (75)
<223> Xaa equals any one of the naturally occurring L-amino acids

<400> 341
Glu Asn Phe Leu Leu Arg Tyr Lys Gly Pro Ser Asp His Trp Ile Gly
1               5                   10                  15

Leu Ser Arg Glu Gln Gly Gln Pro Trp Lys Trp Ile Asn Gly Thr Glu
            20                  25                  30

Trp Thr Arg Gln Leu Val Met Lys Glu Asp Gly Ala Asn Leu Tyr Val
        35                  40                  45

Ala Lys Val Ser Gln Val Pro Arg Met Asn Pro Xaa Leu Ser Trp Val

50                  55                  60

Leu Leu Cys Tyr Pro Gly Trp Ser Ala Val Xaa Thr Ile Val Ala His
65                  70                  75                  80

Cys Ser Leu Asp Phe Pro Gly Ser Lys
                85

<210> 342
<211> 76
<212> PRT
<213> Homo sapiens

<220>
<221> misc feature
<222> (9)
<223> Xaa equals any one of the naturally occurring L-amino acids

<220>
<221> misc feature
<222> (22)
<223> Xaa equals any one of the naturally occurring L-amino acids

<220>
<221> misc feature
<222> (29)
<223> Xaa equals any one of the naturally occurring L-amino acids

<400> 342
Ser Trp Thr Ser Ser Leu Leu Asn Xaa Cys Leu His Ser Lys Glu His
1               5                   10                  15

Ser Ile Lys Ala Thr Xaa Ile Trp Arg Leu Phe Phe Xaa Ile Leu Thr
            20                  25                  30

Ile Ile Leu Cys Gly Met Val Ala Ala Leu Ser Ala Ile Arg Ala Asn
        35                  40                  45

Cys His Gln Glu Pro Ser Val Cys Ser Ser Ser Cys Met Pro Arg Lys
    50                  55                  60

Leu Asp Trp Phe Ser Lys Lys Val Phe Leu Phe Phe
65                  70                  75

<210> 343
<211> 109
<212> PRT
<213> Homo sapiens

<220>
<221> misc feature
<222> (24)
<223> Xaa equals any one of the naturally occurring L-amino acids

<220>
<221> misc feature
<222> (25)
<223> Xaa equals any one of the naturally occurring L-amino acids

<220>
<221> misc feature
<222> (31)
<223> Xaa equals any one of the naturally occurring L-amino acids

<400> 343
Leu Lys Gly Arg Glu Ala Gly Ala Gly Pro Gly Thr Ala Gly Ala Pro
1               5                   10                  15

Gly Arg Glu Asp Ala Asn Gly Xaa Xaa Arg Gly Arg Gly Gly Xaa His
            20                  25                  30

Gln Leu Tyr Leu Trp Val Asp Asn Ile Pro Leu Ser Arg Pro Lys Arg
        35                  40                  45

Asn Leu Ser Arg Asp Phe Ser Asp Gly Val Leu Val Ala Glu Val Ile
    50                  55                  60

Lys Phe Tyr Phe Pro Lys Met Val Glu Met His Asn Tyr Val Gly Thr
65                  70                  75                  80

Ser Ser Leu Gln Gln Lys Leu Ser Asn Trp Gly His Leu Asn Arg Lys
                85                  90                  95

Val Leu Lys Arg Leu Asn Phe Ser Val Pro Asp Asp Val
            100                 105

<210> 344
<211> 187
<212> PRT
<213> Homo sapiens

<400> 344
Ala Lys Asn Ser Gln Lys Glu Glu Asn Pro Glu His Val Glu Ile Gln
1               5                   10                  15

Lys Met Met Asp Ser Leu Phe Leu Lys Leu Asp Ala Leu Ser Asn Phe
            20                  25                  30

His Phe Ile Pro Lys Pro Pro Val Pro Glu Ile Lys Val Val Ser Asn
        35                  40                  45

Leu Pro Ala Ile Thr Met Glu Glu Val Ala Pro Val Ser Val Ser Asp
    50                  55                  60

Ala Ala Leu Leu Ala Pro Glu Glu Ile Lys Glu Lys Asn Lys Ala Gly
65                  70                  75                  80

Asp Ile Lys Thr Ala Ala Glu Lys Thr Ala Thr Asp Lys Lys Arg Glu
                85                  90                  95

Arg Arg Lys Lys Lys Tyr Gln Lys Arg Met Lys Ile Lys Glu Lys Glu
            100                 105                 110

Lys Arg Arg Lys Leu Leu Glu Lys Ser Ser Val Asp Gln Ala Gly Lys
        115                 120                 125

Tyr Ser Lys Thr Val Ala Ser Glu Lys Leu Lys Gln Leu Thr Lys Thr

130                 135                 140

Gly Lys Ala Ser Phe Ile Lys Val Arg Thr Arg Glu Arg Lys Leu Leu
145                 150                 155                 160

Lys Gly Thr Phe Val Gly Glu Val Asp Ser Lys Cys Trp Val Thr Gly
                165                 170                 175

Met Ser Glu Pro Ala Asp Ser Pro Pro Val Gly
            180                 185

<210> 345
<211> 51
<212> PRT
<213> Homo sapiens

<400> 345

<210> 346
<211> 29
<212> PRT
<213> Homo sapiens

<400> 346
Asp Glu Gly Lys Asp Lys Ala Leu Lys Ser Ser Gln Ala Phe Phe Ser
1               5                   10                  15

Lys Leu Gln Asp Gln Val Lys Met Gln Ile Asn Asp Ala
            20                  25

<210> 347
<211> 28
<212> PRT
<213> Homo sapiens

<400> 347
Glu Glu Asn Pro Glu His Val Glu Ile Gln Lys Met Met Asp Ser Leu
1               5                   10                  15

Phe Leu Lys Leu Asp Ala Leu Ser Asn Phe His Phe
            20                  25

<210> 348
<211> 13
<212> PRT

<213> Homo sapiens

<400> 348
Ser Asn Leu Pro Ala Ile Thr Met Glu Glu Val Ala Pro
1               5                   10

<210> 349
<211> 31
<212> PRT
<213> Homo sapiens

<400> 349
Ser Ser Val Asp Gln Ala Gly Lys Tyr Ser Lys Thr Val Ala Ser Glu
1               5                   10                  15

Lys Leu Lys Gln Leu Thr Lys Thr Gly Lys Ala Ser Phe Ile Lys
            20                  25                  30

<210> 350
<211> 23
<212> PRT
<213> Homo sapiens

<400> 350
Val Ser Val Ser Asp Ala Ala Leu Leu Ala Pro Glu Glu Ile Lys Glu
1               5                   10                  15

Lys Asn Lys Ala Gly Asp Ile
            20

<210> 351
<211> 20
<212> PRT
<213> Homo sapiens

<400> 351
Met Ala Ile Pro Ala Phe Ser Ser Cys Gln Gln Ile Ser Ser Ala Ala
1               5                   10                  15

Ala Leu Gln Ile
            20

<210> 352
<211> 14
<212> PRT
<213> Homo sapiens

<400> 352
Cys Asn Gly Pro Phe Lys His Phe Ser Phe Thr Val Ser Thr
1               5                   10

<210> 353
<211> 11
<212> PRT
<213> Homo sapiens

<400> 353
Cys Arg Trp Arg Pro Glu Ser Ala Ala Pro Cys
1               5                   10


<210> 354
<211> 12
<212> PRT
<213> Homo sapiens

<400> 354
Thr Arg Pro Gly Arg Gly Ala Gln Ala Pro Val Lys
1               5                   10


<210> 355
<211> 21
<212> PRT
<213> Homo sapiens

<400> 355
Met Val Ser Trp Met Ile Ser Arg Ala Val Val Leu Val Phe Gly Met
1               5                   10                  15

Leu Tyr Pro Ala Tyr
            20


<210> 356
<211> 17
<212> PRT
<213> Homo sapiens

<400> 356
Gly Met Leu Tyr Pro Ala Tyr Tyr Ser Tyr Lys Ala Val Lys Thr Lys
1               5                   10                  15

Asn


<210> 357
<211> 17
<212> PRT
<213> Homo sapiens

<400> 357
Glu Tyr Val Arg Trp Met Met Tyr Trp Ile Val Phe Ala Leu Tyr Thr
1               5                   10                  15

Val


<210> 358
<211> 17
<212> PRT
<213> Homo sapiens

<400> 358
Tyr Pro Ala Tyr Tyr Ser Tyr Lys Ala Val Lys Thr Lys Asn Val Lys
1               5                   10                  15

Glu

<210> 359
<211> 13
<212> PRT
<213> Homo sapiens

<400> 359
Val Ala Trp Phe Pro Leu Tyr Tyr Glu Leu Lys Ile Ala
1               5                   10

<210> 360
<211> 186
<212> PRT
<213> Homo sapiens

<220>
<221> misc feature
<222> (181)
<223> Xaa equals any one of the naturally occurring L-amino acids

<400> 360
Met Val Ser Trp Met Ile Ser Arg Ala Val Val Leu Val Phe Gly Met
1               5                   10                  15

Leu Tyr Pro Ala Tyr Tyr Ser Tyr Lys Ala Val Lys Thr Lys Asn Val
            20                  25                  30

Lys Glu Tyr Val Arg Trp Met Met Tyr Trp Ile Val Phe Ala Leu Tyr
        35                  40                  45

Thr Val Ile Glu Thr Val Ala Asp Gln Thr Val Ala Trp Phe Pro Leu
    50                  55                  60

Tyr Tyr Glu Leu Lys Ile Ala Phe Val Ile Trp Leu Leu Ser Pro Tyr
65                  70                  75                  80

Thr Lys Gly Ala Ser Leu Ile Tyr Arg Lys Phe Leu His Pro Leu Leu
                85                  90                  95

Ser Ser Lys Glu Arg Glu Ile Asp Asp Tyr Ile Val Gln Ala Lys Glu
            100                 105                 110

Arg Gly Tyr Glu Thr Met Val Asn Phe Gly Arg Gln Gly Leu Asn Leu
        115                 120                 125

Ala Ala Thr Ala Ala Val Thr Ala Ala Val Lys Ser Gln Gly Ala Ile
    130                 135                 140

Thr Glu Arg Leu Arg Ser Phe Ser Met His Asp Leu Thr Thr Ile Gln
145                 150                 155                 160

Gly Asp Glu Pro Val Gly Gln Arg Pro Tyr Gln Pro Leu Pro Glu Ala

                165                 170                 175

Lys Lys Lys Ser Xaa Gln Pro Pro Val Asn
            180                 185

<210> 361
<211> 15
<212> PRT
<213> Homo sapiens

<400> 361
Gln Pro Tyr Gln Val Leu Pro Ser Arg Gln Val Phe Ala Leu Ile
1               5                   10                  15

<210> 362
<211> 24
<212> PRT
<213> Homo sapiens

<400> 362
Val Phe Ser Cys Ile Tyr Gly Glu Gly Tyr Ser Asn Ala His Glu Ser
1               5                   10                  15

Lys Gln Met Tyr Cys Val Phe Asn
            20

<210> 363
<211> 18
<212> PRT
<213> Homo sapiens

<400> 363
Arg Asn Glu Asp Ala Cys Arg Tyr Gly Ser Ala Ile Gly Val Leu Ala
1               5                   10                  15

Phe Leu

<210> 364
<211> 17
<212> PRT
<213> Homo sapiens

<400> 364
Leu Val Val Asp Ala Tyr Phe Pro Gln Ile Ser Asn Ala Thr Asp Arg
1               5                   10                  15

Lys

<210> 365
<211> 25
<212> PRT
<213> Homo sapiens

<400> 365
Ser Ala Leu Trp Thr Phe Leu Trp Phe Val Gly Phe Cys Phe Leu Thr
1               5                   10                  15

Asn Gln Trp Ala Val Thr Asn Pro Lys
            20                  25


<210> 366
<211> 13
<212> PRT
<213> Homo sapiens

<400> 366
Ser Leu Gln Tyr Arg Ile Arg Ile Pro Gly Arg Pro Thr
1               5                   10


<210> 367
<211> 22
<212> PRT
<213> Homo sapiens

<400> 367
Asp Leu Val Thr Tyr Thr Ser Ser Leu Gln Tyr Arg Ile Arg Ile Pro
1               5                   10                  15

Gly Arg Pro Thr Arg Pro
            20


<210> 368
<211> 36
<212> PRT
<213> Homo sapiens

<400> 368
Leu Gly Asn Lys Lys Tyr Ile Asn Ile Arg Cys Leu Glu Met Gln Val
1               5                   10                  15

Thr Leu Lys Ile Leu Cys Glu Ile Glu Lys Lys Glu Arg Arg Gly Thr
            20                  25                  30

His Cys Leu Val
        35


<210> 369
<211> 22
<212> PRT
<213> Homo sapiens

<400> 369
Val Lys Thr Ala Glu Cys Tyr Ser Ile Pro Leu Gly Ser Cys Pro Val
1               5                   10                  15

Asn Ile Gln Arg Val Arg
            20

<210> 370
<211> 12
<212> PRT
<213> Homo sapiens

<400> 370
Ile Thr Leu Cys Leu Val Cys Ile Val Ala Asn Ala
1               5                   10


<210> 371
<211> 24
<212> PRT
<213> Homo sapiens

<400> 371
Val Thr Ala Tyr Gln Asn Gln Gln Ile Thr Arg Leu Lys Ile Asp Arg
1               5                   10                  15

Asn Pro Phe Ala Lys Gly Phe Arg
            20


<210> 372
<211> 16
<212> PRT
<213> Homo sapiens

<400> 372
Gly Thr Ala Thr Val Thr Ala Tyr Gln Asn Gln Gln Ile Thr Arg Leu
1               5                   10                  15


<210> 373
<211> 24
<212> PRT
<213> Homo sapiens

<400> 373
Lys Ile Asp Arg Asn Pro Phe Ala Lys Gly Phe Arg Asp Ser Gly Arg
1               5                   10                  15

Asn Arg Met Gly Leu Glu Ala Leu
            20


<210> 374
<211> 21
<212> PRT
<213> Homo sapiens

<400> 374
Ser Thr Leu Leu Gln Val Leu Gly Met Ala Phe Leu Pro Leu Thr Leu
1               5                   10                  15

Thr Phe Cys Leu Ala
            20

<210> 375
<211> 30
<212> PRT
<213> Homo sapiens

<400> 375
Val Glu Ser Tyr Ala Phe Trp Arg Pro Ser Leu Arg Thr Leu Thr Phe
1               5                   10                  15

Glu Asp Ile Pro Gly Ile Pro Lys Gln Gly Asn Ala Ser Ser
            20                  25                  30


<210> 376
<211> 14
<212> PRT
<213> Homo sapiens

<400> 376
Gln Ala Gln Ser Asp Cys Ser Cys Ser Thr Val Ser Pro Gly
1               5                   10


<210> 377
<211> 24
<212> PRT
<213> Homo sapiens

<400> 377
Val Leu Ala Gly Ile Val Met Gly Asp Leu Val Leu Thr Val Leu Ile
1               5                   10                  15

Ala Leu Ala Val Tyr Phe Leu Gly
            20


<210> 378
<211> 37
<212> PRT
<213> Homo sapiens

<400> 378
Val Pro Arg Gly Arg Gly Ala Ala Glu Ala Thr Arg Lys Gln Arg Ile
1               5                   10                  15

Thr Glu Thr Glu Ser Pro Tyr Gln Glu Leu Gln Gly Gln Arg Ser Asp
            20                  25                  30

Val Tyr Ser Asp Leu
        35


<210> 379
<211> 22
<212> PRT
<213> Homo sapiens

<400> 379

Glu Thr Glu Ser Pro Tyr Gln Glu Leu Gln Gly Gln Arg Ser Asp Val
1               5                   10                  15

Tyr Ser Asp Leu Asn Thr
            20


<210> 380
<211> 28
<212> PRT
<213> Homo sapiens

<400> 380
Phe Leu Cys Ala Leu Ser Pro Leu Gly Gln Leu Leu Gln Asp Arg Tyr
1               5                   10                  15

Gly Trp Arg Gly Gly Phe Leu Ile Leu Gly Gly Leu
            20                  25


<210> 381
<211> 27
<212> PRT
<213> Homo sapiens

<220>
<221> misc feature
<222> (22)
<223> Xaa equals any one of the naturally occurring L-amino acids

<400> 381
Leu Leu Asn Cys Cys Val Cys Ala Ala Leu Met Arg Pro Leu Val Val
1               5                   10                  15

Thr Ala Gln Pro Gly Xaa Gly Pro Pro Arg Pro
            20                  25


<210> 382
<211> 25
<212> PRT
<213> Homo sapiens

<220>
<221> misc feature
<222> (5)
<223> Xaa equals any one of the naturally occurring L-amino acids

<400> 382
Ser Arg Arg Leu Xaa Asp Leu Ser Val Phe Arg Asp Arg Gly Phe Val
1               5                   10                  15

Leu Tyr Ala Val Ala Ala Ser Val Met
            20                  25


<210> 383
<211> 57
<212> PRT
<213> Homo sapiens

<400> 383
Met Met Ala Thr Pro Ser Thr Arg Pro Pro Pro Pro Ala Ala Ser Thr
1               5                   10                  15

Thr Ser Ala Thr Ala Pro Ala Leu Pro Pro Arg Pro Pro Trp Pro Trp
            20                  25                  30

Pro Pro Ser Ser Trp Pro Pro Ser Gly Val Ser Ser Lys Ala Pro Glu
        35                  40                  45

Ala Asp Pro Leu Lys Asn Lys Ala Leu
    50                  55


<210> 384
<211> 76
<212> PRT
<213> Homo sapiens

<400> 384
Leu Leu Leu Thr Ser Pro Leu Pro Arg Cys Pro Pro Ala Cys Ser His
1               5                   10                  15

Asp Ala Pro Ala His Pro Asp Pro Gly Gly Pro His Gly Leu Thr Ser
            20                  25                  30

Gly Pro Gly Leu Gly Leu Pro Arg Val Cys Leu Gln Arg Arg Gln Leu
        35                  40                  45

Leu Gln Pro His Ala Leu Pro Gly Tyr Gly Cys Leu Leu His Asp His
    50                  55                  60

Ala His Leu Leu His Pro His Gln Asp Glu Gly Gln
65                  70                  75


<210> 385
<211> 56
<212> PRT
<213> Homo sapiens

<400> 385
Trp Leu Leu Gln Ala Arg Val His His Leu Leu Leu Pro Val Arg Pro
1               5                   10                  15

Leu Gln Arg His Arg Pro Cys His Pro Gly His Pro Gly Pro Gly Pro
            20                  25                  30

His Pro Pro Gly His Pro Leu Gly Ser Pro Leu Lys Pro Pro Arg Gln
        35                  40                  45

Thr His Ser Arg Thr Lys Leu Ser
    50                  55


<210> 386
<211> 52
<212> PRT
<213> Homo sapiens

<400> 386
Gln Glu Phe Gln Thr Gly Leu Gly Asn Met Val Lys Pro Cys Leu Tyr
1               5                   10                  15

Glu Lys Tyr Arg Asn Ile Ser Trp Leu Trp Trp His Thr Pro Val Val
            20                  25                  30

Pro Ala Thr Trp Glu Ala Glu Val Gly Gly Ser Leu Glu Pro Gly Arg
        35                  40                  45

Leu Arg Leu Gln
    50


<210> 387
<211> 65
<212> PRT
<213> Homo sapiens

<400> 387
Ile Leu Gly Gly Glu Ser Ile Leu Ile Leu Ser Trp Val Phe Ser Tyr
1               5                   10                  15

Ile Phe Phe Arg Ile Ala Leu Glu Ile Thr Ile Tyr Ile Leu Asn Val
            20                  25                  30

Ser Pro Phe Cys Leu Gly Arg Trp Leu Met Pro Val Ile Pro Ala Leu
        35                  40                  45

Trp Glu Ala Glu Val Gly Gly Leu Pro Glu Leu Arg Ser Ser Arg Pro
    50                  55                  60

Ala
65


<210> 388
<211> 15
<212> PRT
<213> Homo sapiens

<400> 388
Met Pro Lys Gln Leu Ala Gln Leu Leu Tyr Arg Leu Pro Arg Gly
1               5                   10                  15


<210> 389
<211> 46
<212> PRT
<213> Homo sapiens

<400> 389
Leu Phe Gln Ala Ile Ser Val Ser Gly Ser His Arg Gln Gly Ser Arg
1               5                   10                  15

Thr Trp Asn Thr Leu Thr Glu Gly Asn Ala Glu Ala Ala Cys Thr Val
            20                  25                  30

Ala Leu Gln Thr Ser Lys Arg Leu Ile Leu Ala Ser Arg Trp

        35                  40                  45

<210> 390
<211> 50
<212> PRT
<213> Homo sapiens

<400> 390
Thr Leu Ser Phe Met Asn Ser His Cys Val Pro Ile Lys Ala Leu Phe
1               5                   10                  15

Phe Leu Ser Val Val Ser Tyr Ile Phe Ile Met Pro His His Ile Phe
            20                  25                  30

Phe Thr Val Lys Ile Leu Lys Ser Cys Phe Gln Val Gly Gln Leu Met
        35                  40                  45

Lys Leu
    50

<210> 391
<211> 109
<212> PRT
<213> Homo sapiens

<400> 391

<210> 392
<211> 62
<212> PRT
<213> Homo sapiens

<400> 392

<210> 393
<211> 87
<212> PRT
<213> Homo sapiens

<400> 393
Val Pro Tyr His Ile Ala Gly Thr Leu Pro His Cys Cys Ser Leu Pro
1               5                   10                  15

Val Gly Tyr Gly Gly Met Ser Val Arg Leu Gln Gly Cys Arg Tyr Val
            20                  25                  30

Gly Asn Val Gly Pro Gln Gly Asn Met Gln Ser Gly Arg Ser Trp Ala
        35                  40                  45

Leu Lys Met Val Leu Leu Cys Asn Ser Cys Leu Gly Leu Gly Val Gly
    50                  55                  60

Ser Val Gly Pro Ser Met Ser Ser Leu Phe Gly Ala Val Leu Ser Glu
65                  70                  75                  80

Thr Pro Gly Ser Ser Val Tyr
                85

<210> 394
<211> 29
<212> PRT
<213> Homo sapiens

<400> 394
Met Leu Asp Pro Arg Ala Thr Cys Asn Leu Val Gly Val Gly Leu Ser
1               5                   10                  15

Lys Trp Cys Cys Cys Val Thr Ala Ala Trp Val Leu Gly
            20                  25

<210> 395
<211> 65
<212> PRT
<213> Homo sapiens

<220>
<221> misc feature
<222> (48)
<223> Xaa equals any one of the naturally occurring L-amino acids

<400> 395
His Gly Asp Trp Ile Tyr Val His Ile Val Glu Gln Leu Asn Gln Ala
1               5                   10                  15

Asn Asn Lys Ser Val Thr Ser His Thr Tyr Phe Val Val Lys Thr Cys
            20                  25                  30

Lys Ile His Ser Leu Ser Asn Phe Gln Ala Ser Asn Thr Leu Leu Xaa
        35                  40                  45

Thr Val Val Thr Met Leu Tyr Asn Arg Ser Leu Glu Leu Ile Leu Pro
    50                  55                  60

Val
65

<210> 396
<211> 68
<212> PRT
<213> Homo sapiens

<220>
<221> misc feature
<222> (26)
<223> Xaa equals any one of the naturally occurring L-amino acids

<400> 396
Thr Tyr Ser Ser Cys Leu Thr Lys Ile Leu Tyr Ser Leu Ile Asn Ile
1               5                   10                  15

Tyr Pro Ile Pro His Cys Ser Pro Ala Xaa Ile Thr Thr Ile Leu Leu
            20                  25                  30

Ser Ala Ser Met Asn Leu Thr Phe Phe Phe Phe Arg Phe His Ile Cys
        35                  40                  45

Glu Ile Ala Gln Tyr Leu Ser Phe Cys Ala Trp Leu Ile Ser Leu Asn
    50                  55                  60

Ile Lys Ser Leu
65

<210> 397
<211> 33
<212> PRT
<213> Homo sapiens

<400> 397
Met Asn Leu Thr Phe Phe Phe Phe Arg Phe His Ile Cys Glu Ile Ala
1               5                   10                  15

Gln Tyr Leu Ser Phe Cys Ala Trp Leu Ile Ser Leu Asn Ile Lys Ser
            20                  25                  30

Leu

<210> 398
<211> 58
<212> PRT

<213> Homo sapiens

<400> 398
Leu Val Cys Tyr Cys Ser Thr Lys Lys Glu Lys Lys Leu His Glu Ile
1               5                   10                  15

Ala Ile Gln Gln Gly Gln Asn Trp Arg Trp Leu Leu Phe Tyr Lys Glu
            20                  25                  30

Ile Ser Val Pro Gly Phe Gln Ser Val Trp Cys Ser Tyr Lys Cys Leu
        35                  40                  45

Cys Val Val Trp Lys Ala Gly Glu Gly Gly
    50                  55


<210> 399
<211> 36
<212> PRT
<213> Homo sapiens

<400> 399


<210> 400
<211> 37
<212> PRT
<213> Homo sapiens

<400> 400


<210> 401
<211> 77
<212> PRT
<213> Homo sapiens

<400> 401
Ser Lys Asn Lys Thr Arg Gly Gly Asp Trp Cys Val Thr Val Leu Arg
1               5                   10                  15

Lys Arg Arg Lys Ser Phe Met Lys Ser Pro Phe Ser Lys Asp Arg Thr
            20                  25                  30

Gly Asp Gly Phe Ser Phe Thr Lys Lys Ser Leu Ser Gln Ala Phe Ser
        35                  40                  45

Leu Phe Gly Val His Thr Ser Val Cys Val Leu Cys Gly Arg Arg Gly
    50                  55                  60

Lys Ala Gly Glu Gly Gly Pro Val Gln Gly Pro Leu Trp
65                  70                  75


<210> 402
<211> 55
<212> PRT
<213> Homo sapiens

<400> 402


<210> 403
<211> 24
<212> PRT
<213> Homo sapiens

<400> 403
Met Gly Glu Ser Glu Cys Tyr Arg Arg Leu Ser Gly Ala Ser Cys Thr
1               5                   10                  15

Trp Thr Val His Val Asp Phe Ala
            20


<210> 404
<211> 33
<212> PRT
<213> Homo sapiens

<400> 404
Met His Cys Gly Thr Arg Val Trp Lys Thr Met Lys His Asp Tyr Phe
1               5                   10                  15

Leu Leu Ala Cys Leu Ser Met Thr Ser Thr Gly Gly Ile Leu Cys Thr
            20                  25                  30

Leu


<210> 405
<211> 67

<212> PRT
<213> Homo sapiens

<400> 405
Ser Thr Leu Ser Leu Ile Pro Thr Ser Ser Ser Leu Ser Phe Trp Pro
1               5                   10                  15

Trp Cys Thr Ala Ile Ile Gly Ser Ile Phe Thr Tyr Cys Val Cys Val
            20                  25                  30

Cys Val Cys Phe Val Val Met Asn Arg Thr Cys Tyr Leu Pro Asn Ser
        35                  40                  45

Ile Ile Tyr His Asn Ser Lys Leu Ala Thr Ile Ile Asp Lys Ser Met
    50                  55                  60

Thr Leu Ser
65


<210> 406
<211> 20
<212> PRT
<213> Homo sapiens

<400> 406


<210> 407
<211> 62
<212> PRT
<213> Homo sapiens

<400> 407
Met Ser Thr Gly Asp Gly Arg Asp Ala Glu Lys Gly Trp Pro Val Ser
1               5                   10                  15

Glu Glu Glu Asn Gln Arg Ser Val Tyr Pro Gly Tyr Pro Glu Cys Asp
            20                  25                  30

Glu Arg Gln Ala Val Pro Gln His Cys Ala Ile Ala Ser Pro Ser Ser
        35                  40                  45

Leu Gln Ser His His Pro Ala Ser Ala Cys Val Pro Arg Arg
    50                  55                  60


<210> 408
<211> 38
<212> PRT
<213> Homo sapiens

<400> 408
Gln Gln Met Thr Leu Gly Thr Lys Ile Lys Trp Gly Gln Leu Gln Arg
1               5                   10                  15

Gly Gln Glu Ile Pro Thr Gly Asp Phe Thr Val Arg Asn Phe Met Arg
            20                  25                  30

Phe Ser Ile Ile Tyr Cys
        35


<210> 409
<211> 31
<212> PRT
<213> Homo sapiens

<220>
<221> misc feature
<222> (11)
<223> Xaa equals any one of the naturally occurring L-amino acids

<400> 409
Pro Phe Leu Phe Cys Ala Ser Arg Ile Arg Xaa Gln Gly Ile Gly Ile
 1               5                  10                  15

His Gly Gln Val Ala Cys Ser Ala Val Arg Met Tyr Asn Asn Arg
            20                  25                  30


<210> 410
<211> 45
<212> PRT
<213> Homo sapiens

<400> 410
Val Leu Cys Glu Glu Ala Gly Gln Lys Val Pro Ser Thr Pro Ser Trp
 1               5                  10                  15

Ser Ser Trp Thr Leu Gln Lys Arg Leu Arg Gly Ser Pro Ala Glu Ala
            20                  25                  30

Asn Cys Ser Pro Ser Phe Pro Ala Pro Pro Gly Lys Glu
        35                  40                  45


<210> 411
<211> 103
<212> PRT
<213> Homo sapiens

<400> 411
Met Ser Leu Ser Ala Leu Ala Cys Asp Phe Thr Pro Ile Gln Pro Trp
 1               5                  10                  15

Glu Trp Glu Glu Tyr Glu Gln Ile Thr Leu Gly Leu Thr Ala Pro Ser
            20                  25                  30

Asn Leu Leu Glu Ser Asn Tyr Leu Gly Gln Ala Ser Glu Cys Phe Val
        35                  40                  45

Arg Lys Leu Val Arg Arg Phe Pro Gln Leu Leu Pro Gly Pro Pro Gly
    50                  55                  60

His Cys Arg Lys Asp Leu Gly Asp Pro Gln Gln Arg Pro Ile Ala Leu
65                  70                  75                  80

Leu Pro Ser Leu Pro His Gln Glu Arg Asn Asn Val His Arg Leu Glu
                85                  90                  95

Ala Asp Ser Glu Val Asp Leu
            100

<210> 412
<211> 46
<212> PRT
<213> Homo sapiens

<400> 412
Cys Val Asp Phe Asp Glu Tyr Phe Ser Ser Trp Glu Pro Leu Leu Lys
1               5                   10                  15

Met Met Phe Lys Gly Val Val Gly Gly Lys Met Lys Ala Trp Arg Arg
            20                  25                  30

Lys Lys Arg Arg Lys Pro Leu Pro Tyr Lys Ile His Ala Asp
        35                  40                  45

<210> 413
<211> 30
<212> PRT
<213> Homo sapiens

<400> 413

<210> 414
<211> 37
<212> PRT
<213> Homo sapiens

<400> 414
Leu Ile Ser Ser Val Asn Lys Thr Lys Gln Lys Arg Ser Asp Ala Thr
1               5                   10                  15

Leu Ser His Lys His Asp Arg Leu Leu Asn His Phe Val Phe Phe Gly
            20                  25                  30

Asn Ser Tyr Asn Tyr
        35

<210> 415
<211> 127
<212> PRT
<213> Homo sapiens

<220>
<221> misc feature
<222> (95)
<223> Xaa equals any one of the naturally occurring L-amino acids

<400> 415
Ser Ser Lys Phe Pro Ser Asp Met Leu Leu Arg Ile Gln Gln Ile Ile
1               5                   10                  15

Tyr Cys His Lys Leu Thr Ile Ile Leu Thr Lys Trp Arg Asn Thr Ala
            20                  25                  30

Arg His Lys Ser Lys Lys Lys Glu Asp Glu Leu Ile Leu Lys His Glu
        35                  40                  45

Leu Gln Leu Lys Lys Trp Lys Asn Arg Leu Ile Leu Lys Arg Ala Ala
    50                  55                  60

Ala Glu Glu Ser Asn Phe Pro Glu Arg Ser Ser Ser Glu Val Phe Leu
65                  70                  75                  80

Val Asp Glu Thr Leu Lys Cys Asp Ile Ser Leu Leu Pro Glu Xaa Ala
                85                  90                  95

Ile Leu Gln Val Cys Met Asn Ser Val Tyr Ile Ile Tyr Tyr Asn Leu
            100                 105                 110

Pro Ser Val Val Val His Ala Cys Asn Pro Ser Cys Leu Gly Gly
        115                 120                 125


<210> 416
<211> 11
<212> PRT
<213> Homo sapiens

<400> 416
Ser Leu Glu Ser Thr Asn Ala Ile Lys Ser Asn
1               5                   10


<210> 417
<211> 19
<212> PRT
<213> Homo sapiens

<400> 417
Ile Arg Pro Asn Lys Asn Asp Gln Met Arg His Cys Leu Ile Asn Met
1               5                   10                  15

Ile Asp Tyr


<210> 418
<211> 37
<212> PRT
<213> Homo sapiens

<400> 418

Ile Thr Leu Cys Phe Leu Glu Thr Ala Ile Thr Ile Asn Ile Tyr Ser
1               5                   10                  15

Asn Leu Val Asn Phe Leu Gln Ile Cys Tyr Cys Gly Tyr Asn Arg Ser
            20                  25                  30

Ser Ile Val Thr Ser
        35


<210> 419
<211> 31
<212> PRT
<213> Homo sapiens

<400> 419
Ile Ser Phe Arg Tyr Ala Ile Ala Asp Thr Thr Asp His Leu Leu Ser
1               5                   10                  15

Gln Ala Asn His Tyr Pro Asn Lys Met Ala Glu Tyr Ser Lys Thr
            20                  25                  30


<210> 420
<211> 86
<212> PRT
<213> Homo sapiens

<220>
<221> misc feature
<222> (18)
<223> Xaa equals any one of the naturally occurring L-amino acids

<400> 420
Pro Gln Ile Lys Leu Leu Asn Ser Asp Ala Leu Gly Met Arg Thr Thr
1               5                   10                  15

Ser Xaa Asp Leu Val Pro Cys Asn Gln Cys Phe Ile Pro Leu Pro Pro
            20                  25                  30

Ser Cys Asn Arg Ile Ala Ser Arg Lys Ala Val Asn Trp Lys Gln Gln
        35                  40                  45

Arg Leu Pro Ala Val Arg Gly Leu Leu Asn Asn Ala Pro His Arg Arg
    50                  55                  60

Pro Pro Thr Pro Arg Thr Pro Cys Val Phe Pro Ser Glu Gly Pro Lys
65                  70                  75                  80

Gly Tyr Gly Phe His Val
                85


<210> 421
<211> 39
<212> PRT
<213> Homo sapiens

<220>
<221> misc feature

<222> (5)
<223> Xaa equals any one of the naturally occurring L-amino acids

<400> 421
Glu Gln Leu Ala Xaa Ile Ser Cys Arg Val Ile Asn Val Ser Phe Arg
1               5                   10                  15

Cys Leu His His Val Ile Glu Ser Leu Pro Glu Arg Gln Leu Thr Gly
            20                  25                  30

Ser Ser Arg Gly Ser Gln Pro
        35


<210> 422
<211> 73
<212> PRT
<213> Homo sapiens

<220>
<221> misc feature
<222> (45)
<223> Xaa equals any one of the naturally occurring L-amino acids

<400> 422
Glu Asp Cys Ser Thr Met Pro Pro Ile Ala Ala Pro Pro Pro Leu Ala
1               5                   10                  15

Pro Leu Val Phe Ser Pro Leu Arg Gly Pro Arg Val Met Ala Phe Met
            20                  25                  30

Ser Arg Cys Gly Asp Arg Gly Gly Arg Gly Arg Ser Xaa Ala Gly Arg
        35                  40                  45

Gly Trp Pro Trp Ser Glu Ser Gly Val Ile Asn Ala His Pro Lys Lys
    50                  55                  60

Arg Pro Cys Pro Gly Pro Met Leu Ser
65                  70


<210> 423
<211> 48
<212> PRT
<213> Homo sapiens

<400> 423

<210> 424
<211> 42
<212> PRT
<213> Homo sapiens

<400> 424
Ala Thr Val Pro Gly Ser Ile Tyr Asn Tyr Phe Tyr His Tyr Asn Ala
1               5                   10                  15

Gly Ala Leu Lys Pro Glu His Ala Ser Glu Ser Pro Arg Gly Leu Cys
            20                  25                  30

Ala Gln Thr Ala Gly Pro Phe Pro Ser Phe
        35                  40


<210> 425
<211> 56
<212> PRT
<213> Homo sapiens

<400> 425
Ile Arg His Glu Pro Pro Pro Pro Arg Phe Lys Arg Phe Ser Cys Leu
1               5                   10                  15

Ser Leu Leu Ser Ser Trp Asp Tyr Arg Arg Ala Pro Pro His Val Ala
            20                  25                  30

Ile Phe Cys Thr Leu Ser Arg Asp Gly Val Leu Pro His Trp Pro Gly
        35                  40                  45

Trp Ser Gln Thr Pro Asp Leu Lys
    50                  55


<210> 426
<211> 72
<212> PRT
<213> Homo sapiens

<400> 426


<210> 427
<211> 26
<212> PRT

<213> Homo sapiens

<400> 427
Leu Arg Val Gly Gly Thr Ser Glu Asn Cys Cys Arg Gly Glu Cys Cys
1               5                   10                  15

Gly Ser Val Cys Ile Pro Pro Gly Arg Leu
            20                  25


<210> 428
<211> 46
<212> PRT
<213> Homo sapiens

<400> 428
Gly Leu Cys Met Val His Ser Leu Leu Thr Ser Ser Leu Gly Gly Arg
1               5                   10                  15

Cys Cys Asn Tyr Pro Tyr Ile Ala Asp Lys Asp Ile Glu Thr Glu Val
            20                  25                  30

Lys Pro Pro Ser Gln Gly His Thr Trp His Leu His Cys Ser
        35                  40                  45


<210> 429
<211> 75
<212> PRT
<213> Homo sapiens

<400> 429
Gln Leu Trp Cys Ile Thr Ala Leu Pro Ser Thr Arg His Cys Ser Lys
1               5                   10                  15

Gly Phe Ala Trp Phe Thr His Ser Leu Arg His Pro Ser Val Ala Gly
            20                  25                  30

Ala Val Ile Ile Leu Ile Leu Gln Thr Arg Thr Leu Arg Gln Arg Ser
        35                  40                  45

Ser His Leu Pro Lys Gly Thr His Gly Ile Cys Thr Ala Pro Asp Arg
    50                  55                  60

Pro Thr Glu Arg Ala Ala Val Thr Ile Leu Lys
65                  70                  75


<210> 430
<211> 39
<212> PRT
<213> Homo sapiens

<400> 430
Ser Phe Asp Asn Asn Asn Ser Tyr Gly Val Ser Gln Leu Tyr Gln Val
1               5                   10                  15

Pro Asp Thr Val Leu Arg Ala Leu His Gly Ser Leu Thr Pro Tyr Val
            20                  25                  30

Ile Pro Arg Trp Gln Val Leu
        35

<210> 431
<211> 38
<212> PRT
<213> Homo sapiens

<400> 431
Asp Arg Gly Gln Ala Thr Phe Pro Arg Ala His Met Ala Ser Ala Leu
1               5                   10                  15

Leu Leu Thr Asp Arg Gln Arg Glu Leu Leu Ser Arg Ser Ser Asn Glu
            20                  25                  30

Leu Cys Met Ser Lys Val
        35

<210> 432
<211> 73
<212> PRT
<213> Homo sapiens

<220>
<221> misc feature
<222> (66)
<223> Xaa equals any one of the naturally occurring L-amino acids

<400> 432
Leu Leu Leu Ile Leu Arg Pro Phe Leu Asn Ser Gln Phe Lys Leu Gln
1               5                   10                  15

Leu Pro Leu Val Leu Phe His Ser Ser Cys Thr Tyr Ile Cys Leu Leu
            20                  25                  30

Tyr Asn Tyr Glu Leu Phe His Ile Val Ala Leu Thr Gly Lys Leu Met
        35                  40                  45

Asn Leu Gly Leu His Leu Phe Ala His His Leu Ile Leu Ala Val Ala
    50                  55                  60

His Xaa Gly Cys Ser Ile Pro Ile Tyr
65                  70

<210> 433
<211> 37
<212> PRT
<213> Homo sapiens

<400> 433
Thr His Asn Ser Asn Tyr Ser Ser Leu Trp Phe Ser Ser Thr Ala Val
1               5                   10                  15

Val Leu Thr Tyr Val Tyr Tyr Ile Ile Met Asn Cys Phe Ile Leu Ser
            20                  25                  30

Pro Leu Gln Val Asn

        35

<210> 434
<211> 53
<212> PRT
<213> Homo sapiens

<400> 434
Thr Leu Val Ala Gly Ser Pro Cys Ser Leu Ser Arg Trp Ile Met Ala
1               5                   10                  15

Gly Phe Cys His Gly Glu Leu Val Gln Ser Asp Met Glu Ser Gln Glu
            20                  25                  30

Trp Glu Arg Gly Gln Val Val Leu Ser His Thr Ser Leu Pro Trp Cys
        35                  40                  45

Tyr Val Ser Pro Arg
    50


<210> 435
<211> 39
<212> PRT
<213> Homo sapiens

<400> 435
Met Ala Gly Phe Cys His Gly Glu Leu Val Gln Ser Asp Met Glu Ser
1               5                   10                  15

Gln Glu Trp Glu Arg Gly Gln Val Val Leu Ser His Thr Ser Leu Pro
            20                  25                  30

Trp Cys Tyr Val Ser Pro Arg
        35


<210> 436
<211> 94
<212> PRT
<213> Homo sapiens

<400> 436

<210> 437
<211> 82
<212> PRT
<213> Homo sapiens

<400> 437
Arg Ser Lys Arg Gln Ser Gln Gly Ser Arg Cys Ser Val Pro Leu Leu
1               5                   10                  15

Ala Gln Gln Ser Arg Ser Pro Pro Val Pro Leu Gln Ala Gln Pro Ala
            20                  25                  30

Trp Leu Leu Gly Ser Glu Thr Ile Ala Trp Ser Gly Gly Gly Ser Gly
        35                  40                  45

Trp Glu Gly Pro Arg Asp Pro Gly Thr Ser Thr Ala Ala Gly Asn Ser
    50                  55                  60

Gly Pro Gly Ile Gly Met Gly His Arg Thr Pro Pro Pro Ser His Thr
65                  70                  75                  80

Gly Arg

<210> 438
<211> 30
<212> PRT
<213> Homo sapiens

<400> 438
Arg Trp Asp Pro Ala Trp Gly Leu Asp Ile Pro Glu Ser Ser Cys Pro
1               5                   10                  15

Val Thr Met Gly Glu Leu Arg Ser Gly Asp Gly Ile Val Leu
            20                  25                  30

<210> 439
<211> 50
<212> PRT
<213> Homo sapiens

<400> 439
Gly Ala Leu Leu Trp Asp Asn Ser Met Ile Ser Ala Pro Arg Gly Ser
1               5                   10                  15

His Arg Glu Ala Gly Ala Leu Phe Pro Ser Trp Leu Ser Asn Pro Ala
            20                  25                  30

Val Leu Pro Ser Arg Ser Arg Pro Ser Gln Pro Gly Cys Leu Asp Pro
        35                  40                  45

Arg Gln
    50

<210> 440
<211> 49
<212> PRT
<213> Homo sapiens

<400> 440


<210> 441
<211> 11
<212> PRT
<213> Homo sapiens

<400> 441
Met Ala Arg Ala Ser Glu Ala Ala Gly Thr Gly
1               5                   10


<210> 442
<211> 84
<212> PRT
<213> Homo sapiens

<400> 442
Cys Phe Thr Thr Ala Phe Gln Lys Ala Leu Arg Asp Pro Arg Pro Thr
1               5                   10                  15

Leu Pro Asp Thr His Gly Ser Leu Arg Asn Ala Pro Leu Lys Ser Leu
            20                  25                  30

Thr Leu Pro Ala Ala Phe Val Val Ser Phe Phe Phe Leu Ser Leu Leu
        35                  40                  45

Gln Asp Gly Ile Lys Glu Arg Ser Gln Thr Gln Asn Ala Thr Phe Phe
    50                  55                  60

Phe His Asp Arg Ser Asp Ile Glu Gly Leu Ser Glu Glu Pro Cys Ser
65                  70                  75                  80

Gly Thr Thr Pro


<210> 443
<211> 95
<212> PRT
<213> Homo sapiens

<400> 443

Leu Ala Leu Gln Glu Ala Val Thr Gly Lys Gln Val Leu Cys Ser Pro
1               5                   10                  15

Pro Gly Ser Ala Ile Pro Gln Ser Ser Arg Pro Ala Pro Gly Pro Ala
            20                  25                  30

Ser Leu Ala Ala Trp Ile Arg Asp Asn Ser Leu Val Trp Arg Arg Leu
        35                  40                  45

Arg Val Gly Gly Thr Gln Gly Pro Gly His Gln Tyr Ser Ser Trp Glu
    50                  55                  60

Phe Arg Pro Arg Asp Arg Asp Gly Ala Gln Asp Thr Thr Pro Ile Ser
65                  70                  75                  80

His Arg Glu Met Lys Val Gly Ser Ser Met Gly Thr Gly His Pro
                85                  90                  95


<210> 444
<211> 42
<212> PRT
<213> Homo sapiens

<400> 444
Met Phe Tyr Ser Lys Ile Phe Tyr Phe Leu Leu Leu Asn Ser Asp Thr
1               5                   10                  15

Ser Asn Asn Val Thr Ser Lys Thr Leu Val Ser Ser Ile Ser Ser Ser
            20                  25                  30

Asn Asn Arg Leu Ala Val Ser Ile Val Phe
        35                  40


<210> 445
<211> 47
<212> PRT
<213> Homo sapiens

<400> 445
Ser Arg Gln Lys Asn Leu Leu Lys Leu His Ser Asn Pro Asn Cys Asp
1               5                   10                  15

Asn Phe Cys Phe Ile Phe Asn Tyr Lys Pro Lys Tyr Ile Cys Ile Phe
            20                  25                  30

Lys Leu Ile Cys Leu Lys Ile Leu Leu Tyr Ile Phe Gly Ser Gly
        35                  40                  45


<210> 446
<211> 56
<212> PRT
<213> Homo sapiens

<220>
<221> misc feature
<222> (24)
<223> Xaa equals any one of the naturally occurring L-amino acids

<400> 446
Met Leu Leu Ser Leu Leu Met Val Phe Thr Ser Glu Leu Tyr Val Lys
1               5                   10                  15

Arg His Ile Ser Phe Lys Ser Xaa Asp Lys Pro His Cys His Lys Asn
            20                  25                  30

Gln Asp Ile Asp Val Leu Phe Arg Lys Leu Leu Glu Lys His Phe Lys
        35                  40                  45

Val Ile Asn Met Ile Cys Phe Pro
    50                  55


<210> 447
<211> 12
<212> PRT
<213> Homo sapiens

<400> 447
Phe Arg Glu Tyr Gly Phe Tyr Asn Leu His Phe Cys
1               5                   10


<210> 448
<211> 38
<212> PRT
<213> Homo sapiens

<400> 448
Leu Val Thr Thr Asp Tyr Tyr Asp Gly Cys Asn Glu Asp Tyr Glu Tyr
1               5                   10                  15

Asn Trp Ser Tyr Met Phe Leu Asn Ser Glu Gln Leu Phe Ile Lys Phe
            20                  25                  30

Tyr Pro Thr Phe Phe Cys
        35


<210> 449
<211> 52
<212> PRT
<213> Homo sapiens

<400> 449
Asn Val Ile Ala Pro Gly Leu Glu Ser Ser Cys Ala Asn Ser Leu Phe
1               5                   10                  15

Leu Leu Phe Val Cys Leu Pro Val Ala His His Arg His Asn Phe Leu
            20                  25                  30

Phe Ile Lys His Ser Leu Tyr Asn His Leu Arg Asp Tyr Glu Ser Asp
        35                  40                  45

Phe Asp Lys Ile
    50

<210> 450
<211> 34
<212> PRT
<213> Homo sapiens

<400> 450
Pro Lys Val Leu Ala Val Leu Lys Lys Lys Asn His Val Ala Leu Ser
1               5                   10                  15

Ile Phe Glu Leu Leu Ser Asn Asp Ile Cys Ser Phe Ile Ser Phe Phe
            20                  25                  30

Met Ser


<210> 451
<211> 28
<212> PRT
<213> Homo sapiens

<400> 451
Glu Gly Pro Asp Ile Asn Ser Asn Leu Lys Phe Leu Leu Cys Leu Lys
1               5                   10                  15

Lys Lys Ile Met Trp Pro Phe Gln Tyr Leu Asn Cys
            20                  25


<210> 452
<211> 47
<212> PRT
<213> Homo sapiens

<400> 452
Leu Leu Ser Leu Ile Leu Leu Arg Ile Trp Tyr Asp Phe Ser Lys Gln
1               5                   10                  15

Thr Val Phe Trp Phe Phe Leu Asn Val Phe Asn Phe Phe Ser Ser Cys
            20                  25                  30

Asn Asn Asp Gly Ala Cys Ser Tyr Lys Tyr Arg Lys Val Gln Ile
        35                  40                  45


<210> 453
<211> 12
<212> PRT
<213> Homo sapiens

<400> 453
His Thr Leu Phe Ile Ser Phe Leu Trp Ala Glu Gly
1               5                   10


<210> 454
<211> 28
<212> PRT
<213> Homo sapiens

<400> 454
Met Leu Pro Val Phe Val Leu Phe Phe Cys Phe Thr Tyr Ser Ala Arg
1               5                   10                  15

Lys Gln Ser Val Phe Lys Lys Gly Asn Val Phe Glu
            20                  25


<210> 455
<211> 63
<212> PRT
<213> Homo sapiens

<400> 455
Ser Pro Cys Ser Ala Ala Glu Cys His Asn Leu Ser Leu Leu Ser Ser
1               5                   10                  15

Cys Ser Leu Val Ser Ser Asn Ile Leu Phe Ser Phe Pro Phe Phe Gly
            20                  25                  30

Gln Lys Ala Arg Cys Cys Leu Phe Leu Phe Tyr Phe Ser Ala Ser His
        35                  40                  45

Ile Ala His Glu Ser Arg Val Tyr Ser Lys Lys Glu Met Cys Leu
    50                  55                  60


<210> 456
<211> 65
<212> PRT
<213> Homo sapiens

<400> 456
His Lys Cys Phe Gln Cys Phe Ile Leu Ala Asn Gly Phe Leu Lys Val
1               5                   10                  15

Ile Lys Pro Phe Gln Arg Asn Trp Ser Asp Lys Thr Phe Phe Leu Val
            20                  25                  30

Cys Leu Asn Lys Ala Ile Ser Glu Ala Leu Leu Ser Lys Met Thr Phe
        35                  40                  45

Leu Ser Phe Phe Lys Thr Asn Leu Leu Leu Leu Glu Thr Phe Cys Thr
    50                  55                  60

Ile
65


<210> 457
<211> 99
<212> PRT
<213> Homo sapiens

<400> 457
Leu Leu Gly Val Leu Lys Pro Leu Tyr Phe Ser Val Glu Pro Val Leu
1               5                   10                  15

Gly Glu Arg Ser Val Ala Phe Glu Glu Val Arg Glu Lys Asn His Gly
            20                  25                  30

Thr Ser Gly Phe Leu Ser Leu Tyr Ser Leu Ala Ala Ile Val Cys Gly
        35                  40                  45

His Leu Met Phe Phe His Thr Leu Leu Gly Arg Gly Gly Asn Asp His
    50                  55                  60

Pro Gly Gln Ser Pro Leu Pro Gly Met Arg Pro Leu Arg Gly Gly Leu
65                  70                  75                  80

Ala Gly Gln Ala Pro Ser Gly His Pro Trp Met Gln Pro Leu Asp Thr
                85                  90                  95

Cys Leu Leu


<210> 458
<211> 43
<212> PRT
<213> Homo sapiens

<400> 458


<210> 459
<211> 49
<212> PRT
<213> Homo sapiens

<400> 459
Glu Lys Arg Ile Met Val Pro Gln Gly Phe Phe Pro Phe Thr Arg Trp
1               5                   10                  15

Gln Pro Leu Ser Val Gly Thr Ser Cys Phe Ser Thr Leu Tyr Trp Ala
            20                  25                  30

Val Glu Val Thr Ile Thr Gln Ala Ser Leu Leu Cys Leu Gly Cys Ala
        35                  40                  45

Leu


<210> 460
<211> 123
<212> PRT
<213> Homo sapiens

<400> 460
Met Thr Leu Asp Glu Trp Lys Asn Leu Gln Glu Gln Thr Arg Pro Lys
1               5                   10                  15

Pro Glu Phe Asn Ile Arg Lys Pro Glu Ser Thr Val Pro Ser Lys Ala
                20                  25                  30

Val Val Ile Arg Glu Ser Lys Tyr Arg Asp Asp Met Val Lys Asp Asp
            35                  40                  45

Tyr Glu Asp Asp Ser His Val Phe Arg Lys Pro Ala Asn Asp Ile Thr
    50                  55                  60

Ser Gln Leu Glu Ile Asn Phe Gly Asn Leu Pro Arg Pro Gly Arg Gly
65                  70                  75                  80

Ala Arg Gly Gly Thr Arg Gly Gly Arg Gly Arg Ile Arg Arg Ala Glu
                85                  90                  95

Asn Tyr Gly Pro Arg Ala Glu Val Val Met Gln Asp Val Ala Pro Asn
            100                 105                 110

Pro Asp Asp Pro Glu Asp Phe Pro Ala Leu Ser
        115                 120


<210> 461
<211> 100
<212> PRT
<213> Homo sapiens

<400> 461
Cys Lys Met Leu Pro Pro Thr Gln Met Thr Arg Lys Ile Ser Leu Arg
1               5                   10                  15

Cys Leu Glu Arg Ala Leu Phe Pro Ser Thr Ala Glu Leu His Cys Thr
            20                  25                  30

Pro Val Gly Arg Leu Phe Gln Leu Gly Gln Gly Ser Gln Thr Leu Arg
        35                  40                  45

Thr Ile Asp Val Ala Phe Pro Val Ser Cys Lys Phe Val Ala Leu Phe
    50                  55                  60

Trp Ala Glu Leu Leu Glu Gly Leu Leu Gln Arg Leu Glu Ser Arg Pro
65                  70                  75                  80

Phe Pro Lys Lys Met Lys Asn Gly Asp Cys Val Phe Ile Glu Gly Ile
                85                  90                  95

Ser Asn Glu Glu
            100


<210> 462
<211> 41
<212> PRT
<213> Homo sapiens

<400> 462
Pro Pro Ser Ser Trp Ala Trp Ser Gln Arg Arg His Pro Gly Arg Pro
1               5                   10                  15

Gly Lys Asp Gln Glu Gly Arg Glu Leu Trp Thr Gln Ser Arg Ser Gly
            20                  25                  30

Asp Ala Arg Cys Cys Pro Gln Pro Arg
        35                  40


<210> 463
<211> 22
<212> PRT
<213> Homo sapiens

<400> 463
Cys Leu Lys Cys Val Tyr Arg Asp Ser Ile Asp Ser Ser Ala Glu Ala
1               5                   10                  15

Trp Arg Glu Arg Arg Leu
            20


<210> 464
<211> 29
<212> PRT
<213> Homo sapiens

<400> 464
Leu Ser Tyr Ser Val Leu Leu Ile Leu Pro Leu Phe His Ser Leu Pro
1               5                   10                  15

Thr Leu Lys Asp Thr His Thr His Asn Lys Trp Val Glu
            20                  25


<210> 465
<211> 61
<212> PRT
<213> Homo sapiens

<400> 465
Glu Val Asn Gly Val Gly Tyr Lys His Ser Cys Phe Ser Asp Ile Ser
1               5                   10                  15

Ser Val Leu Glu Asn Lys Asp Ser Arg Met Arg Ala Pro His Tyr Ala
            20                  25                  30

Ser Phe Gln His Phe Phe Ser Val Leu Leu Lys Leu Ser Pro Gln Ala
        35                  40                  45

Cys Leu Thr Glu Ser Gln Cys Ile Pro Leu Thr Phe Tyr
    50                  55                  60


<210> 466
<211> 37
<212> PRT
<213> Homo sapiens

<400> 466
Lys Thr His Thr His Thr Ile Ser Gly Trp Ser Lys Lys Ser Thr Glu
1               5                   10                  15

Leu Asp Ile Ser Ile Pro Ala Phe Leu Thr Ser Pro Val Ser Trp Arg
            20                  25                  30

Thr Arg Ile Leu Glu
        35

<210> 467
<211> 29
<212> PRT
<213> Homo sapiens

<400> 467
Ile Arg His Glu Leu Gly Ser Ser Asp Pro Pro Ala Glu Ala Ser Gln
 1               5                  10                  15

Ile Ala Gly Thr Ala Ala Val Ser His His Ala Gln Pro
            20                  25

<210> 468
<211> 25
<212> PRT
<213> Homo sapiens

<400> 468
Met Leu Tyr Leu Ile Leu Ile Ser Leu Ser Ser Leu Ser Phe Ser Phe
 1               5                  10                  15

Ser Leu Pro Pro Phe Ser Ile Ile Ile
            20                  25

<210> 469
<211> 24
<212> PRT
<213> Homo sapiens

<400> 469
Ser Ser Tyr Phe Leu Arg His Phe Arg Ile Tyr His Thr Cys Pro Lys
 1               5                  10                  15

Tyr Phe Ser Met Asn Ile Ile Asn
            20

<210> 470
<211> 69
<212> PRT
<213> Homo sapiens

<400> 470
Lys Leu Thr Leu Thr Lys Gly Asn Lys Ser Trp Ser Ser Thr Ala Val
 1               5                  10                  15

Ala Ala Ala Leu Glu Leu Val Asp Pro Pro Gly Cys Arg Asn Ser Ala
            20                  25                  30

Arg Asp Ser Leu Pro Asn Ser Thr Met Met Phe Tyr Tyr Ala Cys Phe

        35                  40                  45

Ile Leu Tyr Ser Ser Leu Ser Pro Leu Ser Leu Ser Leu Ser Pro Ser
    50                  55                  60

Leu Leu Ser Leu Leu
65

<210> 471
<211> 14
<212> PRT
<213> Homo sapiens

<400> 471
Gln Phe His Thr Gly Asn Ser Tyr Asp His Asp Tyr Ala Lys
1               5                   10

<210> 472
<211> 35
<212> PRT
<213> Homo sapiens

<400> 472
Ala Val Cys Thr Gly Gly Tyr Cys Glu Ser Cys Arg Cys Glu His Cys
1               5                   10                  15

Val Cys Val Cys Val Asp Leu Cys Val Leu Phe Ser Gly Lys Glu Leu
            20                  25                  30

Arg Val Arg
        35

<210> 473
<211> 72
<212> PRT
<213> Homo sapiens

<400> 473

<210> 474
<211> 99
<212> PRT

<213> Homo sapiens

<400> 474



<210> 475
<211> 74
<212> PRT
<213> Homo sapiens

<400> 475
Met Gly His Leu Phe Val Val Cys Leu Leu Ser Ser Trp Trp Thr Phe
1               5                   10                  15

Arg Pro Phe Ala Leu Ala Val Thr Val Asn His Val Ala Val Asn Ile
            20                  25                  30

Val Cys Val Ser Ala Trp Thr Cys Val Ser Cys Ser Leu Gly Arg Ser
        35                  40                  45

Cys Gly Leu Glu Gly Ser Phe Leu Phe Pro Leu Glu Thr Leu Trp Phe
    50                  55                  60

Pro His Met Val Val Leu Cys Leu Thr Phe
65                  70


<210> 476
<211> 51
<212> PRT
<213> Homo sapiens

<400> 476
His Asp Val Leu Gly Ala Arg Asn Ala Ala Cys Val Cys Cys Ser Phe
1               5                   10                  15

Leu Leu Gln Gln Asn Arg Ile Leu Leu Phe Gly Trp Ala Thr Cys Leu
            20                  25                  30

Leu Ser Val Tyr Ser Pro Ala Gly Gly His Leu Gly Arg Leu His Trp

        35                  40                  45

Arg Leu Leu
    50


<210> 477
<211> 130
<212> PRT
<213> Homo sapiens

<400> 477


<210> 478
<211> 28
<212> PRT
<213> Homo sapiens

<400> 478
Leu Thr Arg Asn Ser Phe Pro Glu Asn Arg Thr His Lys Ser Thr Gln
1               5                   10                  15

Thr His Thr Gln Cys Ser Gln Arg His Asp Ser Gln
            20                  25

<210> 479
<211> 90
<212> PRT
<213> Homo sapiens

<400> 479
Ile Arg His Glu Gly Gln Ser Ser Ser Arg Gly Ser Ser His Cys Asp
1               5                   10                  15

Ser Pro Ser Pro Gln Glu Asp Gly Gln Ile Met Phe Asp Val Glu Met
            20                  25                  30

His Thr Ser Arg Asp His Ser Ser Gln Ser Glu Glu Glu Val Val Glu
        35                  40                  45

Gly Glu Lys Glu Val Glu Ala Leu Lys Lys Ser Ala Asp Trp Val Ser
    50                  55                  60

Asp Trp Ser Ser Arg Pro Glu Asn Ile Pro Pro Lys Glu Phe His Phe
65                  70                  75                  80

Arg His Pro Lys Arg Ser Val Ser Leu Ser
                85                  90


<210> 480
<211> 40
<212> PRT
<213> Homo sapiens

<400> 480
Gly Ile Leu Leu Thr Leu Tyr Pro Phe Trp Pro Glu Asp Ile Leu Glu
1               5                   10                  15

Phe Pro Asn Arg Val Tyr Cys Cys Leu Glu Ile Cys Lys Gly Phe Phe
            20                  25                  30

Ser Ala Asn Ala Thr Ser Arg Leu
        35                  40

<210> 481
<211> 47
<212> PRT
<213> Homo sapiens

<400> 481
Glu Phe Gly Thr Arg Asp Arg Val Val Pro Glu Ala Val Leu Thr Val
1               5                   10                  15

Thr Ala Leu Arg His Lys Lys Met Gly Arg Ser Cys Leu Met Trp Lys
            20                  25                  30

Cys Thr Pro Ala Gly Thr Ile Ala Leu Ser Gln Lys Lys Lys Leu
        35                  40                  45

<210> 482
<211> 52
<212> PRT
<213> Homo sapiens

<400> 482
Ala His Pro Leu Pro Ala Pro Thr Glu Gly Lys Glu Lys Pro Leu Glu
1               5                   10                  15

Met Arg Val Thr Cys Glu Val Val Tyr Cys His Ser Ser Leu Phe Glu
            20                  25                  30

Leu Glu Thr Ile Val Ser Met Thr Gln Pro Thr Thr Leu Phe Leu His
        35                  40                  45

Ile Gln Phe Gln
    50

<210> 483
<211> 68
<212> PRT
<213> Homo sapiens

<400> 483
Thr Phe Cys Val Phe Lys His Glu Glu Lys Trp Ser His Glu Glu Arg
1               5                   10                  15

Gly Tyr Phe Leu Arg Arg Ile Ser Glu Gly Val His Ser Ile Ser Leu
            20                  25                  30

Pro Phe Ser Cys Phe Gly Phe Gly Ala Arg His Leu Tyr Trp Lys Ala
        35                  40                  45

Thr Glu His Thr Leu Cys Gln His Leu Leu Arg Glu Arg Lys Ser Pro
    50                  55                  60

Trp Lys Cys Val
65

<210> 484
<211> 64
<212> PRT
<213> Homo sapiens

<400> 484
Gln Ser Leu Leu Leu Phe Arg Asn Leu Gln Gly Leu Leu Phe Arg Lys
1               5                   10                  15

Cys His Gln Gln Ile Ile Ile Leu Ser Ala Met Leu Leu Ser Leu Ile
            20                  25                  30

Ser Ala Thr Arg Leu Asp Leu Tyr His Ser Trp Tyr Lys Phe Tyr Ser
        35                  40                  45

Cys Asn Ile Thr Thr Ile Ser Leu Leu Lys Arg Asp Gln Val Ser Lys
    50                  55                  60

<210> 485
<211> 22
<212> PRT
<213> Homo sapiens

<400> 485
Ile Arg His Glu Glu Ser Phe Asn Pro Leu Thr Cys Gly Phe Ser Leu
1               5                   10                  15

Phe Phe Ser Leu Phe Ser
            20

<210> 486
<211> 27
<212> PRT
<213> Homo sapiens

<400> 486
Met Glu Thr Leu Leu Leu Leu Leu Phe Phe Leu Ser Leu Leu Ile Phe
1               5                   10                  15

Arg Phe Arg Ile Leu Val Ser Gln Cys Ile Asn
            20                  25

<210> 487
<211> 65
<212> PRT
<213> Homo sapiens

<400> 487
Phe Leu Leu Thr Thr Val Leu Leu Phe Ser Ser Lys Val Arg Asp Pro
1               5                   10                  15

Arg Ala Asn Phe Asp Gln Ser Leu Arg Val Leu Lys His Ala Lys Lys
            20                  25                  30

Val Gln Pro Asp Val Ile Ser Lys Thr Ser Ile Met Leu Gly Leu Gly
        35                  40                  45

Glu Asn Asp Glu Gln Val Tyr Ala Thr Met Lys Gly Lys Glu Ile Glu
    50                  55                  60

Lys
65

<210> 488
<211> 23
<212> PRT
<213> Homo sapiens

<400> 488
Gln Gln Ser Cys Cys Phe Pro Val Arg Phe Val Ile Leu Gly Pro Ile
1               5                   10                  15

Leu Ile Ser Pro Tyr Val Tyr
            20

<210> 489
<211> 59
<212> PRT
<213> Homo sapiens

<400> 489
Val Trp Leu Leu Ser Ser Ile Leu Leu Arg Val Leu Trp Asn Arg Tyr
1               5                   10                  15

Thr Leu Gln Glu Leu Ser Phe Trp Leu Pro Trp Phe Ala Ser Arg Ala
            20                  25                  30

Thr Ser Leu Val Leu Gln His Gly Asp Asn Tyr Leu Leu Phe Leu Phe
        35                  40                  45

Cys Phe Val Cys Phe Val Leu Ala Met Pro Phe
    50                  55

<210> 490
<211> 26
<212> PRT
<213> Homo sapiens

<400> 490
Ile Arg His Glu Val Ser Met Ala Phe Val Phe His Leu Ala Gln Gly
1               5                   10                  15

Thr Leu Glu Pro Leu Tyr Ile Ala Gly Ala
            20                  25

<210> 491
<211> 40
<212> PRT
<213> Homo sapiens

<400> 491

<210> 492
<211> 26
<212> PRT
<213> Homo sapiens

<400> 492
His Glu Leu Thr Val Pro Ser Arg Met Gly Ser Lys Gly Lys Pro Tyr
1               5                   10                  15

Pro Cys Gly Phe Tyr Ser Ser Leu Ile Pro
            20                  25

<210> 493
<211> 103
<212> PRT
<213> Homo sapiens

<400> 493
Lys Cys Ile Tyr Pro Lys Pro Ala Arg Thr His His Cys Ser Ile Cys
1               5                   10                  15

Asn Arg Cys Val Leu Lys Met Asp His His Cys Pro Trp Leu Asn Asn
            20                  25                  30

Cys Val Gly His Tyr Asn His Arg Tyr Phe Phe Ser Phe Cys Phe Phe
        35                  40                  45

Met Thr Leu Gly Cys Val Tyr Cys Ser Tyr Gly Ser Trp Asp Leu Phe
    50                  55                  60

Arg Glu Ala Tyr Ala Ala Ile Glu Lys Met Lys Gln Leu Asp Lys Asn
65                  70                  75                  80

Lys Leu Gln Ala Val Ala Asn Gln Thr Tyr His Gln Thr Pro Pro Pro
                85                  90                  95

Thr Phe Ser Phe Arg Glu Arg
            100

<210> 494
<211> 38
<212> PRT
<213> Homo sapiens

<400> 494
Ala Arg Gly His Trp Asn Leu Ile Leu Ile Val Phe His Tyr Tyr Gln
1               5                   10                  15

Ala Ile Thr Thr Pro Pro Gly Tyr Pro Pro Gln Gly Arg Asn Asp Ile
            20                  25                  30

Ala Thr Val Ser Ile Cys
        35

<210> 495
<211> 33
<212> PRT
<213> Homo sapiens

<400> 495
Trp Gln Cys Glu Leu Asp Cys Val Ser His Asp Ser Ser Thr His Ser
1               5                   10                  15

Ala Pro Tyr Val Ile Ser Arg Ala Ser Lys Gly Ser Phe Ser Gln Asn
            20                  25                  30

Pro

<210> 496
<211> 83
<212> PRT
<213> Homo sapiens

<400> 496
Ser Lys Arg Ala Ser Gly Pro Ala Leu Gly Tyr His Ala Gly Gln Phe
1               5                   10                  15

Lys Asp Gln Pro Phe Tyr His Cys Arg Arg Lys Thr Gln Cys Gly Glu
            20                  25                  30

Ile Leu Gly Leu Thr Ser Leu Tyr Ser Gly Lys Gln Lys Phe Gln Pro
        35                  40                  45

Gln Thr Arg Gly Gln Ala Ala Ser Tyr Leu Pro Cys Pro Val Leu Thr
    50                  55                  60

Arg Thr Ser Ser Arg Ile Gln His Trp Ser Trp Pro Pro Pro Leu Leu
65                  70                  75                  80

Leu Ala Val

<210> 497
<211> 31
<212> PRT
<213> Homo sapiens

<400> 497
Glu Ser Leu Gln Leu Arg Leu Leu Gly Gln Leu Glu Gly Ile Pro Gly
1               5                   10                  15

Cys Gly Tyr Arg Lys Ala Leu Ala Tyr Ser Gly Ala Leu Thr Phe
            20                  25                  30

<210> 498
<211> 66
<212> PRT
<213> Homo sapiens

<400> 498
Ser Leu Ala Pro Trp Glu Trp Asn Glu Leu Gly Ala Pro Ser Leu Gly
1               5                   10                  15

Asp Cys Ser Leu Ser Leu Cys Asp Gly Ser Val Ser Trp Thr Val Ser
            20                  25                  30

Ala Thr Thr Arg Ala Leu Ile Leu Leu Pro Met Leu Phe Gln Gly Pro
        35                  40                  45

Pro Arg Ala Ala Phe Leu Arg Ile Leu Asp Gln Lys Glu Pro Val Gly
    50                  55                  60

Leu Pro
65

<210> 499
<211> 72
<212> PRT
<213> Homo sapiens

<400> 499
Thr Ala Thr Leu Asn Ser Phe Phe Gly Gly Trp Gly Leu Ala Leu Leu
1               5                   10                  15

Leu Arg Leu Glu Cys Ser Asp Thr Ile Met Asp His Cys Ser Leu Asp
            20                  25                  30

Leu Leu Gly Ser Ser Asn Pro Pro Ala Ser Ala Ser Gln Val Val Gly
        35                  40                  45

Thr Thr Gly Ala Arg His His Ala Gln Leu Ile Phe Cys Phe Phe Val
    50                  55                  60

Gln Thr Arg Ser His Ser Val Ala

65                  70

<210> 500
<211> 47
<212> PRT
<213> Homo sapiens

<400> 500
Met Asp His Cys Ser Leu Asp Leu Leu Gly Ser Ser Asn Pro Pro Ala
1               5                   10                  15

Ser Ala Ser Gln Val Val Gly Thr Thr Gly Ala Arg His His Ala Gln
            20                  25                  30

Leu Ile Phe Cys Phe Phe Val Gln Thr Arg Ser His Ser Val Ala
        35                  40                  45

<210> 501
<211> 14
<212> PRT
<213> Homo sapiens

<400> 501
Gly Val Leu Lys Gln Ser Ser His Leu Val Leu Ser Lys Gly
1               5                   10

<210> 502
<211> 21
<212> PRT
<213> Homo sapiens

<400> 502
Asp Tyr Ser Cys Glu Ser Leu Cys Pro Ala Leu Leu Ser Ile Ala Pro
1               5                   10                  15

Asp Ile Val Leu Asn
            20

<210> 503
<211> 27
<212> PRT
<213> Homo sapiens

<400> 503
Thr Thr Ile His Lys Thr Gln Leu Gly Ser Tyr Lys Ile Leu Trp Glu
1               5                   10                  15

Pro Lys Glu Gly Tyr His Asn Ser Thr Trp Ile
            20                  25

<210> 504
<211> 9
<212> PRT
<213> Homo sapiens

<400> 504
Ile Arg Glu Ile Phe Leu Arg Arg Pro
1               5

<210> 505
<211> 24
<212> PRT
<213> Homo sapiens

<400> 505
Leu Lys Phe Gln Lys Pro Gly Lys Ile Gln Met Arg Gly Gly Gly Arg
1               5                   10                  15

Val Phe Trp Tyr Lys Asn Cys Lys
            20

<210> 506
<211> 30
<212> PRT
<213> Homo sapiens

<400> 506
Asn Ser Ala Arg Val Thr Gln Lys Gly Glu Ser Val Gly Ser Val Gly
1               5                   10                  15

Cys Met Arg Ala Ile Ala Gly Phe Asp Asn Tyr Pro Leu Phe
            20                  25                  30

<210> 507
<211> 33
<212> PRT
<213> Homo sapiens

<400> 507
Gly Thr Ile Gly Ile Phe Trp Pro Leu Pro Val Ala Ile Leu Ser Ser
1               5                   10                  15

Gly Asp Tyr Leu Gln Thr Gln Ile His Arg Pro Leu Leu His Arg Gly
            20                  25                  30

Thr

<210> 508
<211> 20
<212> PRT
<213> Homo sapiens

<400> 508

<210> 509
<211> 46
<212> PRT
<213> Homo sapiens

<400> 509
Ser Tyr Phe Phe Val Tyr Asn Leu Ile Leu Lys Ile Ile Gln Gly Asp
1               5                   10                  15

His Ala Ser Ile Ile Leu Leu Ala Thr Ile Pro Ile Phe Gly Asp Ile
            20                  25                  30

Tyr Tyr Val Lys Gly Gln Leu Ala Ser Phe Gly Pro Tyr Leu
        35                  40                  45

<210> 510
<211> 21
<212> PRT
<213> Homo sapiens

<400> 510
Leu Phe Tyr His Leu Glu Ile Ile Ser Arg His Lys Ser Ile Ala His
1               5                   10                  15

Cys Ser Ile Glu Ala
            20

<210> 511
<211> 12
<212> PRT
<213> Homo sapiens

<400> 511
Cys Ser Cys His Cys Pro Ser Arg Ala Phe Ser Thr
1               5                   10

<210> 512
<211> 29
<212> PRT
<213> Homo sapiens

<400> 512

<210> 513
<211> 48
<212> PRT
<213> Homo sapiens

<400> 513

<210> 514
<211> 41
<212> PRT
<213> Homo sapiens

<400> 514
Asn Ser Ala Arg Asp Val Phe Phe Thr Gln Lys Ile Leu Tyr Ser Gln
1               5                   10                  15

Thr Cys Ile Phe Phe Pro Cys Leu Val Pro Phe Ser Phe Leu Phe Ser
            20                  25                  30

Phe Phe Phe Phe Leu Ser Phe Val Gly
        35                  40

<210> 515
<211> 56
<212> PRT
<213> Homo sapiens

<400> 515

<210> 516
<211> 39
<212> PRT
<213> Homo sapiens

<400> 516
Ile Gln Leu Leu Tyr Leu Lys Gly Ala Ala Met Lys Tyr Leu Ser Tyr
1               5                   10                  15

Val Ala Arg Leu Leu Phe Leu Lys Ala Leu Asp Leu Phe Ala Pro Lys
            20                  25                  30

Met Val Gln Ile Asp Ser Phe
        35

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein, a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1 % SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI-anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

This gene maps to chromosome 12, and therefore, may be used as a marker in linkage analysis for chromosome 12.

This gene is expressed primarily in cerebellum.

Therefore, polynucleotides and polypeptides of the invention are useful as reagents for differential identification of the tissue(s) or cell type(s) present in a biological sample and for diagnosis of diseases and conditions which include, but are not limited to, damage to the cerebellum or additional CNS tissues caused by insults such as trauma or ischemia. Similarly, polypeptides and antibodies directed to these polypeptides are useful in providing immunological probes for differential identification of the tissue(s) or cell type(s). For a number of disorders of the above tissues or cells, particularly of the central nervous system (CNS), expression of this gene at significantly higher or lower levels may be routinely detected in certain tissues (e.g., neural cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:150 as residues: Pro-7 to Cys-21, Leu-25 to Ser-30.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia. paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo - particularly those associated with neuromuscular junctions, sexually-linked disorders, or disorders of the cardiovascular system. Protein, as well as, antibodies directed against the protein may show utility as a tumor marker and/or immunotherapy targets for the above listed tumors and tissues. Many polynucleotide sequences, such as EST sequences, are publicly available and accessible through sequence databases. Some of these sequences are related to SEQ ID NO: 11 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1868 of SEQ ID NO:11, b is an integer of 15 to 1882, where both a and b correspond to the positions of nucleotide residues shown in SEQ ID NO:11, and where b is greater than or equal to a + 14.

### FEATURES OF PROTEIN ENCODED BY GENE NO: 2

This gene is expressed primarily in PHA stimulated T-cells.

Therefore, polynucleotides and polypeptides of the invention are useful as reagents for differential identification of the tissue(s) or cell type(s) present in a biological sample and for diagnosis of diseases and conditions which include, but are not limited to, autoimmune disease. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., immune cells and tissue, and cancerous and wounded tissues) or bodily fluids (e.g., lymph, serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in tonsils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer e.g., by boosting immune responses. Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tumors and tissues. Many polynucleotide sequences, such as EST sequences, are publicly available and accessible through sequence databases. Some of these sequences are related to SEQ ID NO: 12 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1576 of SEQ ID NO:12, b is an integer of 15 to 1590, where both a and b correspond to the positions of nucleotide residues shown in SEQ ID NO: 12, and where b is greater than or equal to a + 14.

### FEATURES OF PROTEIN ENCODED BY GENE NO: 3

The translation product of this gene was shown to have homology to the human retrovirus-related reverse transcriptase pseudogene (See Genbank Accession No. pirlA25313IGNHUL1). In specific embodiments, polypeptides of the invention comprise the amino acid sequence:
STHASVQKKDLTKFSAHSWLKKKKTFRKMIMEEIFLNLIKNIYKSPYSQCNT (SEQ ID NO:289), VRSEKGFDKIQCPFMVK (SEQ ID NO:290),
FSKPSSYKTYIPKINLHF YILLMNIWETIKIVPLNNCFTKMNYLGI (SEQ ID NO:291), KKETKLSLFANDMI (SEQ ID NO:292), and/or
SPLLFNILLEVLSSAVRKEKELK (SEQ ID NO:293). An additional embodiment is the polynucleotides encoding these polypeptides.

This gene is expressed primarily in PHA activated T cells.

Therefore, polynucleotides and polypeptides of the invention are useful as reagents for differential identification of the tissue(s) or cell type(s) present in a biological sample and for diagnosis of diseases and conditions which include, but are not limited to, inflammation and autoimmune diseases. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues (e.g., immune cells and tissue, and cancerous and wounded tissues) or bodily fluids (e.g., lymph, serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 152 as residues: Ile-14 to Thr-24.

The tissue distribution indicates that polynucleotides and polypeptides corresponding to this gene are useful for the diagnosis and treatment of a variety of immune system disorders. Expression of this gene product in tonsils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer e.g., by boosting immune responses. Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Alternatively, the homology to a reverse transcriptase human gene may implicate this gene as providing utility in the understanding of host-viral interactions, particularly those involving retroviruses and other integration-dependent viruses. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:13 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1359 of SEQ ID NO:13, b is an integer of 15 to 1373, where both a and b correspond to the positions of nucleotide residues shown in SEQ ID NO: 13, and where b is greater than or equal to a + 14.

### FEATURES OF PROTEIN ENCODED BY GENE NO: 4

The translation product of this gene shares sequence homology with npdcf-1 which is thought to be important in promoting the survival of bi-potential glial progenitor cells (See Genbank Accession No. gil456107). In specific embodiments, polypeptides of the invention comprise the amino acid sequence:
LRRPSTPLRRPWLHLQLPRISLGDQRLAQSAEMYHYQHQRQQMLSLERHKEPP KELDTALRMRRMRTETS RCTSARAWPRPGKWRCATICSTTPHCPRPC RPPAHRLH CHDLEADRRPLAPR (SEQ ID NO:294), RATQGAGHGSSDEENED GDFTVYECPGM APTGEMEVRNHLFD HAALSAPLPAPSSPLALP (SEQ ID NO:295), KAEYATAK ALATPAATPDLAWGPAPGTERGDVPLPAPTATDV VPGAA (SEQ ID NO:296), SAEM YHYQHQRQQML (SEQ ID NO:297), LERHKEPPKEL (SEQ ID NO:298), AKCPPGA HACGP (SEQ ID NO:299), PVHMSPLEP (SEQ ID NO:300), WCRLQREIRLTQ (SEQ ID NO:301), SSDEENEDGDFTVYECPG (SEQ ID NO:302), APTGEMEVRN (SEQ ID NO:303),
and/or CPGSLDCALK (SEQ ID NO:304). Additional embodiments is the polynucleotides encoding these polypeptides.

It has been discovered that this gene is expressed primarily in cerebellum and synovial sarcoma and to a lesser extent in several other cancer cell lines.

Therefore, polynucleotides and polypeptides of the invention are useful as reagents for differential identification of the tissue(s) or cell type(s) present in a biological sample and for diagnosis of diseases and conditions which include, but are not limited to, tumors characterized by cells of a relatively undifferentiated state, and/or neural tumors. Similarly, polypeptides and antibodies directed to these polypeptides are useful in providing immunological probes for differential identification of the tissue(s) or cell type(s). For a number of disorders of the above tissues or cells, particularly of the synovial fluid, prostate, breast and uterus, expression of this gene at significantly higher or lower levels may be routinely detected in certain tissues (e.g., neural cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 153 as residues: Pro-6 to Arg-11, Glu-52 to Gly-59.

The tissue distribution and homology to npdcf- 1 indicates that polynucleotides and polypeptides corresponding to this gene are useful for diagnosing and treating tumors that contain relatively undifferentiated cells. In addition,The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Alternatively, the expression of this gene product in synovium would suggest a role in the detection and treatment of disorders and conditions affecting the skeletal system, in particular the connective tissues (e.g., arthritis, trauma, tendonitis, chrondomalacia and inflammation). Protein, as well as, antibodies directed against the protein may show utility as a tumor marker and/or immunotherapy targets for the above listed tumors and tissues. Many polynucleotide sequences, such as EST sequences, are publicly available and accessible through sequence databases. Some of these sequences are related to SEQ ID NO:14 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1128 of SEQ ID NO: 14, b is an integer of 15 to 1142, where both a and b correspond to the positions of nucleotide residues shown in SEQ ID NO: 14, and where b is greater than or equal to a + 14.

### FEATURES OF PROTEIN ENCODED BY GENE NO: 5

This gene is expressed primarily in colon, pituitary, and to a lesser extent in fetal lung and fibrosarcoma.

Therefore, polynucleotides and polypeptides of the invention are useful as reagents for differential identification of the tissue(s) or cell type(s) present in a biological sample and for diagnosis of diseases and conditions which include, but are not limited to, endocrine disorders effecting the Gut/ pituitary/hypothalamic axis. Similarly, polypeptides and antibodies directed to these polypeptides are useful in providing immunological probes for differential identification of the tissue(s) or cell type(s). For a number of disorders of the above tissues or cells, particularly of the digestive system and regulation of feeding, expression of this gene at significantly higher or lower levels may be routinely detected in certain tissues (e.g., gastrointesinal tissue, endocrine tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:154 as residues: Asn-26 to Cys-32, Cys-100 to Leu-112, Cys-128 to Ser-135.

The tissue distribution indicates that polynucleotides and polypeptides corresponding to this gene are useful for treating disorders related to the intake and utilization of food since this gene is expressed in the digestive tract and a CNS site involved in regulation of weight homeostasis. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 15 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1020 of SEQ ID NO: 15. b is an integer of 15 to 1034, where both a and b correspond to the positions of nucleotide residues shown in SEQ ID NO:15, and where b is greater than or equal to a + 14.

### FEATURES OF PROTEIN ENCODED BY GENE NO: 6

The translation product of this gene shares sequence homology with Cortical granule lectin which is thought to be important in blocking polyspermy during fertilization of the egg (See Genbank Accession No. gnIlPIDle1 181610). Preferred polypeptides comprise the following amino acid sequence: RSCKEIKD (SEQ ID NO:305), GGGWTLVASVHEN (SEQ ID NO:306), ADYPEGDGNWANYNTFGSA (SEQ ID NO:307), ATSDDYKNPGYYDI (SEQ ID NO:308), CIGGGGYFPEA (SEQ ID NO:309), and/or EITEAAVLLFY (SEQ ID NO:310). Also preferred are the polynucleotides encoding these polypeptides.

This gene is expressed primarily in benign and metastatic colon, and to a lesser extent in HEL cells.

Therefore, polynucleotides and polypeptides of the invention are useful as reagents for differential identification of the tissue(s) or cell type(s) present in a biological sample and for diagnosis of diseases and conditions which include, but are not limited to, cancer, or inflammatory conditions of the colon. Similarly, polypeptides and antibodies directed to these polypeptides are useful in providing immunological probes for differential identification of the tissue(s) or cell type(s). For a number of disorders of the above tissues or cells, particularly of the digestive system, expression of this gene at significantly higher or lower levels may be routinely detected in certain tissues (e.g., gastrointestinal tissue, and proliferating, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:155 as residues: Arg-15 to Ser-33, Pro-35 to Cys-41.

The tissue distribution and homology to cortical granule lectins indicates that polynucleotides and polypeptides corresponding to this gene are useful for treating disorders of the colon. These may include diseases related to damage or chronic inflammation as well as tumors of the colon. The product may also be useful for the identification of colon cancer metastasis and, as a secreted protein, may have diagnostic and prognostic applications. Protein, as well as, antibodies directed against the protein may show utility as a tumor marker and/or immunotherapy targets for the above listed tumors and tissues. Many polynucleotide sequences, such as EST sequences, are publicly available and accessible through sequence databases. Some of these sequences are related to SEQ ID NO: 16 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1184 of SEQ ID NO: 16, b is an integer of 15 to 1198, where both a and b correspond to the positions of nucleotide residues shown in SEQ ID NO:16, and where b is greater than or equal to a + 14.

### FEATURES OF PROTEIN ENCODED BY GENE NO: 7

This gene is expressed primarily in eight week human embryos.

Therefore, polynucleotides and polypeptides of the invention are useful as reagents for differential identification of the tissue(s) or cell type(s) present in a biological sample and for diagnosis of diseases and conditions which include, but are not limited to, fetal and/or developmental abnormalities. Similarly, polypeptides and antibodies directed to these polypeptides are useful in providing immunological probes for differential identification of the tissue(s) or cell type(s). For a number of disorders of the above tissues or cells, particularly of the developing fetus, expression of this gene at significantly higher or lower levels may be routinely detected in certain tissues (e.g., developing and/or differentiating cells or tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for detecting embryonic abnormalities. Expression within embryonic tissue and other cellular sources marked by proliferating cells indicates that this protein may play a role in the regulation of cellular division. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 17 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1433 of SEQ ID NO:17, b is an integer of 15 to 1447, where both a and b correspond to the positions of nucleotide residues shown in SEQ ID NO: 17, and where b is greater than or equal to a + 14.

### FEATURES OF PROTEIN ENCODED BY GENE NO: 8

This gene is expressed primarily in endothelial cells. and to a lesser extent in lymph node, tonsils, heart and spinal cord.

Therefore, polynucleotides and polypeptides of the invention are useful as reagents for differential identification of the tissue(s) or cell type(s) present in a biological sample and for diagnosis of diseases and conditions which include, but are not limited to, vascular disease such as restenosis, including disorders of the integumentary system. Similarly, polypeptides and antibodies directed to these polypeptides are useful in providing immunological probes for differential identification of the tissue(s) or cell type(s). For a number of disorders of the above tissues or cells, particularly of the vascular system, expression of this gene at significantly higher or lower levels may be routinely detected in certain tissues amd cell types (e.g., integumentary tissue, lymph tissue and other cells and tissue of the immune system, cells and tissue of the nervous system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating diseases of the vasculature including problems associated with diabetes and restenosis following angioplasty. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 18 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1408 of SEQ ID NO:18, b is an integer of 15 to 1422, where both a and b correspond to the positions of nucleotide residues shown in SEQ ID NO: 18, and where b is greater than or equal to a + 14.

### FEATURES OF PROTEIN ENCODED BY GENE NO: 9

The translation product of this gene was shown to have homology to the Gcap1 gene product of Mus musculus, which is specifically expressed in cerebellum and appears to be developmentally regulated (See Genbank Accession No. gil862343).

This gene is expressed primarily in fetal lung and endothelial cells and to a lesser extent in astrocytes and fetal brain.

Therefore, polynucleotides and polypeptides of the invention are useful as reagents for differential identification of the tissue(s) or cell type(s) present in a biological sample and for diagnosis of diseases and conditions which include, but are not limited to, endothelial cell proliferation as in restenosis. Similarly, polypeptides and antibodies directed to these polypeptides are useful in providing immunological probes for differential identification of the tissue(s) or cell type(s). For a number of disorders of the above tissues or cells, particularly of the vascular system, expression of this gene at significantly higher or lower levels may be routinely detected in certain tissues (e.g., developmental tissue, neural tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal brain, in addition to the homology to a brain-specific regulatory protein indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tumors and tissues. Alternatively, The tissue distribution indicates that polynucleotides and polypeptides corresponding to this gene are useful for treating abnormal proliferation of endothelial cells such as occurrs upon injury to the lung or arteries. Many polynucleotide sequences, such as EST sequences, are publicly available and accessible through sequence databases. Some of these sequences are related to SEQ ID NO:19 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1093 of SEQ ID NO: 19, b is an integer of 15 to 1107, where both a and b correspond to the positions of nucleotide residues shown in SEQ ID NO:19, and where b is greater than or equal to a + 14.

### FEATURES OF PROTEIN ENCODED BY GENE NO: 10

This gene maps to chromosome 12, and therefore, may be used as a marker in linkage analysis for chromosome 12.

This gene is expressed primarily in infant brain and fetal tissues.

Therefore, polynucleotides and polypeptides of the invention are useful as reagents for differential identification of the tissue(s) or cell type(s) present in a biological sample and for diagnosis of diseases and conditions which include, but are not limited to, developmental abnormalities during gestation such as spina bifida. Similarly, polypeptides and antibodies directed to these polypeptides are useful in providing immunological probes for differential identification of the tissue(s) or cell type(s). For a number of disorders of the above tissues or cells, particularly of the Central nervous system, expression of this gene at significantly higher or lower levels may be routinely detected in certain tissues (e.g., developing and/or differentiating cells and tissue, and nervous tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tumors and tissues. Alternatively, The tissue distribution indicates that polynucleotides and polypeptides corresponding to this gene are useful for the diagnosis and treatment of cancer and other proliferative disorders. Expression within embryonic tissue and other cellular sources marked by proliferating cells indicates that this protein may play a role in the regulation of cellular division. Similarly, embryonic development also involves decisions involving cell differentiation and/or apoptosis in pattern formation. Thus this protein may also be involved in apoptosis or tissue differentiation and could again be useful in cancer therapy. Many polynucleotide sequences, such as EST sequences, are publicly available and accessible through sequence databases. Some of these sequences are related to SEQ ID NO:20 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1169 of SEQ ID NO:20, b is an integer of 15 to 1183, where both a and b correspond to the positions of nucleotide residues shown in SEQ ID NO:20, and where b is greater than or equal to a + 14.

### FEATURES OF PROTEIN ENCODED BY GENE NO: 11

This gene is expressed primarily in fetal kidney.

Therefore, polynucleotides and polypeptides of the invention are useful as reagents for differential identification of the tissue(s) or cell type(s) present in a biological sample and for diagnosis of diseases and conditions which include, but are not limited to, renal failure, tumors of the kidney, and/or developmental abnormalities associated with the kidney. Similarly, polypeptides and antibodies directed to these polypeptides are useful in providing immunological probes for differential identification of the tissue(s) or cell type(s). For a number of disorders of the above tissues or cells, particularly of the renal system, expression of this gene at significantly higher or lower levels may be routinely detected in certain tissues (e.g., urological tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:160 as residues: Gln-26 to Gln-34.

The tissue distribution indicates that polynucleotides and polypeptides corresponding to this gene are useful for the diagnosis and treatment of cancer and other proliferative disorders, particularly renal disorders. Expression within embryonic tissue and other cellular sources marked by proliferating cells indicates that this protein may play a role in the regulation of cellular division. Similarly, embryonic development also involves decisions involving cell differentiation and/or apoptosis in pattern formation. Thus this protein may also be involved in apoptosis or tissue differentiation and could again be useful in cancer therapy. Many polynucleotide sequences, such as EST sequences, are publicly available and accessible through sequence databases. Some of these sequences are related to SEQ ID NO:21 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1406 of SEQ ID NO:21, b is an integer of 15 to 1420, where both a and b correspond to the positions of nucleotide residues shown in SEQ ID NO:21, and where b is greater than or equal to a + 14.

### FEATURES OF PROTEIN ENCODED BY GENE NO: 12

This gene is expressed primarily in breast, fetal kidney, and T-cells.

Therefore, polynucleotides and polypeptides of the invention are useful as reagents for differential identification of the tissue(s) or cell type(s) present in a biological sample and for diagnosis of diseases and conditions:autoimmune disease, chronic inflammatory conditions, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., immune cells and tissue, and reproductive tissue, and cancerous and wounded tissues) or bodily fluids (e.g., breast milk, lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:161 as residues: His-2 to Lys-7, Ser-28 to Glu-35.

The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer e.g., by boosting immune responses. Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:22 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1561 of SEQ ID NO:22, b is an integer of 15 to 1575, where both a and b correspond to the positions of nucleotide residues shown in SEQ ID NO:22, and where b is greater than or equal to a + 14.

### FEATURES OF PROTEIN ENCODED BY GENE NO: 13

This gene is expressed primarily in the frontal cortex of the brain.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disorders, ischemia, Alzheimer's, Parkinson's. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., neural tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 162 as residues: Glu-31 to Gly-37.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tumors and tissues. Many polynucleotide sequences, such as EST sequences, are publicly available and accessible through sequence databases. Some of these sequences are related to SEQ ID NO:23 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 527 of SEQ ID NO:23, b is an integer of 15 to 541, where both a and b correspond to the positions of nucleotide residues shown in SEQ ID NO:23, and where b is greater than or equal to a + 14.

### FEATURES OF PROTEIN ENCODED BY GENE NO: 14

This gene maps to chromosome 1, and therefore, may be used as a marker in linkage analysis for chromosome 1.

This gene is expressed primarily in ovary and to a lesser extent in infant brain.

Therefore, polynucleotides and polypeptides of the invention are useful as reagents for differential identification of the tissue(s) or cell type(s) present in a biological sample and for diagnosis of diseases and conditions:cancers and other diseases of the female reproductive system including ovarian cysts and hormonal disorders. Similarly, polypeptides and antibodies directed to these polypeptides are useful in providing immunological probes for differential identification of the tissue(s) or cell type(s). For a number of disorders of the above tissues or cells, particularly of the female reproductive system, expression of this gene at significantly higher or lower levels may be routinely detected in certain tissues (e.g., reproductive tissue, neural cells and tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:163 as residues: Ser-32 to Glu-37.

The tissue distribution in ovarian tissue indicates that polynucleotides and polypeptides corresponding to this gene are useful for diagnosis and intervention of ovarian tumors, in addition to other tumors where expression has been indicated. Protein, as well as, antibodies directed against the protein may show utility as a tissue-specific marker and/or immunotherapy target for the above listed tissues. Alternatively, expression within the fetal brain indicates that the protein product of this gene is useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Many polynucleotide sequences, such as EST sequences, are publicly available and accessible through sequence databases. Some of these sequences are related to SEQ ID NO:24 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 819 of SEQ ID NO:24, b is an integer of 15 to 833, where both a and b correspond to the positions of nucleotide residues shown in SEQ ID NO:24, and where b is greater than or equal to a + 14.

### FEATURES OF PROTEIN ENCODED BY GENE NO: 15

The translation product of this gene was shown to have homology to the highly conserved ras gene which is known to be important in the regulation of cell growth, and thus has been shown to serve as an inducible oncogene in eukaryotic tissues (See Genbank Accession No. gblZ11804lDDRASX). When tested against PC12 (rat pheochromocytoma cells) cell lines, supernatants removed from cells containing this gene activated the EGR1 (early growth response gene 1) pathway. Thus, it is likely that this gene activates sensory neuron cells through the EGR1 signal transduction pathway. The EGR1 (early growth response gene 1) is a separate signal transduction pathway from Jaks-STAT, genes containing the EGR1 promoter are induced in various tissues and cell types upon activation, leading the cells to undergo differentiation and proliferation.

This gene is expressed primarily in T-cells.

Therefore, polynucleotides and polypeptides of the invention are useful as reagents for differential identification of the tissue(s) or cell type(s) present in a biological sample and for diagnosis of diseases and conditions which include, but are not limited to, diseases involving immune regulation including autoimmune diseases such as rheumatoid arthritis, lupus, and leukemia. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues (e.g., immune cells and tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:164 as residues: Ala-28 to His-41, Pro-43 to Gln-64.

The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer - particularly considering the homology to a conserved ras gene, combined with the detected EGR1 biological activity. Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:25 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1541 of SEQ ID NO:25, b is an integer of 15 to 1555, where both a and b correspond to the positions of nucleotide residues shown in SEQ ID NO:25, and where b is greater than or equal to a + 14.

### FEATURES OF PROTEIN ENCODED BY GENE NO: 16

This gene maps to chromosome 13, and therefore, may be used as a marker in linkage analysis for chromosome 13.

This gene is expressed primarily in kidney cortex.

Therefore, polynucleotides and polypeptides of the invention are useful as reagents for differential identification of the tissue(s) or cell type(s) present in a biological sample and for diagnosis of diseases and conditions which include, but are not limited to, diseases of the kidney including cancer and renal dysfunction. Similarly, polypeptides and antibodies directed to these polypeptides are useful in providing immunological probes for differential identification of the tissue(s) or cell type(s). For a number of disorders of the above tissues or cells, particularly of the renal system, expression of this gene at significantly higher or lower levels may be routinely detected in certain tissues (e.g., urogenital tissue, and cancerous and wounded tissues) or bodily fluids (e.g., urine, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/diagnosis of diseases of the kidney including kidney failure. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:26 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1529 of SEQ ID NO:26, b is an integer of 15 to 1543, where both a and b correspond to the positions of nucleotide residues shown in SEQ ID NO:26, and where b is greater than or equal to a + 14.

### FEATURES OF PROTEIN ENCODED BY GENE NO: 17

This gene is expressed primarily in T-cell lymphoma and to a lesser extent in bone marrow stromal cells.

Therefore, polynucleotides and polypeptides of the invention are useful as reagents for differential identification of the tissue(s) or cell type(s) present in a biological sample and for diagnosis of diseases and conditions which include, but are not limited to, cancer including lymphomas and leukemias. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., immune cells and tissue, and bone marrow, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Expression in bone marrow cells suggest that the protein product of this gene is useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Many polynucleotide sequences, such as EST sequences, are publicly available and accessible through sequence databases. Some of these sequences are related to SEQ ID NO:27 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1248 of SEQ ID NO:27, b is an integer of 15 to 1262, where both a and b correspond to the positions of nucleotide residues shown in SEQ ID NO:27, and where b is greater than or equal to a + 14.

### FEATURES OF PROTEIN ENCODED BY GENE NO: 18

This gene is expressed primarily in a medulloblastoma.

Therefore, polynucleotides and polypeptides of the invention are useful as reagents for differential identification of the tissue(s) or cell type(s) present in a biological sample and for diagnosis of diseases and conditions which include, but are not limited to, disorders of the central nervous system including cancers. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., neural cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 167 as residues: Phe-22 to Leu-28.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:28 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 739 of SEQ ID NO:28, b is an integer of 15 to 753, where both a and b correspond to the positions of nucleotide residues shown in SEQ ID NO:28, and where b is greater than or equal to a + 14.

### FEATURES OF PROTEIN ENCODED BY GENE NO: 19

The translation product of this gene was shown to have homology to the mammalian notch I protein which has been shown to be important in the regulation of cell-fate during pattern formation and development (See Genbank Accession No. gil57635). One embodiment of this gene comprises polypeptides of the following amino acid sequence: KHEXHQVSDGALRCFASLADRFTRRGVDPAPLAKHGLTEE LLSRMAAAGGTVSGPSSACKPXRSTTGAPSTTADSKLSNQVSTTVSLLSTLCRG SPVVTHDLLRSELPDSIESALQGDERCVLDTMRLVDFLLVLLFEGRKALPKSSA GSTG RIPGLRRLDSSGERSHRQLIDCIRSKDTDALIDAIDTGAFEVN FMDDVGQTLLNWA SAFGTQEMVEFLCERGADVNRGQRSSSLHYAACF GRPQVAKTLLRHGANPDLRDEDGKTPLDKARERGHSEVVAILQSPGDW MCPVNKGDDK (SEQ ID NO:31 1), PLDKARERGHSEWAIL (SEQ ID NO:312), and/or AKTLLRHGANPDLRD (SEQ ID NO:313). Additional embodiments are directed to polynucleotides encoding these polypeptides.

This gene is expressed primarily in endothelial cells.

Therefore, polynucleotides and polypeptides of the invention are useful as reagents for differential identification of the tissue(s) or cell type(s) present in a biological sample and for diagnosis of diseases and conditions which include, but are not limited to, diseases involving angiogenic abnormalities including diabetic retinopathy, macular degeneration, and other diseases including arterioscerosis and cancer. Similarly, polypeptides and antibodies directed to these polypeptides are useful in providing immunological probes for differential identification of the tissue(s) or cell type(s). For a number of disorders of the above tissues or cells, particularly of the vascular system, expression of this gene at significantly higher or lower levels may be routinely detected in certain tissues (e.g., endothelial cells, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 168 as residues: Asp-17 to Phe-23.

The tissue distribution indicates that polynucleotides and polypeptides corresponding to this gene are useful for treating diseases where an increase or decrease in angiogenesis is indicated and as a factor in the wound healing process. Protein, as well as, antibodies directed against the protein may show utility as a tumor marker and/or immunotherapy targets for the above listed tissues. Alternatively, considering the homology to the Notch I protein, this gene may show utility in the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:29 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1607 of SEQ ID NO:29, b is an integer of 15 to 1621, where both a and b correspond to the positions of nucleotide residues shown in SEQ ID NO:29, and where b is greater than or equal to a + 14.

### FEATURES OF PROTEIN ENCODED BY GENE NO: 20

This gene is expressed primarily in meningioma tissues.

Therefore, polynucleotides and polypeptides of the invention are useful as reagents for differential identification of the tissue(s) or cell type(s) present in a biological sample and for diagnosis of diseases and conditions which include, but are not limited to, cancers of the central nervous system and endothelium. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., neural cells and tissue, and endothelial, cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:30 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 907 of SEQ ID NO:30, b is an integer of 15 to 921, where both a and b correspond to the positions of nucleotide residues shown in SEQ ID NO:30, and where b is greater than or equal to a + 14.

### FEATURES OF PROTEIN ENCODED BY GENE NO: 21

The translation product of this gene was shown to have homology to the retinoic acid receptor gamma-2 which is thought to be important in development, and may be a key determinant for human breast cancer during aberrant activation (See Genbank Accession No. AA176435).

This gene is expressed primarily in ovary and to a lesser extent in meningioma.

Therefore, polynucleotides and polypeptides of the invention are useful as reagents for differential identification of the tissue(s) or cell type(s) present in a biological sample and for diagnosis of diseases and conditions which include, but are not limited to, ovarian cancer, as well as, other cancers of the female reproductive system, and endothelial tissue in general. Similarly, polypeptides and antibodies directed to these polypeptides are useful in providing immunological probes for differential identification of the tissue(s) or cell type(s). For a number of disorders of the above tissues or cells, particularly of the female reproductive system, expression of this gene at significantly higher or lower levels may be routinely detected in certain tissues (e.g., reproductive tissue, and neural tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:170 as residues: Leu-8 to Gln-18, Thr-26 to Lys-33, Met-39 to Cys-46, Ala-62 to Pro-69, Pro-83 to Glu-90.

The tissue distribution in ovary tissues indicates that polynucleotides and polypeptides corresponding to this gene are useful for diagnosis and intervention of tumors within these tissues, in addition to other tumors where expression has been indicated. Protein, as well as, antibodies directed against the protein may show utility as a tissue-specific marker and/or immunotherapy target for the above listed tumors and tissues. Many polynucleotide sequences, such as EST sequences, are publicly available and accessible through sequence databases. Some of these sequences are related to SEQ ID NO:31 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2081 of SEQ ID NO:31, b is an integer of 15 to 2095, where both a and b correspond to the positions of nucleotide residues shown in SEQ ID NO:31, and where b is greater than or equal to a + 14.

### FEATURES OF PROTEIN ENCODED BY GENE NO: 22

This gene is expressed primarily in the spongy tissue from Alzheimer's brain.

Therefore, polynucleotides and polypeptides of the invention are useful as reagents for differential identification of the tissue(s) or cell type(s) present in a biological sample and for diagnosis of diseases and conditions which include, but are not limited to, Alzheimer's disease and other neurodegenerative diseases. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., neural tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 171 as residues: Ser-31 to Ala-37, Ala-50 to Tyr-55, Phe-63 to Arg-68, His-83 to Pro-89.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:32 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1824 of SEQ ID NO:32, b is an integer of 15 to 1838, where both a and b correspond to the positions of nucleotide residues shown in SEQ ID NO:32, and where b is greater than or equal to a + 14.

### FEATURES OF PROTEIN ENCODED BY GENE NO: 23

This gene is expressed primarily in bone marrow cells.

Therefore, polynucleotides and polypeptides of the invention are useful as reagents for differential identification of the tissue(s) or cell type(s) present in a biological sample and for diagnosis of diseases and conditions which include, but are not limited to, hematological disorders. Similarly, polypeptides and antibodies directed to these polypeptides are useful in providing immunological probes for differential identification of the tissue(s) or cell type(s). For a number of disorders of the above tissues or cells, particularly of the hematological and immune systems, expression of this gene at significantly higher or lower levels may be routinely detected in certain tissues (e.g., immune cells and tissue, haematopoeitic cells and tissue, and cancerous and wounded tissues) or bodily fluids (e.g., lymph, serum, plasma, urine, synovial fluid and spinal fluid) or another tissue or cell sample taken from an individual having such a disorder, relative to the standard-gene expression level, i.e., the expression level in healthy tissue or bodily fluid from an individual not having the disorder. Preferred epitopes include those comprising a sequence shown in SEQ ID NO:172 as residues: Glu-22 to Ser-33, Leu-47 to Ser-55, Thr-87 to Arg-104.

The tissue distribution indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:33 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 768 of SEQ ID NO:33, b is an integer of 15 to 782, where both a and b correspond to the positions of nucleotide residues shown in SEQ ID NO:33, and where b is greater than or equal to a + 14.

### FEATURES OF PROTEIN ENCODED BY GENE NO: 24

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, including inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., immune cells and tissue, haematopoeitic cells and tissue, blood cells, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder: Preferred epitopes include those comprising a sequence shown in SEQ ID NO: 173 as residues: Gln-36 to Lys-41.

The tissue distribution indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:34 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1546 of SEQ ID NO:34, b is an integer of 15 to 1560, where both a and b correspond to the positions of nucleotide residues shown in SEQ ID NO:34, and where b is greater than or equal to a + 14.

### FEATURES OF PROTEIN ENCODED BY GENE NO: 25

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, including inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., immune cells and tissue, haematopoeitic cells and tissue, blood cells, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:35 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1078 of SEQ ID NO:35, b is an integer of 15 to 1092, where both a and b correspond to the positions of nucleotide residues shown in SEQ ID NO:35, and where b is greater than or equal to a + 14.

### FEATURES OF PROTEIN ENCODED BY GENE NO: 26

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, including inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., immune cells and tissue, haematopoeitic cells and tissue, blood cells, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 175 as residues: Lys-9 to Leu-16, Ser-33 to Met-43.

The tissue distribution indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:36 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1139 of SEQ ID NO:36, b is an integer of 15 to 1153, where both a and b correspond to the positions of nucleotide residues shown in SEQ ID NO:36, and where b is greater than or equal to a + 14.

### FEATURES OF PROTEIN ENCODED BY GENE NO: 27

The translation product of this gene was shown to have homology to the intrinsic factor-B 12 receptor precursor of Rattus norvegicus which is thought to be important in development (See Genbank Accession No. gil2961490 (AF022247)). One embodiment of this gene comprises polypeptides of the following amino acid sequence: DCNRDYHKAFGNLRSPGWPDNYDNDXDCXVTLTAPQNHHSGIVENAETISWR (SEQ ID NO:314), FGNLRSPGWPDNYDN (SEQ ID NO:315), and/or APQNHXLK CRNDFLEV (SEQ ID NO:316). Additional embodiments are directed to polynucleotides encoding these polypeptides:

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, including inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely defected in certain tissues (e.g., immune cells and tissue, haematopoetic cells and tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:37 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 971 of SEQ ID NO:37, b is an integer of 15 to 985, where both a and b correspond to the positions of nucleotide residues shown in SEQ ID NO:37, and where b is greater than or equal to a + 14.

### FEATURES OF PROTEIN ENCODED BY GENE NO: 28

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune and/or haematological disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g.. immune cells and tissue, haematopoeitic cells and tissue, blood cells, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:177 as residues: Pro-55 to Ser-66.

The tissue distribution indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:38 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1108 of SEQ ID NO:38, b is an integer of 15 to 1122, where both a and b correspond to the positions of nucleotide residues shown in SEQ ID NO:38, and where b is greater than or equal to a + 14.

### FEATURES OF PROTEIN ENCODED BY GENE NO: 29

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune and haematological disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., immune cells and tissue, blood cells, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:39 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 584 of SEQ ID NO:39, b is an integer of 15 to 598, where both a and b correspond to the positions of nucleotide residues shown in SEQ ID NO:39, and where b is greater than or equal to a + 14.

### FEATURES OF PROTEIN ENCODED BY GENE NO: 30

This gene is expressed primarily in ovarian cancer. This gene also maps to chromosome 7, and therefore can be used as a marker in linkage analysis for chromosome 7.

Therefore, polynucleotides and polypeptides of the invention are useful as reagents for differential identification of the tissue(s) or cell type(s) present in a biological sample and for diagnosis of diseases and conditions which include, but are not limited to, ovarian cancer. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of ovarian cancer. Many polynucleotide sequences, such as EST sequences, are publicly available and accessible through sequence databases. Some of these sequences are related to SEQ ID NO:40 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1115 of SEQ ID NO:40, b is an integer of 15 to 1129, where both a and b correspond to the positions of nucleotide residues shown in SEQ ID NO:40, and where b is greater than or equal to a + 14.

### FEATURES OF PROTEIN ENCODED BY GENE NO: 31

This gene is expressed primarily in ovarian cancer.

Therefore, polynucleotides and polypeptides of the invention are useful as reagents for differential identification of the tissue(s) or cell type(s) present in a biological sample and for diagnosis of diseases and conditions which include, but are not limited to, ovarian cancer. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of ovarian cancer. Many polynucleotide sequences, such as EST sequences, are publicly available and accessible through sequence databases. Some of these sequences are related to SEQ ID NO:41 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1144 of SEQ ID NO:41, b is an integer of 15 to 1158, where both a and b correspond to the positions of nucleotide residues shown in SEQ ID NO:41, and where b is greater than or equal to a + 14.

### FEATURES OF PROTEIN ENCODED BY GENE NO: 32

This gene is expressed primarily in ovarian tumor.

Therefore, polynucleotides and polypeptides of the invention are useful as reagents for differential identification of the tissue(s) or cell type(s) present in a biological sample and for diagnosis of diseases and conditions which include, but are not limited to, ovarian cancer. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels maybe routinely detected in certain tissues (e.g., reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of ovarian cancer. Many polynucleotide sequences, such as EST sequences, are publicly available and accessible through sequence databases. Some of these sequences are related to SEQ ID NO:42 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1753 of SEQ ID NO:42, b is an integer of 15 to 1767, where both a and b correspond to the positions of nucleofide residues shown in SEQ ID NO:42, and where b is greater than or equal to a + 14.

### FEATURES OF PROTEIN ENCODED BY GENE NO: 33

The translation product of this gene shares sequence homology with uroplakin III which is thought to be important in urothelial differentiation. (See Accession No. d10226610) Preferred polypeptide fragments comprise the amino acid sequence: ASIDTWPGRRSGGMIVITSI (SEQ ID NO:317) and/or GSPQAETRWSDPIALHQ GKSPASIDTWPGRRSGGMIVITSI (SEQ ID NO:318).

This gene is expressed primarily in ovarian tumor.

Therefore, polynucleotides and polypeptides of the invention are useful as reagents for differential identification of the tissue(s) or cell type(s) present in a biological sample and for diagnosis of diseases and conditions which include, but are not limited to, ovarian cancer. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to uroplakin III indicates that polynucleotides and polypeptides corresponding to this gene are useful for diagnosis and treatment of ovarian cancer.Many polynucleotide sequences, such as EST sequences, are publicly available and accessible through sequence databases. Some of these sequences are related to SEQ ID NO:43 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 903 of SEQ ID NO:43, b is an integer of 15 to 917, where both a and b correspond to the positions of nucleotide residues shown in SEQ ID NO:43, and where b is greater than or equal to a + 14.

### FEATURES OF PROTEIN ENCODED BY GENE NO: 34

The translation product of this gene shares sequence homology with estrogen-responsive finger protein. which is thought to be important in uterine implantation. (See Accession No. 1088467; and J. Biol. Chem. 270 (41), 24406-24413 (1995), herein incorporated by reference in its entirety.) Preferred polypeptide fragments comprise the amino acid sequence:
VXDITFDPDTAHKYLRLQEENRKVTNTTPWEHPYPDLPSRFLH (SEQ ID NO:319); LYLHRYYFEVEIFGAGTYV (SEQ ID NO:320); SCISGNNFSW SLQWNGKEFTAW (SEQ ID NO:321); TPLKAGPFWSSGSILTS (SEQ ID NO:322); SVSEVKAVAEMQFGELLAAVRKAQ KEQAAL (SEQ ID NO:323); EKSKQELETMA AISNTVQFLEEYCKFKNTEDITFPSVYIGLKD (SEQ ID NO:324); LENYKKKLQEF SKEEEYDIRTQVSAXVQR (SEQ ID NO:325); and/or GVYIDFPGGILSFYGVEYDS MTLVHKFACKFSEPVYAA (SEQ ID NO:326). Also preferred are polynucleotide fragments encoding these polypeptide fragments.

This gene is expressed primarily in ovarian cancer.

Therefore, polynucleotides and polypeptides of the invention are useful as reagents for differential identification of the tissue(s) or cell type(s) present in a biological sample and for diagnosis of diseases and conditions which include, but are not limited to, ovarian cancer and other disorders of the reproductive system. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to estrogen-responsive finger protein indicates that polynucleotides and polypeptides corresponding to this gene are useful for diagnosis and treatment of ovarian cancer and other disorders of the reproductive system. Many polynucleotide sequences, such as EST sequences, are publicly available and accessible through sequence databases. Some of these sequences are related to SEQ ID NO:44 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1973 of SEQ ID NO:44, b is an integer of 15 to 1987, where both a and b correspond to the positions of nucleotide residues shown in SEQ ID NO:44, and where b is greater than or equal to a + 14.

### FEATURES OF PROTEIN ENCODED BY GENE NO: 35

This gene shows sequence homology to a Caenorhabditis elegans gene, called D1054.3. (See Accession No. gnIlPIDle348554.) Preferred polypeptide fragments comprise the amino acid sequence:
SKIKYDWYQTESQVVTTLMIKNVQKNDVNVEFSEKELSALVKLPSGEDYNLKL ELLHPIIPEQSTFKVLSTKIEIKLKKPEAVRWEKLEGQGDVPTPKQFVADVKNLY PSSSPYTRNWDKLVGEIKEEEKNEKLEGDAALNRLFQQIYSDGSDEVKR AMNKSFMESGGTVLSTNWSDVGKRKVEINPPDDMEWKKY (SEQ ID NO:327); GDAALN RLFQQIYSDGSDEVKRAMNKSFMESGGTVLSTN (SEQ ID NO:328); and/or DWYQTESQ VVITLMIKNVQKNDV (SEQ ID NO:329). Also preferred are polynucleotide fragments encoding these polypeptide fragments.

This gene is expressed primarily in osteoclastoma.

Therefore, polynucleotides and polypeptides of the invention are useful as reagents for differential identification of the tissue(s) or cell type(s) present in a biological sample and for diagnosis of diseases and conditions which include, but are not limited to, osteoclastoma and other forms of cancer. Similarly, polypeptides and antibodies directed to these polypeptides are useful in providing immunological probes for differential identification of the tissue(s) or cell type(s). For a number of disorders of the above tissues or cells, particularly of the bone system, expression of this gene at significantly higher or lower levels may be routinely detected in certain tissues (e.g., bone cells, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of osteoclastoma and other forms of cancers. Many polynucleotide sequences, such as EST sequences, are publicly available and accessible through sequence databases. Some of these sequences are related to SEQ ID NO:45 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2039 of SEQ ID NO:45, b is an integer of 15 to 2053, where both a and b correspond to the positions of nucleotide residues shown in SEQ ID NO:45, and where b is greater than or equal to a + 14.

### FEATURES OF PROTEIN ENCODED BY GENE NO: 36

This gene is expressed primarily in Human Placenta.

Therefore, polynucleotides and polypeptides of the invention are useful as reagents for differential identification of the tissue(s) or cell type(s) present in a biological sample and for diagnosis of diseases and conditions which include, but are not limited to, diseases of embryonic and reproductive systems. Similarly, polypeptides and antibodies directed to these polypeptides are useful in providing immunological probes for differential identification of the tissue(s) or cell type(s). For a number of disorders of the above tissues or cells, particularly of the embryonic and reproductive systems, expression of this gene at significantly higher or lower levels may be routinely detected in certain tissues (e.g., reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of the disorders of embryonic and reproductive systems. Many polynucleotide sequences, such as EST sequences, are publicly available and accessible through sequence databases. Some of these sequences are related to SEQ ID NO:46 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1258 of SEQ ID NO:46, b is an integer of 15 to 1272, where both a and b correspond to the positions of nucleotide residues shown in SEQ ID NO:46, and where b is greater than or equal to a + 14.

### FEATURES OF PROTEIN ENCODED BY GENE NO: 37

This gene is expressed primarily in Anergic T-cell.

Therefore, polynucleotides and polypeptides of the invention are useful as reagents for differential identification of the tissue(s) or cell type(s) present in a biological sample and for diagnosis of diseases and conditions which include, but are not limited to, blood cell disorders, especially those involved with T-cells. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cells and tissue of the immune system, blood cells, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of T cell related disorders. Many polynucleotide sequences, such as EST sequences, are publicly available and accessible through sequence databases. Some of these sequences are related to SEQ ID NO:47 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 759 of SEQ ID NO:47, b is an integer of 15 to 773, where both a and b correspond to the positions of nucleotide residues shown in SEQ ID NO:47, and where b is greater than or equal to a + 14.

### FEATURES OF PROTEIN ENCODED BY GENE NO: 38

The translation product of this gene shares sequence homology with a murine bone-related sulphatase. (See Accession No. 3046314.)

This gene is expressed primarily in retina.

Therefore, polynucleotides and polypeptides of the invention are useful as reagents for differential identification of the tissue(s) or cell type(s) present in a biological sample and for diagnosis of diseases and conditions which include, but are not limited to, eye dieases. Similarly, polypeptides and antibodies directed to these polypeptides are useful in providing immunological probes for differential identification of the tissue(s) or cell type(s). For a number of disorders of the above tissues or cells, particularly of the eye, expression of this gene at significantly higher or lower levels may be routinely detected in certain tissues (e.g., bone cells and tissue, retinal cellsand other cells and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:187 as residues: Ala-21 to Arg-27, Asp-40 to Arg-45, Glu-97 to Thr-110, Glu-117 to Lys-128, Arg-175 to Lys-182, Pro-207 to Gly-220, Val-253 to Ile-272.

The tissue distribution and homology to sulphatases indicates that polynucleotides and polypeptides corresponding to this gene are useful for diagnosis and treatment of eye disorders. Moreover, this gene may be involved in bone-related disorders, osteoporosis, Paget's disease, osteomalacia, and diagnosis. Many polynucleotide sequences, such as EST sequences, are publicly available and accessible through sequence databases. Some of these sequences are related to SEQ ID NO:48 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2105 of SEQ ID NO:48, b is an integer of 15 to 2119, where both a and b correspond to the positions of nucleotide residues shown in SEQ ID NO:48, and where b is greater than or equal to a + 14.

### FEATURES OF PROTEIN ENCODED BY GENE NO: 39

This gene is expressed primarily in human stomach cancers.

Therefore, polynucleotides and polypeptides of the invention are useful as reagents for differential identification of the tissue(s) or cell type(s) present in a biological sample and for diagnosis of diseases and conditions which include, but are not limited to, cancer, particularly of the gastrointestinal system. Similarly, polypeptides and antibodies directed to these polypeptides are useful in providing immunological probes for differential identification of the tissue(s) or cell type(s). For a number of disorders of the above tissues or cells, particularly of the cancer, expression of this gene at significantly higher or lower levels may be routinely detected in certain tissues (e.g., gastrointestinal tissue, endothelial cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in tumors of the stomach indicates that polynucleotides and polypeptides corresponding to this gene are useful for diagnosis and intervention of these tumors, in addition to other tumors where expression has been indicated. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:49 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1174 of SEQ ID NO:49, b is an integer of 15 to 1188, where both a and b correspond to the positions of nucleotide residues shown in SEQ ID NO:49, and where b is greater than or equal to a + 14.

### FEATURES OF PROTEIN ENCODED BY GENE NO: 40

This gene is expressed primarily in human synovial membrane.

Therefore, polynucleotides and polypeptides of the invention are useful as reagents for differential identification of the tissue(s) or cell type(s) present in a biological sample and for diagnosis of diseases and conditions which include, but are not limited to, diseases of synovial membrane and musculoskeletal disorders. Similarly, polypeptides and antibodies directed to these polypeptides are useful in providing immunological probes for differential identification of the tissue(s) or cell type(s). For a number of disorders of the above tissues or cells, particularly of the synovial membrane system, expression of this gene at significantly higher or lower levels may be routinely detected in certain tissues (e.g., synovial cells and tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:189 as residues: Pro-10 to Ser-20.

The tissue distribution within synovial tissue indicates the product of this gene may a role in the detection and treatment of disorders and conditions affecting the skeletal system, in particular the connective tissues (e.g., arthritis, trauma, tendonitis, chrondomalacia and inflammation).Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:50 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 464 of SEQ ID NO:50, b is an integer of 15 to 478, where both a and b correspond to the positions of nucleotide residues shown in SEQ ID NO:50, and where b is greater than or equal to a + 14.

### FEATURES OF PROTEIN ENCODED BY GENE NO: 41

The translation product of this gene shares sequence homology with adipose specific collagen-like factor as well as the human adipocyte complement related protein Acrp30, the latter of which is known to be important in energy balance and homeostasis involving food intake, particularly in carbohydrate and lipid catabolism/anabolism (See Genbank Accession Nos.gnIlPIDld1008822 and W09108, respectively). In specific embodiments, polypeptides of the invention comprise the amino acid sequence:
XLWDPGLPGVCRCGSIVLKSAFSVGITTSYPEXRLPIIFNKVLLPRGXALQPCH R GSSSVLSQGIYYFSYDITLANKHLAIGLVHNGQYRIKTFDANTGNHDVASG STVIYLQPEDEVWLEIFFTDQNGLFSDPGWADSLFSGFLLYVDTDYLDSISE DDEL (SEQ ID NO:330), GSIVLKSAFSVGITT (SEQ ID NO:331), GIYYFSYDITLA NK (SEQ ID NO:332), DSLFSGFLLYVDT (SEQ ID NO:333), and/or NHDVASGST VIYL (SEQ ID NO:334). Additional embodiments are directed to the polynucleotides encoding these polypeptides.

This gene is expressed primarily in human schwanoma.

Therefore, polynucleotides and polypeptides of the invention are useful as reagents for differential identification of the tissue(s) or cell type(s) present in a biological sample and for diagnosis of diseases and conditions which include, but are not limited to, neurofibroma, and other neural disorders. Similarly, polypeptides and antibodies directed to these polypeptides are useful in providing immunological probes for differential identification of the tissue(s) or cell type(s). For a number of disorders of the above tissues or cells, particularly of the diseases relating to peripheral or sympathetic nervous system, expression of this gene at significantly higher or lower levels may be routinely detected in certain tissues and locations (e.g., neural cells and tissue, integumentary tissue, extracellular matrix, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:190 as residues: Gly-16 to Pro-30, Pro-42 to Gly-56, Gly-62 to Gly-77, Glu-93 to Gly-104, Glu-109 to Glu-114, Pro-121 to Asp-126.

The tissue distribution combined with the homology to a conserved human adipose specific collagen-like factor as well as to the human adipocyte complement related protein Acrp30, indicates that polynucleotides and polypeptides corresponding to this gene are useful for the detection/treatment of neurodegenerative disease states and behavioural disorders particularly neuroschwannoma, and including Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Alternatively, considering the homology to a conserved adipose specific collagen-like factor, would suggest that this protein may also be important in the diagnosis or treatment of various autoimmune disorders such as rheumatoid arthritis, lupus, scleroderma, and dermatomyositis as well as dwarfism, spinal deformation, and specific joint abnormalities as well as chondrodysplasias ie. spondyloepiphyseal dysplasia congenita, familial osteoarthritis, Atelosteogenesis type II, metaphyseal chondrodysplasia type Schmid. Many polynucleotide sequences, such as EST sequences, are publicly available and accessible through sequence databases. Some of these sequences are related to SEQ ID NO:51 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1319 of SEQ ID NO:51, b is an integer of 15 to 1333, where both a and b correspond to the positions of nucleotide residues shown in SEQ ID NO:51, and where b is greater than or equal to a + 14.

### FEATURES OF PROTEIN ENCODED BY GENE NO: 42

This gene is expressed primarily in human activated T-Cells.

Therefore, polynucleotides and polypeptides of the invention are useful as reagents for differential identification of the tissue(s) or cell type(s) present in a biological sample and for diagnosis of diseases and conditions which include, but are not limited to, immunodeficiency, and other immune disorders. Similarly, polypeptides and antibodies directed to these polypeptides are useful in providing immunological probes for differential identification of the tissue(s) or cell type(s). For a number of disorders of the above tissues or cells, particularly of the disorders of the immune system, expression of this gene at significantly higher or lower levels may be routinely detected in certain tissues (e.g., immune cells and tissue, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:52 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1241 of SEQ ID NO:52, b is an integer of 15 to 1255, where both a and b correspond to the positions of nucleotide residues shown in SEQ ID NO:52, and where b is greater than or equal to a + 14.

### FEATURES OF PROTEIN ENCODED BY GENE NO: 43

This gene is expressed primarily in human activated T-Cells.

Therefore, polynucleotides and polypeptides of the invention are useful as reagents for differential identification of the tissue(s) or cell type(s) present in a biological sample and for diagnosis of diseases and conditions which include, but are not limited to, immunodeficiencies and other immune disorders. Similarly, polypeptides and antibodies directed to these polypeptides are useful in providing immunological probes for differential identification of the tissue(s) or cell type(s). For a number of disorders of the above tissues or cells, particularly of the disorders of the immune system, expression of this gene at significantly higher or lower levels may be routinely detected in certain tissues (e.g., immune cells and tissue, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:192 as residues: Glu-15 to Arg-23, Asn-79 to Gly-84.

The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:53 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1126 of SEQ ID NO:53, b is an integer of 15 to 1140, where both a and b correspond to the positions of nucleotide residues shown in SEQ ID NO:53, and where b is greater than or equal to a + 14.

### FEATURES OF PROTEIN ENCODED BY GENE NO: 44

This gene is expressed primarily in human tonsil.

Therefore, polynucleotides and polypeptides of the invention are useful as reagents for differential identification of the tissue(s) or cell type(s) present in a biological sample and for diagnosis of diseases and conditions which include, but are not limited to, inflammatory and immune diseases and/or disorders. Similarly, polypeptides and antibodies directed to these polypeptides are useful in providing immunological probes for differential identification of the tissue(s) or cell type(s). For a number of disorders of the above tissues or cells, particularly of the immune diseases, expression of this gene at significantly higher or lower levels may be routinely detected in certain tissues (e.g., immune cells and tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 193 as residues: Ile-2 to Lys-9, Gln-43 to Phe-49, Asn-59 to His-69, Gly-87 to Asp-93.

The tissue distribution indicates that polynucleotides and polypeptides corresponding to this gene are useful for the diagnosis and treatment of a variety of immune system disorders. Expression of this gene product in tonsils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:54 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1206 of SEQ ID NO:54, b is an integer of 15 to 1220, where both a and b correspond to the positions of nucleotide residues shown in SEQ ID NO:54, and where b is greater than or equal to a + 14.

### FEATURES OF PROTEIN ENCODED BY GENE NO: 45

The translation product of this gene shares sequence homology with a novel human G52-24 secreted protein as well as the early lymphocyte activation antigen CD69, the latter of which has been shown to be important in lymphocyte proliferation and functions as a signal trasmitting receptor in lymphocytes, natural killer cells, and platelets (See Genbank Accession Nos. W27288 and gil558352, respectively). Preferred polypeptides comprise the following amino acid sequence:
ENFLLRYKGPSDHWIGLSREQGQPWKWINGTEWTRQLVMKEDGANLYVAKV SQVPRMNPXLS WVLLCYPGWSAVXTIVAHCSLDFPGSK (SEQ ID NO:335), ELTAIK SHQYVLQAACPESWIGFQRKCFYFSDDTKNWTSSQRFCDSQDADLA QVESFQELVRK (SEQ ID NO:336), WIGLSREQGQPWKWING (SEQ ID NO:337), CPESWIG FQRKC (SEQ ID NO:338), NFLLRYKGPSDHWIGL (SEQ ID NO:339), ASHLRLL SSWDYRFPILGAGECAYLNDKGASSARHYTERKWICSKSDIHV (SEQ ID NO:340), ENFLLRYKGPSDHWIGLSREQGQPWKWINGTEWTRQL VMKEDG ANLYVAKVSQVPRMNPXLSWVLLCYPGWSAVXTIVAHCSLDF PGSK (SEQ ID NO:341), and/or SWTSSLLNXCLHSKEHSIKATIWRLFFXILTIIL. CGMVAALSA IRANCHQEPSVCSSSCMPRKLDWFSKKVFLFF (SEQ ID NO:342). Also preferred are the polynucleotides encoding these polypeptides.

This gene is expressed primarily in human testis.

Therefore, polynucleotides and polypeptides of the invention are useful as reagents for differential identification of the tissue(s) or cell type(s) present in a biological sample and for diagnosis of diseases and conditions which include, but are not limited to, relating to male reproductive endocrine and immune disorders. Similarly, polypeptides and antibodies directed to these polypeptides are useful in providing immunological probes for differential identification of the tissue(s) or cell type(s). For a number of disorders of the above tissues or cells, particularly of the disorders of reproductive system, expression of this gene at significantly higher or lower levels may be routinely detected in certain tissues (e.g., immune cells and tissue, reproductive tissue, and cancerous and wounded tissues) or bodily fluids (e.g., lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 194 as residues: Asn-20 to Pro-25, Ser-48 to Asp-65.

The tissue distribution indicates that polynucleotides and polypeptides corresponding to this gene are useful for the diagnosis and treatment of a variety of immune system disorders. Homology of this gene product to the early lymphocyte activation antigen CD69 indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Alternatively, the tissue distribution within human testis may be indicative of a role for this gene product in normal testicular function, and may implicate this gene product in male fertility, and could even suggest a use as a male contraceptive. Many polynucleotide sequences, such as EST sequences, are publicly available and accessible through sequence databases. Some of these sequences are related to SEQ ID NO:55 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 680 of SEQ ID NO:55, b is an integer of 15 to 694, where both a and b correspond to the positions of nucleotide residues shown in SEQ ID NO:55, and where b is greater than or equal to a + 14.

### FEATURES OF PROTEIN ENCODED BY GENE NO: 46

This gene is expressed primarily in human testes.

Therefore, polynucleotides and polypeptides of the invention are useful as reagents for differential identification of the tissue(s) or cell type(s) present in a biological sample and for diagnosis of diseases and conditions which include, but are not limited to, male reproductive and endocrine disorders. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., endocrine tissue, reproductive tissue, and cancerous and wounded tissues) or bodily fluids (e.g.seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:195 as residues: Pro-20 to Trp-25, Arg-33 to Thr-38, Asn-51 to Ile-56, Gly-82 to Ser-91, Lys-151 to Arg-156.

The tissue distribution indicates that polynucleotides and polypeptides corresponding to this gene are useful for the detection, treatment, and/or prevention of various endocrine disorders and cancers, particularly Addisonís disease, Cushingís Syndrome, and disorders and/or cancers of the pancrease (e.g., diabetes mellitus), adrenal cortex, ovaries, pituitary (e.g., hyper-, hypopituitarism), thyroid (e.g., hyper-, hypothyroidism), parathyroid (e.g., hyper-,hypoparathyroidism), hypothallamus, and testes. Protein, as well as, antibodies directed against the protein may show utility as a tumor marker and/or immunotherapy targets for the above listed tissues. Alternatively, expression within the human testis may be indicative of a role for this gene product in normal testicular function, and may implicate this gene product in male fertility, and could even suggest a use as a male contraceptive. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:56 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 974 of SEQ ID NO:56, b is an integer of 15 to 988, where both a and b correspond to the positions of nucleotide residues shown in SEQ ID NO:56, and where b is greater than or equal to a + 14.

### FEATURES OF PROTEIN ENCODED BY GENE NO: 47

One embodiment of this gene comprises polypeptides of the following amino acid sequence:
LKGREAGAGPGTAGAPGREDANGXXRGRGGXHQLYLWVDNIPLSRPKRNLS RDFSDGVLVAEVIKFYFPKMVEMHNYVGTSSLQQKLSNWGHLNRKVLKRLN FSVPDDV (SEQ ID NO:343). An additional embodiment is the polynucleotides encoding these polypeptides.

This gene is expressed primarily in human adult testis.

Therefore, polynucleotides and polypeptides of the invention are useful as reagents for differential identification of the tissue(s) or cell type(s) present in a biological sample and for diagnosis of diseases and conditions which include, but are not limited to, relating to male reproductive and endocrine disorders. Similarly, polypeptides and antibodies directed to these polypeptides are useful in providing immunological probes for differential identification of the tissue(s) or cell type(s). For a number of disorders of the above tissues or cells, particularly of the disorders of the reproductive system, expression of this gene at significantly higher or lower levels may be routinely detected in certain tissues (e.g., reproductive tissue, and endocrine, cancerous and wounded tissues) or bodily fluids (e.g., seminal fluid, serum, plasma, urine, synovial fluid and spinal fluid) or another tissue or cell sample or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:196 as residues: Gln-21 to Gly-33, Gln-55 to Glu-60.

The tissue distribution indicates that polynucleotides and polypeptides corresponding to this gene are useful for the reproductive system, and may be indicative of a role for this gene product in normal testicular function, male fertility, and/or as a male contraceptive. Protein, as well as, antibodies directed against the protein may show utility as a tissue-specific marker and/or immunotherapy target for the above listed tissues.. Many polynucleotide sequences, such as EST sequences, are publicly available and accessible through sequence databases. Some of these sequences are related to SEQ ID NO:57 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1486 of SEQ ID NO:57, b is an integer of 15 to 1500, where both a and b correspond to the positions of nucleotide residues shown in SEQ ID NO:57, and where b is greater than or equal to a + 14.

### FEATURES OF PROTEIN ENCODED BY GENE NO: 48

The translation product of this gene shares sequence homology with the human M phase phosphoprotein 10 as well as ORF YJR002w of *Saccharomyces cerevisiae* (See Genbank Accession No.gnIlPIDle266673). Preferred polypeptides comprise the following amino acid sequence:
AKNSQKEENPEHVEIQKMMDSLFLKLDALSNFHFPKPPVPEIKVVSNLPAITM EE VAPVSVSDAALLAPEEIKEKNKAGDIKTAAEKTATDKKRERRKKKYQK RMKIKEKEKRRKLLEKSSVDQAGKYSKTVASEKLKQLTKTGKASFIKVRTRE RKLLKGTFVGEVDSKCWVTGMSEPADSPPVG (SEQ ID NO:344), LQDEGKD KALKSSQAFFSKLQDQVKMQINDAKKTEKKKKKRQDISVHKLKL (SEQ ID NO:345), DEGK DKALKSSQAFFSKLQDQVKMQINDA (SEQ ID NO:346), EENPEHVEIQKMMDSLFL KLDALSNFHF (SEQ ID NO:347), SSVDQAGKYSK TVASEKLKQLTKTGKASFIK (SEQ ID NO:349), VSVSDAALLAPEEIKEK NKAGDI (SEQ ID NO:350), and/or SNLPATTMEEVAP (SEQ ID NO:348). Also preferred are the polynucleotides encoding these polypeptides.

This gene is expressed primarily in human thyroid.

Therefore, polynucleotides and polypeptides of the invention are useful as reagents for differential identification of the tissue(s) or cell type(s) present in a biological sample and for diagnosis of diseases and conditions which include, but are not limited to, diseases relating to the thyroid gland, particularly hyper- and hypothyroidism. Similarly, polypeptides and antibodies directed to these polypeptides are useful in providing immunological probes for differential identification of the tissue(s) or cell type(s). For a number of disorders of the above tissues or cells, particularly of the disorders of the endocrine system, expression of this gene at significantly higher or lower levels may be routinely detected in certain tissues (e.g., endocrine tissue, and cancerous and wounded tissues) or bodily fluids (e.g., lymph, serum, plasma, urine, synovial fluid and spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for metabolic disorders, particularly hyper-, hypothyroidism, Graves disease Hashimoto's thyroiditis, and/or cancer or neoplasias of the thyroid, and/or other endocrine organs and immune system. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:58 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1377 of SEQ ID NO:58, b is an integer of 15 to 1391, where both a and b correspond to the positions of nucleotide residues shown in SEQ ID NO:58, and where b is greater than or equal to a + 14.

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-may have commercial utility in the expansion of stem cells and committed progenitors of various blood lineages, and in the differentiation and/or proliferation of various cell types.. Many polynucleotide sequences, such as EST sequences, are publicly available and accessible through sequence databases. Some of these sequences are related to SEQ ID NO:59 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1565 of SEQ ID NO:59, b is an integer of 15 to 1579, where both a and b correspond to the positions of nucleotide residues shown in SEQ ID NO:59, and where b is greater than or equal to a + 14.

### FEATURES OF PROTEIN ENCODED BY GENE NO: 50

The translation product of this gene was shown to have homology to the chicken LRP/alpha-2-macroglobulin receptor which is thought to play a pivitol role on the metabolism of alpha-2-macroglubulins, as well as, complexes between plasminogen activators and their endogenous inhibitors (See GenbankAccession No gblX74904lGGLRPA2MR).

This gene is expressed primarily in neuronal tissues and to a lesser extent in uterine cancer.

Therefore, polynucleotides and polypeptides of the invention are useful as reagents for differential identification of the tissue(s) or cell type(s) present in a biological sample and for diagnosis of diseases and conditions which include, but are not limited to, neuronal disorders and uterine cancer. Similarly, polypeptides and antibodies directed to these polypeptides are useful in providing immunological probes for differential identification of the tissue(s) or cell type(s). For a number of disorders of the above tissues or cells, particularly of the central neuron system, expression of this gene at significantly higher or lower levels may be routinely detected in certain tissues (e.g., neural cells and tissue, reproductive tissue, and cancerous and wounded tissues) or bodily fluids (e.g., amniotic fluid, serum, plasma, urine, synovial fluid and spinal fluid) or another tissue or cell sample or another tissue or cell sample taken from an individual having such a disorder, relative to the standard gene expression level. i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO: 60 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1227 of SEQ ID NO:60, b is an integer of 15 to 1241, where both a and b correspond to the positions of nucleotide residues shown in SEQ ID NO:60, and where b is greater than or equal to a + 14.

### FEATURES OF PROTEIN ENCODED BY GENE NO: 51

This gene is expressed primarily in uterine cancer and to a lesser extent in other tissues.

Therefore, polynucleotides and polypeptides of the invention are useful as reagents for differential identification of the tissue(s) or cell type(s) present in a biological sample and for diagnosis of diseases and conditions which include, but are not limited to, uterine cancer, and other reproductive disorders. Similarly, polypeptides and antibodies directed to these polypeptides are useful in providing immunological probes for differential identification of the tissue(s) or cell type(s). For a number of disorders of the above tissues or cells, particularly of the uterine cancer, expression of this gene at significantly higher or lower levels may be routinely detected in certain tissues (e.g., reproductive tissue, and cancerous and wounded tissues) or bodily fluids (e.g., amniotic fluid, serum, plasma, urine, synovial fluid and spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution in tumors of the uterus indicates that polynucleotides and polypeptides corresponding to this gene are useful for diagnosis and intervention of these tumors, in addition to other tumors where expression has been indicated. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:61 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 916 of SEQ ID NO:61, b is an integer of 15 to 930, where both a and b correspond to the positions of nucleotide residues shown in SEQ ID NO:61, and where b is greater than or equal to a + 14.

### FEATURES OF PROTEIN ENCODED BY GENE NO: 52

This gene is expressed primarily in Wilm's tumor and to a lesser extent in other tissues.

Therefore, polynucleotides and polypeptides of the invention are useful as reagents for differential identification of the tissue(s) or cell type(s) present in a biological sample and for diagnosis of diseases and conditions which include, but are not limited to, Wilm's tumor, and other urogenital disorders. Similarly, polypeptides and antibodies directed to these polypeptides are useful in providing immunological probes for differential identification of the tissue(s) or cell type(s). For a number of disorders of the above tissues or cells, particularly of the Wilm's tumor, expression of this gene at significantly higher or lower levels may be routinely detected in certain tissues (e.g., urogenital tissue, and cancerous and wounded tissues) or bodily fluids (e.g., serum, plasma, urine, synovial fluid and spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of Wilm's tumor. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:62 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 984 of SEQ ID NO:62, b is an integer of 15 to 998, where both a and b correspond to the positions of nucleotide residues shown in SEQ ID NO:62, and where b is greater than or equal to a + 14.

### FEATURES OF PROTEIN ENCODED BY GENE NO: 53

The translation product of this gene was shown to have homology to the MEK kinase 3 of *Mus musculus,* mutations of which and/or aberrant regulation of, may provide a predisposition to cancer. This gene maps to chromosome 17, and therefore,may be used as a marker in linkage analysis for chromosome 17.

This gene is expressed primarily in pituitary and to a lesser extent in ulcerative colitis, hemapoietic cells, and some other tissues.

Therefore, polynucleotides and polypeptides of the invention are useful as reagents for differential identification of the tissue(s) or cell type(s) present in a biological sample and for diagnosis of diseases and conditions which include, but are not limited to, immune, gastrointestinal, haematopoeitic diseases. Similarly, polypeptides and antibodies directed to these polypeptides are useful in providing immunological probes for differential identification of the tissue(s) or cell type(s). For a number of disorders of the above tissues or cells, particularly of the neuronal and immune tissues, expression of this gene at significantly higher or lower levels may be routinely detected in certain tissues (e.g., immune cells and tissue, and cancerous and wounded tissues) or bodily fluids (e.g., lymph, serum, plasma, urine, synovial fluid and spinal fluid) or another tissue or cell sample or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in ulcerative colitis indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:63 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1179 of SEQ ID NO: 63, b is an integer of 15 to 1193, where both a and b correspond to the positions of nucleotide residues shown in SEQ ID NO:63, and where b is greater than or equal to a + 14.

### FEATURES OF PROTEIN ENCODED BY GENE NO: 54

When tested against Jurkat T-cell cell lines, supernatants removed from cells containing this gene activated the GAS (gamma activation site) pathway. Thus, it is likely that this gene activates T-cells through the Jaks-STAT signal transduction pathway. GAS (gamma activation site) is a promoter element found upstream in many genes which are involved in the Jaks-STAT pathway. The Jaks-STAT pathway is a large, signal transduction pathway involved in the differentiation and proliferation of cells. Therefore, activation of the Jaks-STATs pathway, reflected by the binding of the GAS element, can be used to indicate proteins involved in the proliferation and differentiation of cells.

This gene is expressed primarily in fetal spleen, adipose, and to a lesser extent in other tissues.

Therefore, polynucleotides and polypeptides of the invention are useful as reagents for differential identification of the tissue(s) or cell type(s) present in a biological sample and for diagnosis of diseases and conditions which include, but are not limited to, immune, metabolic, and developmental disorders. Similarly, polypeptides and antibodies directed to these polypeptides are useful in providing immunological probes for differential identification of the tissue(s) or cell type(s). For a number of disorders of the above tissues or cells, particularly of the fetal spleen and adipose tissues, expression of this gene at significantly higher or lower levels may be routinely detected in certain tissues (e.g., immune cells and tissue, developing cells and tissue, and cancerous and wounded tissues) or bodily fluids (e.g., lymph, serum, plasma, urine, synovial fluid and spinal fluid) or another tissue or cell sample or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:203 as residues: Tyr-41 to Phe-47.

The tissue distribution combined with the detection of GAS promoter activation activity indicates that polynucleotides and polypeptides corresponding to this gene are useful for the diagnosis and treatment of a variety of immune system disorders. Expression of this gene product in fetal spleen indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:64 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 816 of SEQ ID NO:64, b is an integer of 15 to 830, where both a and b correspond to the positions of nucleotide residues shown in SEQ ID NO:64, and where b is greater than or equal to a + 14.

### FEATURES OF PROTEIN ENCODED BY GENE NO: 55

This gene is expressed primarily in IL-1/TNF stimulated synovial and human adipose.

Therefore, polynucleotides and polypeptides of the invention are useful as reagents for differential identification of the tissue(s) or cell type(s) present in a biological sample and for diagnosis of diseases and conditions which include, but are not limited to, rheumatoid arthritis or obessity. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., synovial and adipose cells and tissues, and cancerous and wounded tissues) or bodily fluids (e.g., serum, plasma, urine, synovial fluid and spinal fluid) or another tissue or cell sample or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:204 as residues: Leu-37 to Arg-45, Ser-60 to Ser-65.

The tissue distribution indicates that polynucleotides and polypeptides corresponding to this gene are useful for the diagnosis or treatment of rheumatoid arthritis or other immune diseases. Many polynucleotide sequences, such as EST sequences, are publicly available and accessible through sequence databases. Some of these sequences are related to SEQ ID NO:65 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 853 of SEQ ID NO:65, b is an integer of 15 to 867, where both a and b correspond to the positions of nucleotide residues shown in SEQ ID NO:65, and where b is greater than or equal to a + 14.

### FEATURES OF PROTEIN ENCODED BY GENE NO: 56

This gene is expressed primarily in aortic endothelium and to a lesser extent in melanocytes.

Therefore, polynucleotides and polypeptides of the invention are useful as reagents for differential identification of the tissue(s) or cell type(s) present in a biological sample and for diagnosis of diseases and conditions which include, but are not limited to, cardiovascular diseases. Similarly, polypeptides and antibodies directed to these polypeptides are useful in providing immunological probes for differential identification of the tissue(s) or cell type(s). For a number of disorders of the above tissues or cells, particularly of the cardiovascular system, expression of this gene at significantly higher or lower levels may be routinely detected in certain tissues (e.g., cardiovascular tissue, and melanocytes, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:205 as residues: Met-1 to Trp-12, Arg-33 to Ser-53.

The tissue distribution in human aortic endothelial cells indicates that polynucleotides and polypeptides corresponding to this gene are useful for the detection or intervention of cardiovascular diseases, such as hypertension, cadiovascular injuries, congenital heart diseases, ischemic heart diseases, rheumatic and other hypersensitivity diseases, cardiomyopathy. Many polynucleotide sequences, such as EST sequences, are publicly available and accessible through sequence databases. Some of these sequences are related to SEQ ID NO:66 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 671 of SEQ ID NO:66, b is an integer of 15 to 685, where both a and b correspond to the positions of nucleotide residues shown in SEQ ID NO:66, and where b is greater than or equal to a + 14.

### FEATURES OF PROTEIN ENCODED BY GENE NO: 57

The translation product of this gene shares sequence homology with prostaglandin EP3-9 receptor which is thought to be important in prostaglandin hormonal reaction. In specific embodiments, polypeptides of the invention comprise the sequence:MAIPAFSSCQQISSAAALQI (SEQ ID NO:351), and/or CNGPFKHFSFTVST (SEQ ID NO:352). Polynucleotides encoding these polypeptides are also encompassed by the invention.

This gene is expressed primarily in human retina.

Therefore, polynucleotides and polypeptides of the invention are useful as reagents for differential identification of the tissue(s) or cell type(s) present in a biological sample and for diagnosis of diseases and conditions which include, but are not limited to, glaucoma or other ocular diseases. Similarly, polypeptides and antibodies directed to these polypeptides are useful in providing immunological probes for differential identification of the tissue(s) or cell type(s). For a number of disorders of the above tissues or cells, particularly of the ocular system, expression of this gene at significantly higher or lower levels may be routinely detected in certain tissues (e.g., retinal and other optic tissue cells, and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in eyes and homology to prostaglandin receptor indicates that polynucleotides and polypeptides corresponding tothis gene are useful for detection and intervention of ocular diseases like glaucoma. Specifically the receptor can be used for the identification of agonists or antagonists, anti-inflammatories for the eyes, and vasoconstrictive agents, etc. Many polynucleotide sequences, such as EST sequences, are publicly available and accessible through sequence databases. Some of these sequences are related to SEQ ID NO:67 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 787 of SEQ ID NO:67, b is an integer of 15 to 801, where both a and b correspond to the positions of nucleotide residues shown in SEQ ID NO:67, and where b is greater than or equal to a + 14.

### FEATURES OF PROTEIN ENCODED BY GENE NO: 58

The translation product of this gene shares weak sequence homology with *Hemophilus influenzae* outer protein P6 which is thought to be important in host cell interaction.

This gene is expressed primarily in human adrenal gland tumor.

Therefore, polynucleotides and polypeptides of the invention are useful as reagents for differential identification of the tissue(s) or cell type(s) present in a biological sample and for diagnosis of diseases and conditions which include, but are not limited to, adrenal insufficiency or hyperfunction. Similarly, polypeptides and antibodies directed to these polypeptides are useful in providing immunological probes for differential identification of the tissue(s) or cell type(s). For a number of disorders of the above tissues or cells, particularly of the endocrine systems, expression of this gene at significantly higher or lower levels may be routinely detected in certain tissues (e.g., adrenal gland, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in adrenal gland tumor and homology to *Hemophilus influenzae* outer membrane protein suggest that polynucleotides and polypeptides corresponding to this gene are useful in treating adrenal insuficiencies or hyperfunction because a secretory protein from an endocrine organ may function as a hormone. Many polynucleotide sequences, such as EST sequences, are publicly available and accessible through sequence databases. Some of these sequences are related to SEQ ID NO:68 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 894 of SEQ ID NO:68, b is an integer of 15 to 908, where both a and b correspond to the positions of nucleotide residues shown in SEQ ID NO:68, and where b is greater than or equal to a + 14.

### FEATURES OF PROTEIN ENCODED BY GENE NO: 59

When tested against a Jurkat T-cell line, supernatants removed from cells containing this gene activated the GAS (gamma activation site) pathway. Thus, it is likely that this gene activates T-cells through the Jaks-STAT signal transduction pathway. The GAS is a promoter element found upstream in many genes which are involved in the Jaks-STAT pathway. The Jaks-STAT pathway is a complex, signal transduction pathway involved in the differentiation and proliferation of cells. Therefore, activation of the Jaks-STATs pathway, reflected by the binding of the GAS element, can be used to indicate proteins involved in the proliferation and differentiation of cells.

This gene is expressed primarily in human kidney pyramid and to a lesser extent in human brain.

Therefore, polynucleotides and polypeptides of the invention are useful as reagents for differential identification of the tissue(s) or cell type(s) present in a biological sample and for diagnosis of diseases and conditions which include, but are not limited to, nephrotic, nephritic syndromes, renal failure, hypertensive nephrosclerosis. Similarly, polypeptides and antibodies directed to these polypeptides are useful in providing immunological probes for differential identification of the tissue(s) or cell type(s). For a number of disorders of the above tissues or cells, particularly of the renal system, expression of this gene at significantly higher or lower levels may be routinely detected in certain tissues (eg., hepatic tissue, brain and other tissue of the nervous system, T-cells and other cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in kidney indicates that polynucleotides and polypeptides corresponding to this gene are useful for renal diseases, including nephrotic, nephritic syndromes, renal failure, hypertensive nephrosclerosis. Additionally, the gene product may have endocrine functions related to renal function, metabolism and homeostasis. Many polynucleotide sequences, such as EST sequences, are publicly available and accessible through sequence databases. Some of these sequences are related to SEQ ID NO:69 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 682 of SEQ ID NO:69, b is an integer of 15 to 696, where both a and b correspond to the positions of nucleotide residues shown in SEQ ID NO:69, and where b is greater than or equal to a + 14.

### FEATURES OF PROTEIN ENCODED BY GENE NO: 60

This gene is expressed primarily in both normal or cancerous human breast tissue.

Therefore, polynucleotides and polypeptides of the invention are useful as reagents for differential identification of the tissue(s) or cell type(s) present in a biological sample and for diagnosis of diseases and conditions which include, but are not limited to, Non-neoplastic breast diseases or breast cancers. Similarly, polypeptides and antibodies directed to these polypeptides are useful in providing immunological probes for differential identification of the tissue(s) or cell type(s). For a number of disorders of the above tissues or cells, particularly of the breast, expression of this gene at significantly higher or lower levels may be routinely detected in certain tissues (e.g., mammary tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:209 as residues: Pro-20 to Ser-28.

The tissue distribution in breast indicates that polynucleotides and polypeptides corresponding to this gene are useful for either non-neoplastic breast diseases, such as congentital anomalities, gynecomastia, mastitis and abscess, duct ectasia and fat necrosis, or neoplasia in the breast. Many polynucleotide sequences, such as EST sequences, are publicly available and accessible through sequence databases. Some of these sequences are related to SEQ ID NO:70 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 441 of SEQ ID NO:70, b is an integer of 15 to 455, where both a and b correspond to the positions of nucleotide residues shown in SEQ ID NO:70, and where b is greater than or equal to a + 14.

### FEATURES OF PROTEIN ENCODED BY GENE NO: 61

When tested against a K562 kidney cell line, supernatants removed from cells containing this gene activated the ISRE (interferon-sensitive responsive element) pathway. Thus, it is likely that this gene activates kidney cells through the Jaks-STAT signal transduction pathway. The ISRE is a promoter element found upstream in many genes which are involved in the Jaks-STAT pathway. The Jaks-STAT pathway is a complex, signal transduction pathway involved in the differentiation and proliferation of cells. Therefore, activation of the Jaks-STATs pathway, reflected by the binding of the ISRE element, can be used to indicate proteins involved in the proliferation and differentiation of cells. Polynucleotides encoding these polylpeptides are also encompassed by the invention.

This gene is expressed primarily in activated T-cells and osteoarthritis, and to a lesser extent in aortic endothelium, placenta and number of tissues or cell lines.

Therefore, polynucleotides and polypeptides of the invention are useful as reagents for differential identification of the tissue(s) or cell type(s) present in a biological sample and for diagnosis of diseases and conditions which include, but are not limited to, inflammatory conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., T-cells and other cells and tissue of the immune system, bone tissue, endothelium and placenta, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:2 10 as residues: Gln-36 to Glu-49, Glu-51 to Leu-66, Asp-68 to Ser-73.

The tissue distribution in activated T-cells and under inflammatory conditions like osteoarthritis suggest that the protein product of this gene is involved in the inflammatory reactions. Therefore it may be useful in the diagnosis or intervention in the inflammatory diseases with the involvement of T-cells, including osteoarthritis. Many polynucleotide sequences, such as EST sequences, are publicly available and accessible through sequence databases. Some of these sequences are related to SEQ ID NO:71 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 399 of SEQ ID NO:71, b is an integer of 15 to 413, where both a and b correspond to the positions of nucleotide residues shown in SEQ ID NO:71, and where b is greater than or equal to a + 14.

### FEATURES OF PROTEIN ENCODED BY GENE NO: 62

This gene is expressed primarily in breast lymphnodes, B-cell lymphoma and to a lesser extent in neutrophils and bone marrow cells.

Therefore, polynucleotides and polypeptides of the invention are useful as reagents for differential identification of the tissue(s) or cell type(s) present in a biological sample and for diagnosis of diseases and conditions which include, but are not limited to, inflammation, immunodeficiency, allergy. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, hematopoietic cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in the cells of immunological functions indicates that polynucleotides and polypeptides corresponding to this gene are useful for diagnosis or intervention of immunologically mediated disorders, such as allergy, immunodeficiency, immune surveillance, etc. Many polynucleotide sequences, such as EST sequences, are publicly available and accessible through sequence databases. Some of these sequences are related to SEQ ID NO:72 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 835 of SEQ ID NO:72, b is an integer of 15 to 849, where both a and b correspond to the positions of nucleotide residues shown in SEQ ID NO:72, and where b is greater than or equal to a + 14.

### FEATURES OF PROTEIN ENCODED BY GENE NO: 63

The translation product of this gene shares weak sequence homology with Interferon induced 1-8 gene encoded polypeptide which is thought to be important in retroviral REV responsive element binding and thus viral replication.

This gene is expressed primarily in B-cell lymphoma.

Therefore, polynucleotides and polypeptides of the invention are useful as reagents for differential identification of the tissue(s) or cell type(s) present in a biological sample and for diagnosis of diseases and conditions which include, but are not limited to, immune response to viral infections and other immunologically replated disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., B-cells and other cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:212 as residues: Pro-47 to Asn-53.

The tissue distribution in B-cell lymphoma and homology to interferon induced 1-8 gene indicates that polynucleotides and polypeptides corresponding tothis gene are useful for intervention of viral infection and other immunologically related disorders. The homology with interferon induced 1-8 REV response element binding gene indicates the gene product may bind to viral components to interfere with the entry, packaging, replication, or induce the host cell anti-viral response by intereferon mediated pathways. Many polynucleotide sequences, such as EST sequences, are publicly available and accessible through sequence databases. Some of these sequences are related to SEQ ID NO:73 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 491 of SEQ ID NO:73, b is an integer of 15 to 505, where both a and b correspond to the positions of nucleotide residues shown in SEQ ID NO:73, and where b is greater than or equal to a + 14.

### FEATURES OF PROTEIN ENCODED BY GENE NO: 64

This gene is expressed primarily in bone marrow.

Therefore, polynucleotides and polypeptides of the invention are useful as reagents for differential identification of the tissue(s) or cell type(s) present in a biological sample and for diagnosis of diseases and conditions which include, but are not limited to, hemapoiesis disorders. Similarly, polypeptides and antibodies directed to these polypeptides are useful in providing immunological probes for differential identification of the tissue(s) or cell type(s). For a number of disorders of the above tissues or cells, particularly of the hemapoietic system, expression of this gene at significantly higher or lower levels may be routinely detected in certain tissues (e.g., bone marrow, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:213 as residues: Thr-45 to Tyr-50.

The tissue distribution in bone marrow indicates that polynucleotides and polypeptides corresponding to this gene are useful for hemapoiesis disorders. The gene product may function as a growth factor or mobilization agent for the cells of myeloid or lymphoid lineages. Many polynucleotide sequences, such as EST sequences, are publicly available and accessible through sequence databases. Some of these sequences are related to SEQ ID NO:74 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 705 of SEQ ID NO:74, b is an integer of 15 to 719, where both a and b correspond to the positions of nucleotide residues shown in SEQ ID NO:74, and where b is greater than or equal to a + 14.

### FEATURES OF PROTEIN ENCODED BY GENE NO: 65

The translation product of this gene shares sequence homology with the familial adenomatous polyposis gene which is thought to be important in tumorigenesis of colon cancer (see, e.g., Fulton, Nature 368, 32-38 (1994); accession no. U28412; Joslyn et al., Cell 66 (3), 601-613 (1991); accession no. M73547; and Spirio et al., Nucleic Acids Res. 19 (22), 6348 (1991)). In specific embodiments, polypeptides of the invention comprise the sequence: CRWRPESAAPC (SEQ ID NO:353), TRPGRGAQAPVK (SEQ ID NO:354), MVSWMISRAVVLVFGMLYPAY (SEQ ID NO:355), GMLYPAYYSYKAVKTKN (SEQ ID NO:356), EYVRWMMYWIV FALYTV (SEQ ID NO:357), YPAYYSYKAVKTKNVKE (SEQ ID NO:358), VAWFPLYYELKIA (SEQ ID NO:359), and/or MVSWMISRAVVLVFGMLYPAY YSYKAVKTKNVKEYVRWMMYWIVFALYTVIETVADQTVAWFPLYYELKIAFVI WLLSPYTKGASLIYRKFLHIPLLSSKEREIDDYIVQAKERGYETMVNFGRQGLNL AATAAVTAAVKSQGAITERLRSFSMHDLTTIQGDEPVGQRPYQPLPEAKKKSXQ PPVN (SEQ ID NO:360). Polynucleotides encoding these polypeptides are also encompassed by the invention.

This gene is expressed primarily in osteoclastoma, prostate, bone marrow and to a lesser extent in testes and dendritic cells.

Therefore, polynucleotides and polypeptides of the invention are useful as reagents for differential identification of the tissue(s) or cell type(s) present in a biological sample and for diagnosis of diseases and conditions which include, but are not limited to, colonb cancer and cancers of various origin, including osteoclastoma and prostate cancer. Similarly, polypeptides and antibodies directed to these polypeptides are useful in providing immunological probes for differential identification of the tissue(s) or cell type(s). For a number of disorders of the above tissues or cells, particularly of the tumorigenesis, expression of this gene at significantly higher or lower levels may be routinely detected in certain tissues and cell types (e.g., bone, prostate, bone marrow, colon and other gastrointestinal tissue, tissue of the nervous system, and testis and other reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:214 as residues: Ser-59 to De-64, Ala-71 to Tyr-76, Pro-125 to Ser-141.

The tissue distribution in osteoclastoma, prostate, bone marrow and homology to familial adenomatous polyposis gene indicates polynucleotides and polypeptides corresponding to this gene are useful for diagnosis and intervention of tumors of various origins, including colon cancer, osteoclastoma and prostate cancer. Many polynucleotide sequences, such as EST sequences, are publicly available and accessible through sequence databases. Some of these sequences are related to SEQ ID NO:75 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1260 of SEQ ID NO:75, b is an integer of 15 to 1274, where both a and b correspond to the positions of nucleotide residues shown in SEQ ID NO:75, and where b is greater than or equal to a + 14.

### FEATURES OF PROTEIN ENCODED BY GENE NO: 66

The translation product of this gene shares regional and weak sequence homology with neu differentiation factor and a serine protease N-terminal fragment which contains a EGF-like domain and is thought to be important in growth and differentiation of several cell types, including colon epithelial cells and Schwann cells.

This gene is expressed primarily in fetal lung, bone marrow, fetal liver and to a lesser extent in brain.

Therefore, polynucleotides and polypeptides of the invention are useful as reagents for differential identification of the tissue(s) or cell type(s) present in a biological sample and for diagnosis of diseases and conditions which include, but are not limited to, tissue injuries or diseases in lung, bone marrow, or liver. Similarly, polypeptides and antibodies directed to these polypeptides are useful in providing immunological probes for differential identification of the tissue(s) or cell type(s). For a number of disorders of the above tissues or cells, particularly of the liver and lung, expression of this gene at significantly higher or lower levels may be routinely detected in certain tissues and cell types (e.g., lung and pulmonary tissue, bone marrow, hepatic tissue, neural tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to neu differentiation factor indicates that polynucleotides and polypeptides corresponding to this gene are useful for diagnosis or intervention of liver or lung injuries, including hepatic failure, recovery from hepatitis, cirrhosis, and complication from liver transplantation. Many polynucleotide sequences, such as EST sequences, are publicly available and accessible through sequence databases. Some of these sequences are related to SEQ ID NO:76 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 505 of SEQ ID NO:76, b is an integer of 15 to 519, where both a and b correspond to the positions of nucleotide residues shown in SEQ ID NO:76, and where b is greater than or equal to a + 14.

### FEATURES OF PROTEIN ENCODED BY GENE NO: 67

This gene is expressed primarily in activated T-cells.

Therefore, polynucleotides and polypeptides of the invention are useful as reagents for differential identification of the tissue(s) or cell type(s) present in a biological sample and for diagnosis of diseases and conditions which include, but are not limited to, arthritis, asthma, auto-immune and immunodeficiency diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., T-cells and other cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The expression of this gene in T-cells indicates a potential role in the treament/detection of immune disorders such as such as arthritis, asthma, for hypersensitivity reactions and transplant rejection, and also in immune deficiency diseases such as AIDS, and leukemia. Many polynucleotide sequences, such as EST sequences, are publicly available and accessible through sequence databases. Some of these sequences are related to SEQ ID NO:77 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 375 of SEQ ID NO:77, b is an integer of 15 to 389, where both a and b correspond to the positions of nucleotide residues shown in SEQ ID NO:77, and where b is greater than or equal to a + 14.

### FEATURES OF PROTEIN ENCODED BY GENE NO: 68

This gene maps to chromosome 7, accordingly, polynucleotides of the invention may be used in linkage analysis as a marker for chromosome 7.

This gene is expressed primarily in brain and to a lesser extent in breast.

Therefore, polynucleotides and polypeptides of the invention are useful as reagents for differential identification of the tissue(s) or cell type(s) present in a biological sample and for diagnosis of diseases and conditions which include, but are not limited to, neurodegenerative conditions and behavioural disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues and cell types (e.g., brain and other tissue of the nervous system, mammary tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:217 as residues: Leu-40 to His-46.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, schizophrenia, mania, dementia, paranoia, obsessive compulsive disorder and panic disorder. Many polynucleotide sequences, such as EST sequences, are publicly available and accessible through sequence databases. Some of these sequences are related to SEQ ID NO:78 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 809 of SEQ ID NO:78, b is an integer of 15 to 823, where both a and b correspond to the positions of nucleotide residues shown in SEQ ID NO:78, and where b is greater than or equal to a + 14.

### FEATURES OF PROTEIN ENCODED BY GENE NO: 69

The translation product of this gene shares sequence homology with a rat secretory carrier membrane protein which is believed to play a role in cell surface recycling. See e.g., Brand et al., EMBO J, 1993, Oct;12(10):3753-3761. Secretory carrier membrane proteins (SCAMPs) are widely distributed as components of post-Golgi membranes that function as recycling carriers to the cell surface. In fibroblasts, SCAMPs are concentrated in compartments involved in the endocytosis and recycling of cell surface receptors while in neurons and other cell types having regulated transport pathways, SCAMPs are also components of regulated carriers (synaptic vesicles, secretion granules and transporter vesicles). Their presence in multiple pathways distinguishes them from proteins (e.g., recycling cell surface receptors and synaptic vesicle proteins) which are concentrated in selected pathways. The SCAMPs also do not appear to reside beyond the boundaries of these pathways. This distribution indicates that SCAMPs are general markers of membranes that function in cell surface recycling. Accordingly, polpeptides of the invention and antibodies thereto, may be used to identify membranes that function in cell surface recycling.

This gene is expressed primarily in hematopoietic cell types.

Therefore, polynucleotides and polypeptides of the invention are useful as reagents for differential identification of the tissue(s) or cell type(s) present in a biological sample and for diagnosis of diseases and conditions which include, but are not limited to, immune and hematopoetic disorders. Similarly, polypeptides and antibodies directed to these polypeptides are useful in providing immunological probes for differential identification of the tissue(s) or cell type(s). For a number of disorders of the above tissues or cells, particularly of the immune and hematopoetic systems, expression of this gene at significantly higher or lower levels may be routinely detected in certain tissues or cell types (e.g., hematopoietic cells, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:218 as residues: Ser-25 to Gly-31, Gln-149 to Ser-155.

The hematopoetic tissue distribution and homology to a cell surface molecule indicates polynucleotides and polypeptides corresponding to this gene are useful for the detection and/or treatment of immune or hematopoietic disorders including arthritis, asthma and immunodeficiency diseases. Many polynucleotide sequences, such as EST sequences, are publicly available and accessible through sequence databases. Some of these sequences are related to SEQ ID NO:79 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2441 of SEQ ID NO:79, b is an integer of 15 to 2455, where both a and b correspond to the positions of nucleotide residues shown in SEQ ID NO:79, and where b is greater than or equal to a + 14.

### FEATURES OF PROTEIN ENCODED BY GENE NO: 70

This gene maps to chromosome 4. Accordingly, polynucleotides of the invention may be used in linkage analysis as a marker for chromosome 4. When tested against a Jurkat T-cell line, supernatants removed from cells containing this gene activated the GAS (gamma activation site) pathway. Thus, it is likely that this gene activates T-cells through the Jaks-STAT signal transduction pathway. The GAS is a promoter element found upstream in many genes which are involved in the Jaks-STAT pathway. The Jaks-STAT pathway is a complex, signal transduction pathway involved in the differentiation and proliferation of cells. Therefore, activation of the Jaks-STATs pathway, reflected by the binding of the GAS element, can be used to indicate proteins involved in the proliferation and differentiation of cells.

This gene is expressed primarily in brain.

Therefore, polynucleotides and polypeptides of the invention are useful as reagents for differential identification of the tissue(s) or cell type(s) present in a biological sample and for diagnosis of diseases and conditions which include, but are not limited to, neurodegenerative conditions and behavioural disorders. Similarly, polypeptides and antibodies directed to these polypeptides are useful in providing immunological probes for differential identification of the tissue(s) or cell type(s). For a number of disorders of the above tissues or cells, particularly of the brain and central nervous system, expression of this gene at significantly higher or lower levels may be routinely detected in certain tissues and cell types (e.g., brain and other tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:219 as residues: Asp-57 to Gly-64.

The tissue distribution of this gene primarily in brain indicates that polynucleotides and polypeptides corresponding to this gene are useful for the treatement and/or detection of neurodegenerative disease states and behavioural disorders such as Alzheimers Disease, Parkinsons Disease, Huntingtons Disease, schizophrenia, mania, dementia, paranoia, obsessive compulsive disorder and panic disorder. Many polynucleotide sequences, such as EST sequences, are publicly available and accessible through sequence databases. Some of these sequences are related to SEQ ID NO:80 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 907 of SEQ ID NO:80, b is an integer of 15 to 921, where both a and b correspond to the positions of nucleotide residues shown in SEQ ID NO:80, and where b is greater than or equal to a + 14.

### FEATURES OF PROTEIN ENCODED BY GENE NO: 71

This gene is expressed primarily in hematopoietic cells.

Therefore, polynucleotides and polypeptides of the invention are useful as reagents for differential identification of the tissue(s) or cell type(s) present in a biological sample and for diagnosis of diseases and conditions which include, but are not limited to, autoimmune and immunodeficiency disease states. Similarly, polypeptides and antibodies directed to these polypeptides are useful in providing immunological probes for differential identification of the tissue(s) or cell type(s). For a number of disorders of the above tissues or cells, particularly of the hematopoetic system, expression of this gene at significantly higher or lower levels may be routinely detected in certain tissues and cell types (e.g., hematopoietic cells, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene predominantly in hematopoietic cell types indicates that polynucleotides and polypeptides corresponding to this gene are important for the treatment or detection of immune or hematopoietic disorders including arthritis, asthma, immunodeficiency diseases, leukemia, hypersensitivity and transplant rejection. Many polynucleotide sequences, such as EST sequences, are publicly available and accessible through sequence databases. Some of these sequences are related to SEQ ID NO:81 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 664 of SEQ ID NO:81, b is an integer of 15 to 678, where both a and b correspond to the positions of nucleotide residues shown in SEQ ID NO:81, and where b is greater than or equal to a + 14.

### FEATURES OF PROTEIN ENCODED BY GENE NO: 72

This gene is expressed primarily in hematopoietic cells.

Therefore, polynucleotides and polypeptides of the invention are useful as reagents for differential identification of the tissue(s) or cell type(s) present in a biological sample and for diagnosis of diseases and conditions which include, but are not limited to, auto-immune and immunodeficiency disease staes. Similarly, polypeptides and antibodies directed to these polypeptides are useful in providing immunological probes for differential identification of the tissue(s) or cell type(s). For a number of disorders of the above tissues or cells, particularly of the hematopoietic system, expression of this gene at significantly higher or lower levels may be routinely detected in certain tissues and cell types (e.g., hematopoietic cells, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene predominantly in hematopoietic cell types indicates that polynucleotides and polypeptides corresponding to this gene are important for the treatment or detection of immune or hematopoietic disorders including arthritis, asthma, immunodeficiency diseases, leukemia, and transplant rejection. Many polynucleotide sequences, such as EST sequences, are publicly available and accessible through sequence databases. Some of these sequences are related to SEQ ID NO:82 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 843 of SEQ ID NO:82, b is an integer of 15 to 857, where both a and b correspond to the positions of nucleotide residues shown in SEQ ID NO:82, and where b is greater than or equal to a + 14.

### FEATURES OF PROTEIN ENCODED BY GENE NO: 73

The translation product of this gene shares sequence homology with rat synaptogyrin which is thought to be important in membrane trafficking (see e.g., Stenius et al., J. Cell Biol. 131 (6 Pt 2), 1801-1809 (1995)). In specific embodiments, polypeptides of the invention comprise the sequence: QPYQVLPSRQVFALI (SEQ ID NO:361), VFSCI YGEGYSNAHESKQMYCVFN (SEQ ID NO:362), RNEDACRYGSAIGVLAFL (SEQ ID NO:363), LWDAYFPQISNATDRK (SEQ ID NO:364), and/or SALWTFLWFVGFC FLTNQWAVTNPK (SEQ ID NO:365). Polynucleotides encoding these polypeptides are also encompassed by the invention.

This gene is expressed primarily in breast and ovary and to a lesser extent in most hematopoietic tissue types.

Therefore, polynucleotides and polypeptides of the invention are useful as reagents for differential identification of the tissue(s) or cell type(s) present in a biological sample and for diagnosis of diseases and conditions which include, but are not limited to, female infertility and female reproductive abnormalities. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., mammary tissue, and ovary and other reproductive tissue, haematopoietic cells and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:222 as residues: Pro-9 to Trp-18, Thr-20 to Ala-27.

The tissue distribution in ovary and breast and homology to a protein involved in membrane trafficking indicates that polynucleotides and polypeptides corresponding to this gene play a role in the detection/treatment of female fertility disorders, endocrine disorders, ovarian failure, amenorrhea, ovarian cancer, and also potentially in both non-neoplastic breast diseases such as congenital abnormalities and neoplasia in the breast.. Many polynucleotide sequences, such as EST sequences, are publicly available and accessible through sequence databases. Some of these sequences are related to SEQ ID NO:83 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1963 of SEQ ID NO:83, b is an integer of 15 to 1977, where both a and b correspond to the positions of nucleotide residues shown in SEQ ID NO:83, and where b is greater than or equal to a + 14.

### FEATURES OF PROTEIN ENCODED BY GENE NO: 74

This gene maps to chromosome 12, and therefore polynucleotides of the invention may be used in linkage analysis as a marker for chromosome 12.

This gene is expressed primarily in brain and to a lesser extent in placenta and spleen.

Therefore, polynucleotides and polypeptides of the invention are useful as reagents for differential identification of the tissue(s) or cell type(s) present in a biological sample and for diagnosis of diseases and conditions which include, but are not limited to, behavioural disorders and neurodegerative disease states. Similarly, polypeptides and antibodies directed to these polypeptides are useful in providing immunological probes for differential identification of the tissue(s) or cell type(s). For a number of disorders of the above tissues or cells, particularly of the brain and central nervous system, expression of this gene at significantly higher or lower levels may be routinely detected in certain tissues (e.g., brain and other tissue of the nervous system, spleen and other cells and tissue of the immune system, placenta, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, schizophrenia, mania, dementia, paranoia, obsessive compulsive disorder and panic disorder. Many polynucleotide sequences, such as EST sequences, are publicly available and accessible through sequence databases. Some of these sequences are related to SEQ ID NO:84 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1135 of SEQ ID NO:84, b is an integer of 15 to 1149, where both a and b correspond to the positions of nucleotide residues shown in SEQ ID NO:84, and where b is greater than or equal to a + 14.

### FEATURES OF PROTEIN ENCODED BY GENE NO: 75

When tested against a K562 kidney cell line, supernatants removed from cells containing this gene activated the ISRE (interferon-sensitive responsive element) pathway. Thus, it is likely that this gene activates kidney cells through the Jaks-STAT signal transduction pathway. The ISRE is a promoter element found upstream in many genes which are involved in the Jaks-STAT pathway. The Jaks-STAT pathway is a complex, signal transduction pathway involved in the differentiation and proliferation of cells. Therefore, activation of the Jaks-STATs pathway, reflected by the binding of the GAS element, can be used to indicate proteins involved in the proliferation and differentiation of cells.

This gene is expressed primarily in bone marrow and spleen.

Therefore, polynucleotides and polypeptides of the invention are useful as reagents for differential identification of the tissue(s) or cell type(s) present in a biological sample and for diagnosis of diseases and conditions which include, but are not limited to, autoimmune diseases, transplant rejection and immundeficiency disease states. Similarly, polypeptides and antibodies directed to these polypeptides are useful in providing immunological probes for differential identification of the tissue(s) or cell type(s). For a number of disorders of the above tissues or cells, particularly of the hematopoetic and immune systems, expression of this gene at significantly higher or lower levels may be routinely detected in certain tissues (e.g., bone marrow, cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:224 as residues: Pro-22 to His-33, Ser-42 to Trp-48.

The tissue distribution of this gene predominantly in hematopoietic cell types indicates that polynucleotides and polypeptides corresponding to this gene are important for the treatment or detection of immune or hematopoietic disorders including arthritis, asthma, immunodeficiency diseases, leukemia, and transplant rejection. Many polynucleotide sequences, such as EST sequences, are publicly available and accessible through sequence databases. Some of these sequences are related to SEQ ED NO:85 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 753 of SEQ ID NO:85, b is an integer of 15 to 767, where both a and b correspond to the positions of nucleotide residues shown in SEQ ID NO:85, and where b is greater than or equal to a + 14.

### FEATURES OF PROTEIN ENCODED BY GENE NO: 76

In specific embodiments, polypeptides of the invention comprise the sequence: SLQYRIRIPGRPT (SEQ ID NO:366), DLVTYTSSLQYRIRIPGRPTRP (SEQ ID NO:367). VKTAECYSIPLGSCPVNIQRVR (SEQ ID NO:369), and/or LGNKKYIN IRCLEMQVTLKILCEIEKKERRGTHCLV (SEQ ID NO:368). Polynucleotides encoding these polypeptides are also encompassed by the invention.

This gene is expressed primarily in primary dendritic cells.

Therefore, polynucleotides and polypeptides of the invention are useful as reagents for differential identification of the tissue(s) or cell type(s) present in a biological sample and for diagnosis of diseases and conditions which include, but are not limited to, auto-immune disorders such as asthma and arthritis, in transplant rejection, leukemia and immunodeficiency disease states. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g.; dendritic cells and other cells and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:225 as residues: Gly-2 to Glu-7, Arg-27 to Gly-34.

The tissue distribution of this gene predominantly in hematopoietic cell types indicates that polynucleotides and polypeptides corresponding tot his gene are important for the treatment or detection of immune or hematopoietic disorders including arthritis, asthma, immunodeficiency diseases, leukemia, hypersensitivity and graft rejection. Many polynucleotide sequences, such as EST sequences, are publicly available and accessible through sequence databases. Some of these sequences are related to SEQ ID NO:86 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 714 of SEQ ID NO:86, b is an integer of 15 to 728, where both a and b correspond to the positions of nucleotide residues shown in SEQ ID NO:86, and where b is greater than or equal to a + 14.

### FEATURES OF PROTEIN ENCODED BY GENE NO: 77

This gene is expressed primarily in 12 week old early stage human.

Therefore, polynucleotides and polypeptides of the invention are useful as reagents for differential identification of the tissue(s) or cell type(s) present in a biological sample and for diagnosis of diseases and conditions which include, but are not limited to, developmental abnormalities. Similarly, polypeptides and antibodies directed to these polypeptides are useful in providing immunological probes for differential identification of the tissue(s) or cell type(s). For a number of disorders of the above tissues or cells, particularly of the developmental system, expression of this gene at significantly higher or lower levels may be routinely detected in certain tissues (e.g., developing and/or differentiating cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:226 as residues: Thr-14 to Thr-19.

The expression of this gene primarily in the embryo, indicates a key role in embryo development and could be used in the treatment and or detection of developmental disorders. Many polynucleotide sequences, such as EST sequences, are publicly available and accessible through sequence databases. Some of these sequences are related to SEQ ID NO:87 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 721 of SEQ ID NO: 87, b is an integer of 15 to 735, where both a and b correspond to the positions of nucleotide residues shown in SEQ ID NO:87, and where b is greater than or equal to a + 14.

### FEATURES OF PROTEIN ENCODED BY GENE NO: 78

This gene is expressed primarily in T-cells, and to a lesser extent in cord blood and osteosarcoma.

Therefore, polynucleotides and polypeptides of the invention are useful as reagents for differential identification of the tissue(s) or cell type(s) present in a biological sample and for diagnosis of diseases and conditions which include, but are not limited to, auto-immune diseases, immunodeficiency diseases and host-graft rejection. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell tpyes (e.g., cells and tissue of the immune system, and bone, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:227 as residues: Pro-36 to Ala-41.

The expression of this gene in T-cells indicates a potential role in the treament/detection of immune disorders such as such as arthritis, asthma, immune deficiency diseases such as AIDS, leukemia and transplant rejection. Many polynucleotide sequences, such as EST sequences, are publicly available and accessible through sequence databases. Some of these sequences are related to SEQ ID NO:88 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 875 of SEQ ID NO:88, b is an integer of 15 to 889, where both a and b correspond to the positions of nucleotide residues shown in SEQ ID NO:88, and where b is greater than or equal to a + 14.

### FEATURES OF PROTEIN ENCODED BY GENE NO: 79

This gene is expressed primarily in placenta and 9 week old embryo and to a lesser extent in fetal spleen.

Therefore, polynucleotides and polypeptides of the invention are useful as reagents for differential identification of the tissue(s) or cell type(s) present in a biological sample and for diagnosis of diseases and conditions which include, but are not limited to, developmental disorders. Similarly, polypeptides and antibodies directed to these polypeptides are useful in providing immunological probes for differential identification of the tissue(s) or cell type(s). For a number of disorders of the above tissues or cells, particularly of the developmental system, expression of this gene at significantly higher or lower levels may be routinely detected in certain tissues (e.g., developing and differentiating tissues, and spleen and other cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The expression of this gene primarily in the embryo, indicates a key role in embryo development and could be used in the treatment and or detection of developmental disorders.Many polynucleotide sequences, such as EST sequences, are publicly available and accessible through sequence databases. Some of these sequences are related to SEQ ID NO:89 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 555 of SEQ ID NO:89, b is an integer of 15 to 569, where both a and b correspond to the positions of nucleotide residues shown in SEQ ID NO:89, and where b is greater than or equal to a + 14.

### FEATURES OF PROTEIN ENCODED BY GENE NO: 80

This gene is expressed primarily in early stage brain.

Therefore, polynucleotides and polypeptides of the invention are useful as reagents for differential identification of the tissue(s) or cell type(s) present in a biological sample and for diagnosis of diseases and conditions which include, but are not limited to, developmental and neurodegenerative diseases of the brain and nervous system. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues (e.g., brain and other tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and detection of developmental and neurodegenerative diseases, as well as behavioral or nervous system disorders. Examples of such conditions would include: depression, schizophrenia, mania, dementia, paranoia, addictive behavior and sleep disorders. In addition a brain-specific gene product may be useful in the diagnosis of specific brain tumors. Many polynucleotide sequences, such as EST sequences, are publicly available and accessible through sequence databases. Some of these sequences are related to SEQ ID NO:90 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 320 of SEQ ID NO:90, b is an integer of 15 to 334, where both a and b correspond to the positions of nucleotide residues shown in SEQ ID NO:90, and where b is greater than or equal to a + 14.

### FEATURES OF PROTEIN ENCODED BY GENE NO: 81

This gene is expressed primarily in synovial tissue.

Therefore, polynucleotides and polypeptides of the invention are useful as reagents for differential identification of the tissue(s) or cell type(s) present in a biological sample and for diagnosis of diseases and conditions which include, but are not limited to, arthritis, tendonitis and chrondomalacia. Similarly, polypeptides and antibodies directed to these polypeptides are useful in providing immunological probes for differential identification of the tissue(s) or cell type(s). For a number of disorders of the above tissues or cells, particularly of the synovium, expression of this gene at significantly higher or lower levels may be routinely detected in certain tissues (e.g., synovial tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of connective tissue disorders such as arthritis, tendonitis, chrondomalacia, inflammation and trauma.Many polynucleotide sequences, such as EST sequences, are publicly available and accessible through sequence databases. Some of these sequences are related to SEQ ID NO:91 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 781 of SEQ ID NO:91, b is an integer of 15 to 795, where both a and b correspond to the positions of nucleotide residues shown in SEQ ID NO:91, and where b is greater than or equal to a + 14.

### FEATURES OF PROTEIN ENCODED BY GENE NO: 82

This gene is expressed primarily in frontal cortex of brain.

Therefore, polynucleotides and polypeptides of the invention are useful as reagents for differential identification of the tissue(s) or cell type(s) present in a biological sample and for diagnosis of diseases and conditions which include, but are not limited to, developmental and neurodegenerative diseases of the brain. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., brain and other tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:231 as residues: Ser-4 to Tyr-13.

The tissue distribution indicates that polynucleotides and polypeptides corresponding to this gene are useful for the detection and treatment of developmental and neurodegenerative diseases of the brain and nervous system, including malignancies as well as behavioral disorders. Examples of such conditions might include: depression, schizophrenia, Alzheimer's disease, Parkinson's disease, Huntington's disease, mania, dementia, paranoia, addictive behavior and sleep disorders.Many polynucleotide sequences, such as EST sequences, are publicly available and accessible through sequence databases. Some of these sequences are related to SEQ ID NO:92 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 563 of SEQ ID NO:92, b is an integer of 15 to 577, where both a and b correspond to the positions of nucleotide residues shown in SEQ ID NO:92, and where b is greater than or equal to a + 14.

### FEATURES OF PROTEIN ENCODED BY GENE NO: 83

The translation product of this gene shares sequence homology with the L6 cell surface antigen, which is highly expressed in lung, breast, colon, and ovarian carcinomas. See e.g., Marken et al., Proc Natl Acad Sci U S A 1992 Apr 15;89(8):3503-3507. In a specific embodiment, polypeptides of the invention comprise the sequence: ITLCLVCIVANA (SEQ ID NO:370). Polynucleotides encoding these polypeptides are also encompassed by the invention.

This gene is expressed primarily in tissues of liver origin (fetal liver, hepatoma, hepatocellular tumor).

Therefore, polynucleotides and polypeptides of the invention are useful as reagents for differential identification of the tissue(s) or cell type(s) present in a biological sample and for diagnosis of diseases and conditions which include, but are not limited to, cancers of the liver. Similarly, polypeptides and antibodies directed to these polypeptides are useful in providing immunological probes for differential identification of the tissue(s) or cell type(s). For a number of disorders of the above tissues or cells, particularly of the hepatic system, expression of this gene at significantly higher or lower levels may be routinely detected in certain tissues (e.g., lung and pulmonary tissue, colon and other gastrointestinal tissue, mammary tissue, ovarian tissue and other tissue of the reproductive system, and hepatic tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:232 as residues: Asn-32 to Asn-41, Thr-140 to Ala-147, Asp-188 to His-197.

The murine monoclonal antibody (mAb) L6 recognizes an integral membrane glycoprotein that is highly expressed in lung, breast, colon, and ovarian carcinomas and is referred to as the L6 antigen. This antigen is an attractive target for therapeutic intervention due to its high level expression on malignant cells. The tissue distribution and homology to L6 antigen indicates that the protein product of this gene is useful for detection and treatment of neoplastic tissues - particularly of the liver. Many polynucleotide sequences, such as EST sequences, are publicly available and accessible through sequence databases. Some of these sequences are related to SEQ ID NO:93 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 954 of SEQ ID NO:93, b is an integer of 15 to 968, where both a and b correspond to the positions of nucleotide residues shown in SEQ ID NO:93, and where b is greater than or equal to a + 14.

### FEATURES OF PROTEIN ENCODED BY GENE NO: 84

This gene is expressed primarily in glioblastoma.

Therefore, polynucleotides and polypeptides of the invention are useful as reagents for differential identification of the tissue(s) or cell type(s) present in a biological sample and for diagnosis of diseases and conditions which include, but are not limited to, glioblastoma. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cells and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection and treatment of malignancies, as well as developmental and neurodegenerative diseases of the brain and nervous system. Many polynucleotide sequences, such as EST sequences, are publicly available and accessible through sequence databases. Some of these sequences are related to SEQ ID NO:94 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 539 of SEQ ID NO:94, b is an integer of 15 to 553, where both a and b correspond to the positions of nucleotide residues shown in SEQ ID NO:94, and where b is greater than or equal to a + 14.

### FEATURES OF PROTEIN ENCODED BY GENE NO: 85

The translation product of this gene shares sequence homology with Tbx which is thought to be important in developmental regulation (see e.g., Knezevic et al., Development 124: 411-419 (1997); accession No. U80951). In specific embodiments, polypeptides of the invention comprise the sequence:VTAYQNQQITRLKIDRNPFAKGFR (SEQ ID NO:371), GTATVTAYQ NQQITRL (SEQ ID NO:372), KIDRNPFAKGFRDSGRNRMGLEAL (SEQ ID NO:373), VESYAFWRPSLRTLTFEDIPGIPKQGNASS (SEQ ID NO:375), and/or STLLQVLGMAFLPLTLTFCLA (SEQ ID NO:374). Polynucleotides encoding these polypeptides are also encompassed by the invention.

This gene is expressed primarily in synovial sarcoma and to a lesser extent in osteoclastoma and hemangiopericytoma.

Therefore, polynucleotides and polypeptides of the invention are useful as reagents for differential identification of the tissue(s) or cell type(s) present in a biological sample and for diagnosis of diseases and conditions which include, but are not limited to, osteosarcoma, osteoclastoma, chondrosarcoma. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues and cell types (e.g., bone cells and tissue, and synovial cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:234 as residues: Ala-45 to Asp-50, Arg-57 to Pro-63.

The tissue distribution indicates that polynucleotides and polypeptides corresponding to this gene are useful for the treatment and diagnosis of osteoperosis, fracture, osteosarcoma, osteoclastoma, chondrosarcoma, ossification and osteonecrosis, arthritis, tendonitis, chrondomalacia, inflammation.Many polynucleotide sequences, such as EST sequences, are publicly available and accessible through sequence databases. Some of these sequences are related to SEQ ID NO:95 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 954 of SEQ ID NO:95, b is an integer of 15 to 968, where both a and b correspond to the positions of nucleotide residues shown in SEQ ID NO:95, and where b is greater than or equal to a + 14.

### FEATURES OF PROTEIN ENCODED BY GENE NO: 86

This gene maps to chromosome 19, accordingly, polynucleotides of the invention may be used in linkage analysis as a marker for chromosome 19. The translation product of this gene is a transmembrane protein that forms disulfide-bonded homodimers and contains a motif in its cytoplasmic domain (located at the carboxy terminus of the protein relative to the transmembrane domain) that functions as an adaptor for associating protein complexes involved in triggering cellular activation. The transmembrane domain is predicted to consist of the amino acid sequence:
VLAGIVMGDLVLTVLIALAVYFLG (SEQ ID NO:377). In specific embodiments, polypeptides of the invention comprise, or alternatively, consist of, the sequence: QAQSDCSCSTVSPG (SEQ ID NO:376), VLAGIVMGDLVLTVLIALA VYFLG (SEQ ID NO:377), VPRGRGAAEATRKQRITETESPYQELQGQRSDVYSDL (SEQ ID NO:378), and/or ETESPYQELQGQRSDVYSDLNT (SEQ ID NO:379). Polynculeotides encoding these polypeptides are also encompassed by the invention.

This gene is expressed primarily in macrophages.

Therefore, polynucleotides and polypeptides of the invention are useful as reagents for differential identification of the tissue(s) or cell type(s) present in a biological sample and for diagnosis of diseases and conditions which include, but are not limited to, immunologically mediated disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:235 as residues: Ala-28 to Ser-33, Ala-76 to Lys-111.

The tissue distribution indicates that polynucleotides and polypeptides corresponding to this gene are useful for the diagnosis and treatment of immune disorders including: leukemias, lymphomas, auto-immunities, immunodeficiencies (e.g., AIDS), immuno-supressive conditions (transplantation) and hematopoeitic disorders. Many polynucleotide sequences, such as EST sequences, are publicly available and accessible through sequence databases. Some of these sequences are related to SEQ ID NO:96 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 683 of SEQ ID NO:96, b is an integer of 15 to 697, where both a and b correspond to the positions of nucleotide residues shown in SEQ ID NO:96, and where b is greater than or equal to a + 14.

### FEATURES OF PROTEIN ENCODED BY GENE NO: 87

This gene is expressed primarily in prostate cancer.

Therefore, polynucleotides and polypeptides of the invention are useful as reagents for differential identification of the tissue(s) or cell type(s) present in a biological sample and for diagnosis of diseases and conditions which include, but are not limited to, prostate cancer. Similarly, polypeptides and antibodies directed to these polypeptides are useful in providing immunological probes for differential identification of the tissue(s) or cell type(s). For a number of disorders of the above tissues or cells, particularly of the prostate, expression of this gene at significantly higher or lower levels may be routinely detected in certain tissues (e.g., prostate, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for detection and treatment of prostate cancer and other prostate disorders. Many polynucleotide sequences, such as EST sequences, are publicly available and accessible through sequence databases. Some of these sequences are related to SEQ ID NO:97 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 852 of SEQ ID NO:97, b is an integer of 15 to 866, where both a and b correspond to the positions of nucleotide residues shown in SEQ ID NO:97, and where b is greater than or equal to a + 14.

### FEATURES OF PROTEIN ENCODED BY GENE NO: 88

The translation product of this gene shares sequence homology with retinal epithelial membrane protein (REMP), which is thought to be important in development and maintenance of normal retinal function (See e.g., Philp et al., Exp. Cell Res. 219 (1), 64-73 (1995); and accesion no.U 15685). The translation product of this gene also shares homology with monocarboxylate transporter protein (accesion no.U87627). In specific embodiments, polypeptides of the invention comprise, or alternatively, consist of, the sequence: FLCALSPLGQLLQDRYGWRGGFLILGGL (SEQ ID NO:380), LLNCCVCAALMRPLVVTAQPGXGPPRP (SEQ ID NO:381), and/or SRRLXDLSV FRDRGFVLYAVAASVM (SEQ ID NO:382). Polynucleotides encoding these polypeptides are also encompassed by the invention.

This gene is expressed primarily in neutrophils and to a lesser extent in a variety of other tissues and cell types, including retina.

Therefore, polynucleotides and polypeptides of the invention are useful as reagents for differential identification of the tissue(s) or cell type(s) present in a biological sample and for diagnosis of diseases and conditions which include, but are not limited to, eye disorders. Similarly, polypeptides and antibodies directed to these polypeptides are useful in providing immunological probes for differential identification of the tissue(s) or cell type(s). For a number of disorders of the above tissues or cells, particularly of the eye, expression of this gene at significantly higher or lower levels may be routinely detected in certain tissues and cell types (e.g., retinal cells and other cells and tissue of the nervous system, neutrophils and other blood cells, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to REMP indicates polynucleotides and polypeptides corresponding to this gene are useful for the diagnosis and treatment of eye disorders, including neoplasms, visual impairments and blindness. Many polynucleotide sequences, such as EST sequences, are publicly available and accessible through sequence databases. Some of these sequences are related to SEQ ID NO:98 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1354 of SEQ ID NO:98, b is an integer of 15 to 1368, where both a and b correspond to the positions of nucleotide residues shown in SEQ ID NO:98, and where b is greater than or equal to a + 14.

### FEATURES OF PROTEIN ENCODED BY GENE NO: 89

The translation product of this gene shares sequence homology with human squamous cell E48 antigen which is thought to be important in self-recognition, immune function. Additional embodiments of the invention are directed to polypeptides comprising the sequence:
MMATPSTRPPPPAASTTSATAPALPPRPPWPWPPSSWPPSGVSSKAPEADPLK NKAL (SEQ ID NO:383); LLLTSPLPRCPPACSHDAPAHPDPGGPHGLTSGPGLG LPRVCLQRRQLLQPHALPGYGCLLHDHAHLLHPHQDEGQ (SEQ ID NO:384); and/or WLLQARVHHLLLPVRPLQRHRPCHPGHPGPGPHPPGHPLGSPLKPP RQTHSRTKLS (SEQ ID NO:385). Further embodiments of the invention include polynucleotides encoding these polypeptides.

This gene is expressed primarily in adult brain and to a lesser extent in fetal lung.

Therefore, polynucleotides and polypeptides of the invention are useful as reagents for differential identification of the tissue(s) or cell type(s) present in a biological sample and for diagnosis of diseases and conditions which include, but are not limited to, autoimmune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., brain and other cells and tissue of the immunce system, lung, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:238 as residues: Tyr-28 to Phe-34, Thr-54 to Val-60, Tyr-73 to Thr-82.

The tissue distribution and homology to human squamous cell E48 antigen indicates that the polynucleotides and polypeptides corresponding to this gene are useful for study, diagnosis and treatment of autoimmune diseases and disorders. Many polynucleotide sequences, such as EST sequences, are publicly available and accessible through sequence databases. Some of these sequences are related to SEQ ID NO:99 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 599 of SEQ ID NO:99, b is an integer of 15 to 613, where both a and b correspond to the positions of nucleotide residues shown in SEQ ID NO:99, and where b is greater than or equal to a + 14.

### FEATURES OF PROTEIN ENCODED BY GENE NO: 90

Additional embodiments of the invention include polypeptides comprising of the following amino acid sequences:
QEFQTGLGNMVKPCLYEKYRNISWLWWHTPVVPATWEAEVGGSLEPGRLRLQ (SEQ ID NO:386) and/or ILGGESILILSWVFSYIFFRIALEITIYILNVSPFCLG RWLM PVIPALWEAEVGGLPELRSSRPA (SEQ ID NO:387). Further embodiments include polynucleotides encoding these polypeptides.

This gene is expressed primarily in human adult lymph node tissue.

Therefore, polynucleotides and polypeptides of the invention are useful as reagents for differential identification of the tissue(s) or cell type(s) present in a biological sample and for diagnosis of diseases and conditions which include, but are not limited to, immune disorders and lymphomas. Similarly, polypeptides and antibodies directed to these polypeptides are useful in providing immunological probes for differential identification of the tissue(s) or cell type(s). For a number of disorders of the above tissues or cells, particularly of the immune and metabolic systems, expression of this gene at significantly higher or lower levels may be routinely detected in certain tissues (e.g., lymph tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis and treatment of immune and lymph diseases and disorders such as lymphomas. Many polynucleotide sequences, such as EST sequences, are publicly available and accessible through sequence databases. Some of these sequences are related to SEQ ID NO: 100 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 671 of SEQ ID NO: 100, b is an integer of 15 to 685, where both a and b correspond to the positions of nucleotide residues shown in SEQ ID NO: 100, and where b is greater than or equal to a + 14.

### FEATURES OF PROTEIN ENCODED BY GENE NO: 91

Additional embodiments of the invention include polypeptides comprising of the following amino acid sequences: MPKQLAQLLYRLPRG (SEQ ID NO:388); LFQAIS VSGSHRQGSRTWNTLTEGNAEAACTVALQTSKRLILASRW (SEQ ID NO:389); TLSFMNSHCVPIKALFFLSWSYIMPHHIFFTVKILKSCFQVGQLMKL (SEQ ID NO:390); and/or RPTRPITFSSNISEWVPSTGFQDLEHFNRRKCRSS LHSCFTDFQEADSGFKMEPWSWFFFFFFFFPQRTCGCALCVLFLFSIW GPHGKELLNSFLYELPLCSYKGPFLS (SEQ ID NO:391). Further embodiments of the invention are directed to polynucleotides which encode these polypeptides.

This gene is expressed primarily in placenta and synovium.

Therefore, polynucleotides and polypeptides of the invention are useful as reagents for differential identification of the tissue(s) or cell type(s) present in a biological sample and for diagnosis of diseases of the synovium and placenta. Similarly, polypeptides and antibodies directed to these polypeptides are useful in providing immunological probes for differential identification of the tissue(s) or cell type(s). For a number of disorders of the above tissues or cells, particularly of the placenta and synovium, expression of this gene at significantly higher or lower levels may be routinely detected in certain tissues (e.g., synovial tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis and treatment of growth and development disorders and arthritic and inflammatory conditions.Many polynucleotide sequences, such as EST sequences, are publicly available and accessible through sequence databases. Some of these sequences are related to SEQ ID NO:101 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 632 of SEQ ID NO:101, b is an integer of 15 to 646, where both a and b correspond to the positions of nucleotide residues shown in SEQ ID NO:101, and where b is greater than or equal to a + 14.

### FEATURES OF PROTEIN ENCODED BY GENE NO: 92

Additional embodiments of the invention include polypeptides comprising the following amino acid sequences:
VDPRVRLPLFWWQPSCAVYLFPRVYNNMCTRVLGTLPHCWDLATLLQPSSRI WGNVSEAPGM (SEQ ID NO:392); VPYHIAGTLPHCCSLPVGYGGMSVRL QGCRYVGNVGPQGNMQSGRSWALKMVLLCNSCLGLGVGSVGPSMSSLF GAVLSETPGSSVY (SEQ ID NO:393); and/or MLDPRATCNLVGVGLS KWCCCVTAAVWLG (SEQ ID NO:394). Further embodiments of the invention include polynucleotides which encode these polypeptides.

This gene is expressed primarily in chronic lymphocytic leukemia.

Therefore, polynucleotides and polypeptides of the invention are useful as reagents for differential identification of the tissue(s) or cell type(s) present in a biological sample and for diagnosis of diseases of immune system including cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis and treatment of disorders of the immune system including cancers.Many polynucleotide sequences, such as EST sequences, are publicly available and accessible through sequence databases. Some of these sequences are related to SEQ ID NO: 102 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 812 of SEQ ID NO: 102, b is an integer of 15 to 826, where both a and b correspond to the positions of nucleotide residues shown in SEQ ID NO: 102, and where b is greater than or equal to a + 14.

### FEATURES OF PROTEIN ENCODED BY GENE NO: 93

Additional embodiments of the invention include polypeptides comprising of the following amino acid sequences:
HGDWIYVHIVEQLNQANNKSVTSHTYFVVKTCKIHSLSNFQASNTLLXTVVTM LYNRSLELILPV (SEQ ID NO:395); TYSSCLTKILYSLINIYPIPHCSPAXITTIL LSASMNLTFFFFRFHICEIAQYLSFCAWLISLNIKSL (SEQ ID NO:396); and/or MNLTFFFFRFHICEIAQYLSFCAWLISLNIKSL (SEQ ID NO:397). Further embodimnents include polynucleotides which encode these polypeptides.

This gene is expressed primarily in brain medulloblastoma.

Therefore, polynucleotides and polypeptides of the invention are useful as reagents for differential identification of the tissue(s) or cell type(s) present in a biological sample and for diagnosis of cancer and disorders of the CNS. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., brain and other cells and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of cancers and other disorders and diseases of the CNS. Many polynucleotide sequences, such as EST sequences, are publicly available and accessible through sequence databases. Some of these sequences are related to SEQ ID NO: 103 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 572 of SEQ ID NO: 103, b is an integer of 15 to 586, where both a and b correspond to the positions of nucleotide residues shown in SEQ ID NO:103, and where b is greater than or equal to a + 14.

### FEATURES OF PROTEIN ENCODED BY GENE NO: 94

Additional embodiments of the invention include polypeptides comprising the following amino acid sequences:
LVCYCSTKKEKKLHEIAIQQGQNWRWLLFYKEISVPGFQSVWCSYKCLCVVW KAGEGG (SEQ ID NO:398); RRSCSGPPLVNTAGKILSSSPAKLACKRTDFHIPSI (SEQ ID NO:399); RASILGIDNERGCHFRHFNPLKEYKRKKKENKSFRIV (SEQ ID NO:400); SKNKTRGGDWCVTVLRKRRKSFMKSPFSKDRTGDGFSFTKKS LSQAFSLFGVHTSVCVLCGRRGKAGEGGPVQGPLW (SEQ ID NO:401); and/or MKSPFSKDRTGDGFSFTKKSLSQAFSLFGVHTSVCVLCGRRGKAGEGGPVQG PLW (SEQ ID NO:402). Further embodiments include polynucleotides comprising these amino acid sequences.

This gene is expressed primarily in meningima and neutrophils and to a lesser extent in anergic T cells and CD34 depleted buffy coat.

Therefore, polynucleotides and polypeptides of the invention are useful as reagents for differential identification of the tissue(s) or cell type(s) present in a biological sample and for diagnosis of diseases and conditions which include, but are not limited to, inflammatory, immune and hemopoietic disorders. Similarly, polypeptides and antibodies directed to these polypeptides are useful in providing immunological probes for differential identification of the tissue(s) or cell type(s). For a number of disorders of the above tissues or cells, particularly of the hemopoietic, immune and inflammatory systems, expression of this gene at significantly higher or lower levels may be routinely detected in certain tissues (e.g., T-cells and other cells and tissue of the immune system, meningima, neutrophils and other blood cells, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:243 as residues: Glu-45 to Asn-50.

The tissue distribution indicates that polynucleotides and polypeptides corresponding to this gene are useful for the study, diagnosis and treatment of various disorders and diseases of the immune, inflammatory, and hemopoietic systems. Many polynucleotide sequences, such as EST sequences, are publicly available and accessible through sequence databases. Some of these sequences are related to SEQ ID NO:104 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 614 of SEQ ID NO: 104, b is an integer of 15 to 628, where both a and b correspond to the positions of nucleotide residues shown in SEQ ID NO: 104, and where b is greater than or equal to a + 14.

### FEATURES OF PROTEIN ENCODED BY GENE NO: 95

Additional embodiments of the invention include polypeptides comprising the following amino acid sequences: MGESECYRRLSGASCTWTVHVDFA (SEQ ID NO:403); MHCGTRVWKTMKHDYFLLACLSMTSTGGILCTL (SEQ ID NO:404); STLSLIPTSSSLSFWPWCTAIIGSIFTYCVCVCVCFVVMNRTCYLPNSIIYHNSKL ATIIDKSMTLS (SEQ ID NO:405); and/or MWILPKVSLICIVELGYGKP (SEQ ID NO:406). Further embodiments include polynucleotides encoding these polypeptides.

This gene is expressed primarily in human meningima.

Therefore, polynucleotides and polypeptides of the invention are useful as reagents for differential identification of the tissue(s) or cell type(s) present in a biological sample and for diagnosis of diseases and conditions which include, but are not limited to, meningitis and other inflammatory conditions. Similarly, polypeptides and antibodies directed to these polypeptides are useful in providing immunological probes for differential identification of the tissue(s) or cell type(s). For a number of disorders of the above tissues or cells, particularly of the cerebrospinal membranes, expression of this gene at significantly higher or lower levels may be routinely detected in certain tissues (e.g., meningima, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:244 as residues: Ser-35 to Phe-41.

The tissue distribution indicates that polynucleotides and polypeptides corresponding to this gene are useful for study, treatment, and diagnosis of disorders of the meningima. Many polynucleotide sequences, such as EST sequences, are publicly available and accessible through sequence databases. Some of these sequences are related to SEQ ID NO: 105 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 544 of SEQ ID NO: 105, b is an integer of 15 to 558, where both a and b correspond to the positions of nucleotide residues shown in SEQ ID NO: 105, and where b is greater than or equal to a + 14.

### FEATURES OF PROTEIN ENCODED BY GENE NO: 96

Additional embodiments of the invention are directed to polypeptides comprising the following amino acid sequences:
MSTGDGRDAEKGWPVSEEENQRSVYPGYPECDERQAVPQHCAIASPSSLQSHH PASACVPRR (SEQ ID NO:407); QQMTLGTKIKWGQLQRGQEIPTGDFTVR NFMRFSI IYC (SEQ ID NO:408); and/or PFLFCASRIRXQGIGIHGQ VACSAVRMYNNR (SEQ ID NO:409). Further embodiments of the invention are directed to polynucleotides which encode these polypeptides.

This gene is expressed primarily in activated monocytes.

Therefore, polynucleotides and polypeptides of the invention are useful as reagents for differential identification of the tissue(s) or cell type(s) present in a biological sample and for diagnosis of diseases and conditions which include, but are not limited to, immune and hematopoietic disorders. Similarly, polypeptides and antibodies directed to these polypeptides are useful in providing immunological probes for differential identification of the tissue(s) or cell type(s). For a number of disorders of the above tissues or cells, particularly of the immune and hemopoietic systems, expression of this gene at significantly higher or lower levels may be routinely detected in certain tissues (e.g., monocytes and other cells and tissue of the immune system. and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:245 as residues: Met-1 to Ser-6, Pro-29 to Ser-34.

The tissue distribution indicates that polynucleotides and polypeptides corresponding to this gene are useful for study, diagnosis and treatment of diseases of the immune and hemopoietic systems. Many polynucleotide sequences, such as EST sequences, are publicly available and accessible through sequence databases. Some of these sequences are related to SEQ ID NO: 106 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 742 of SEQ ID NO:106, b is an integer of 15 to 756, where both a and b correspond to the positions of nucleotide residues shown in SEQ ID NO: 106, and where b is greater than or equal to a + 14.

### FEATURES OF PROTEIN ENCODED BY GENE NO: 97

Additional embodiments of the invention are directed to polypeptides comprising the following amino acid sequences:
VLCEEAGQKVPSTPSWSSWTLQKRLRGSPAEANCSPSFPAPPGKE (SEQ ID NO:410); MSLSALACDFT PIQPWEWEEYEQITLGLTAPSNLLESNYLGQASECFV RKLVRRFPQLLPGPPGHCRKDLGDPQQRPIALLPSLPHQERNNVHRLEADSEV DL (SEQ ID NO:411); CVDFDEYFSSWEPLLKMMFKGVVGGKMKAWRRKKR RKPLPYKIHAD (SEQ ID NO:412); and/or MMFKGWGGKMKAWRRK KRRKPLPYKIHAD (SEQ ID NO:413). Further embodiments of the invention are directed to polynucleotides which encode these polypeptides.

This gene is expressed primarily in bone marrow and to a lesser extent in testes.

Therefore, polynucleotides and polypeptides of the invention are useful as reagents for differential identification of the tissue(s) or cell type(s) present in a biological sample and for diagnosis of diseases and conditions which include, but are not limited to, hemopoietic and reproductive disorders. Similarly, polypeptides and antibodies directed to these polypeptides are useful in providing immunological probes for differential identification of the tissue(s) or cell type(s). For a number of disorders of the above tissues or cells, particularly of the hemopoietic and reproductive systems, expression of this gene at significantly higher or lower levels may be routinely detected in certain tissues (e.g., bone marrow, testes and other reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis and treatment of various disorders involving the hemopoietic and reproductive systems. Many polynucleotide sequences, such as EST sequences, are publicly available and accessible through sequence databases. Some of these sequences are related to SEQ ID NO: 107 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1132 of SEQ ID NO: 107, b is an integer of 15 to 1146, where both a and b correspond to the positions of nucleotide residues shown in SEQ ID NO: 107, and where b is greater than or equal to a + 14.

### FEATURES OF PROTEIN ENCODED BY GENE NO: 98

Additional embodiments of the invention are directed to polypeptides comprising the following amino acid sequences:
LISSVNKTKQKRSDATLSHKHDRLLNHFVFFGNSYNY (SEQ ID NO:414); SSKFPS
DMLLRIQQIIYCHKLTIILTKWRNTARHKSKKKEDELILKHELQLKKWKNRLIL KRAAAEESNFPERSSSEVFLVDETLKCDISLLPEXAILQVCMNSVYHYYNLPSW VHACNPSCLGG (SEQ ID NO:415); SLESTNAIKSN (SEQ ID NO:416); IRPNKNDQMRHCLINMIDY (SEQ ID NO:417); ITLCFLETAITINIYSNL VNFLQICYCGYNRSSIVTS (SEQ ID NO:418); and/or ISFRYAIADTTDHLLSQA NHYPNKMAEYSKT (SEQ ID NO:419). Further embodiments of the invention are directed to polynucleotides which encode these polypeptides.

This gene is expressed primarily in nasal polyps.

Therefore, polynucleotides and polypeptides of the invention are useful as reagents for differential identification of the tissue(s) or cell type(s) present in a biological sample and for diagnosis of immune system disorders. Similarly, polypeptides and antibodies directed to these polypeptides are useful in providing immunological probes for differential identification of the tissue(s) or cell type(s). For a number of disorders of the above tissues or cells, particularly of the immune and respiratory systems, expression of this gene at significantly higher or lower levels may be routinely detected in certain tissues (e.g., nasal tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of disorders of the immune system. Many polynucleotide sequences, such as EST sequences, are publicly available and accessible through sequence databases. Some of these sequences are related to SEQ ID NO: 108 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 761 of SEQ ID NO:108, b is an integer of 15 to 775, where both a and b correspond to the positions of nucleotide residues shown in SEQ ID NO: 108, and where b is greater than or equal to a + 14.

### FEATURES OF PROTEIN ENCODED BY GENE NO: 99

An additional embodiment of the invention is directed to polypeptides comprising those which exhibit sequence homology with honeybee venom sacepin. Further embodiments of the invention are directed to polynucleotides which encode these polypeptides. Additional embodiments of the invention are directed to polypeptides comprising the following amino acid sequences:
PQIKLLNSDALGMRTTSXDLVPCNQCFIPLPPSCNRIASRKAVNWKQQRLPAVR GLLNNAPHRRPPTPRTPCVFPSEGPKGYGFHV (SEQ ID NO:420); EQLAXISCR VINVSFRCLHHVIESLPERQLTGSSRGSQP (SEQ ID NO:421); EDCSTMPPI AAPPPLAPLVFSPLRGPRVMAFMSRCGDRGGRGRSXAGRGWPWSESGVINAH PKKRPCPGPMLS (SEQ ID NO:422); and/or EFGTRRQWGTR CFPPLVGRKQ SALRRREGKARAGRCCGKRSVKAGFDA (SEQ ID NO:423). Further embodiments of the invention are directed to polynucleotides which encode these polypeptides.

This gene is expressed primarily in activated and control neutrophils and to a lesser extent in fetal liver and spleen.

Therefore, polynucleotides and polypeptides of the invention are useful as reagents for differential identification of the tissue(s) or cell type(s) present in a biological sample and for diagnosis of disorders of the immune and endocrine systems. Similarly, polypeptides and antibodies directed to these polypeptides are useful in providing immunological probes for differential identification of the tissue(s) or cell type(s). For a number of disorders of the above tissues or cells, particularly of the immune. inflammatory and hormonal systems, expression of this gene at significantly higher or lower levels may be routinely detected in certain tissues (e.g., neutrophils and other blood cells, spleen and other cells and tissue of the immune system, liver, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study, diagnosis and treatment of inflammation and various disorders of the immune and endocrine systems. Many polynucleotide sequences, such as EST sequences, are publicly available and accessible through sequence databases. Some of these sequences are related to SEQ ID NO:109 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 897 of SEQ ID NO: 109, b is an integer of 15 to 911, where both a and b correspond to the positions of nucleotide residues shown in SEQ ID NO:109, and where b is greater than or equal to a + 14.

### FEATURES OF PROTEIN ENCODED BY GENE NO: 100

This gene is expressed primarily in activated neutrophils.

Therefore, polynucleotides and polypeptides of the invention are useful as reagents for differential identification of the tissue(s) or cell type(s) present in a biological sample and for diagnosis of diseases and conditions which include, but are not limited to, immune disorders, inflammation. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of inflammatory and immune conditions. Many polynucleotide sequences, such as EST sequences, are publicly available and accessible through sequence databases. Some of these sequences are related to SEQ ID NO:110 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 442 of SEQ ID NO:110, b is an integer of 15 to 456, where both a and b correspond to the positions of nucleotide residues shown in SEQ ID NO:110, and where b is greater than or equal to a + 14.

### FEATURES OF PROTEIN ENCODED BY GENE NO: 101

Additional embodiments of the invention are directed to polypeptides comprising the following amino acid
sequences:ATVPGSIYNYFYHYNAGALKPEHASESPRGLCAQTAGPFPSF (SEQ ID NO:424); IRHEPPPPRFKRFSCLSLLSSWDYRRAPPHVAIFCTLSRDGVLPHW PGWSQTPDLK (SEQ ID NO:425); STHLGLPRCWDYRHEPLCLAPFTTISIIIMQ GLSNLSMPQNPPEGCAHRLLDLSPASDSVPPEWGSKIAFEV (SEQ ID NO:426); and/or LRVGGTSENCCRGECCGSVCIPPGRL (SEQ ID NO:427). Further embodiments of the invention are directed to polynucleotides which encode these polypeptides.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of immune disorders. Many polynucleotide sequences, such as EST sequences, are publicly available and accessible through sequence databases. Some of these sequences are related to SEQ ID NO:111 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 540 of SEQ ID NO:111, b is an integer of 15 to 554, where both a and b correspond to the positions of nucleotide residues shown in SEQ ID NO:111, and where b is greater than or equal to a + 14.

### FEATURES OF PROTEIN ENCODED BY GENE NO: 102

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:251 as residues: Lys-33 to Lys-41.

The tissue distribution indicates that polynucleotides and polypeptides corresponding to this gene are useful for study and treatment of immune disorders. Many polynucleotide sequences, such as EST sequences, are publicly available and accessible through sequence databases. Some of these sequences are related to SEQ ID NO: 112 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 708 of SEQ ID NO:112, b is an integer of 15 to 722, where both a and b correspond to the positions of nucleotide residues shown in SEQ ID NO: 112, and where b is greater than or equal to a + 14.

### FEATURES OF PROTEIN ENCODED BY GENE NO: 103

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of immune disorders. Many polynucleotide sequences, such as EST sequences, are publicly available and accessible through sequence databases. Some of these sequences are related to SEQ ID NO: 113 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 917 of SEQ ID NO:113, b is an integer of 15 to 931, where both a and b correspond to the positions of nucleotide residues shown in SEQ ID NO: 113, and where b is greater than or equal to a + 14.

### FEATURES OF PROTEIN ENCODED BY GENE NO: 104

Additional embodiments of the invention are directed to polypeptides comprising the following amino acid
sequences:GLCMVHSLLTSSLGGRCCNYPYIADKDIETEVK PPSQGHTVHLHCS (SEQ ID NO:428); QLWCITALPSTRHCSKGFAWFTHSLRH PSVAGAVIILILQT RTLRQRSSHLPKGTHGICTAPDRPTERAAVTILK (SEQ ID NO:429); SFDNN NSYGVSQLYQVPDTVLRALHGSLTPYVIPRWQVL (SEQ ID NO:430); and/or DRGQATFP RAHMASALLLTDRQRELLSRSSNELCMSKV (SEQ ID NO:431). Further embodiments of the invention are directed to polynucleotides which encode these polypeptides.

This gene is expressed primarily in neutrophils.

Therefore, polynucleotides and polypeptides of the invention are useful as reagents for differential identification of the tissue(s) or cell type(s) present in a biological sample and for diagnosis of immune diseases and disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of immune disorders. Many polynucleotide sequences, such as EST sequences, are publicly available and accessible through sequence databases. Some of these sequences are related to SEQ ID NO:114 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 574 of SEQ ID NO: 114, b is an integer of 15 to 588, where both a and b correspond to the positions of nucleotide residues shown in SEQ ID NO:114, and where b is greater than or equal to a + 14.

### FEATURES OF PROTEIN ENCODED BY GENE NO: 105

Additional embodiments of the invention are directed to polypeptides comprising the following amino acid sequences:
LLLILRPFLNSQFKLQLPLVLFHSSCTYICLLYNYELFHIVALTGKLMNLGLHLF AHHLILAVAHXGCSIPIY (SEQ ID NO:432); and/or THNSNYSSLWFSST AVVLTYVYYIIMNCFILSPLQVN (SEQ ID NO:433). Further embodiments of the invention are directed to polynucleotides which encode these polypeptides.

This gene is expressed primarily in neutrophils.

Therefore, polynucleotides and polypeptides of the invention are useful as reagents for differential identification of the tissue(s) or cell type(s) present in a biological sample and for diagnosis of immune disorders and diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of inflammatory and immune disorders. Many polynucleotide sequences, such as EST sequences, are publicly available and accessible through sequence databases. Some of these sequences are related to SEQ ID NO:115 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 798 of SEQ ID NO:115, b is an integer of 15 to 812, where both a and b correspond to the positions of nucleotide residues shown in SEQ ID NO:115, and where b is greater than or equal to a + 14.

### FEATURES OF PROTEIN ENCODED BY GENE NO: 106

Additional embodiments of the invention are directed to polypeptides comprising the following amino acid sequences:
TLVAGSPCSLSRWIMAGFCHGELVQSDMESQEWERGQVVLSHTSLPWCYVSP R (SEQ ID NO:434); MAGFCHGELVQSDMESQEWERGQVVLSHTSLPWCYVSPR (SEQ ID NO:435); and/or MAVWISGSYSSFCSRSNWDVFSPNIVLASLPFSFRS VSKAAKPWWLALPALFPDGLWLDSAMGSLYSQTWKARNGKEVRWFSPT PHCLGAMSHL (SEQ ID NO:436). Further embodiments of the invention are directed to polynucleotides which encode these polypeptides.

This gene is expressed primarily in neutrophils.

Therefore, polynucleotides and polypeptides of the invention are useful as reagents for differential identification of the tissue(s) or cell type(s) present in a biological sample and for diagnosis of immune disorders and diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:255 as residues: Pro-54 to Gly-62.

The tissue distribution indicates that polynucleotides and polypeptides corresponding to this gene are useful for study and treatment of immune disorders. Many polynucleotide sequences, such as EST sequences, are publicly available and accessible through sequence databases. Some of these sequences are related to SEQ ID NO: 116 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 492 of SEQ ID NO:116, b is an integer of 15 to 506, where both a and b correspond to the positions of nucleotide residues shown in SEQ ID NO:116, and where b is greater than or equal to a + 14.

### FEATURES OF PROTEIN ENCODED BY GENE NO: 107

Additional embodiments of the invention are directed to polypeptides comprising the following amino acid sequences:
RSKRQSQGSRCSVPLLAQQSRSPPVPLQAQPAWLLGSETIAWSGGGSGWEGPR DPGTSTAAGNSGPGIGMGHRTPPPSHTGR (SEQ ID NO:437); RWDPAWGLD IPESSCPVTMGELRSGDGIVL (SEQ ID NO:438); GALLWDNSMISAPRG SHREAGALFPSWLSNPAVLPSRSRPSQPGCLDPRQ (SEQ ID NO:439); NSARE PRRWIRPTRGSGETTAPCCFEPLNGGTLVHAAAMARASEAAGTG (SEQ ID NO:440); MARASE AAGTG (SEQ ID NO:441); CFTTAFQKALRDPRPTLPDTHG SLRNAPLKSLTLPAAFWSFFFLSLLQDGIKERSQTQNATFFFHDRSDIE GLSEEPCSGTTP (SEQ ID NO:442); and/or LALQE AVTGKQVLCSPP GSAIPQSSRPAPGPASLAAWIRDNSLVWRRLRVGGTQGPGHQYSSWEFRPRD RDGAQDTTPISHREMKVGSSMGTGHP (SEQ ID NO:443). Further embodiments of the invention are directed to polynucleotides which encode these polypeptides.

This gene is expressed primarily in neutrophils.

Therefore, polynucleotides and polypeptides of the invention are useful as reagents for differential identification of the tissue(s) or cell type(s) present in a biological sample and for diagnosis of immune disorders and diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., neutrophils and other blood cells, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:256 as residues: Met-25 to Gly-30.

The tissue distribution indicates that polynucleotides and polypeptides corresponding to this gene are useful for study and treatment of immune disorders. Many polynucleotide sequences, such as EST sequences, are publicly available and accessible through sequence databases. Some of these sequences are related to SEQ ID NO: 117 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 737 of SEQ ID NO: 117, b is an integer of 15 to 751, where both a and b correspond to the positions of nucleotide residues shown in SEQ ID NO:117, and where b is greater than or equal to a + 14.

### FEATURES OF PROTEIN ENCODED BY GENE NO: 108

Additional embodiments of the invention are directed to polypeptides comprising the following amino acid sequences:
MFYSKIFYFLLLNSDTSNNVTSKTLVSSISSSNNRLAVSIVF (SEQ ID NO:444); SRQKNLLKLHSNPNCDNFCFIFNYKPKYICIFKLICLKILLYIFGSG (SEQ ID NO:445); and/or MLLSLLMVFTSELYVKRHISFKSXDKPHCHKNQDIDVLFRKL LEKHFKVINMICFP (SEQ ID NO:446). Further embodiments of the invention are directed to polynucleotides which encode these polypeptides.

This gene is expressed primarily in fetal liver and to a lesser extent in bone and breast cancer cell lines.

Therefore, polynucleotides and polypeptides of the invention are useful as reagents for differential identification of the tissue(s) or cell type(s) present in a biological sample and for diagnosis of diseases and conditions which include, but are not limited to, cancer and metabolic disorders. Similarly, polypeptides and antibodies directed to these polypeptides are useful in providing immunological probes for differential identification of the tissue(s) or cell type(s). For a number of disorders of the above tissues or cells, particularly of the digestive and skeletal system, expression of this gene at significantly higher or lower levels may be routinely detected in certain tissues (e.g., hepatic tissue, bone, mammary tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of growth and metabolic disorders and neoplasias. Many polynucleotide sequences, such as EST sequences, are publicly available and accessible through sequence databases. Some of these sequences are related to SEQ ID NO: 118 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 946 of SEQ ID NO:118, b is an integer of 15 to 960, where both a and b correspond to the positions of nucleotide residues shown in SEQ ID NO: 118, and where b is greater than or equal to a + 14.

### FEATURES OF PROTEIN ENCODED BY GENE NO: 109

Additional embodiments of the invention are directed to polypeptides comprising the following amino acid sequences: FREYGFYNLHFC (SEQ ID NO:447); LVTTD YYDGCNEDYEYNWSYMFLNSEQLFIKFYPTFFC (SEQ ID NO:448); and/or NVIAPGLESSCANSLFLLFVCLPVAHHRHNFLFIKHSLYN HLRDYESDFDKI (SEQ ID NO:449). Further embodiments of the invention are directed to polynucleotides which encode these polypeptides.

This gene is expressed primarily in T cells, fetal heart and infant brain and to a lesser extent in some transformed cell lines.

Therefore, polynucleotides and polypeptides of the invention are useful as reagents for differential identification of the tissue(s) or cell type(s) present in a biological sample and for diagnosis of growth and immune disorders and diseases. Similarly, polypeptides and antibodies directed to these polypeptides are useful in providing immunological probes for differential identification of the tissue(s) or cell type(s). For a number of disorders of the above tissues or cells, particularly of the immune and cardiovascular systems, expression of this gene at significantly higher or lower levels may be routinely detected in certain tissues (e.g., T-cells and other cells and tissue of the immune system, heart and vescular tissue, brain and other tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of developmental and immune disorders. Many polynucleotide sequences, such as EST sequences, are publicly available and accessible through sequence databases. Some of these sequences are related to SEQ ID NO:119 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1428 of SEQ ID NO:119, b is an integer of 15 to 1442, where both a and b correspond to the positions of nucleotide residues shown in SEQ ID NO: 119, and where b is greater than or equal to a + 14.

### FEATURES OF PROTEIN ENCODED BY GENE NO: 110

Additional embodiments of the invention are directed to polypeptides comprising the following amino acid sequences:
PKVLAVLKKKNHVALSIFELLSNDICSFISFFMS (SEQ ID NO:450); EGPDIN SNLKFLLCLKKKIMWPFQYLNC (SEQ ID NO:451); and/or LLSLILLRIWYD FSKQTVFWFFLNVFNFFSSCNNDGACSYKYRKVQI (SEQ ID NO:452). Further embodiments of the invention are directed to polynucleotides which encode these polypeptides.

This gene is expressed primarily in osteoblasts and to a lesser extent in bone marrow and bladder.

Therefore, polynucleotides and polypeptides of the invention are useful as reagents for differential identification of the tissue(s) or cell type(s) present in a biological sample and for diagnosis of diseases and conditions which include, but are not limited to, skeletal and hemopoietic diseases and disorders. Similarly, polypeptides and antibodies directed to these polypeptides are useful in providing immunological probes for differential identification of the tissue(s) or cell type(s). For a number of disorders of the above tissues or cells, particularly of the skeletal and vascular systems, expression of this gene at significantly higher or lower levels may be routinely detected in certain tissues (e.g., bone, bone marrow, and bladder, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:259 as residues: Gly-33 to Lys-38.

The tissue distribution indicates that polynucleotides and polypeptides corresponding to this gene are useful for the study, diagnosis, and treatment of bone and hematopoietic disorders. Many polynucleotide sequences, such as EST sequences, are publicly available and accessible through sequence databases. Some of these sequences are related to SEQ ID NO:120 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 831 of SEQ ID NO: 120, b is an integer of 15 to 845, where both a and b correspond to the positions of nucleotide residues shown in SEQ ID NO: 120, and where b is greater than or equal to a + 14.

### FEATURES OF PROTEIN ENCODED BY GENE NO: 111

Additional embodiments of the invention are directed to polypeptides comprising the following amino acid sequences: HTLFISFLWAEG (SEQ ID NO:453); MLPVFVLFFCFTYSARKQSVFKKGNVFE (SEQ ID NO:454); and/or SPCSAA ECHNLSLLS SCSLVSSNILFSFPFFGQKARCCLFLFYFSASHIAHESRVYSK KEMCL (SEQ ID NO:455). Further embodiments of the invention are directed to polynucleotides which encode these polypeptides.

This gene is expressed primarily in prostate cancer.

Therefore, polynucleotides and polypeptides of the invention are useful as reagents for differential identification of the tissue(s) or cell type(s) present in a biological sample and for diagnosis of diseases and conditions which include, but are not limited to, prostate and other cancers. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues (e.g., prostate, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for study and treatment of prostate cancer and other neoplasias. Many polynucleotide sequences, such as EST sequences, are publicly available and accessible through sequence databases. Some of these sequences are related to SEQ ID NO: 121 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 346 of SEQ ID NO:121, b is an integer of 15 to 360, where both a and b correspond to the positions of nucleotide residues shown in SEQ ID NO:121, and where b is greater than or equal to a + 14.

### FEATURES OF PROTEIN ENCODED BY GENE NO: 112

Additional embodiments of the invention are directed to polypeptides comprising the following amino acid sequences:
HKCFQCFILANGFLKVIKPFQRNWSDKTFFLVCLNKAISEALLSKMTFLSFFKT NLLLLETFCTI (SEQ ID NO:456); LLGVLKPLYFSVEPVLGERSVAFEEVREKNH GTSGFLSLYSLAAIVCGHLMFFHTLLGRGGNDHPGQSPLPGMRPLRGGL AGQAPSGHPWMQPLDTCLL (SEQ ID NO:457); RPTRPPTRPDRPSLELAPG LCADFLGSSNHCIFLLSLYLGRDQ (SEQ ID NO:458); and/or EKRIMVPQGF FPFTRWQPLSVGTSCFSTLYWAVEVTITQASLLCLGCAL (SEQ ID NO:459). Further embodiments of the invention are directed to polynucleotides which encode these polypeptides.

This gene is expressed primarily in haemopoietic and neural tissues and to a lesser extent in a number of cancers and other tissues.

Therefore, polynucleotides and polypeptides of the invention are useful as reagents for differential identification of the tissue(s) or cell type(s) present in a biological sample and for diagnosis of diseases and conditions which include, but are not limited to, diseases of the haemopoietic and neural systems. Similarly, polypeptides and antibodies directed to these polypeptides are useful in providing immunological probes for differential identification of the tissue(s) or cell type(s). For a number of disorders of the above tissues or cells, particularly of the immune and neural system, expression of this gene at significantly higher or lower levels may be routinely detected in certain tissues (e.g., haematopoietic tissue, and neural cells and tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of diseases of the haemopoietic and neural systems including several cancers. Many polynucleotide sequences, such as EST sequences, are publicly available and accessible through sequence databases. Some of these sequences are related to SEQ ID NO: 122 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 930 of SEQ ID NO: 122, b is an integer of 15 to 944, where both a and b correspond to the positions of nucleotide residues shown in SEQ ID NO: 122, and where b is greater than or equal to a + 14.

### FEATURES OF PROTEIN ENCODED BY GENE NO: 113

The translation product of this gene shares sequence homology with intestinal epithelium proliferating cell-associated mRNA sequence which is thought to be important in growth and development of epithelial cells. Additional embodiments of the invention are directed to polypeptides comprising the following amino acid sequences:
MTLDEWKNLQEQTRPKPEFNIRKPESTVPSKAVVIRESKYRDDMVKDDYEDDS HVFRKPANDITSQLEINFGNLPRPGRGARGGTRGGRGRIRRAENYGPRAEVVM QDVAPNPDDPEDFPALS (SEQ ID NO:460); CKMLPPTQMTRKISLRCLERALFP STAELHCTPVGRLFQLGQGSQTLRTIDVAFPVSCKFVALFWAELLEGLL QRLESRPFPKKMKNGDCVFIEGISNEE (SEQ ID NO:461); PPSSWAWS QRRHPGRPGKDQEGRELWTQSRSGDARCCPQPR (SEQ ID NO:462); and/or CLKCVYRDSIDSSAEAWRERRL (SEQ ID NO:463). Further embodiments of the invention are directed to polynucleotides which encode these polypeptides.

This gene is expressed primarily in brain and central nervous system and to a lesser extent in testis.

Therefore, polynucleotides and polypeptides of the invention are useful as reagents for differential identification of the tissue(s) or cell type(s) present in a biological sample and for diagnosis of diseases and conditions which include, but are not limited to, diseases of the neural system. Similarly, polypeptides and antibodies directed to these polypeptides are useful in providing immunological probes for differential identification of the tissue(s) or cell type(s). For a number of disorders of the above tissues or cells, particularly of the neural and reproductive system, expression of this gene at significantly higher or lower levels may be routinely detected in certain tissues (e.g., brain and other cells and tissue of the nervous system, testis and other reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:262 as residues: Glu-20 to Glu-27, Glu-30 to Trp-44.

The tissue distribution and homology to intestinal epithelium proliferating cell-associated mRNA sequence indicates that polynucleotides andpolypeptides corresponding to this gene are useful for growth and developmental diseases of the brain, central nervous system and reproductive system. Many polynucleotide sequences, such as EST sequences, are publicly available and accessible through sequence databases. Some of these sequences are related to SEQ ID NO: 123 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 900 of SEQ ID NO:123, b is an integer of 15 to 914, where both a and b correspond to the positions of nucleotide residues shown in SEQ ID NO:123, and where b is greater than or equal to a + 14.

### FEATURES OF PROTEIN ENCODED BY GENE NO: 114

Additional embodiments of the invention are directed to polypeptides comprising the following amino acid sequences: LSYSVLLILP LFHSLPTL KDTHTHNKWVE (SEQ ID NO:464); EVNGVGYKHSCFSDISSVLENKDS RMRAPHYASFQHFFSVLLKLSPQACLTESQCIPLTFY (SEQ ID NO:465); KTHTHTISGWSKKSTELDISIP AFLTSPVSWRTRILE (SEQ ID NO:466); and/or IRHELGSSDPPAEASQIAGTAAVSHHAQP (SEQ ID NO:467). Further embodiments of the invention are directed to polynucleotides which encode these polypeptides.

This gene is expressed primarily in spinal cord.

Therefore, polynucleotides and polypeptides of the invention are useful as reagents for differential identification of the tissue(s) or cell type(s) present in a biological sample and for diagnosis of diseases and conditions which include, but are not limited to, spinal cord injuries and diseases of the neural system. Similarly, polypeptides and antibodies directed to these polypeptides are useful in providing immunological probes for differential identification of the tissue(s) or cell type(s). For a number of disorders of the above tissues or cells, particularly of the neural system, expression of this gene at significantly higher or lower levels may be routinely detected in certain tissues (e.g., spinal cord and other cells and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:263 as residues: Pro-45 to Gln-52.

The tissue distribution indicates that polynucleotides and polypeptides corresponding to this gene are useful for diagnosis and treatment of spinal cord injuries and diseases of the neural system. Many polynucleotide sequences, such as EST sequences, are publicly available and accessible through sequence databases. Some of these sequences are related to SEQ ID NO: 124 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 448 of SEQ ID NO: 124, b is an integer of 15 to 462, where both a and b correspond to the positions of nucleotide residues shown in SEQ ID NO: 124, and where b is greater than or equal to a + 14.

### FEATURES OF PROTEIN ENCODED BY GENE NO: 115

Additional embodiments of the invention are directed to polypeptides comprising the following amino acid sequences: MLYLILISLSSLSFSFSLPPFSIII (SEQ ID NO:468); SSYFLRHFRIYHTCPKYFSMNIIN (SEQ ID NO:469); KLTLTKGNKSWSSTAVAAALELVDPPGCRNSARDSLPNSTM MFYYAC FILYSSLSPLSLSLSPSLLSLL (SEQ ID NO:470); and/or QFHTGNSYDHDYAK (SEQ ID NO:471). Further embodiments of the invention are directed to polynucleotides which encode these polypeptides.

This gene is expressed primarily in striatum.

Therefore, polynucleotides and polypeptides of the invention are useful as reagents for differential identification of the tissue(s) or cell type(s) present in a biological sample and for diagnosis of a number of diseases of the neural system. Similarly, polypeptides and antibodies directed to these polypeptides are useful in providing immunological probes for differential identification of the tissue(s) or cell type(s). For a number of disorders of the above tissues or cells, particularly of the neural diseases, expression of this gene at significantly higher or lower levels may be routinely detected in certain tissues (e.g., striatum, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of diseases of the neural system. Many polynucleotide sequences, such as EST sequences, are publicly available and accessible through sequence databases. Some of these sequences are related to SEQ ID NO:125 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 531 of SEQ ID NO:125, b is an integer of 15 to 545, where both a and b correspond to the positions of nucleotide residues shown in SEQ ID NO:125, and where b is greater than or equal to a + 14.

### FEATURES OF PROTEIN ENCODED BY GENE NO: 116

Additional embodiments of the invention are directed to polypeptides comprising the following amino acid sequences:
AVCTGGYCESCRCEHCVCVCVDLCVLFSGKELRVR (SEQ ID NO:472); VSFFFV FKWSFAEIKSREEHWASLTPKPTLLSALLTCDVLKSSIIFKCCESTEDKGFDSFF QASKDGSSSRI (SEQ ID NO:473); RSWGS QRSLCLLFIPFAAESYSVVWMGHL FVVCLLSSVVWTFRPFALAVTVNHVAVNIVCVSAWTCVSCSLGRSCGLEGSFLF PLETLWFPHMVVLCLTF (SEQ ID NO:474); GHLFVVCLLSSWWTFRPFALAVT VNHVAVNIVCVSAWTCVSCSLGRSCGLEGSFLFPLETLWFPHMVVLCLTF (SEQ ID NO:475); HDVLGARNAACVCCSFLLQQNRILLFGWATCLLSVYSPA GGHLGR LHWRLL (SEQ ID NO:476); MLDFKTSQVSKALKRVGFGVRLAQ CSSLDLISAKLHLKTKKKETYTTSTVMTAASLFLSYVTSEFTRSIMATFYCFVL KLHIGEMGTLQTAGGSKMTWPLQKAIWQFLKRLSIKLPYVETRESPGETKNY (SEQ ID NO:477); and/or LTRNSFPENRTHKSTQTHTQCSQRHDSQ (SEQ ID NO:478). Further embodiments of the invention are directed to polynucleotides which encode these polypeptides.

This gene is expressed primarily in intestine and cancer cells.

Therefore, polynucleotides and polypeptides of the invention are useful as reagents for differential identification of the tissue(s) or cell type(s) present in a biological sample and for diagnosis of diseases and conditions which include, but are not limited to, diseases of the gastrointestinal tract and cancer. Similarly, polypeptides and antibodies directed to these polypeptides are useful in providing immunological probes for differential identification of the tissue(s) or cell type(s). For a number of disorders of the above tissues or cells, particularly of the digestive system, expression of this gene at significantly higher or lower levels may be routinely detected in certain tissues (e.g., gastrointestinal tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of diseases of the digestive system and cancer. Many polynucleotide sequences, such as EST sequences, are publicly available and accessible through sequence databases. Some of these sequences are related to SEQ ID NO:126 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 898 of SEQ ID NO:126, b is an integer of 15 to 912, where both a and b correspond to the positions of nucleotide residues shown in SEQ ID NO: 126, and where b is greater than or equal to a + 14.

### FEATURES OF PROTEIN ENCODED BY GENE NO: 117

The translation product of this gene shares sequence homology with a human apoptosis regulating protein which is thought to be important in regulating cell death. Additional embodiments of the invention are directed to polypeptides comprising the following amino acid sequences:
IRHEGQSSSRGSSHCDSPSPQEDGQIMFDVEMHTSRDHSSQSEEEWEGEKEVE ALKKSADWVSDWSSRPENIPPKEFHFRHPKRSVSLS (SEQ ID NO:479); GILLTLYPFWPEDILEFPNRVYCCLEICKGFFSANATSRL (SEQ ID NO:480); EFGTRDRVVPEAVLTVTALRHKKMGRSCLMWKCTPAGTIALSQKKKL (SEQ ID NO:481); AHPLPAPTEGKEKPLEMRVTCEWYCHSSLFELETIVSMTQPTT LFLHIQFQ (SEQ ID NO:482); TFCVFKHEEKWSHEERGYFLRRISEGVHSISLPF SCFGFGARHLYWKATEHTLCQHLLRERKSPWKCV (SEQ ID NO:483); and/or QSLLLFRNLQGLLFRKCHQQIIILSAMLLSLISATRLDLYHSWYKFYSCNI TTISLLKRDQVSK (SEQ ID NO:484). Further embodiments of the invention are directed to polynucleotides which encode these polypeptides.

This gene is expressed primarily in muscle, fibroblast cells, haemopoietic cells, fetal lung and to a lesser extent in several other tissues and cells.

Therefore, polynucleotides and polypeptides of the invention are useful as reagents for differential identification of the tissue(s) or cell type(s) present in a biological sample and for diagnosis of diseases and conditions which include, but are not limited to, diseases of the haemopoietic, muscular and developing system. Similarly, polypeptides and antibodies directed to these polypeptides are useful in providing immunological probes for differential identification of the tissue(s) or cell type(s). For a number of disorders of the above tissues or cells, particularly of the immune and muscular system, expression of this gene at significantly higher or lower levels may be routinely detected in certain tissues (e.g., muscle, fibroblast and other cells and tissue of the nervous system, haemotopoietic cells, and lung, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:266 as residues: Met-1 to Ala-6.

The tissue distribution and homology to apoptosis regulating protein indicates that polynucleotides and polypeptides corresponding to this gene are useful for diagnosis and treatment of diseases of the haemopoietic, muscular and developing system. Many polynucleotide sequences, such as EST sequences, are publicly available and accessible through sequence databases. Some of these sequences are related to SEQ ID NO: 127 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1034 of SEQ ID NO: 127, b is an integer of 15 to 1048, where both a and b correspond to the positions of nucleotide residues shown in SEQ ID NO:127, and where b is greater than or equal to a + 14.

### FEATURES OF PROTEIN ENCODED BY GENE NO: 118

Additional embodiments of the invention are directed to polypeptides comprising the following amino acid sequences: IRHEESFNPLTCGFSLFFSLFS (SEQ ID NO:485); METLLLLLFFLSLLIFRFRILVSQCIN (SEQ ID NO:486); FLLTTVLLFSSKVRDP RANFDQSLRVLKHAKKVQPDVISKTSIMLGLGEND EQVYATMKGKEIEK (SEQ ID NO:487); and/or QQSCCFPVRFVILGPILISPYVY (SEQ ID NO:488). Further embodiments of the invention are directed to polynucleotides which encode these polypeptides.

This gene is expressed primarily in synovium.

Therefore, polynucleotides and polypeptides of the invention are useful as reagents for differential identification of the tissue(s) or cell type(s) present in a biological sample and for diagnosis of diseases and conditions which include, but are not limited to, arthritis and other diseases of the musculo-skeletal system. Similarly, polypeptides and antibodies directed to these polypeptides are useful in providing immunological probes for differential identification of the tissue(s) or cell type(s). For a number of disorders of the above tissues or cells, particularly of the musculo-skeletal system, expression of this gene at significantly higher or lower levels may be routinely detected in certain tissues (e.g., synovial tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of diseases of the muscular-skeletal system. Many polynucleotide sequences, such as EST sequences, are publicly available and accessible through sequence databases. Some of these sequences are related to SEQ ID NO: 128 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 708 of SEQ ID NO:128, b is an integer of 15 to 722, where both a and b correspond to the positions of nucleotide residues shown in SEQ ID NO: 128, and where b is greater than or equal to a + 14.

### FEATURES OF PROTEIN ENCODED BY GENE NO: 119

Additional embodiments of the invention are directed to polypeptides comprising the following amino acid sequences:
VWLLSSILLRVLWNRYTLQELSFWLPWFASRATSLVLQHGDNYLLFLFCFVCF VLAMPF (SEQ ID NO:489); IRHEVSMAFVFHLAQGTLEPLYIAGA (SEQ ID NO:490); NSARGEYGFCLPSCSGYFGTAIHCRSLASGYHGLLPEQQA (SEQ ID NO:491); and/or HELTVPSRMGSKGKPYPCGFYSSLIP (SEQ ID NO:492). Further embodiments of the invention are directed to polynucleotides which encode these polypeptides.

This gene is expressed primarily in rejected kidney, stromal cells, and infant brain.

Therefore, polynucleotides and polypeptides of the invention are useful as reagents for differential identification of the tissue(s) or cell type(s) present in a biological sample and for diagnosis of diseases and conditions which include, but are not limited to, diseases of the renal, central nervous and immune systems. Similarly, polypeptides and antibodies directed to these polypeptides are useful in providing immunological probes for differential identification of the tissue(s) or cell type(s). For a number of disorders of the above tissues or cells, particularly of the immune, renal and central nervous system, expression of this gene at significantly higher or lower levels may be routinely detected in certain tissues (e.g., kidney, stromal cells, brain and other cells and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:268 as residues: Ser-6 to Arg-15.

The tissue distribution indicates that polynucleotides and polypeptides corresponding to this gene are useful for diagnosis and treatment of diseases of the renal, central nervous, and immune systems. Many polynucleotide sequences, such as EST sequences, are publicly available and accessible through sequence databases. Some of these sequences are related to SEQ ID NO:129 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 463 of SEQ ID NO:129, b is an integer of 15 to 477, where both a and b correspond to the positions of nucleotide residues shown in SEQ ID NO:129, and where b is greater than or equal to a + 14.

### FEATURES OF PROTEIN ENCODED BY GENE NO: 120

The protein of the invention has sequence identity to the Saccharomyces cerevisiae ankyrin repeat-containing protein (gil466522). Additional embodiments of the invention are directed to polypeptides comprising the following amino acid sequences:
KCIYPKPARTHHCSICNRCVLKMDHHCPWLNNCVGHYNHRYFFSFCFFMTLG CVYCSYGSWDLFREAYAAIEKMKQLDKNKLQAVANQTYHQTPPPTFSFRER (SEQ ID NO: 493); ARGHWNLILIVFHYYQAITTPPGYPPQGRNDIATVSIC (SEQ ID NO:494); WQCELDCVSHDSSTHSAPYVISRASKGSFSQNP (SEQ ID NO:495); SKRASGPALGYHAGQFKDQPFYHCRRKTQCGEILGLTSLYSGKQKFQPQTR GQAASYLPCPVLTRTSSRIQHWSWPPPLLLAV (SEQ ID NO:496); ESLQLRLLGQ LEGIPGCGYRKALAYSGALTF (SEQ ID NO:497); and/or SLAPWEWNELGA PSLGDCSLSLCDGSVSWTVSATTRALILLPMLFQGPPRAAFLRILDQKEPVGLP (SEQ ID NO:498). Further embodiments of the invention are directed to polynucleotides which encode these polypeptides.

This gene is expressed primarily in endometrial tumor and to a lesser extent in several other tissues and organs.

Therefore, polynucleotides and polypeptides of the invention are useful as reagents for differential identification of the tissue(s) or cell type(s) present in a biological sample and for diagnosis of a number of types of cancers, particularly endometrial cancers. Similarly, polypeptides and antibodies directed to these polypeptides are useful in providing immunological probes for differential identification of the tissue(s) or cell type(s). For a number of disorders of the above tissues or cells, particularly of the endometrium, expression of this gene at significantly higher or lower levels may be routinely detected in certain tissues (e.g., endometrium and other reproductive tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:269 as residues: Asn-43 to Arg-49, Phe-57 to Cys-65, Pro-93 to Ser-99.

The tissue distribution indicates that polynucleotides and polypeptides corresponding to this gene are useful for diagnosis and treatment of diseases and cancers of the endometrium and cancers of several different organs and tissue. Many polynucleotide sequences, such as EST sequences, are publicly available and accessible through sequence databases. Some of these sequences are related to SEQ ID NO:130 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1282 of SEQ ID NO: 130, b is an integer of 15 to 1296, where both a and b correspond to the positions of nucleotide residues shown in SEQ ID NO: 130, and where b is greater than or equal to a + 14.

### FEATURES OF PROTEIN ENCODED BY GENE NO: 121

The translation product of this gene shares sequence homology with adrenalin receptor. Additional embodiments of the invention are directed to polypeptides comprising the following amino acid sequences:
TATLNSFFGGWGLALLLRLECSDTIMDHCSLDLLGSSNPPASASQVVGTTGAR HHAQLIFCFFVQTRSHSVA (SEQ ID NO:499); MDHCSLDLLGSSNPPASASQV VGTTGARHHAQLIFCFFVQTRSHSVA (SEQ ID NO:500); GVLKQSSHLVLSKG (SEQ ID NO:501); DYSCESLCPALLSIAPDIVLN (SEQ ID NO:502); TTIHKTQLGS YKILWEPKEGYHNSTWI (SEQ ID NO:503); IREIFLRRP (SEQ ID NO:504); and/or LKFQKPGKIQMRGGGRVFWYKNCK (SEQ ID NO:505). Further embodiments of the invention are directed to polynucleotides which encode these polypeptides.

This gene is expressed primarily in synovial sarcoma.

Therefore, polynucleotides and polypeptides of the invention are useful as reagents for differential identification of the tissue(s) or cell type(s) present in a biological sample and for diagnosis of diseases and conditions which include, but are not limited to, arthritis and other diseases of the synovium including cancers. Similarly, polypeptides and antibodies directed to these polypeptides are useful in providing immunological probes for differential identification of the tissue(s) or cell type(s). For a number of disorders of the above tissues or cells, particularly of the immune and muscular-skeletal system, expression of this gene at significantly higher or lower levels may be routinely detected in certain tissues (e.g., synovial tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to adrenalin receptor indicates that polynucleotides and polypeptides corresponding to this gene are useful for diagnosis and treatment of diseases of the synovium, immune system and musculo-skeletal system including cancers of these tissues and systems. It may also be useful for identifying and therapeutically using antagonists and agonists for this receptor family. Many polynucleotide sequences, such as EST sequences, are publicly available and accessible through sequence databases. Some of these sequences are related to SEQ ID NO: 131 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 724 of SEQ ID NO: 131, b is an integer of 15 to 738, where both a and b correspond to the positions of nucleotide residues shown in SEQ ID NO: 131, and where b is greater than or equal to a + 14.

### FEATURES OF PROTEIN ENCODED BY GENE NO: 122

Additional embodiments of the invention are directed to polypeptides comprising the following amino acid sequences:
NSARVTQKGESVGSVGCMRAIAGFDNYPLF (SEQ ID NO:506); GTIGIFWPLPVAILSSGDYLQTQIHRPLLHRGT (SEQ ID NO:507); LPLPLSSLLHIATCNPFPKT (SEQ ID NO:508); SYFFVYNLILKIIQGDHASIILLATI PIFGDIYYVKGQLASFGPYL (SEQ ID NO:509); LFYHLEIISRHKSIAHCSIEA (SEQ ID NO:510); CSCHCPSRAFST (SEQ ID NO:511); and/or PHAIHSQKPSSIFLIT DVFPDPPVGIYLL (SEQ ID NO:512). Further embodiments of the invention are directed to polynucleotides which encode these polypeptides.

This gene is expressed primarily in chronic synovitis.

Therefore, polynucleotides and polypeptides of the invention are useful as reagents for differential identification of the tissue(s) or cell type(s) present in a biological sample and for diagnosis of diseases and conditions which include, but are not limited to, inflammatory diseases and disorders of the musculo-skeletal system. Similarly, polypeptides and antibodies directed to these polypeptides are useful in providing immunological probes for differential identification of the tissue(s) or cell type(s). For a number of disorders of the above tissues or cells, particularly of the inflammatory and musculo-skeletal system, expression of this gene at significantly higher or lower levels may be routinely detected in certain tissues (e.g., synovial tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:271 as residues: Ser-39 to Pro-44.

The tissue distribution indicates that polynucleotides and polypeptides corresponding to this gene are useful for treatment and diagnosis of disorders and diseases of the inflammatory and musculo-skeletal system. Many polynucleotide sequences, such as EST sequences, are publicly available and accessible through sequence databases. Some of these sequences are related to SEQ ID NO: 132 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 428 of SEQ ID NO:132, b is an integer of 15 to 442, where both a and b correspond to the positions of nucleotide residues shown in SEQ ID NO:132, and where b is greater than or equal to a + 14.

### FEATURES OF PROTEIN ENCODED BY GENE NO: 123

Additional embodiments of the invention are directed to polypeptides comprising the following amino acid sequences:
RKLFHKINSKSFHLSGMHILISVWIVRSRIIKVKYELLLCFFDVIFYV (SEQ ID NO:513); NSARDVFFTQKILYSQTCIFFPCLVPFSFLFSFFFFLSFVG (SEQ ID NO:514); MFSSLKKFYILKHVYSFPVLFHFLFFFLFSFSFLSWAEKGAGKMKLA TENCKMVKS (SEQ ID NO:515); and/or
IQLLYLKGAAMKYLSYVARLLFLKALDLF APKMVQIDSF (SEQ ID NO:516). Further embodiments of the invention are directed to polynucleotides which encode these polypeptides.

This gene is expressed primarily in kidney and infant brain and to a lesser extent in several other tissues and organs.

Therefore, polynucleotides and polypeptides of the invention are useful as reagents for differential identification of the tissue(s) or cell type(s) present in a biological sample and for diagnosis of diseases and conditions which include, but are not limited to, diseases of the renal and central nervous systems. Similarly, polypeptides and antibodies directed to these polypeptides are useful in providing immunological probes for differential identification of the tissue(s) or cell type(s). For a number of disorders of the above tissues or cells, particularly of the neural and renal system, expression of this gene at significantly higher or lower levels may be routinely detected in certain tissues (e.g., renal tissue, and brain and other cells and tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:272 as residues: Gly-24 to Lys-31.

The tissue distribution indicates that polynucleotides and polypeptides corresponding to this gene are useful for diagnosis and treatment of diseases of the neural and renal systems. Many polynucleotide sequences, such as EST sequences, are publicly available and accessible through sequence databases. Some of these sequences are related to SEQ ID NO: 133 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 868 of SEQ ID NO: 133, b is an integer of 15 to 882, where both a and b correspond to the positions of nucleotide residues shown in SEQ ID NO:133, and where b is greater than or equal to a + 14.

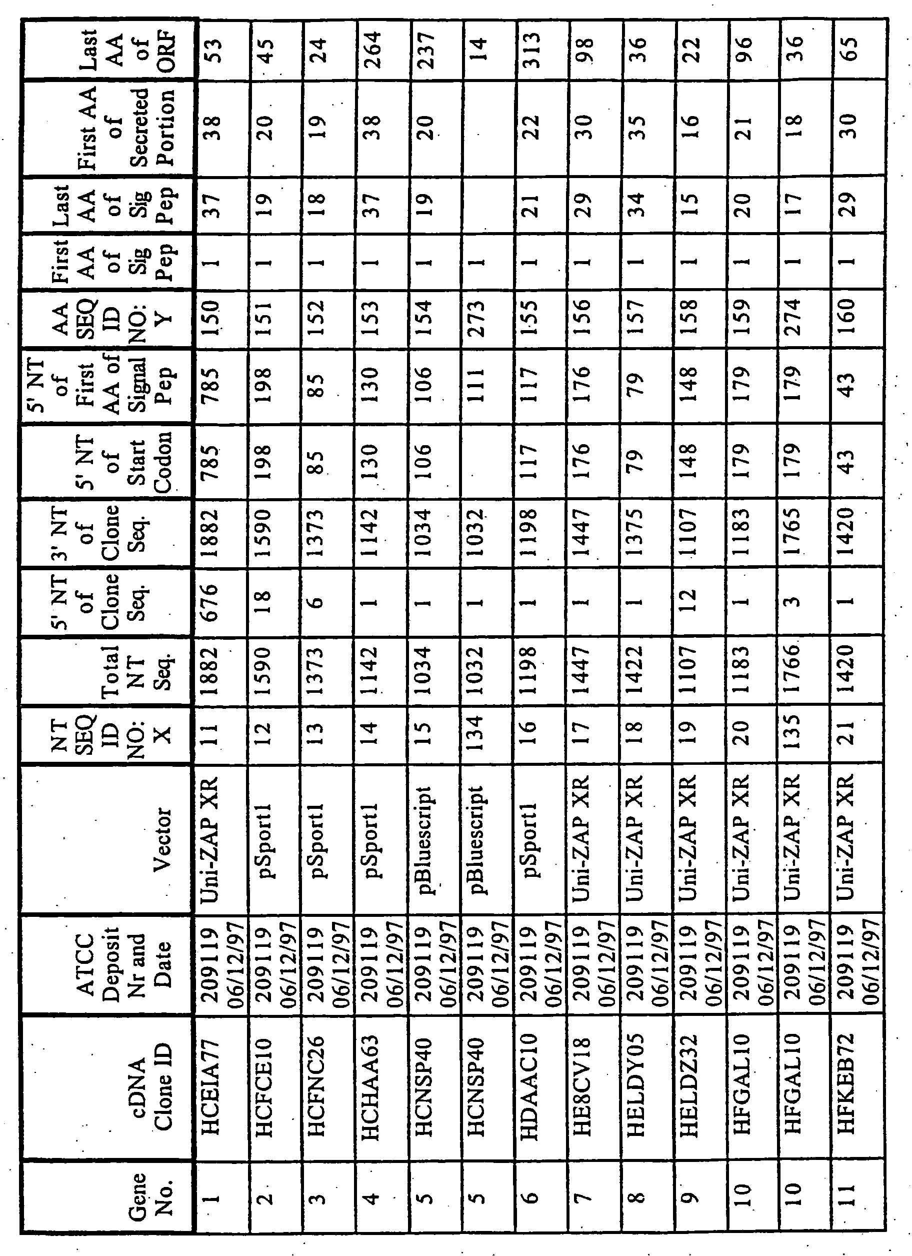

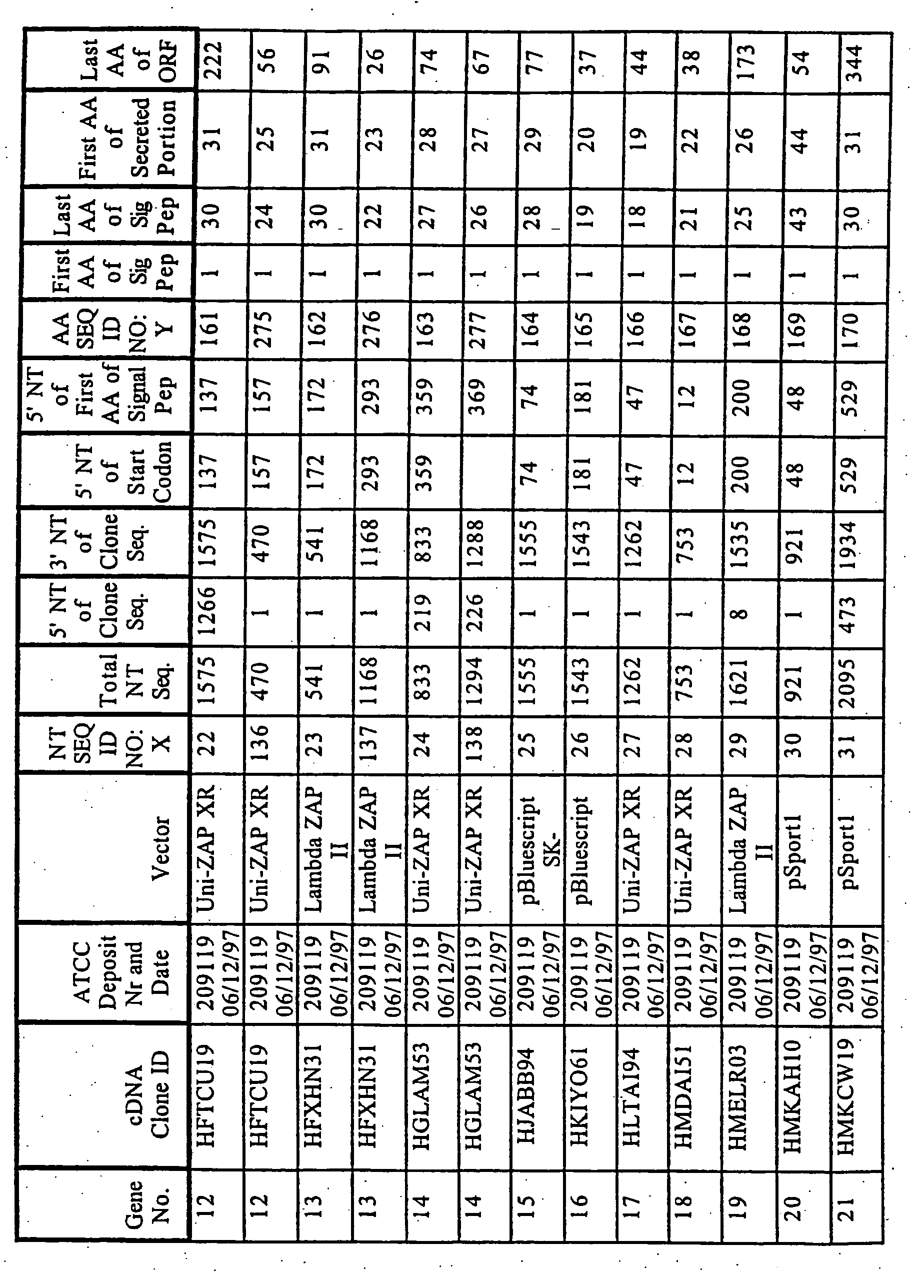

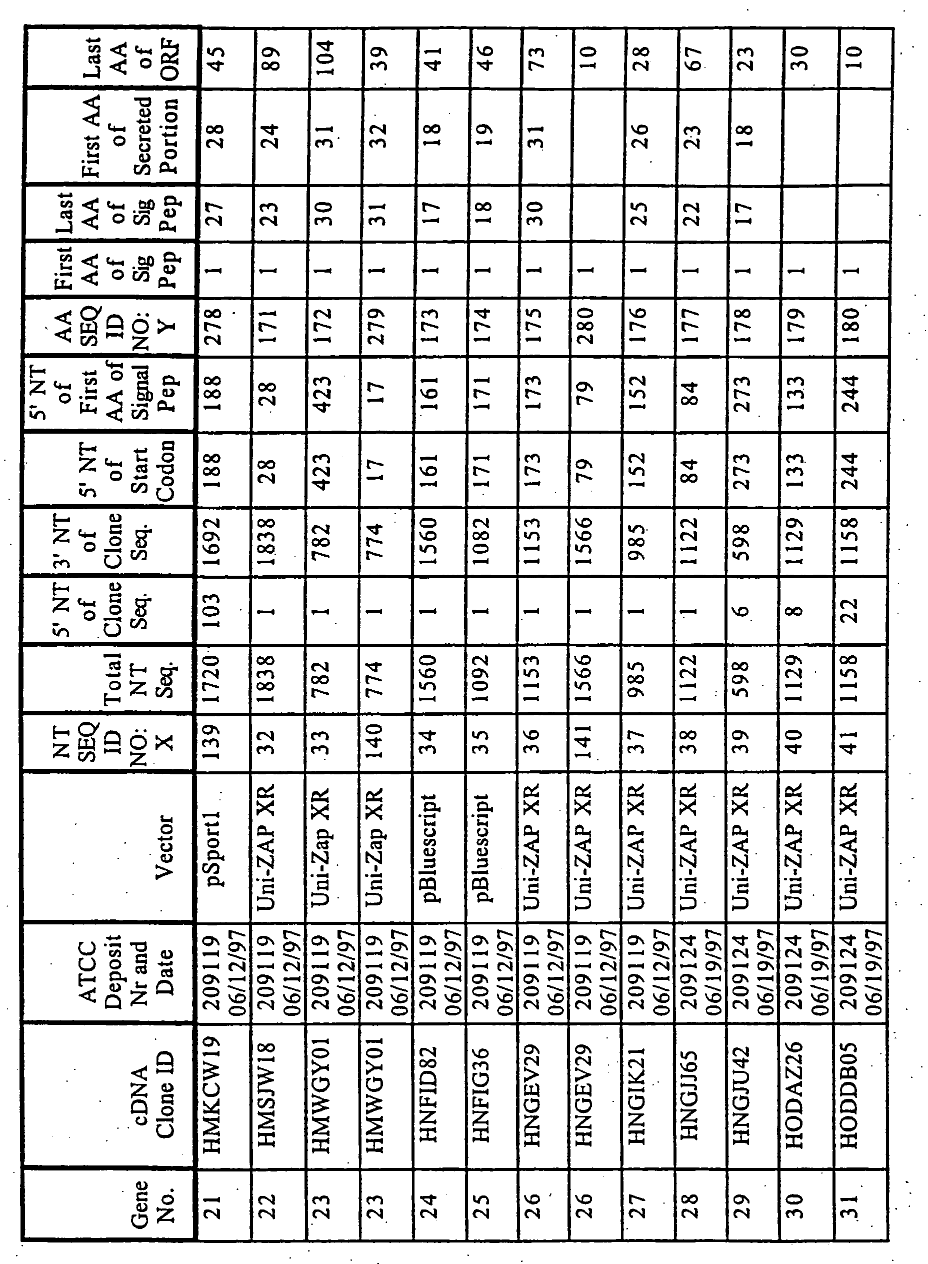

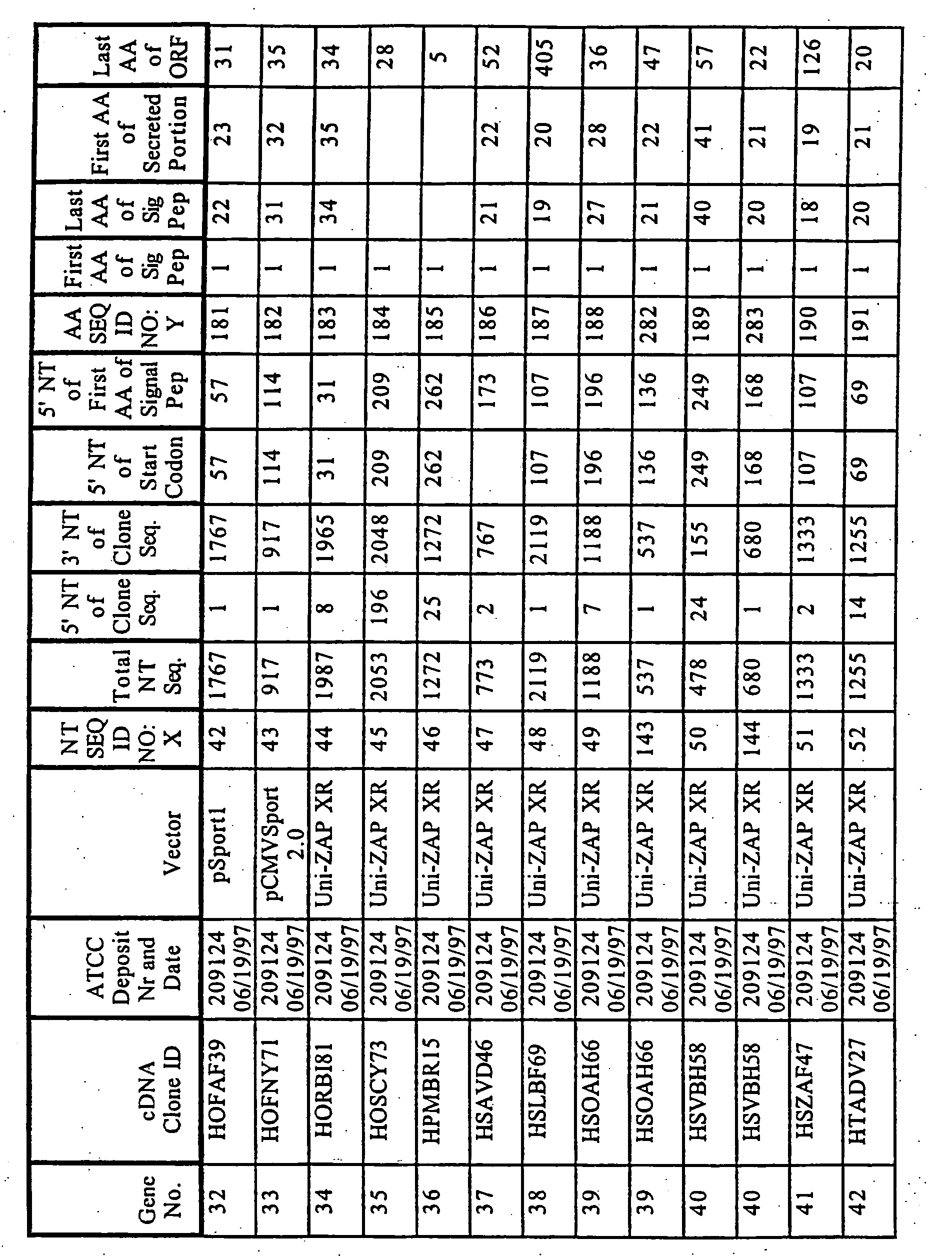

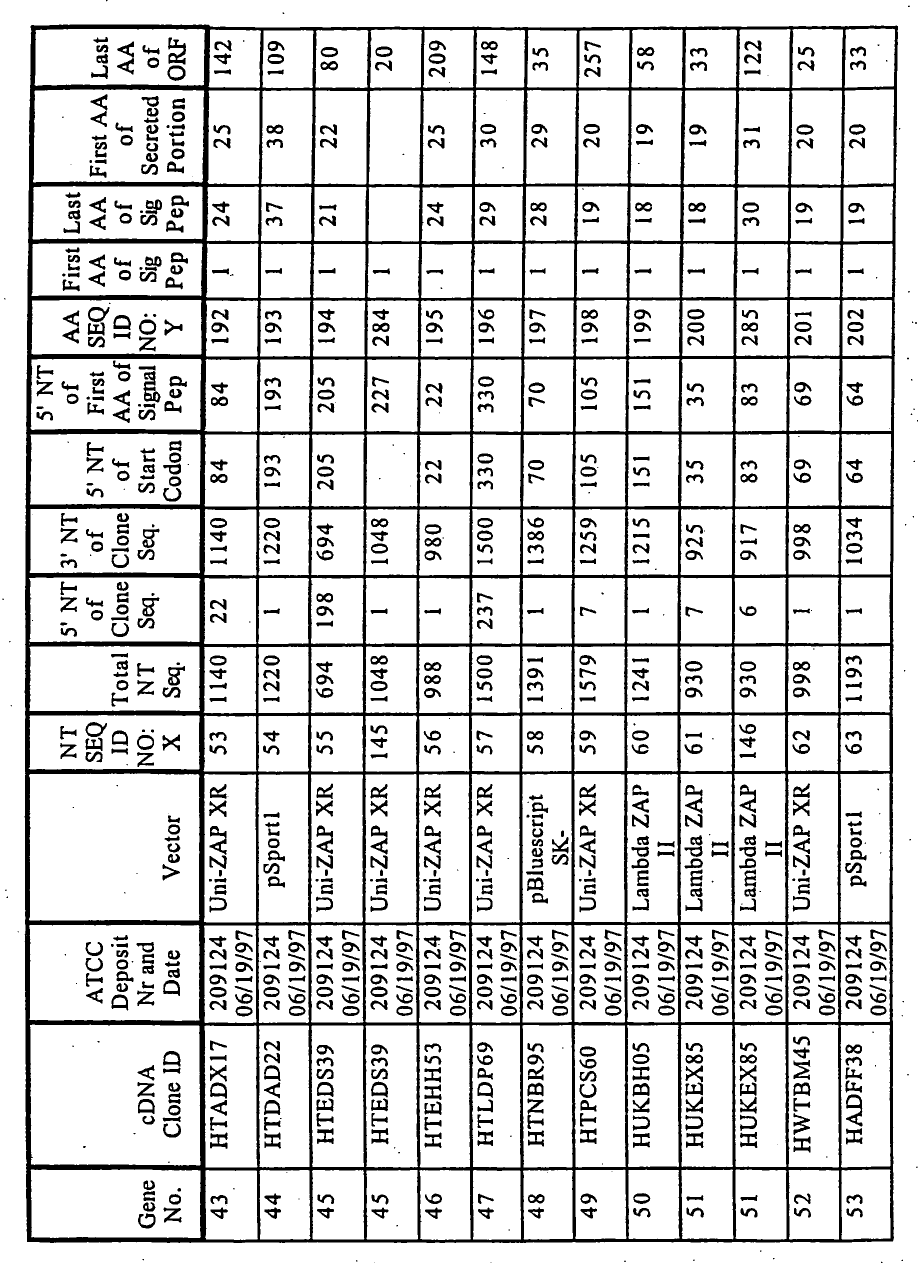

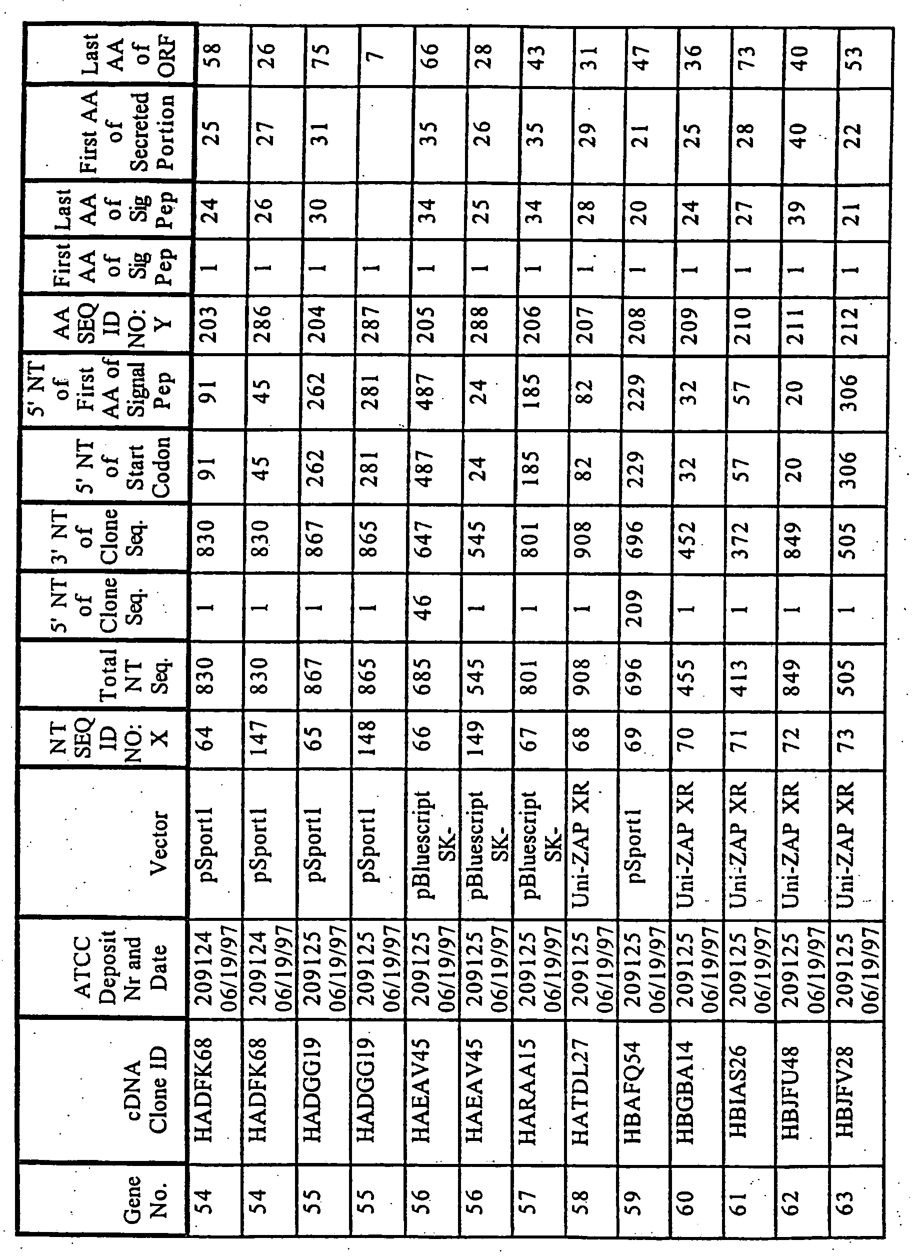

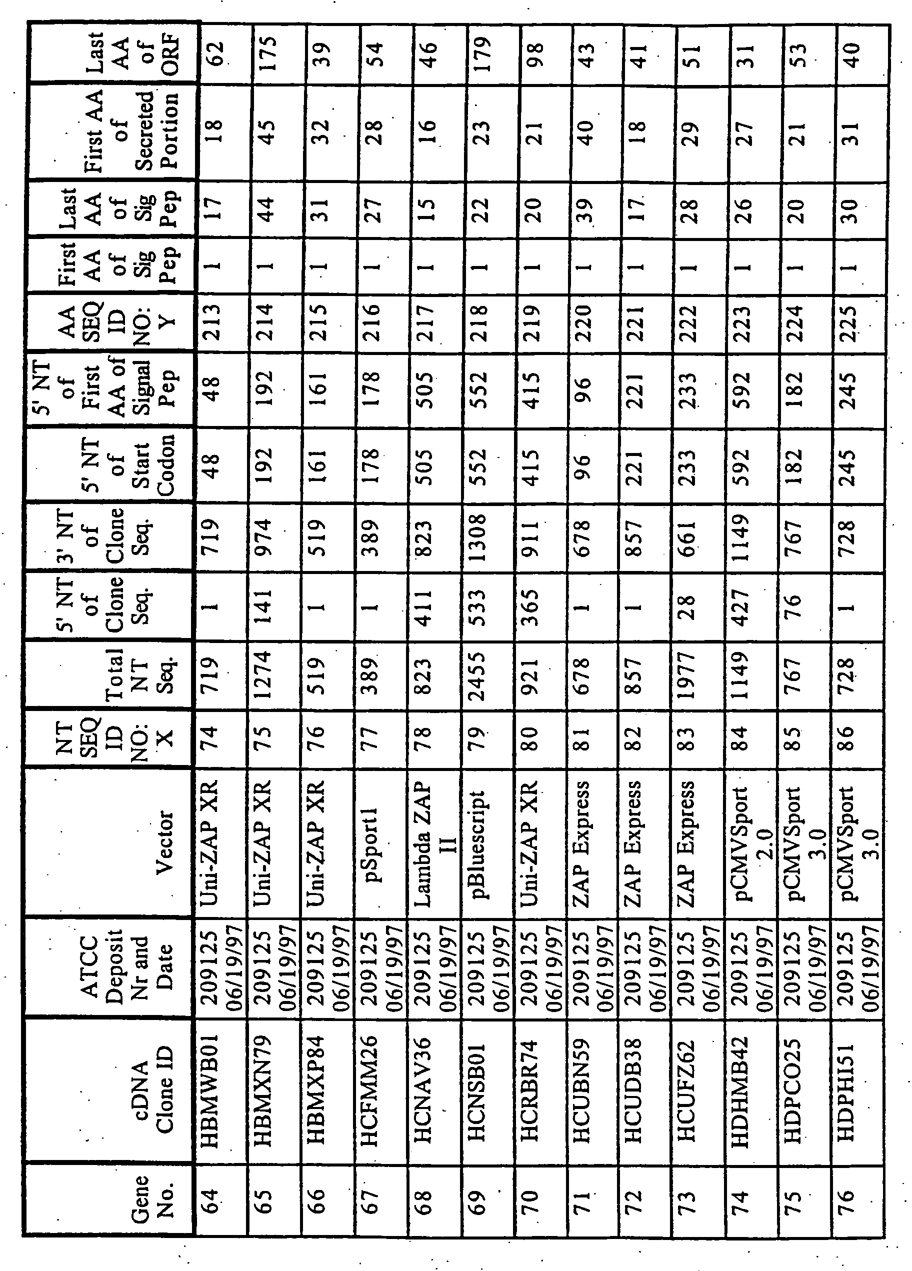

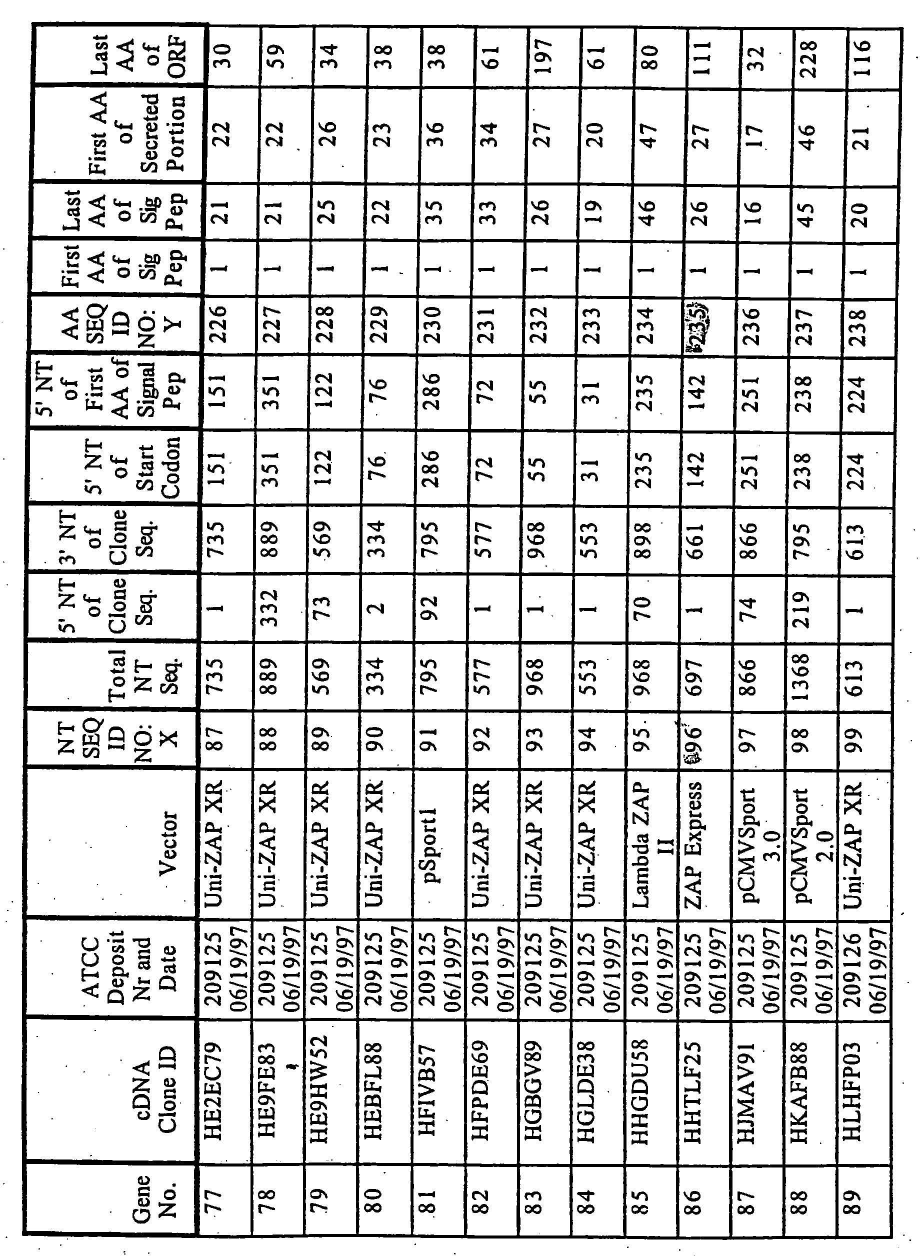

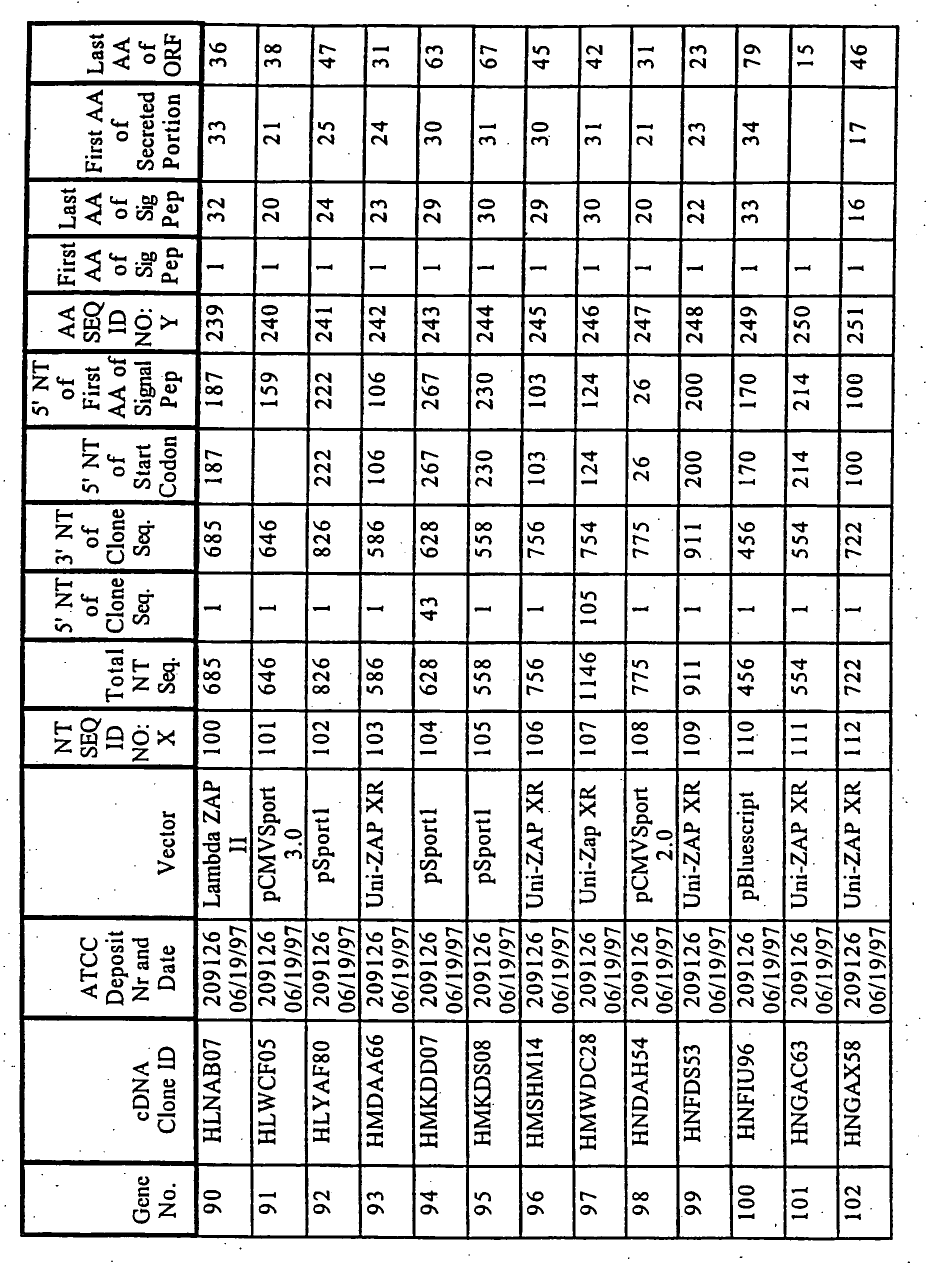

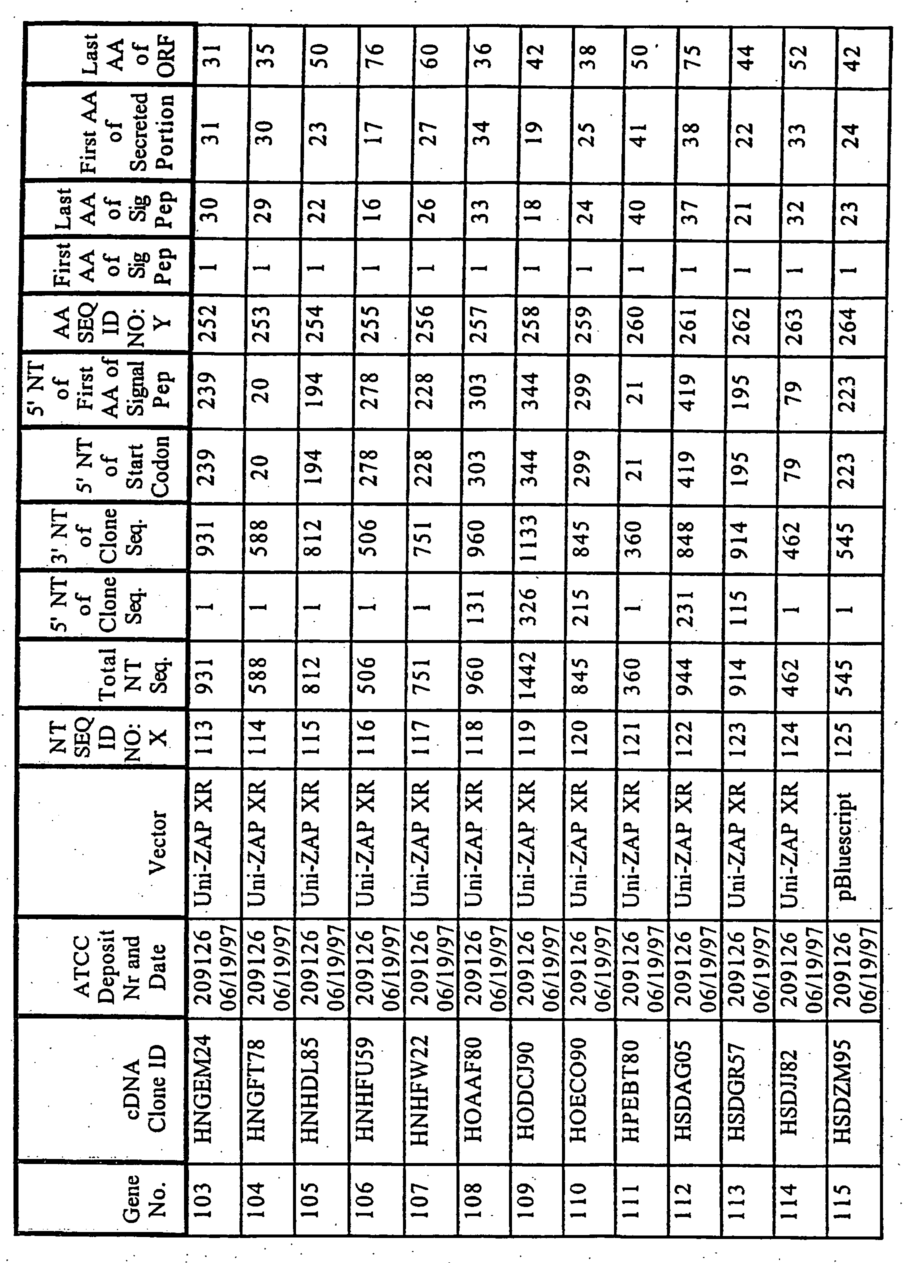

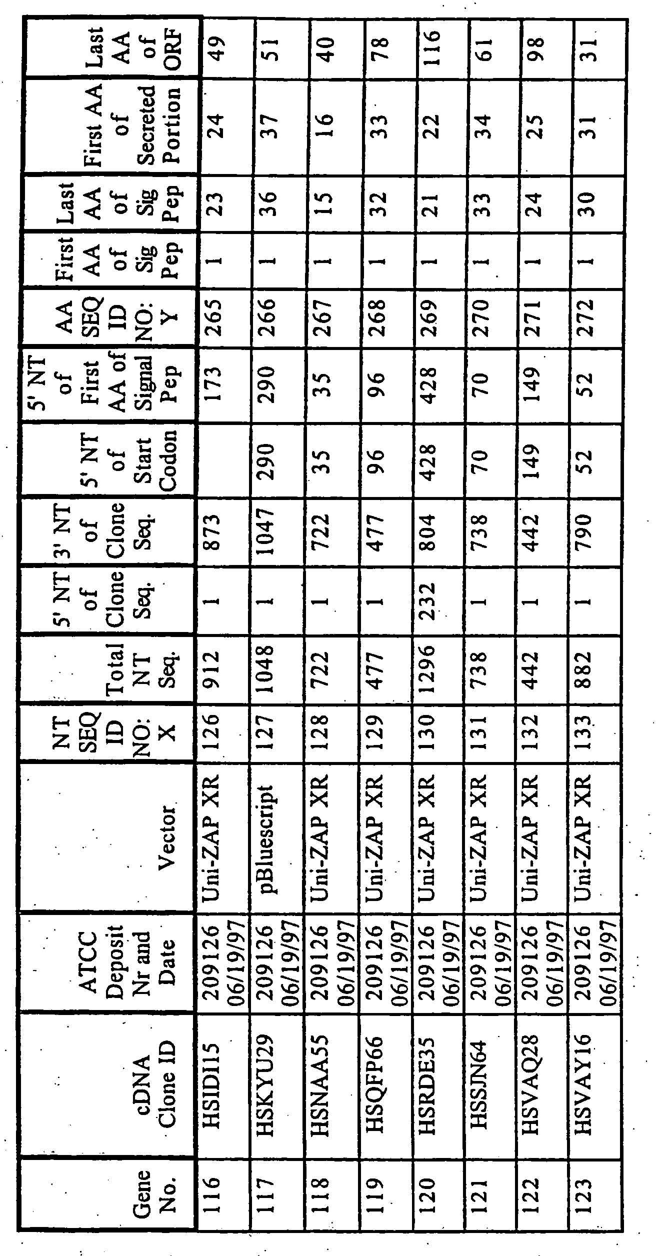

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

''Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly, the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as ''First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification, such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragement specified as described herein.

As a practical matter, whether any particular nucleic acid molecule or polypeptide is at least 90%, 95%, 96%, 97%, 98% or 99% identical to a nucleotide sequence of the presence invention can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are:
Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is becuase the FASTDB program does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignement of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in Table 1 or to the amino acid sequence encoded by deposited DNA clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1 Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N- and C-terminal truncations of the subject sequence when calculating global percent identity. For subject sequences truncated at the N- and C-termini, relative to the the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5,4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus. or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Particularly, N-terminal deletions of the polypeptide of the present invention can be described by the general formula m-p, where p is the total number of amino acids in the polypeptide and m is an integer from 2 to (p-1), and where both of these integers (m & p) correspond to the position of the amino acid residue identified in SEQ ID NO:Y.

Moreover, C-terminal deletions of the polypeptide of the present invention can also be described by the general formula 1-n, where n is an integer from 2 to (p-1), and again where these integers (n & p) correspond to the position of the amino acid residue identified in SEQ ID NO:Y.

The invention also provides polypeptides having one or more amino acids deleted from both the amino and the carboxyl termini, which may be described generally as having residues m-n of SEQ ID NO:Y, where m and n are integers as described above.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998- 4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors. Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected . individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell. Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thtombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye. head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picomaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox, hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, bums, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity, and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA, clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO: Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95 % identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR® 2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT 1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*)*.*

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4° C or frozen at -80° C.

In addition to the above expression vector, the present invention further includes an expression vector comprising phage operator and promoter elements operatively linked to a polynucleotide of the present invention, called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example I, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E*. *coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1 % agarose gel.

The plasmid is digested with the corresponding restriction enzymes and optionally, can be dephosphorylated using calf intestinal phosphatase, using routine procedures known in the art. The DNA is then isolated from a 1 % agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1 % agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB 101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)
Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC
AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA

GGTGGCAGCAGGGGAACGTCTTCTCATGCTCCGTGATGCATGAGGCTCTGC
ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Kohler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604FBiowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 GibcoBRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (116.6 mg/L of CaCl2 (anhyd); 0.00130 mg/L CuSO₄-5H₂O; 0.050 mg/L of Fe(NO₃)₃-9H₂O; 0.417 mg/L of FeSO₄-7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO4; .4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂O; 6.65 mg/ml of L-Aspartic Acid; 29.56 mg/ml of L-Cystine-2HCL-H₂O; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂O; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂O; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | | JAKs | | | STATS | GAS(elements) or ISRE |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g II-10 | + + ? ? | + | + | - | 1 1,3 | GAS (IRF1>Lys6>IFP) |

| gp 130 family | | | | | | |
|---|---|---|---|---|---|---|
| IL-6 (Pleiotrohic) | + | + | + | ? | 1,3 | GAS (IRF1>Lys6>IFP) |
| II-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleibtrohic) | ? | + | ? | ? | 1,3 | |
| IL-12(Pleiotrohic) | + | - | + | + | 1,3 | |

| g-C family | | | | | | |
|---|---|---|---|---|---|---|
| IL-2 (lymphocytes) | - | + | - | + | 1,3,5 | GAS |
| IL-4 (lymph/myeloid) - | | + | - | + | 6 | GAS (IRF1 = IFP >>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| gp140 family | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) - | | - | + | - | 5 | GAS |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda eL al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC 12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC 12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC 12 cells through EGR can be used, such as 50 ng/ul of Neuronal Growth Factor (NGF). Over fifty-fold induction of SEAP is typically seen in the positive control wells. SEAP assay the supematant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAAGCTT:
3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes SalI and NotI, and inserted into a vector containing neomycin resistance. Particularly, the NF-κB/SV40/SEAP cassette was inserted into pGFP-1 (Clontech), replacing the GFP gene, after restricting pGFP-1 with SalI and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1 % SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (lug/ml) which specifically recognizes the phosphorylated epitope of the Erk-1 and Erk-2 kinases (1hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrastemal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

### Example 27: Method of Treatment Using Gene Therapy - In Vivo

Another aspect of the present invention is using *in vivo* gene therapy methods to treat disorders, diseases and conditions. The gene therapy method relates to the introduction of naked nucleic acid (DNA, RNA, and antisense DNA or RNA) sequences into an animal to increase or decrease the expression of the polypeptide of the present invention. A polynucleotide of the present invention may be operatively linked to a promoter or any other genetic elements necessary for the expression of the encoded polypeptide by the target tissue. Such gene therapy and delivery techniques and methods are known in the art, see, for example, W090/11092, W098/11779; U.S. Patent NO. 5693622, 5705151, 5580859; Tabata H. et al. (1997) Cardiovasc. Res. 35(3):470-479, Chao J et al. (1997) Pharmacol. Res. 35(6):517-522, Wolff J.A. (1997) Neuromuscul. Disord. 7(5):314-318, Schwartz B. et al. (1996) Gene Ther. 3(5):405-411, Tsurumi Y. et al. (1996) Circulation 94(12):3281-3290 (incorporated herein by reference).

The polynucleotide constructs of the present invention may be delivered by any method that delivers injectable materials to the cells of an animal, such as, injection into the interstitial space of tissues (heart, muscle, skin, lung, liver, intestine and the like). These polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the polynucleotides may also be delivered in liposome formulations (such as those taught in Felgner P.L. et al. (1995) Ann. NY Acad. Sci. 772:126-139 and Abdallah B. et al. (1995) Biol. Cell 85(1):1-7) which can be prepared by methods well known to those skilled in the art.

The polynucleotide vector constructs of the present invention used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct of the present invention can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked polynucleotide injection, an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in muscle *in vivo* is determined as follows. Suitable template DNA for production of mRNA coding for the polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supernatants from injected and control mice. The results of the above experimentation in mice can be use to extrapolate proper dosages and other treatment parameters in humans and other animals using naked DNA of the present invention.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

Further, the Sequence Listing submitted herewith in paper and computer readable forms are herein incorporated by reference in their entireties.

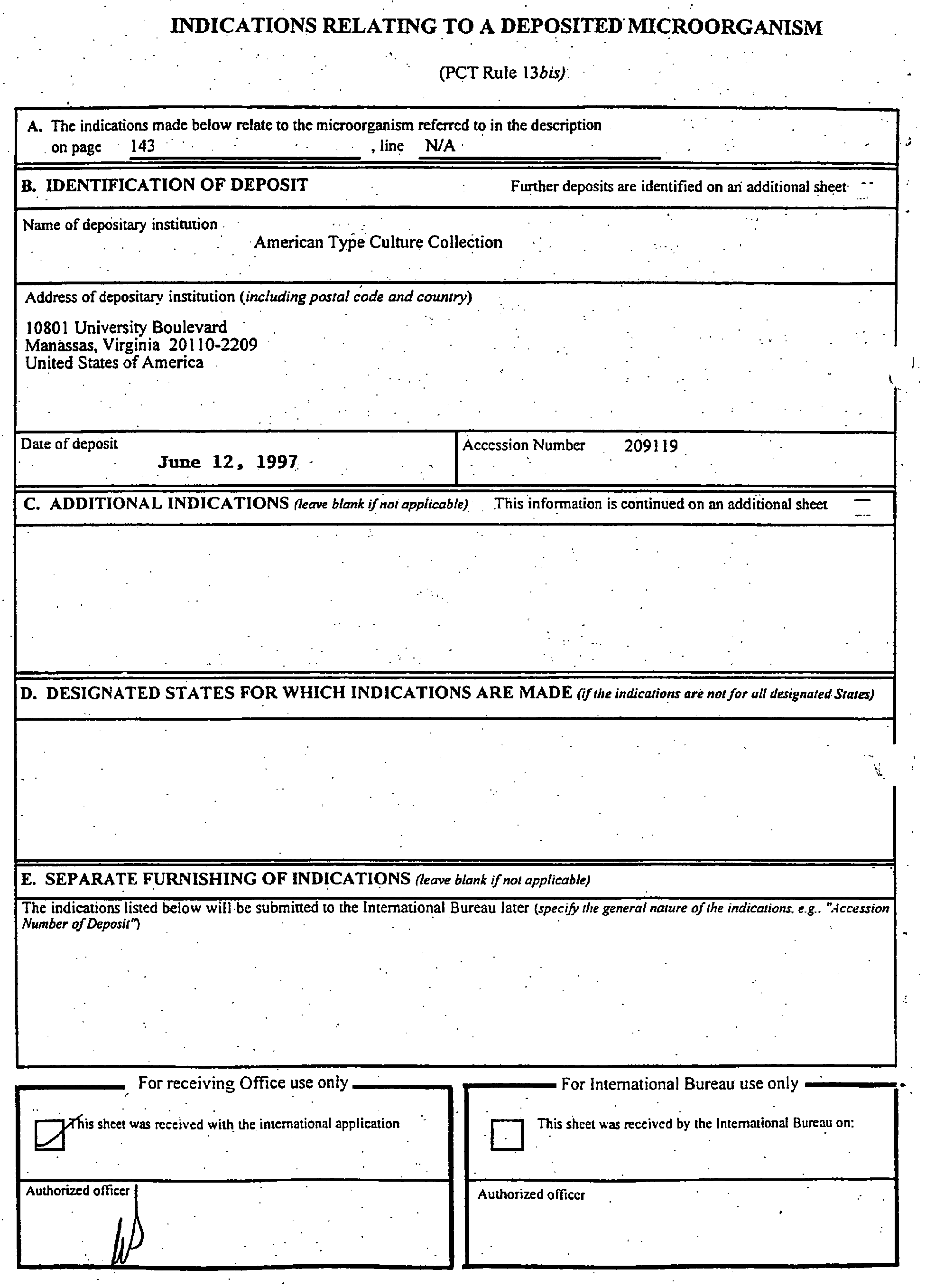

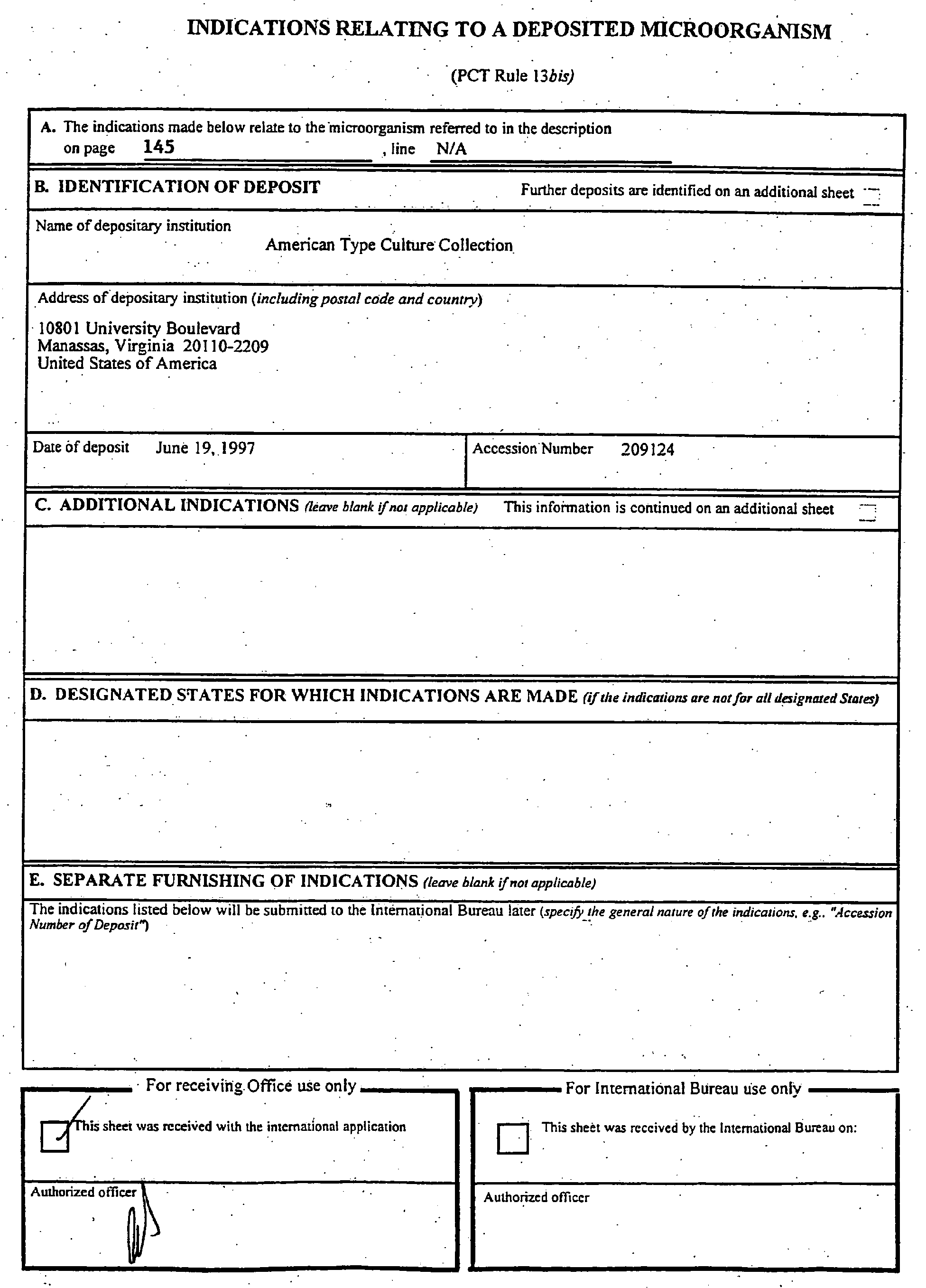

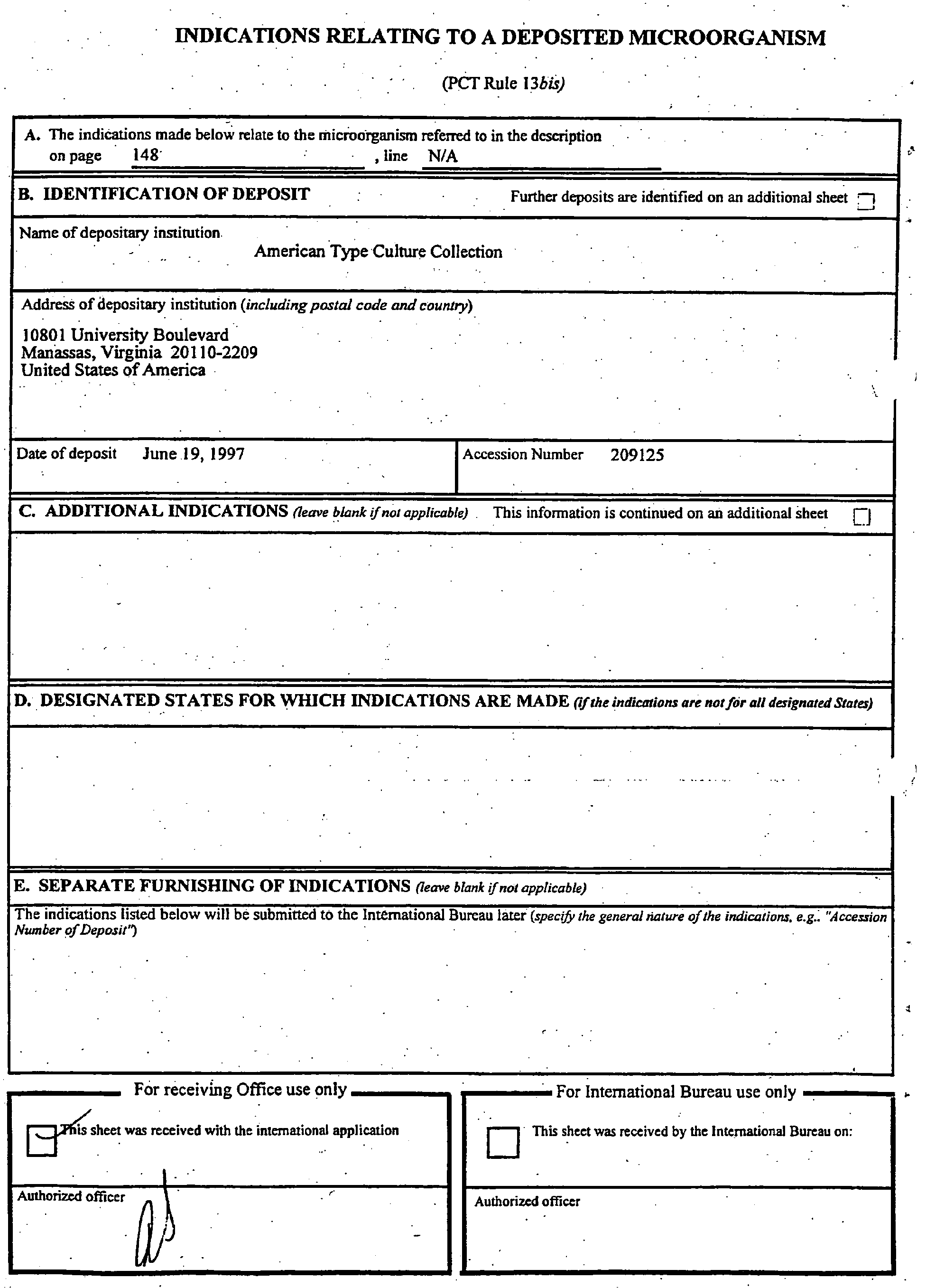

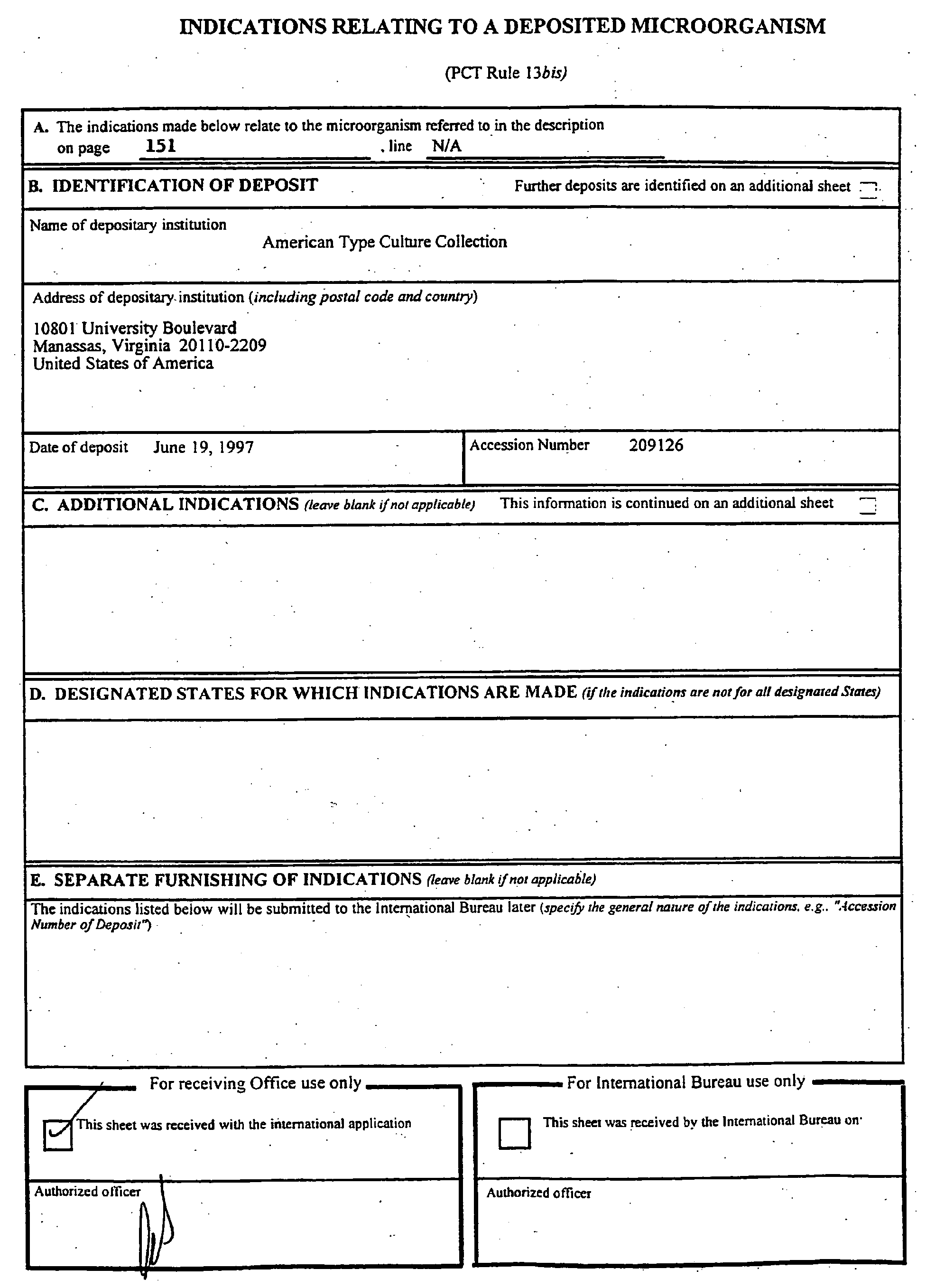

## Claims

1. A nucleic acid molecule comprising a polynucleotide having a nucleotide sequence selected from the group consisting of:
(a) a polynucleotide having the entire nucleotide sequence of SEQ ID NO: 96;
(b) a polynucleotide having the entire cDNA sequence included in ATCC Deposit No. 209125 which is hybridizable to SEQ ID NO: 96;
(c) a polynucleotide fragment of SEQ ID NO: 96;
(d) a polynucleotide fragment of the cDNA sequence included in ATCC Deposit No. 209125, which is hybridizable to SEQ ID NO: 96;
(e) a polynucleotide encoding a polypeptide fragment of SEQ ID NO: 235 ;
(f) a polynucleotide encoding a polypeptide fragment encoded by the cDNA sequence included in ATCC Deposit No. 209125, which is hybridizable to SEQ ID NO: 96;
(g) a polynucleotide encoding a polypeptide fragment of SEQ ID NO: 235 or a polypeptide fragment encoded by the cDNA sequence included in ATCC Deposit No. 209125, said cDNA sequence being hybridizeable to SEQ ID NO: 96, having biological activity;
(h) a polynucleotide encoding a polypeptide fragment of SEQ ID NO: 235 or a polypeptide fragment encoded by the cDNA sequence included in ATCC Deposit No. 209125, said cDNA sequence being hybridizeable to SEQ ID NO: 96, which is secreted;
(i) a polynucleotide encoding the full length polypeptide of SEQ ID NO: 235 or being encoded by the cDNA sequence included in ATCC Deposit No. 209125, said cDNA sequence being hybridizable to SEQ ID NO. 96;
(j) a polynucleotide encoding a polypeptide domain of SEQ ID NO: 235 ;
(k) a polynucleotide encoding a polypeptide domain encoded by the cDNA sequence included in ATCC Deposit No. 209125, which is hybridizable to SEQ ID NO: 96;
(I) a polynucleotide encoding a polypeptide epitope of SEQ ID NO: 235 ;
(m) a polynucleotide encoding a polypeptide epitope encoded by the cDNA sequence included in ATCC Deposit No. 209125, which is hybridizable to SEQ ID NO: 96;
(n) a polynucleotide which is a variant of SEQ ID NO: 96;
(o) a polynucleotide encoding a variant of SEQ ID NO: 235;
(p) a polynucleotide which is an allelic variant of SEQ ID NO: 96;
(q) a polynucleotide encoding an allelic variant of SEQ ID NO: 235;
(r) a polynucleotide which encodes a species homologue of SEQ ID NO: 235 ;
(s) a polynucleotide which is at least 90% identical to a polynucleotide as defined in any one of (a) to (r);
(t) a polynucleotide encoding a polypeptide having an amino acid sequence at least 95% identical to the amino acid sequence of a polypeptide encoded by a polynucleotide of any one of (a) to (s); and
(u) the polynucleotide of any one of (a) to (t), wherein the polynucleotide fragment comprises a nucleotide sequence encoding a secreted protein;
(v) a polynucleotide capable of hybridizing under stringent conditions to any one of the polynucleotides specified in (a) to (t), wherein said polynucleotide does not hybridize under stringent conditions to a nucleic acid molecule having a nucleotide sequence of only A residues or of only T residues;
or the complementary strand of polynucleotide (a) to (u).

2. The nucleic acid molecule of claim 1, wherein the nucleotide sequence comprises sequential nucleotide deletions from either the 5 prime terminus or the 3 prime terminus.

3. The nucleic acid molecule of claim 1 or 2 which is DNA or RNA.

4. A gene corresponding to the cDNA sequence of SEQ ID NO: 96 or encoding the polypeptide having the amino acid sequence of SEQ ID NO: 235.

5. A vector comprising the nucleic acid molecule of any one of claims 1 to 3.

6. A method of making a recombinant host cell comprising introducing the nucleic acid molecule of any one of claims 1 to 3, or the vector of claim 5.

7. A recombinant host cell containing the nucleic acid molecule of claims 1 to 3 or the vector of claim 5 or produced by the method of claim 6.

8. The recombinant host cell of claim 7 which expresses a polypeptide encoded by the nucleic acid molecule of any one of claims 1 to 3.

9. A method of making a polypeptide encoded by the nucleic acid molecule of any one of claims 1 to 3 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3 or obtainable by the method of claim 9.

11. An antibody that binds specifically to the polypeptide of claim 10.

12. An antagonist of the polypeptide of claim 10 comprising a molecule closely related to the natural ligand of said polypeptide or a polypeptide related to the natural receptor of the polypeptide of claim 10 or a fragment thereof.

13. An agonist of the polypeptide of claim 10 comprising a molecule closely related to the natural ligand of said polypeptide or a polypeptide related to the natural receptor of the polypeptide of claim 10 or a fragment thereof.

14. A method for identifying antagonists or agonists of the polypeptide of claim 10 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

15. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

16. An in vitro method of diagnosing a pathological condition or a susceptibility to a pathological condition in a subject comprising:
(a) determining the presence or amount of expression of the polypeptide of claim 10 in a biological sample; and
(b) diagnosing a pathological condition or a susceptibility to a pathological condition based on the presence or amount of expression of the polypeptide.

17. A method for identifying a binding partner to the polypeptide of claim 10 comprising:
(a) contacting the polypeptide of claim 10 with a binding partner; and
(b) determining whether the binding partner effects an activity of the polypeptide.

18. A method of identifying an activity in a biological assay, wherein the method comprises:
(a) expressing the nucleic acid molecule of any one of claims 1 to 3, the gene of claim 4 or the vector of claim 5 in a cell;
(b) isolating the supernatant;
(c) detecting an activity in a biological assay; and
(d) identifying the protein in the supernatant having the activity.

19. The product produced by the method of any one of claims 14, 17 and 18.

20. A pharmaceutical composition comprising the nucleic acid molecule of any one of claims 1 to 3, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

21. Use of the nucleic acid molecule of any one of claims 1 to 3, the polypeptide of claim 10, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

22. A diagnostic composition comprising the nucleic acid molecule of any one of claims 1 to 3 or the antibody of claim 11.

23. A method for the production of a pharmaceutical composition comprising the steps of the method of claim 14 and (d) formulating the compound identified in step (c) in a pharmaceutically acceptable form.
